# EUROPEAN PATENT APPLICATION

(11) **EP 1 584 619 A1**
(43) Date of publication of application: **12.10.2005**
(21) Application number: 05013587.0
(22) Date of filing: 03.05.2000
(51) Int. Cl.: C07D 215/54, A61K 31/47, A61P 43/00, C07D 405/12, C07D 401/12, C07D 417/12, C07D 413/12, C07D 409/12

(54) **Derivatives of quinoline as inhibitors for MEK**

(30) Priority: 08.05.1999 GB 9910577
(62) Divisional of application: 00927491.1
(71) Applicant: AstraZeneca AB, 151 85 Södertälje (SE)
(72) Inventor: Boyle, Francis Thomas, Cheshire SK10 4TG (GB); Gibson, Keith Hopkinson, Cheshire SK10 4TG (GB); Poyser, Jeffrey Philip, Cheshire SK10 4TG (GB); Turner, Paul, Cheshire SK10 4TG (GB)
(74) Representative: Bryant, Tracey

(57) **Abstract**

1. A compound of formula (I) 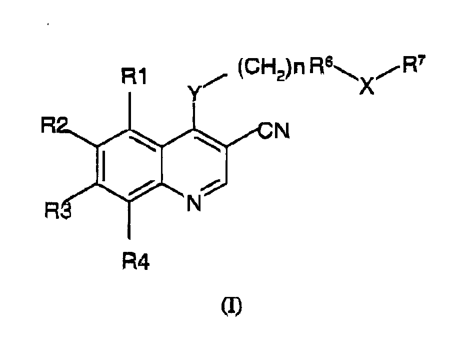 or a pharmaceutically acceptable salt thereof.
wherein:
n is 0-1;
X and Y are independently selected from -NH-, -O-, -S-, or -NR⁸- where R⁸ is alkyl of 1-6 carbon atoms and X may additionally comprise a CH₂ group;
R⁷ is a group (CH₂)ₘR⁹ where m is 0,or an integer of from 1-3 and R⁹ is a substituted aryl group, an optionally substituted cycloalkyl ring of up to 10 carbon atoms, or an optionally substituted heterocyclic ring or an N-oxide of any nitrogen containing ring;
R⁶ is a divalent cycloalkyl of 3 to 7 carbon atoms, which may be optionally further substituted with one or more alkyl of 1 to 6 carbon atom groups; or is a divalent pyridinyl, pyimidinyl, or phenyl ring; wherein the pyridinyl, pyrimidinyl, or phenyl ring may be optionally further substituted with one or more specified groups;
R₁, R₂, R₃ and R₄ are each independently selected from hydrogen or various specified organic groups.

Compounds are useful as pharmaceuticals for the inhibition of MEK activity.

## Description

The present invention relates to certain novel quinoline derivatives as well as to their use as pharmaceuticals, in particular as inhibitors of specific kinase enzymes, such as MEK enzymes. Further aspects of the invention include pharmaceutical compositions and methods of treatment of proliferative disease such as cancer using said compounds.

Cancer is a disease in which cells grow and divide in an uncontrolled fashion. This uncontrolled growth arises from abnormalities in signal transduction pathways that are used by normal cells to regulate cell growth and division in response to various signalling molecules. Normal cells do not proliferate unless stimulated to do so by specific signal molecules located outside the cell derived from nearby cells or tissues. Growth factors bind to the cell membrane via specific receptors which have intrinsic enzyme activity. These receptors relay the growth signal to the cell nucleus via a series of signalling proteins. In cancer, a number of defects in signal pathways are apparent. For example, cancer cells may produce their own growth factors which bind to their cognate receptors, resulting in an autocrine loop, or receptors may be mutated or overexpressed leading to an increased, continuous signal to proliferate. In addition, negative regulators of cell growth may be lost.

Oncogenes are cancer related genes which often encode abnormal versions of signal pathway components, such as receptor tyrosine kinases, serine-threonine kinases, or downstream signaling molecules such as the ras genes, which code for closely related small guanine nucleotide binding proteins which hydrolyse bound guanosine triphosphate (GTP) to guanosine diphosphate (GDP). Ras proteins are active in promoting cell growth and transformation when they are bound to GTP and inactive when they are bound to GDP. Transforming mutants of p21ras are defective in their GTPase activity and hence remain in the active GTP bound state. The ras oncogene is known to play an integral role in certain cancers, and has been found to contribute to the formation of over 20% of all cases of human cancer.

When activated by ligand, cell surface receptors which are coupled to the mitogenic response, such as growth factor receptors, initiate a chain of reactions which leads to the activation of guanine nucleotide exchange activity on ras. When in its active GTP-bound state, a number of proteins interact directly with ras at the plasma membrane resulting in signal transmission through several distinct pathways. The best characterised effector protein is the product of the raf proto-oncogene. The interaction of raf and ras is a key regulatory step in the control of cell proliferation. Ras-mediated activation of the raf serine-threonine kinase in turn activates the dual-specificity MEK (MEK1 and MEK2), which is the immediate upstream activator of mitogen activated protein kinase (MAPKs known as extracellular signal regulated protein kinases or ERK1 and ERK2). To date, no substrates of MEK other than MAPK have been identified, though recent reports indicate that MEK may also be activated by other upstream signal proteins such as MEK kinase or MEKK1 and PKC. Activated MAPK translocates and accumulates in the nucleus, where it can phosphorylate and activate transcription factors such as Elk-1 and Sapla, leading to the enhanced expression of genes such as that for c-fos.

The ras-dependent raf-MEK-MAPK cascade is one of the key signalling pathways responsible for transmitting and amplifying mitogenic signals from cell surface to the nucleus resulting in changes in gene expression and cell fate. This ubiquitous pathway appears essential for normal cell proliferation and constitutive activation of this pathway is sufficient to induce cellular transformation. Transforming mutants of p21ras are constitutively active, resulting in raf, MEK and MAPK activity and cell transformation. Inhibition of MEK activity using either antisense raf, a dominant negative MEK mutant or the selective inhibitor PD098059 have been shown to block the growth and morphological transformation of ras-transformed fibroblasts.

The mechanism of activation of raf, MEK and MAPK is through phosphorylation on specific serine, threonine or tyrosine residues. Activated raf and other kinases phosphorylate MEK1 on S218 and S222 and MEK2 on S222 and S226. This results in MEK activation and subsequent phosphorylation and activation of ERK1 on T190 and Y192 and ERK2 on T183 and Y185 by the dual specificity MEKs. Whilst MEK can be activated by a number of protein kinases, and active MAPKs phosphorylate and activate a number of substrate proteins including transcription factors and other protein kinases, MEKs appear specific and sole activators of MAPKs and could act as a focal point for cross-cascade regulation. MEK1 and MEK2 isoforms show unusual specificity and also contain a proline-rich insert between catalytic subdomains IX and X which is not present in any of the other known MEK family members. These differences between MEK and other protein kinases, together with the known role of MEK in proliferative signalling suggest that it may be possible to discover and employ selective MEK inhibitors as therapeutic agents for use in proliferative disease.

WO 98/43960 discloses a range of 3-cyano quinoline compounds and their use in the treatment of cancer. Certain of the compounds are demonstrated as being inhibitors of Epidermal Growth Factor Receptor Kinase, and to inhibit cancer cell growth. Other quinoline derivatives which inhibit the effect of growth factors such as VEGF are described in WO98/13350.

This invention provides compounds which are inhibitors of the kinase activity of MEK and as a result, can produce therapeutically useful effects in the treatment of proliferative disease and in particular cancer.

According to the present invention there is provided a compound of formula (I) 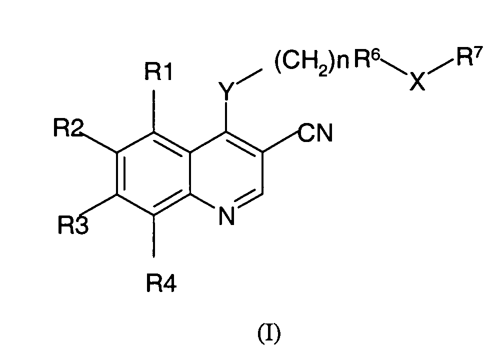 or a pharmaceutically acceptable salt thereof.
wherein:
n is 0-1;
X and Y are independently selected from -NH-, -O-, -S-, or -NR⁸- where R⁸ is alkyl of 1-6 carbon atoms and X may additionally comprise a CH₂ group;
R⁷ is a group (CH₂)ₘR⁹ where m is 0,or an integer of from 1-3 and R⁹ is a substituted aryl group, an optionally substituted cycloalkyl ring of up to 10 carbon atoms, or an optionally substituted heterocyclic ring or an N-oxide of any nitrogen containing ring;
R⁶ is a divalent cycloalkyl of 3 to 7 carbon atoms, which may be optionally further substituted with one or more alkyl of 1 to 6 carbon atom groups; or is a divalent pyridinyl, pyimidinyl, or phenyl ring; wherein the pyridinyl, pyrimidinyl, or phenyl ring may be optionally further substituted with one or more groups selected from halogen, alkyl of 1-6 carbon atoms, alkenyl of 2-6 carbon atoms, alkynyl of 2-6 carbon atoms, azido, hydroxyalkyl of 1-6 carbon atoms, halomethyl, alkoxymethyl of 2-7 carbon atoms, alkanoyloxymethyl of 2-7 carbon atoms, alkoxy of 1-6 carbon atoms, alkylthio of 1-6 carbon atoms, hydroxy, trifluoromethyl, cyano, nitro, carboxy, carboalkoxy of 2-7 carbon atoms, carboalkyl of 2-7 carbon atoms, phenoxy, phenyl, thiophenoxy, benzoyl, benzyl, amino, alkylamino of 1-6 carbon atoms, dialkylamino of 2 to 12 carbon atoms, phenylamino, benzylamino, alkanoylamino of 1-6 carbon atoms, alkenoylamino of 3-8 carbon atoms, alkynoylamino of 3-8 carbon atoms, and benzoylamino;
R₁, R₂, R₃ and R₄ are each independently selected from hydrogen, hydroxy, halogeno, cyano, nitro, trifluoromethyl, C₁₋₃alkyl, -NR¹¹R¹² (wherein R¹¹ and R¹², which may be the same or different, each represents hydrogen or C₁₋₃alkyl), or a group R¹³-X¹-(CH₂)ₓ wherein x is 0 to 3, X¹ represents -O-, -CH₂-, -OCO-, carbonyl, -S-, -SO-, -SO₂-, -NR¹⁴CO-, -CONR¹⁵-, -SO₂NR¹⁶-, -NR¹⁷SO₂- or -NR¹⁸- (wherein R¹⁴, R¹⁵, R¹⁶, R¹⁷ and R¹⁸ each independently represents hydrogen, C₁₋₃alkyl or C₁₋₃alkoxyC₂₋₃alkyl) and R¹³ is selected from one of the following sixteen groups:
   1) C₁₋₅alkyl which may be unsubstituted or which may be substituted with one or more groups selected from hydroxy, fluoro and amino;
   2) C₁₋₅alkylX²COR¹⁹ (wherein X² represents -O- or -NR²⁰- (wherein R²⁰ represents hydrogen, C₁₋₃alkyl or C₁₋₃alkoxyC₂₋₃alkyl) and R¹⁹ represents -NR²¹R²²- or -OR²³- (wherein R²¹, R²² and R²³ which may be the same or different each represents hydrogen, C₁₋₃alkyl or C₁₋₃alkoxyC₂₋₃alkyl));
   3) C₁₋₅alkylX³R²⁴ (wherein X³ represents -O-, -S-, -SO-, -SO₂-, -OCO-, -NR²⁵CO-, -CONR²⁶-, -SO₂NR²⁷-, -NR²⁸SO₂- or -NR²⁹- (wherein R²⁵, R²⁶, R²⁷, R²⁸ and R²⁹ each independently represents hydrogen, C₁₋₃alkyl or C₁₋₃alkoxyC₂₋₃alkyl) and R²⁴ represents hydrogen, C₁₋₃alkyl, cyclopentyl, cyclohexyl or a 5 or 6 membered saturated heterocyclic group with one or two heteroatoms, selected independently from O, S and N, which C₁₋₃alkyl group may bear one or two substituents selected from oxo, hydroxy, halogeno and C₁₋₄alkoxy and which cyclic group may bear one or two substituents selected from oxo, hydroxy, halogeno, C₁₋₄alkyl, C₁₋₄hydroxyalkyl and C₁₋₄alkoxy);
   4) C₁₋₅alkylX⁴C₁₋₅alkylX⁵R³⁰ (wherein X⁴ and X⁵ which may be the same or different are each -O-, -S-, -SO-, -SO₂-, -NR³¹CO-, -CONR³²-, -SO₂NR³³-, -NR³⁴SO₂- or -NR³⁵- (wherein R³¹, R³², R³³, R³⁴ and R³⁵ each independently represents hydrogen, C₁₋₃alkyl or C₁₋₃alkoxyC₂₋₃alkyl) and R³⁰ represents hydrogen or C₁₋₃alkyl);
   5) C₁₋₅alkylR³⁶ (wherein R³⁶ is a 5 or 6 membered saturated heterocyclic group with one or two heteroatoms, selected independently from O, S and N, which heterocyclic group may bear one or two substituents selected from oxo, hydroxy, halogeno, C₁₋₄alkyl, C₁₋₄hydroxyalkyl and C₁₋₄alkoxy);
   6) (CH₂)_{q}X⁶R³⁷ (wherein q is an integer from 0 to 5, X⁶ represents a direct bond, -O-, -S-, -SO-, -SO₂-, -NR³⁸CO-, -CONR³⁹-, -SO₂NR⁴⁰-, -NR⁴¹SO₂- or -NR⁴²- (wherein R³⁸, R³⁹, R⁴⁰, R⁴¹ and R⁴² each independently represents hydrogen, C₁₋₃alkyl or C₁₋₃alkoxyC₂₋₃alkyl) and R³⁷ is a phenyl group, a pyridone group or a 5 or 6 membered aromatic heterocyclic group with 1 to 3 heteroatoms selected from O, N and S, which phenyl, pyridone or aromatic heterocyclic group may carry up to 5 substituents selected from hydroxy, halogeno, amino, C₁₋₄alkyl, C₁₋₄alkoxy, C₁₋₄hydroxyalkyl, C₁₋₄hydroxyalkoxy, C₁₋₄aminoalkyl, C₁₋₄alkylamino, carboxy, cyano, -CONR⁴³R⁴⁴ and -NR⁴⁵COR⁴⁶ (wherein R⁴³, R⁴⁴, R⁴⁵ and R⁴⁶, which may be the same or different, each represents hydrogen, C₁₋₄alkyl or C₁₋₃alkoxyC₂₋₃alkyl));
   7) C₂₋₆alkenylR³⁶ (wherein R³⁶ is as defined hereinbefore);
   8) C₂₋₆alkynylR³⁶ (wherein R³⁶ is as defined hereinbefore);
   9) X⁷R⁴⁷ (wherein X⁷ is -SO₂-, -O- or -CONR⁴⁸R⁴⁹- (wherein R⁴⁸ and R⁴⁹, which may be the same or different, each represents hydrogen, C₁₋₃alkyl or C₁₋₃alkoxyC₂₋₃alkyl) and R⁴⁷ represents C₁₋₅alkyl which may be unsubstituted or which may be substituted with one or more groups selected from hydroxy, fluoro and amino) with the provisos that when X⁷ is -SO₂-, X¹ is -O-, when X⁷ is -O-, X¹ is carbonyl, when X⁷ is -CONR⁴⁸R⁴⁹-, X¹ is -O- or NR¹⁸ (wherein R⁴⁸, R⁴⁹ and R¹⁸ are as defined hereinbefore);
   10) C₂₋₆alkenylR³⁷ (wherein R³⁷ is as defined hereinbefore);
   11) C₂₋₆alkynylR³⁷ (wherein R³⁷ is as defined hereinbefore);
   12) C₂₋₆alkenylX⁸R³⁷ (wherein X⁸ represents -O-, -S-, -SO-, -SO₂-, -NR⁵⁰CO-, -CONR⁵¹-, -SO₂NR⁵²-, -NR⁵³SO₂- or -NR⁵⁴- (wherein R⁵⁰, R⁵¹, R⁵², R⁵³ and R⁵⁴ each independently represents hydrogen, C₁₋₃alkyl or C₁₋₃alkoxyC₂₋₃alkyl) and R³⁷ is as defined hereinbefore);
   13) C₂₋₆alkynylX⁹R³⁷ (wherein X⁹ represents -O-, -S-, -SO-, -SO₂-, -NR⁵⁵CO-, -CONR⁵⁶-, -SO₂NR⁵⁷-, -NR⁵⁸SO₂- or -NR⁵⁹- (wherein R⁵⁵, R⁵⁶, R⁵⁷, R⁵⁸ and R⁵⁹ each independently represents hydrogen, C₁₋₃alkyl or C₁₋₃alkoxyC₂₋₃alkyl) and R³⁷ is as defined hereinbefore);
   14) C₁₋₃alkylX¹⁰C₁₋₃alkylR³⁷ (wherein X¹⁰ represents -O-, -S-, -SO-, -SO₂-, -NR⁶⁰CO-, -CONR⁶¹-, -SO₂NR⁶²-, -NR⁶³SO₂- or -NR⁶⁴- (wherein R⁶⁰, R⁶¹, R⁶², R⁶³ and R⁶⁴ each independently represents hydrogen, C₁₋₃alkyl or C₁₋₃alkoxyC₂₋₃alkyl) and R³⁷ is as defined hereinbefore);
   15) R³⁶ (wherein R³⁶ is as defined hereinbefore); and
   16) C₁₋₃alkylX¹⁰C₁₋₃alkylR³⁶ (wherein X¹⁰ and R³⁶ are as defined hereinbefore).

Suitable pharmaceutically acceptable salts of compounds of formula (I) include acid addition salts such as methanesulfonate, fumarate, hydrochloride, hydrobromide, citrate, maleate and salts formed with phosphoric and sulphuric acid. A preferred pharmaceutically acceptable salt is a hydrochloride salt.

The alkyl portion of the alkyl, alkoxy, alkanoyloxy, alkoxymethyl, alkanoyloxymethyl, alkylsuphinyl, alkylsulphonyl, alkylsulfonamido, carboalkoxy, carboalkyl, alkanoylamino aminoalkyl, alkylaminoalkyl, N,N-dicycloalkylaminoalkyl, hydroxyalkyl, and alkoxyalkyl substituents include both straight chain as well as branched carbon chains. The cycloalkyl portions of N-cycloalkyl-N-alkylaminoalkyl and N,N-dicycloalkylaminoalkyl substituents include both simple carbocycles as well as carbocycles containing alkyl substituents. The alkenyl portion of the alkenyl, alkenoyloxymethyl, alkenyloxy, alkenylsulfonamido, substituents include both straight chain as well as branched carbon chains and one or more sites of unsaturation. The alkynyl portion of the alkynyl, alkynoyloxymethyl, alkynylsulfonamido, alkynyloxy, substituents include both straight chain as well as branched carbon chains and one or more sites of unsaturation. Carboxy is defined as a -CO₂H radical. Carboalkoxy of 2-7 carbon atoms is defined as a -CO₂R" radical, where R" is an alkyl radical of 1-6 carbon atoms. Carboalkyl is defined as a -COR" radical, where R" is an alkyl radical of 1-6 carbon atoms. Alkanoyloxy is defined as a -OCOR" radical, where R" is an alkyl radical of 1-6 carbon atoms. Alkanoyloxymethyl is defined as R"CO₂CH₂- radical, where R" is an alkyl radical of 1-6 carbon atoms. Alkoxymethyl is defined at R"OCH₂- radical, where R" is an alkyl radical of 1-6 carbon atoms. Alkylsulphinyl is defined as R"SO- radical, where R" is an alkyl radical of 1-6 carbon atoms. Alkylsulphonyl is defined as R"SO₂- radical, where R" is alkyl radical of 1-6 carbon atoms. Alkylsulfonamido, alkenylsulfonamido, alkynylsulfonamido are defined as R"SO₂NH- radical, where R" is an alkyl radical of 1-6 carbon atoms, an alkenyl radical of 2-6 carbon atoms, or an alkynyl radical of 2-6 carbon atoms, respectively. N-alkylcarbamoyl is defined as R"NHCO- radical, where R" is an alkyl radical of 1-6 carbon atoms. N,N-dialkylcarbamoyl is defined as R" R'NCO- radical, where R" is an alkyl radical of 1-6 carbon atoms, R' is an alkyl radical of 1-6 carbon atoms and R', and R" may be the same or different. When X is substituted, it is preferred that it is mono-, di-, or tri-substituted, with monosubstituted being most preferred. It is preferred that of the substituents, R₁, R₂, R₃ and R₄ at least one is hydrogen and it is most preferred that two or three be hydrogen. An azacycloalkyl-N-alkyl substituent refers to a monocyclic heterocycle that contains a nitrogen atom on which is substituted a straight or branched chain alkyl radical. A morpholino-N-alkyl substituent is a morpholine ring substituted on the nitrogen atom with a straight or branch chain alkyl radical. A pipeazino-N-alkyl substituent is a piperazine ring substituted on one of the nitrogen atoms with a straight or branch chain alkyl radical. A N-alkyl-piperidino-N-alkyl substituent is a piperidine ring substituted on one of the nitrogen atoms with a straight or branched chain alkyl group and on the other nitrogen atom with a straight or branch chain alkyl radical.

When any group contains an alkyl portion, the alkyl portion contains preferably 1-6 carbon atoms, more preferably 1-4 carbon atoms, particularly methyl, ethyl, n-propyl, isopropyl, n-butyl, iso-butyl, sec-butyl or tert-butyl. When any group contains an alkenyl or alkynyl portion, the alkenyl or alkynyl portion contains preferably 2-6 carbon atoms, more preferably 2-4 carbon atoms.

The compounds of this invention may contain an asymmetric carbon; in such cases, the compounds of this invention cover the racemate and the individual R and S entantiomers, and in the case were more than one asymmetric carbon exists, the individual diasteromers, their racemates and individual entantiomers.

Examples of substituents for aryl groups R⁹ or optional substituents for carbocyclic or heterocyclic groups R⁹ include one or more groups selected from hydroxy; halo; nitro; cyano; carboxy; C₁₋₆alkoxy; C₁₋₆alkyl; C₂₋₆alkenyl; C₂₋₆alkynyl; C₂₋₆alkenyloxy; C₂₋₆alkynyloxy; C₃₋₆cycloalkyl; amino; mono- or di-C₁₋₆alkyl amino; heterocyclyl optionally substituted with C₁₋₆alkyl or oxo; C(O)R^{a}, C(O)OR^{a}, S(O)_{d}R^{a;} NR^{a}C(O)R^{b}; C(O)NR^{a}S(O)_{d}R^{b}, C(O)NR^{a}R^{b;}; NR^{a}C(O)NR^{b}R^{c}; NR^{a}S(O)_{d}R^{b} or N(S(O)_{d}R^{b})S(O)_{d}R^{c} where d is 0, 1 or 2 and R^{a}, R^{b} and R^{c} are independently selected from hydrogen, C₁₋₆alkyl, aryl, C₃₋₆cycloalkyl or heterocylcyl, and wherein any alkyl, alkenyl or alkynyl group or moiety contained within the substituent one R⁹ may themselves be optionally substituted with one or more groups selected from hydroxy; cyano; nitro; halo; carboxy; carboalkoxy of 2-7 carbon atoms, C₃₋₆cycloalkyl, heterocyclyl optionally substituted with C₁₋₆alkyl or oxo; C(O)R^{d}, C(O)OR^{d} NR^{d}R^{e}, S(O)ₑR^{d}, NR^{d}C(O)R^{e}; C(O)NR^{d}R^{e}; NR^{d}C(O)NR^{e}R^{f}; NR^{d}S(O)ₑR^{e} where e is 0, 1 or 2 and R^{d}, R^{e} and R^{f} are independently selected from hydrogen or C₁₋₆alkyl optionally substituted with one or more groups selected from hydroxy; cyano; nitro; halo; carboxy; carboalkoxy of 2-7 carbon atoms, C₃₋₆cycloalkyl, heterocyclyl optionally substituted with C₁₋₆alkyl or oxo; C(O)R^{g}, C(O)OR^{g} NR^{g}R^{h}, S(O)ₑR^{g}, NR^{h}C(O)R^{g}; C(O)NR^{g}R^{h}; NR^{g}C(O)NR^{h}Rⁱ; NR^{g}S(O)ₑR^{h} where e is as defined above and R^{g}, R^{h} and Rⁱ are independently selected from hydrogen or C₁₋₆alkyl. Alternatively, two substituents on adjacent atoms may be joined to form the second ring of a bicyclic ring system wherein the said second ring is optionally substituted with one or more of the groups listed above for R⁹ and optionally contains one or more heteroatoms.

In some embodiments, the level of substitution on the group R⁹ is a chain substituted with complex. Thus, for example, a substituent may comprise an subsituted alkyl chain which is optionally interposed with heteroatoms such as groups of sub-formula (i)

-X^{a}-R⁷⁰-(X^{b}-R⁷¹)_{q}-(X^{c})ₛ-R⁷² (i)

where X^{a} , X^{b} and X^{c} are independently selected from any of the groups listed above for X¹,
R⁷⁰ and R⁷¹ are independently selected from C₁₋₆alkylene, C₂₋₆alkenylene or C₂₋₆alkynylene groups any of which may be optionally substituted with hydroxy; cyano; nitro; halo; carboxy, carboalkoxy of 2-7 carbon atoms or C₃₋₆cycloalkyl;
R⁷² is hydrogen or an C₁₋₆alkyl, C₂₋₆ alkenyl or C₂₋₆alkynyl group any of which may be optionally substituted with hydroxy; cyano; nitro; halo; carboxy or C₃₋₆cycloalkyl;
and q and s are independently 0 or 1.

Preferably R⁹ is an optionally substituted alkoxy group and most preferably, R⁹ is a substituted alkoxy group.

A particular example of compounds of formula (I) are compounds of formula (IA) which are compounds of formula (I) as defined above provided that R⁷ is a group (CH₂)ₘR⁹ where m is 0,or an integer of from 1-3 and R⁹ is a substituted aryl or substituted cycloalkyl ring of up to 10 carbon atoms, wherein the substituents comprise at least one alkoxy group of 1-6 carbon atoms and optionally one or more further substituents, or R⁹ is a heterocyclic ring containing 1 or 2 oxygen atoms and optionally one or more substituents, and where R¹, R², R³ or R⁴ are a group R¹³-X¹-(CH₂)ₓ wherein x is 0 to 3, X¹ represents -O-, -CH₂-, -OCO-, carbonyl, -S-, -SO-, -SO₂-, -NR¹⁴CO-, SO₂NR¹⁶-, -NR¹⁷SO₂- or -NR¹⁸- (wherein R¹⁴, R¹⁵, R¹⁶, R¹⁷, R¹⁸ and R¹³ are as defined above).

Suitable examples of groups Y are -NH-. Suitably X is oxygen.

Preferably n is 0.

Particular examples of groups R⁹ include phenyl or cycloalkyl of from 3-8 and preferably of 6 carbon atoms which are substituted at the position alpha with a alkoxy group, in particular methoxy.

When R⁹ is subsituted phenyl or cycloalkyl, m is preferably 0.

Examples of heterocyclic rings R⁹ include 3- 7 membered rings, up to two of which may be oxygen atoms. Such groups include: 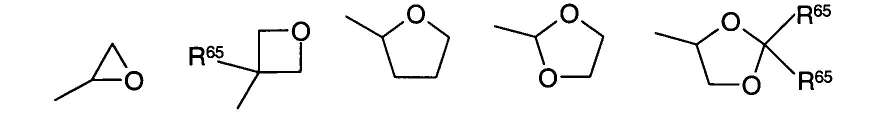 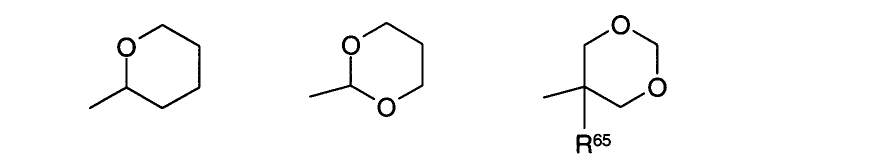 where each R⁶⁵ is independently selected from hydrogen or C₁₋₆alkyl and especially methyl. In such compounds, m is suitably 1, 2 or 3.

Other examples of heterocyclic groups R⁹ include pyridyl, thiazolyl, pyrazinyl, pyrimidinyl, oxadiazole.

Suitable further substituents for R⁷ include those listed above for pyridyl, pyrimidinyl and phenyl groups R^{6.}

Thus a preferred sub-group of compounds of formula (I) are compounds of formula (II) 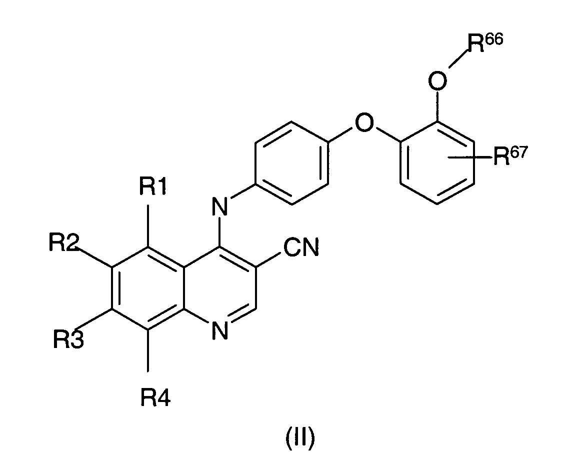 where R¹, R², R³ and R⁴ are as defined above and R⁶⁶ is C₁₋₆ alkyl in particular methyl and R⁶⁷ is selected from hydrogen, halogen, alkyl of 1-6 carbon atoms, alkenyl of 2-6 carbon atoms, alkynyl of 2-6 carbon atoms, azido, hydroxyalkyl of 1-6 carbon atoms, halomethyl, alkoxymethyl of 2-7 carbon atoms, alkanoyloxymethyl of 2-7 carbon atoms, alkoxy of 1-6 carbon atoms, alkylthio of 1-6 carbon atoms, hydroxy, trifluoromethyl, cyano, nitro, carboxy, carboalkoxy of 2-7 carbon atoms, carboalkyl of 2-7 carbon atoms, phenoxy, phenyl, thiophenoxy, benzoyl, benzyl, amino, alkylamino of 1-6 carbon atoms, dialkylamino of 2 to 12 carbon atoms, phenylamino, benzylamino, alkanoylamino of 1-6 carbon atoms, alkenoylamino of 3-8 carbon atoms, alkynoylamino of 3-8 carbon atoms, and benzoylamino.

Suitably R⁶⁶ is C₁₋₆ alkyl such as methyl. Preferably however it is a substituted C₁₋₄ alkyl group, wherein the substitutents are selected from hydroxy, NR^{d}R^{e}, S(O)ₑR^{d}, NR^{d}C(O)R^{e}; C(O)NR^{d}R^{e}; NR^{d}C(O)NR^{e}R^{f}; NR^{d}S(O)ₑR^{e} where e, R^{d}, R^{e} and R^{f} are as defined above.

Preferably R⁶⁷ is hydrogen.

Examples of preferred groups for R¹, R², R³ and R⁴ are set out in WO 98/43960. Preferably x is 0. Conveniently R¹³ is selected from one of the following sixteen groups:
1) C₁₋₅alkyl which may be unsubstituted or substituted with one or more fluorine atoms, or C₂₋₅alkyl which may be unsubstituted or substituted with one or more groups selected from hydroxy and amino;
2) C₂₋₃alkylX²COR¹⁹ (wherein X² is as defined hereinbefore and R¹⁹ represents -NR²¹R²²- or -OR²³- (wherein R²¹, R²² and R²³ which may be the same or different each represents hydrogen, C₁₋₂alkyl or C₁₋₂alkoxyethyl));
3) C₂₋₄alkylX³R²⁴ (wherein X³ is as defined hereinbefore and R²⁴ represents hydrogen, C₁₋₃alkyl, cyclopentyl, cyclohexyl or a 5 or 6 membered saturated heterocyclic group with one or two heteroatoms, selected independently from O, S and N, which C₁₋₃alkyl group may bear one or two substituents selected from oxo, hydroxy, halogeno and C₁₋₃alkoxy and which cyclic group may bear one or two substituents selected from oxo, hydroxy, halogeno, C₁₋₃alkyl, C₁₋₃hydroxyalkyl and C₁₋₃alkoxy);
4) C₂₋₃alkylX⁴C₂₋₃alkylX⁵R³⁰ (wherein X⁴ and X⁵ are as defined hereinbefore and R³⁰ represents hydrogen or C₁₋₃alkyl);
5) C₁₋₅alkylR⁷⁰ (wherein R⁷⁰ is a 5 or 6 membered saturated heterocyclic group with one or two heteroatoms, selected independently from O, S and N, which heterocyclic group is linked to C₁₋₅alkyl through a carbon atom and which heterocyclic group may bear one or two substituents selected from oxo, hydroxy, halogeno, C₁₋₃alkyl, C₁₋₃hydroxyalkyl and C₁₋₃alkoxy) or C₂₋₅alkylR⁷¹ (wherein R⁷¹ is a 5 or 6 membered saturated heterocyclic group with one or two heteroatoms of which one is N and the other is selected independently from O, S and N, which heterocyclic group is linked to C₂₋₅alkyl through a nitrogen atom and which heterocyclic group may bear one or two substituents selected from oxo, hydroxy, halogeno, C₁₋₃alkyl, C₁₋₃hydroxyalkyl and C₁₋₃alkoxy);
6) (CH₂)_{q}X⁶R³⁷ (wherein X⁶ is as defined hereinbefore; q is an integer from 0 to 4 if X⁶ is a direct bond and q is 0, 2 or 3 if X⁶ is other than a direct bond; and R³⁷ is a phenyl group, a pyridone group or a 5 or 6 membered aromatic heterocyclic group with 1 to 3 heteroatoms selected from O, N and S, of which preferably one is N, which phenyl group, pyridone group or aromatic heterocyclic group may be substituted as hereinbefore defined, advantageously substituted with up to 2 substituents as hereinbefore defined, more preferably substituted with one substituent selected from the group of substituents as hereinbefore defined);
7) C₄₋₅alkenylR⁷² (wherein R⁷² represents R⁷⁰ or R⁷¹ as defined hereinbefore);
8) C₄₋₅alkynylR⁷² (wherein R⁷² represents R⁷⁰ or R⁷¹ as defined hereinbefore);
9) X⁷R⁴⁷ (wherein X⁷ is as defined hereinbefore and R⁴⁷ represents C₁₋₃alkyl which may be unsubstituted or which may be substituted with one or more groups selected from hydroxy, fluoro and amino);
10) C₃₋₅alkenylR³⁷ (wherein R³⁷ is as defined hereinbefore);
11) C₃₋₅alkynylR³⁷ (wherein R³⁷ is as defined hereinbefore);
12) C₄₋₅alkenylX⁸R³⁷ (wherein X⁸ and R³⁷ are as defined hereinbefore);
13) C₄₋₅alkynylX⁹R³⁰ (wherein X⁹ and R³⁰ are as defined hereinbefore);
14) C₁₋₃alkylX¹⁰C₁₋₃alkylR³⁷ (wherein X¹⁰ and R³⁷ are as defined hereinbefore);
15) R³⁶ (wherein R³⁶ is as defined hereinbefore); and
16) C₁₋₃alkylX¹¹C₁₋₃alkylR³⁶ (wherein X¹¹ and R³⁶ are as defined hereinbefore).

Advantageously R¹³ is selected from one of the following eleven groups:
1) C₁₋₄alkyl which may be unsubstituted or substituted with one or more fluorine atoms, or C₂₋₄alkyl which may be unsubstituted or substituted with one or two groups selected from hydroxy and amino;
2) C₂₋₃alkylX²COR¹⁹ (wherein X² is as defined hereinbefore and R¹⁹ represents -NR²¹R²²- or -OR²³- (wherein R²¹, R²² and R²³ which may be the same or different each represents hydrogen, C₁₋₂alkyl or C₁₋₂alkoxyethyl));
3) C₂₋₃alkylX³R²⁴ (wherein X³ is as defined hereinbefore and R²⁴ is a group selected from C₁₋₃alkyl, cyclopentyl, cyclohexyl, pyrrolidinyl and piperidinyl which group is linked to X³ through a carbon atom and which C₁₋₃alkyl group may bear one or two substituents selected from oxo, hydroxy, halogeno and C₁₋₂alkoxy and which cyclopentyl, cyclohexyl, pyrrolidinyl or piperidinyl group may carry one substituent selected from oxo, hydroxy, halogeno, C₁₋₂alkyl, C₁₋₂hydroxyalkyl and C₁₋₂alkoxy);
4) C₂₋₃alkylX⁴C₂₋₃alkylX⁵R³⁰ (wherein X⁴ and X⁵ are as defined hereinbefore) and R³⁰ represents hydrogen or C₁₋₂alkyl);
5) C₁₋₄alkylR⁷⁰ (wherein R⁷⁰ is a group selected from pyrrolidinyl, piperazinyl, piperidinyl, 1,3-dioxolan-2-yl, 1,3-dioxan-2-yl, 1,3-dithiolan-2-yl and 1,3-dithian-2-yl, which group is linked to C₁₋₄alkyl through a carbon atom and which group may carry one or two substituents selected from oxo, hydroxy, halogeno, C₁₋₂alkyl, C₁₋₂hydroxyalkyl and C₁₋₂alkoxy) or C₂₋₄alkylR⁷¹ (wherein R⁷¹ is a group selected from morpholino, thiomorpholino, pyrrolidin-1-yl, piperazin-1-yl and piperidino which group may carry one or two substituents selected from oxo, hydroxy, halogeno, C₁₋₂alkyl, C₁₋₂hydroxyalkyl and C₁₋₂alkoxy); and
6) (CH₂)_{q}X⁶R³⁷ (wherein X⁶ is as defined hereinbefore; q is an integer from 1 to 3 if X⁶ is a direct bond and q is 2 or 3 if X⁶ is other than a direct bond; and R³⁷ is a phenyl group, a pyridone group or a 5 or 6 membered aromatic heterocyclic group with 1 to 2 heteroatoms selected from O, N and S, of which preferably one is N, which phenyl group, pyridone group or aromatic heterocyclic group may be substituted as hereinbefore defined, preferably substituted with one substituent selected from hydroxy, halogeno, C₁₋₂alkyl, C₁₋₂alkoxy, C₁₋₂hydroxyalkyl, C₁₋₂hydroxyalkoxy, carboxy, cyano, -CONR⁴³R⁴⁴ and -NR⁴⁵COR⁴⁶ (wherein R⁴³, R⁴⁴, R⁴⁵ and R⁴⁶, which may be the same or different, each represents hydrogen or C₁₋₂alkyl));
7) C₄₋₅alkenylR⁷¹ (wherein R⁷¹ is as defined hereinbefore);
8) C₄₋₅alkynylR⁷¹ (wherein R⁷¹ is as defined hereinbefore);
9) C₁₋₃alkylX¹⁰C₁₋₃alkylR³⁷ (wherein X¹⁰ and R³⁷ are as defined hereinbefore);
10) R³⁶ (wherein R³⁶ is as defined hereinbefore); and
11) C₁₋₃alkylX¹¹C₁₋₃alkylR³⁶ (wherein X¹¹ and R³⁶ are as defined hereinbefore).

Preferably R¹³ is selected from one of the following nine groups:
1) C₁₋₃alkyl which may be unsubstituted or substituted with one or more fluorine atoms, or C₂₋₃alkyl which may be unsubstituted or substituted with one or two groups selected from hydroxy and amino;
2) 2-(3,3-dimethylureido)ethyl, 3-(3,3-dimethylureido)propyl, 2-(3-methylureido)ethyl, 3-(3-methylureido)propyl, 2-ureidoethyl, 3-ureidopropyl, 2-(N,N-dimethylcarbamoyloxy)ethyl, 3-(N,N-dimethylcarbamoyloxy)propyl, 2-(N-methylcarbamoyloxy)ethyl, 3-(N-methylcarbamoyloxy)propyl, 2-(carbamoyloxy)ethyl, 3-(carbamoyloxy)propyl;
3) C₂₋₃alkylX³R²⁴ (wherein X³ is as defined hereinbefore and R²⁴ is a group selected from C₁₋₂alkyl, cyclopentyl, cyclohexyl, pyrrolidinyl and piperidinyl which group is linked to X³ through a carbon atom and which C₁₋₂alkyl group may bear one or two substituents selected from oxo, hydroxy, halogeno and C₁₋₂alkoxy and which cyclopentyl, cyclohexyl, pyrrolidinyl or piperidinyl group may carry one substituent selected from oxo, hydroxy, halogeno, C₁₋₂alkyl, C₁₋₂hydroxyalkyl and C₁₋₂alkoxy);
4) C₂₋₃alkylX⁴C₂₋₃alkylX⁵R³² (wherein X⁴ and X⁵ are as defined hereinbefore) and R³⁰ represents hydrogen or C₁₋₂alkyl);
5) C₁₋₂alkylR⁷⁰ (wherein R⁷⁰ is a group selected from pyrrolidinyl, piperazinyl, piperidinyl, 1,3-dioxolan-2-yl, 1,3-dioxan-2-yl, 1,3-dithiolan-2-yl and 1,3-dithian-2-yl, which group is linked to C₁₋₂alkyl through a carbon atom and which group may carry one substituent selected from oxo, hydroxy, halogeno, C₁₋₂alkyl, C₁₋₂hydroxyalkyl and C₁₋₂alkoxy) or C₂₋₃alkylR⁵⁹ (wherein R⁵⁹ is a group selected from morpholino, thiomorpholino, piperidino, piperazin-1-yl and pyrrolidin-1-yl which group may carry one or two substituents selected from oxo, hydroxy, halogeno, C₁₋₂alkyl, C₁₋₂hydroxyalkyl and C₁₋₂alkoxy);
6) (CH₂)_{q}X⁶R³⁷ (wherein X⁶ is as defined hereinbefore; q is an integer from 1 to 3 if X⁶ is a direct bond and q is 2 or 3 if X⁶ is other than a direct bond; and R³⁷ is a group selected from phenyl, a pyridone group, pyridyl, imidazolyl, thiazolyl, thienyl, triazolyl and pyridazinyl, preferably selected from phenyl, a pyridone group, pyridyl, imidazolyl, thiazolyl and triazolyl which group may be substituted with one substituent selected from hydroxy, halogeno, C₁₋₂alkyl, C₁₋₂alkoxy, C₁₋₂hydroxyalkyl, C₁₋₂hydroxyalkoxy, carboxy, cyano, -CONR⁴³R⁴⁴ and -NR⁴⁵COR⁴⁶ (wherein R⁴³, R⁴⁴, R⁴⁵ and R⁴⁶ are as defined hereinbefore);
7) C₁₋₃alkylX¹⁰C₁₋₃alkylR³⁷ (wherein X¹⁰ and R³⁷ are as defined hereinbefore);
8) R³⁶ (wherein R³⁶ is as defined hereinbefore); and
9) C₁₋₃alkylX¹¹C₁₋₃alkylR³⁶ (wherein X¹¹ and R³⁶ are as defined hereinbefore). More preferably R¹³ represents 2-methylthiazol-4-ylmethyl, 2-acetamidothiazol-4-ylmethyl, 1-methylimidazol-2-ylmethyl, 4-pyridylmethyl, 2-(4-pyridyl)ethyl, 3-(4-pyridyl)propyl, 2-((N-(1-methylimidazol-4-ylsulphonyl)-N-methyl)amino)ethyl, 2-((N-(3-morpholinopropylsulphonyl)-N-methyl)amino)ethyl, 2-((N-methyl-N-4-pyridyl)amino)ethyl, 2-(4-oxidomorpholino)ethyl, 3-(4-oxidomorpholino)propyl, 2-(4-oxo-1,4-dihydro-1-pyridyl)ethyl, 3-(4-oxo-1,4-dihydro-1-pyridyl)propyl, methyl, ethyl, trifluoromethyl, 2,2,2-trifluoroethyl, 2-hydroxyethyl, 3-hydroxypropyl, 2-(N,N-dimethylsulphamoyl)ethyl, 2-(N-methylsulphamoyl)ethyl, (1,3-dioxolan-2-yl)methyl, 2-(1,3-dioxolan-2-yl)ethyl, 2-(2-methoxyethylamino)ethyl, 2-(2-hydroxyethylamino)ethyl, 3-(2-methoxyethylamino)propyl, 3-(2-hydroxyethylamino)propyl, 2-(1,2,4-triazol-1-yl)ethyl, 2-(1,2,4-triazol-4-yl)ethyl, 3-(1,2,4-triazol-1-yl)propyl, 3-(1,2,4-triazol-4-yl)propyl, 2-(4-pyridyloxy)ethyl, 3-(4-pyridyloxy)propyl, 2-(4-pyridylamino)ethyl, 3-(4-pyridylamino)propyl, 2-(2-methylimidazol-1-yl)ethyl, 3-(2-methylimidazol-1-yl)propyl, 2-(5-methyl-1,2,4-triazol-1-yl)ethyl, 3-(5-methyl-1,2,4-triazol-1-yl)propyl, morpholino, N-methylpiperazinyl, piperazinyl, 2-(N,N-dimethylamino)ethyl, 3-(N,N-dimethylamino)propyl, 2-morpholinoethyl, 3-morpholinopropyl, 2-piperidinoethyl, 3-piperidinopropyl, 2-(piperazin-1-yl)ethyl, 3-(piperazin-1-yl)propyl, 2-(pyrrolidin-1-yl)ethyl, 3-(pyrrolidin-1-yl)propyl, 2-methoxyethyl, 3-methoxypropyl, 2-(imidazol-1-yl)ethyl, 2-(1,2,3-triazol-1-yl)ethyl, 2-(1,2,3-triazol-2-yl)ethyl, 3-(imidazol-1-yl)propyl, 3-(1,2,3-triazol-1-yl)propyl, 3-(1,2,3-triazol-2-yl)propyl, 2-thiomorpholinoethyl, 3-thiomorpholinopropyl, 2-(1,1-dioxothiomorpholino)ethyl, 3-(1,1-dioxothiomorpholino)propyl, 2-(2-methoxyethoxy)ethyl, 2-(4-methylpiperazin-1-yl)ethyl, 3-(4-methylpiperazin-1-yl)propyl, 3-(methylsulphinyl)propyl, 3-(methylsulphonyl)propyl, 2-(methylsulphinyl)ethyl, benzyl, 2-sulphamoylethyl or 2-(methylsulphonyl)ethyl.

Especially R¹³ represents methyl, ethyl, trifluoromethyl, 2,2,2-trifluoroethyl, 2-hydroxyethyl, 3-hydroxypropyl, 2-methoxyethyl, 3-methoxypropyl, 2-(methylsulphinyl)ethyl, 2-(methylsulphonyl)ethyl, 2-(N,N-dimethylsulphamoyl)ethyl, 2-(N-methylsulphamoyl)ethyl, 2-sulphamoylethyl, 2-(N,N-dimethylamino)ethyl, 3-(N,N-dimethylamino)propyl, 2-morpholinoethyl, 3-morpholinopropyl, 2-piperidinoethyl, 3-piperidinopropyl, 2-(piperazin-1-yl)ethyl, 3-(piperazin-1-yl)propyl, 2-(pyrrolidin-1-yl)ethyl, 3-(pyrrolidin-1-yl)propyl, (1,3-dioxolan-2-yl)methyl, 2-(1,3-dioxolan-2-yl)ethyl, 2-(2-methoxyethylamino)ethyl, 2-(2-hydroxyethylamino)ethyl, 3-(2-methoxyethylamino)propyl, 3-(2-hydroxyethylamino)propyl, 2-methylthiazol-4-ylmethyl, 2-acetamidothiazol-4-ylmethyl, 1-methylimidazol-2-ylmethyl, 2-(imidazol-1-yl)ethyl, 2-(1,2,3-triazol-1-yl)ethyl, 2-(1,2,3-triazol-2-yl)ethyl, 2-(1,2,4-triazol-1-yl)ethyl, 2-(1,2,4-triazol-4-yl)ethyl, 4-pyridylmethyl, 2-(4-pyridyl)ethyl, 3-(4-pyridyl)propyl, 3-(3-pyridyl)propyl, benzyl, 2-(4-pyridyloxy)ethyl, 2-(4-pyridylamino)ethyl, or 2-(4-oxo-1,4-dihydro-1-pyridyl)ethyl.

More especially R¹³ represents methyl, ethyl, trifluoromethyl, 2,2,2-trifluoroethyl, 2-hydroxyethyl, 3-hydroxypropyl, 2-methoxyethyl, 3-methoxypropyl, 2-(methylsulphinyl)ethyl, 2-(methylsulphonyl)ethyl, 2-(N,N-dimethylsulphamoyl)ethyl, 2-(N-methylsulphamoyl)ethyl, 2-sulphamoylethyl, 2-(N,N-dimethylamino)ethyl, 3-(N,N-dimethylamino)propyl, 2-morpholinoethyl, 3-morpholinopropyl, 2-piperidinoethyl, 3-piperidinopropyl, 2-(piperazin-1-yl)ethyl, 3-(piperazin-1-yl)propyl, 2-(pyrrolidin-1-yl)ethyl, 3-(pyrrolidin-1-yl)propyl, (1,3-dioxolan-2-yl)methyl, 2-(1,3-dioxolan-2-yl)ethyl, 2-(2-methoxyethylamino)ethyl, 2-(2-hydroxyethylamino)ethyl, 3-(2-methoxyethylamino)propyl, 3-(2-hydroxyethylamino)propyl, 2-methylthiazol-4-ylmethyl, 2-acetamidothiazol-4-ylmethyl, 1-methylimidazol-2-ylmethyl, 2-(imidazol-1-yl)ethyl, 2-(1,2,3-triazol-1-yl)ethyl, 2-(1,2,3-triazol-2-yl)ethyl, 2-(1,2,4-triazol-1-yl)ethyl, 2-(1,2,4-triazol-4-yl)ethyl, 4-pyridylmethyl, 2-(4-pyridyl)ethyl, 3-(4-pyridyl)propyl, benzyl, 2-(4-pyridyloxy)ethyl, 2-(4-pyridylamino)ethyl, or 2-(4-oxo-1,4-dihydro-1-pyridyl)ethyl.

In particular R¹ and R⁴ are suitably hydrogen.

Examples of preferred groups for R² include C₁₋₆ alkoxy such as methoxy.

The group R³ is suitably selected from hydrogen or C₁₋₆alkoxy.

Preferably both R² and R³ are C₁₋₆ alkoxy and are preferably methoxy.

A further preferred group for R² or R³ is 3-morpholinopropyloxy.

Particular examples of compounds of formula (I) are listed in Tables 1, 2 and 3. In these tables "DMMPO" indicates a 1,6-dimethylmorpholinopropoxy group of formula: 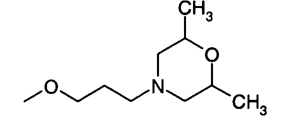 "MPO" is morpholinopropoxy group of formula: 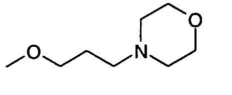 "MEO" is a morpholinoethoxygroup of formula: 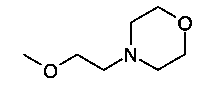 and Me is CH₃

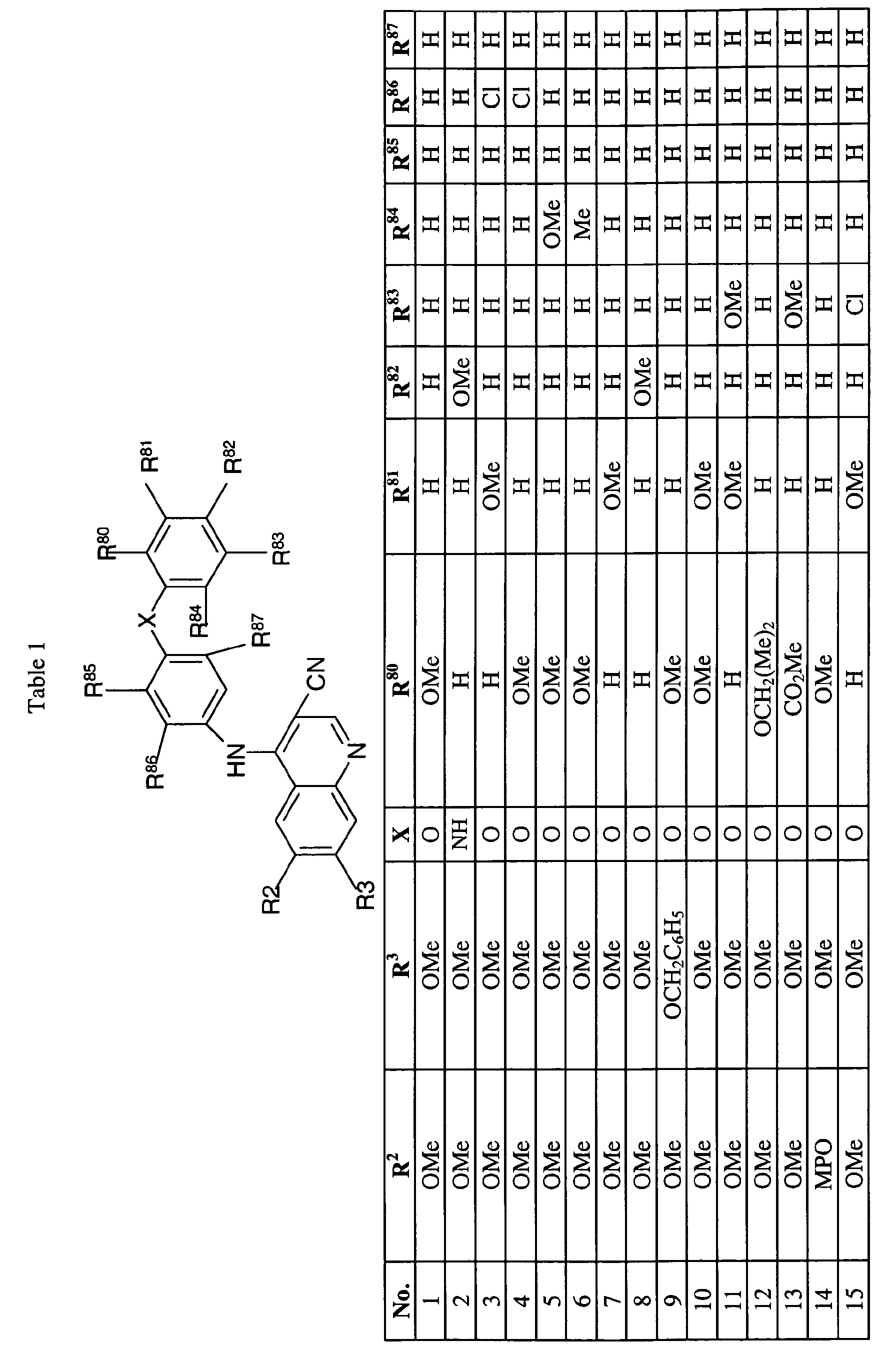

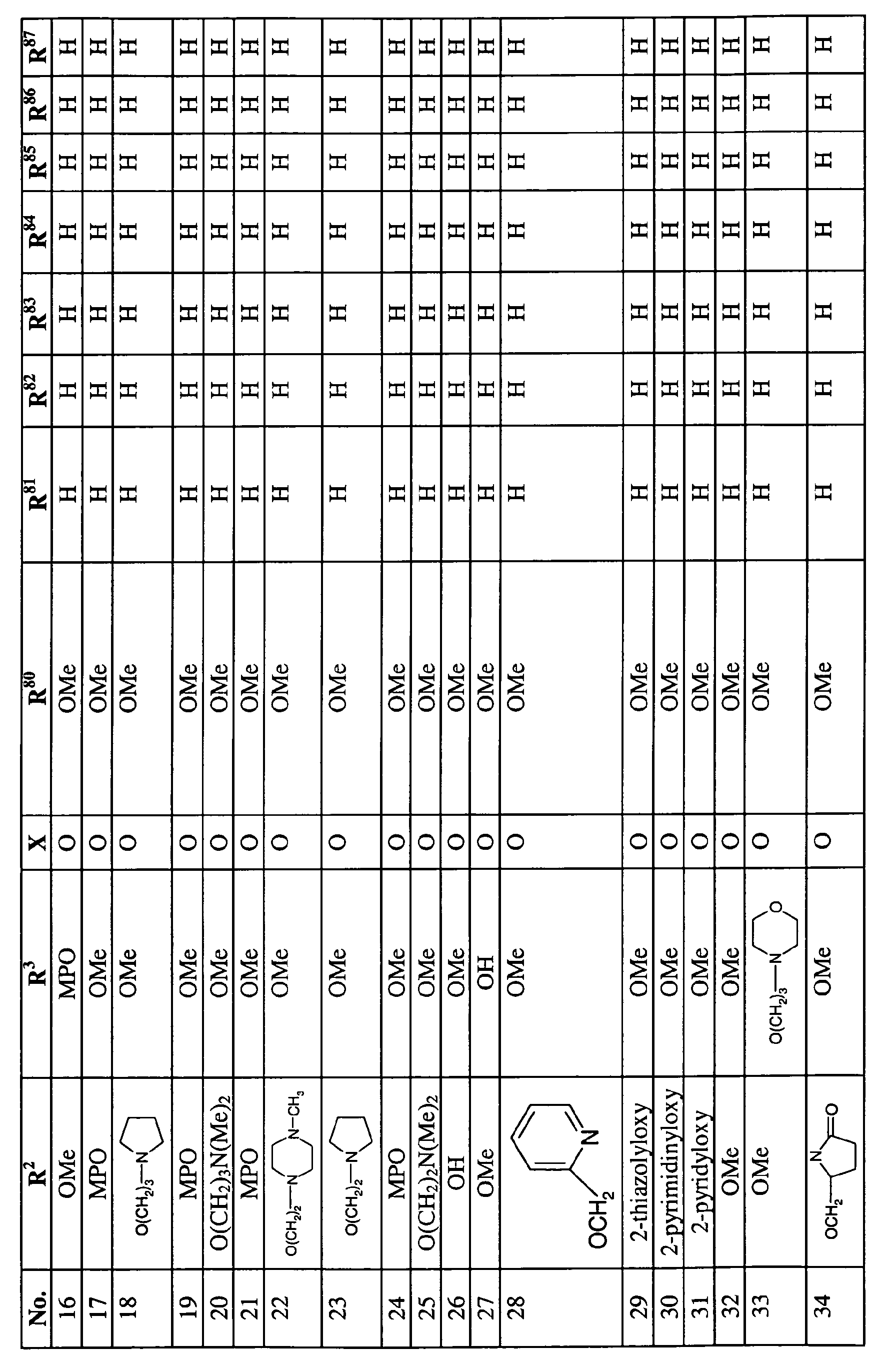

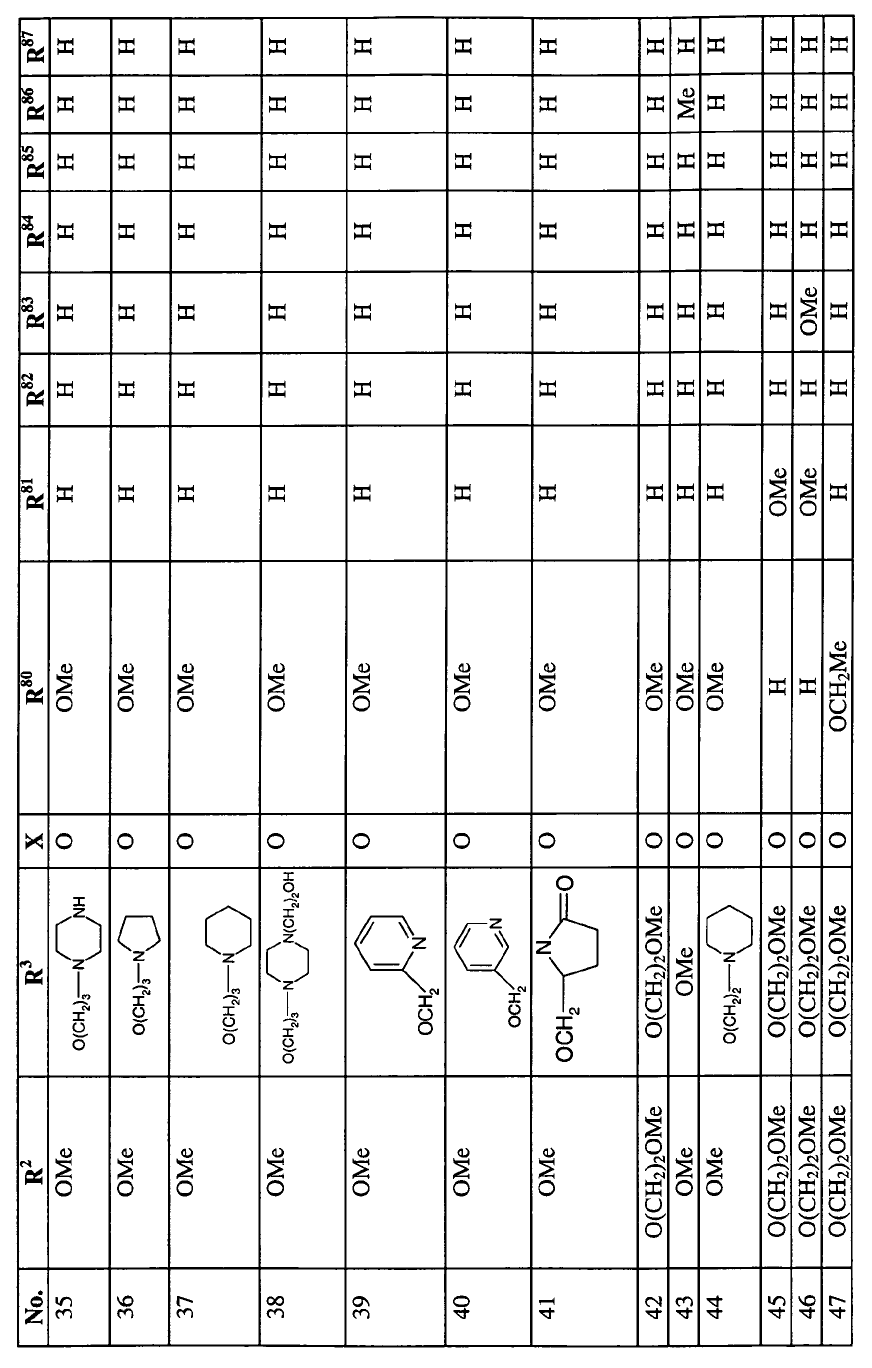

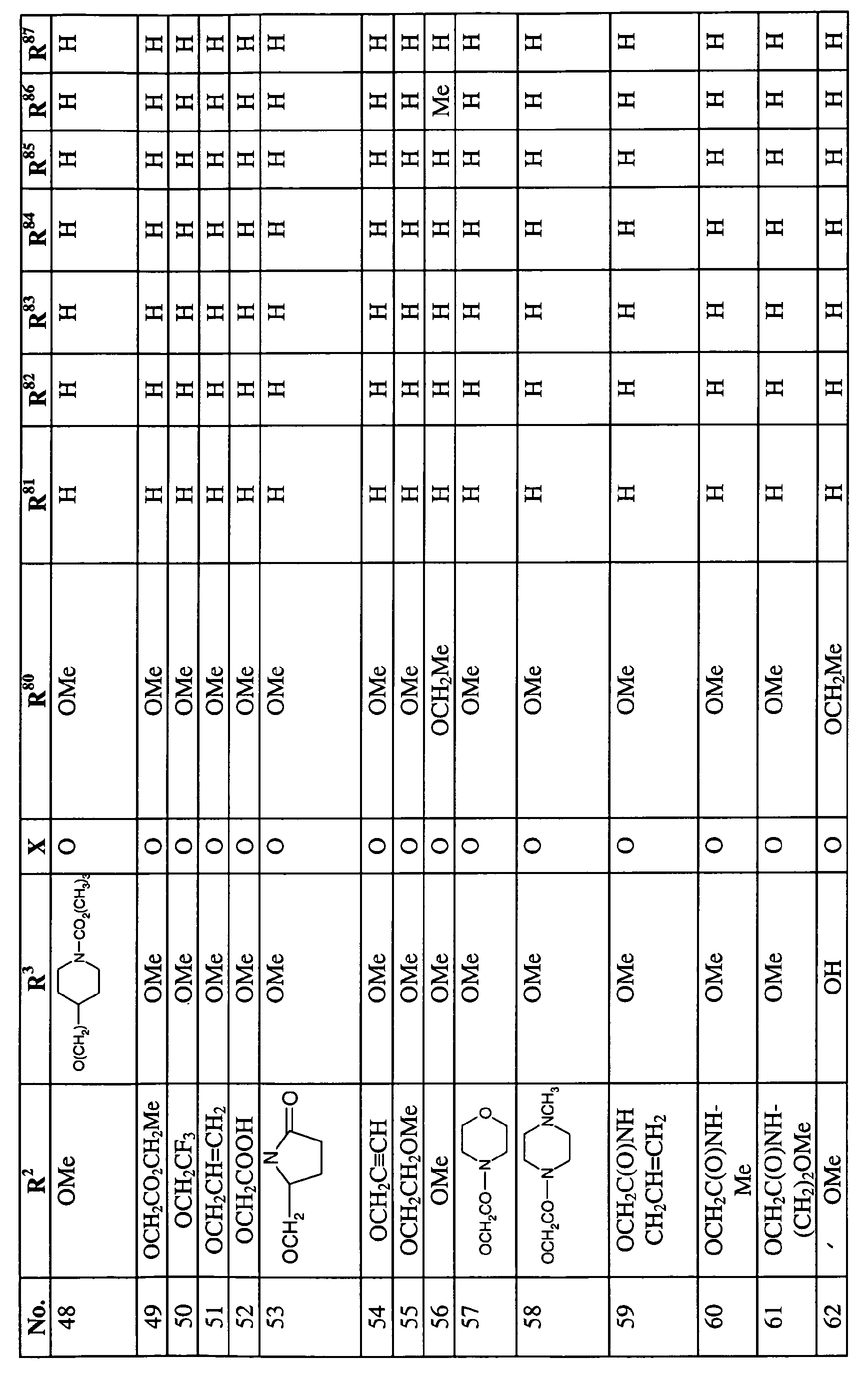

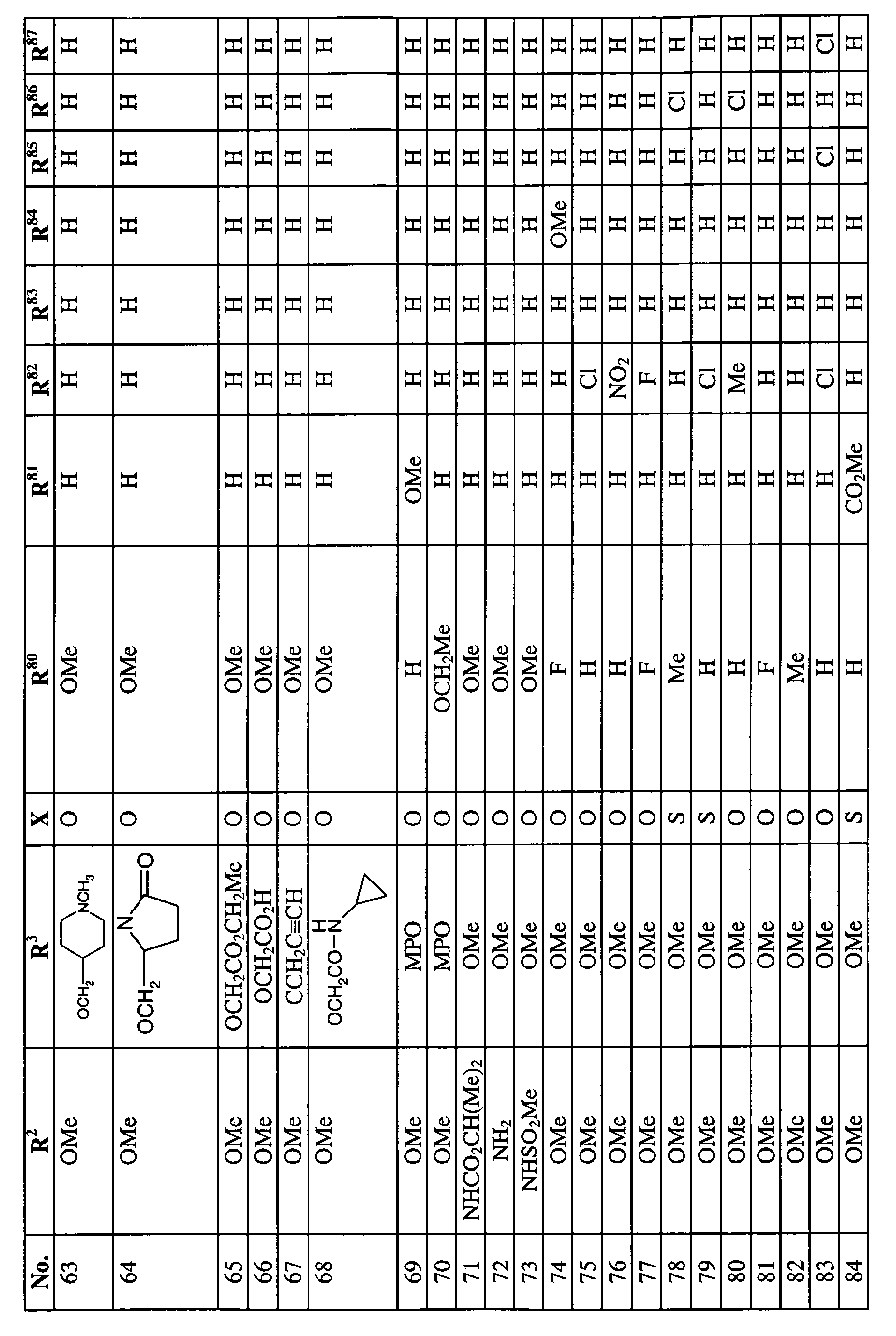

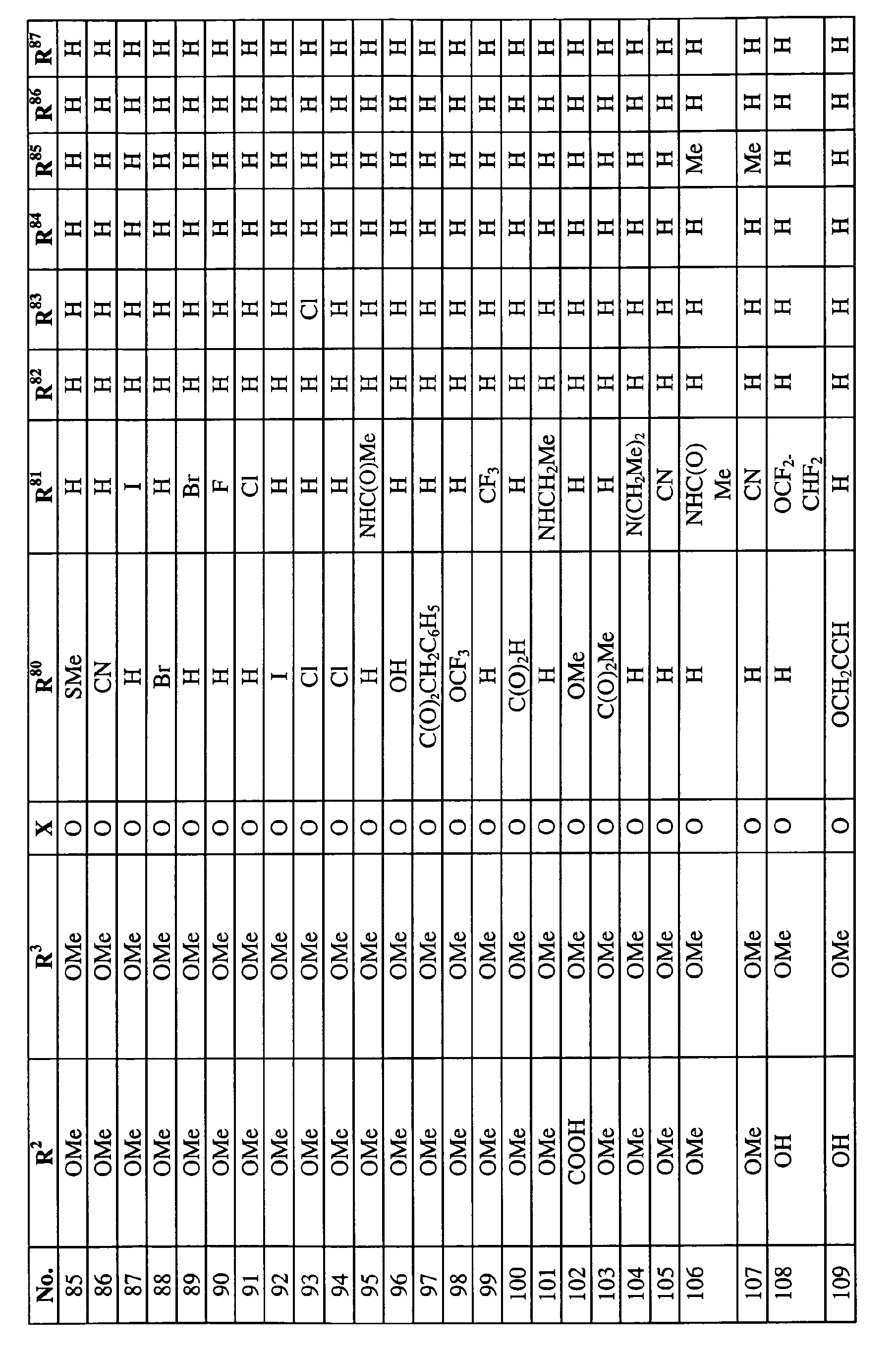

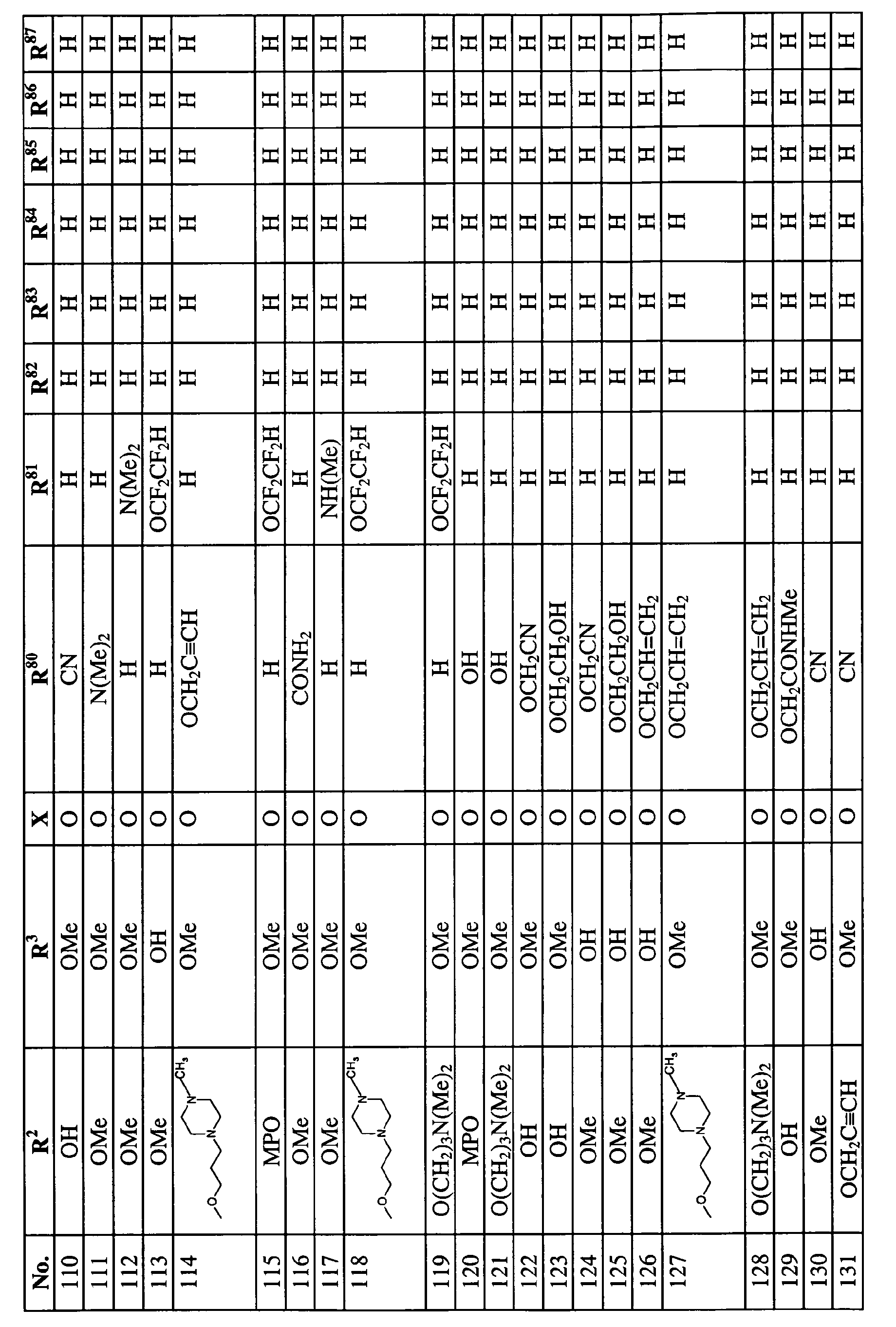

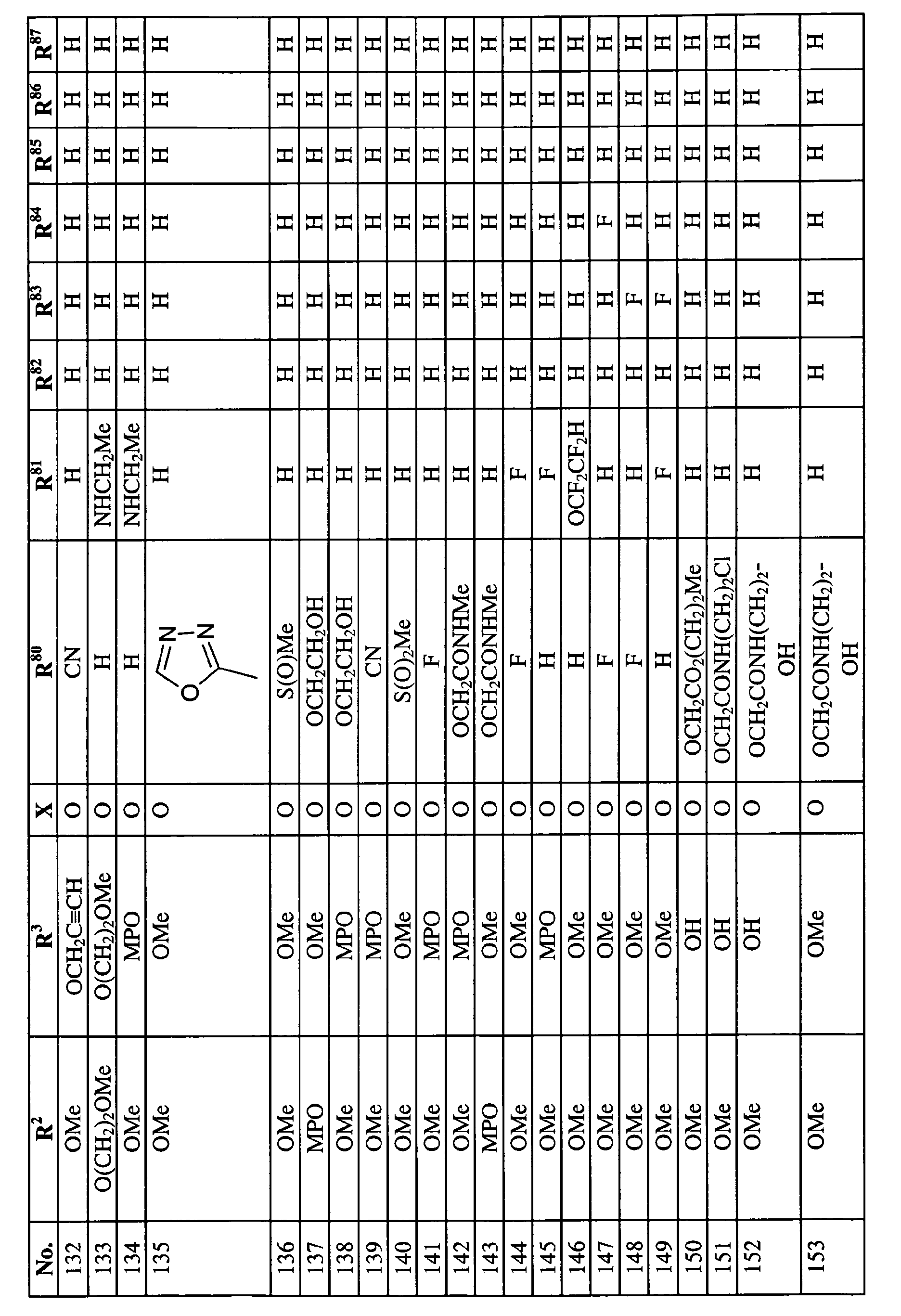

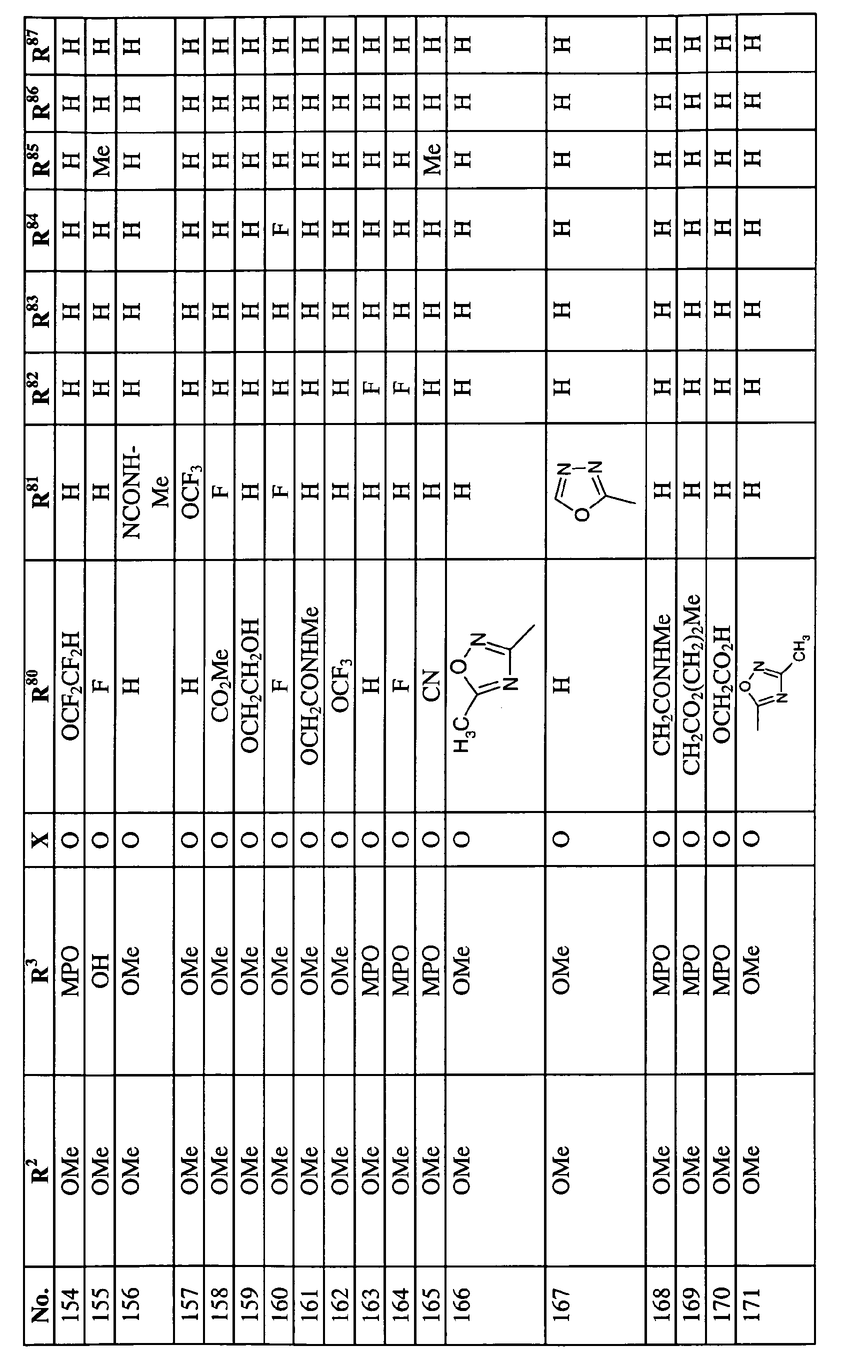

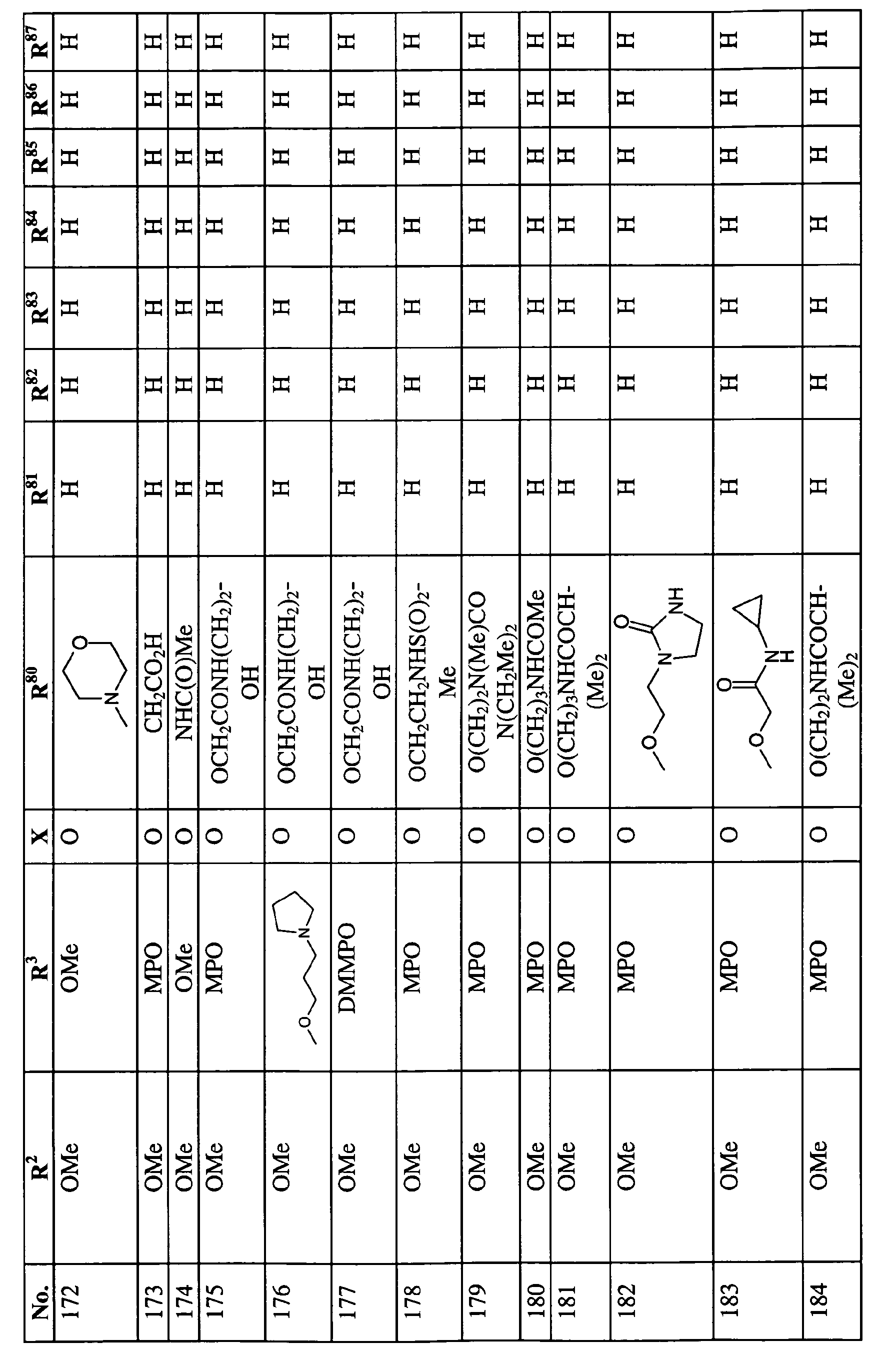

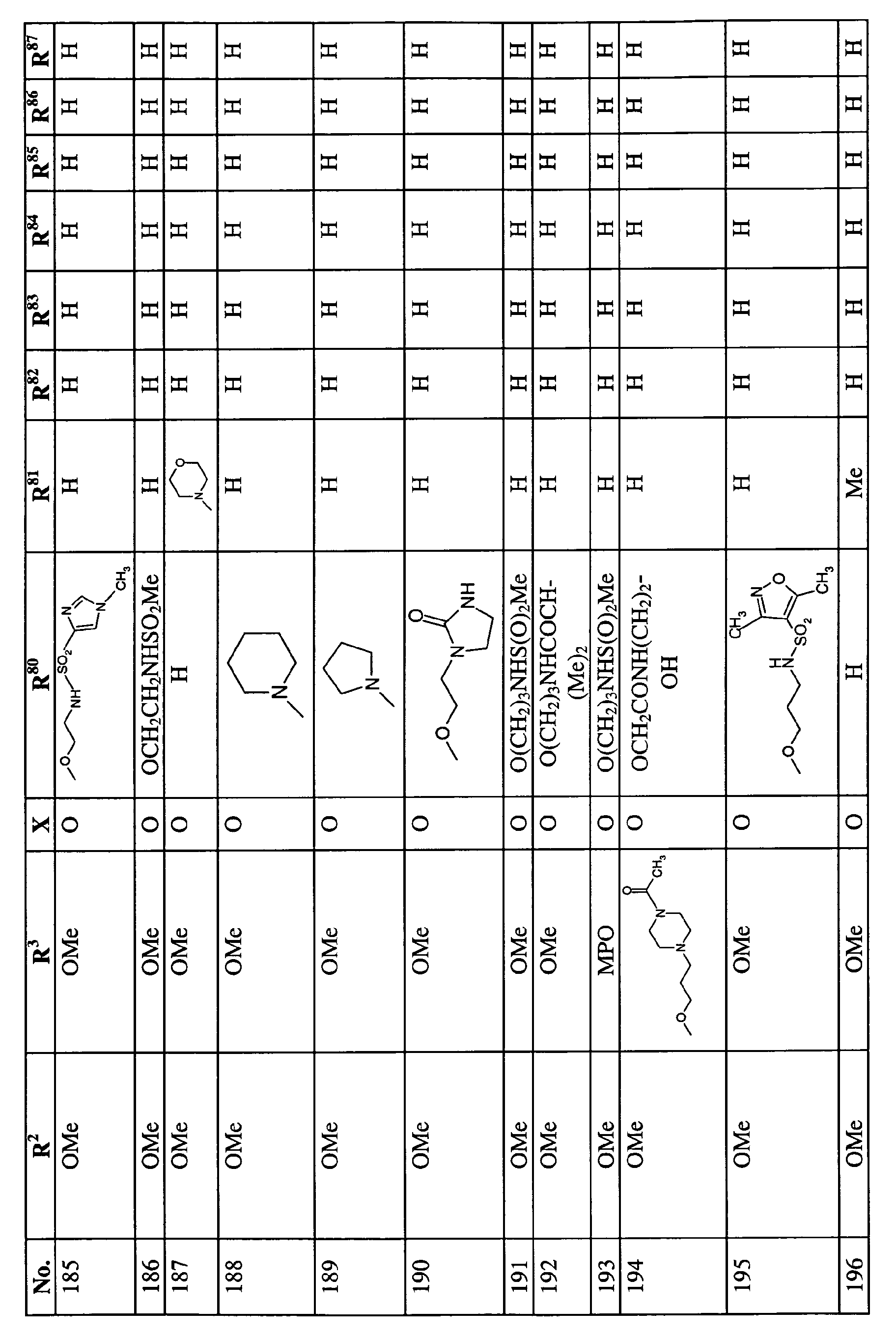

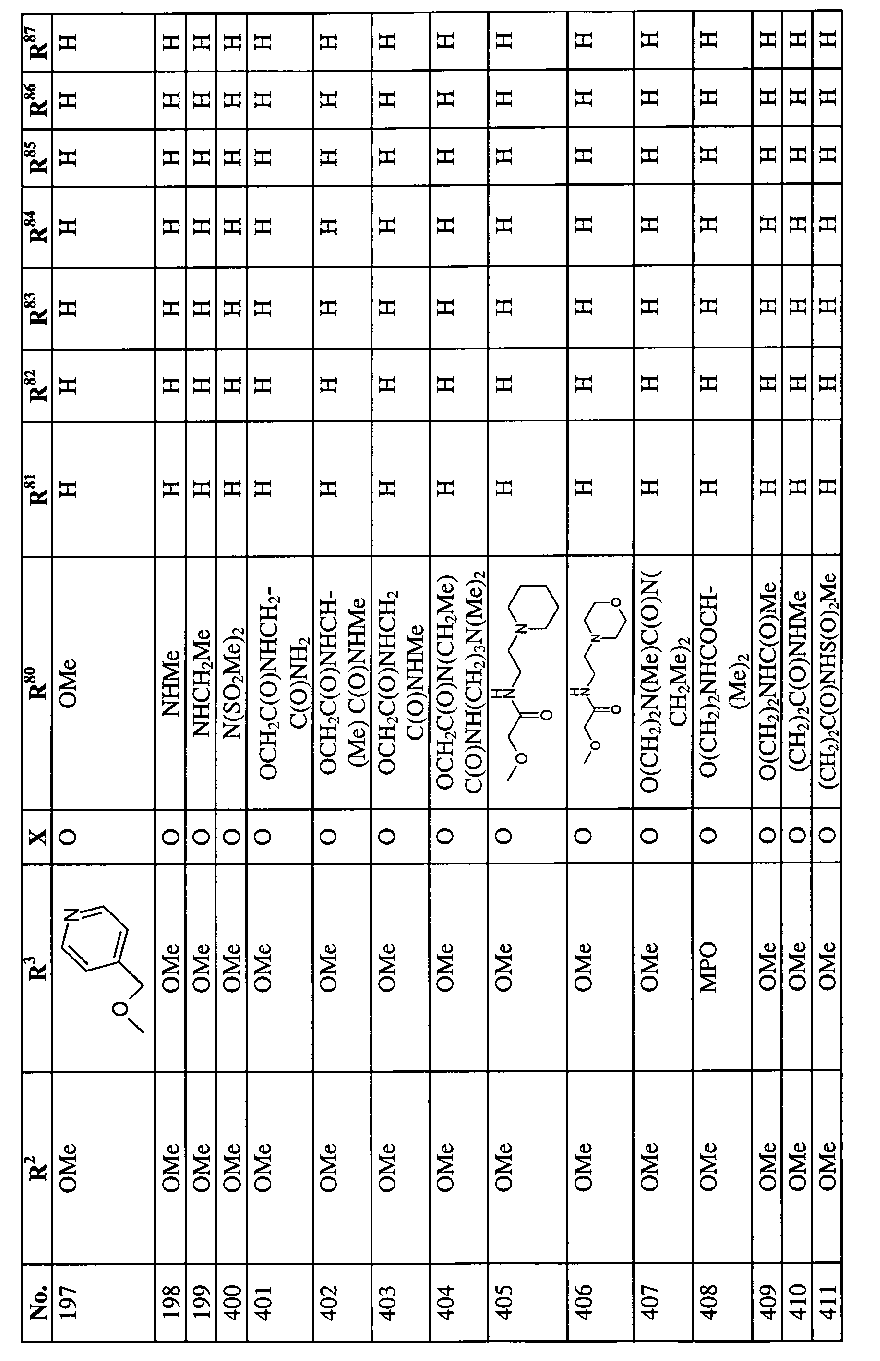

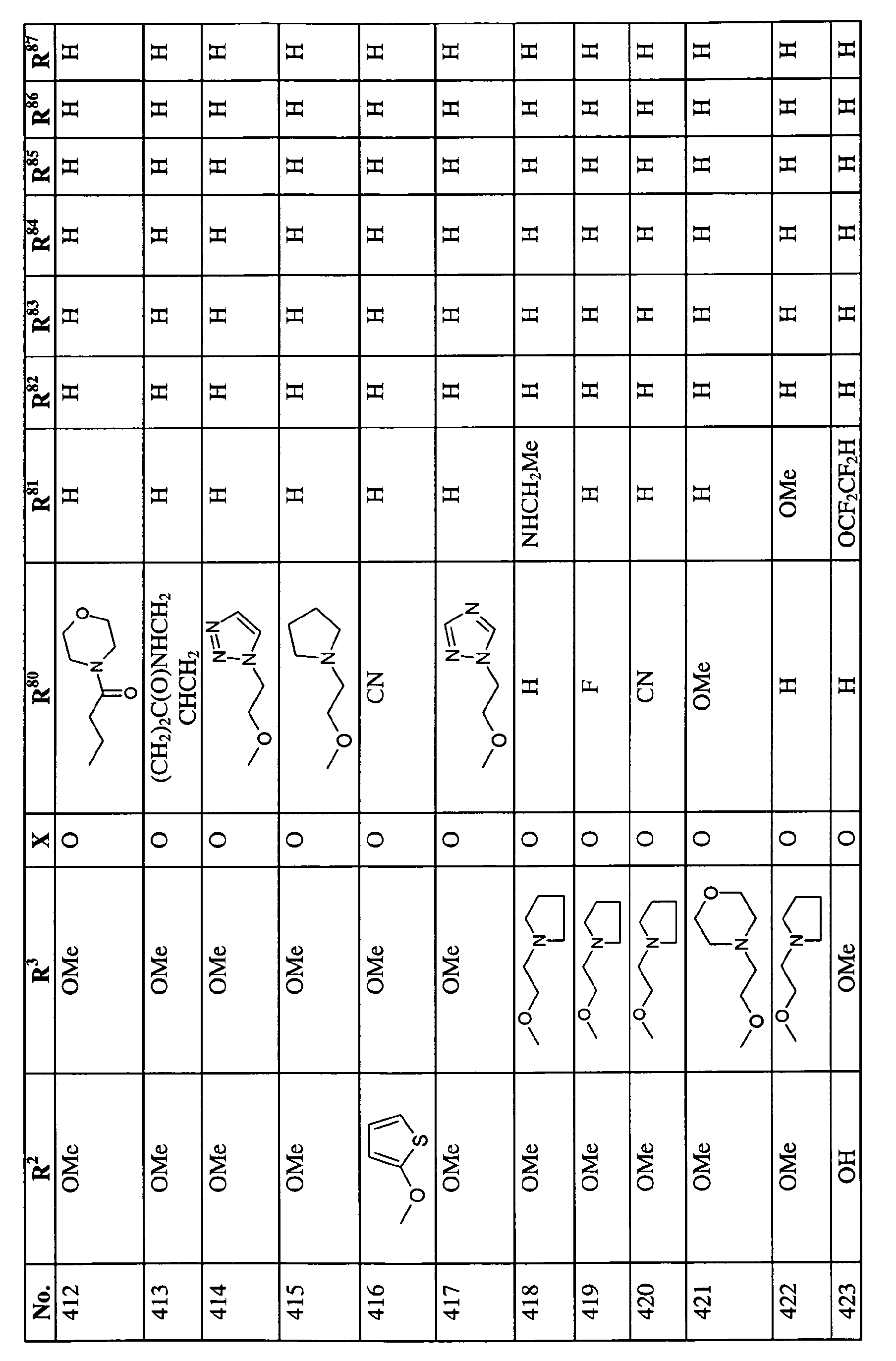

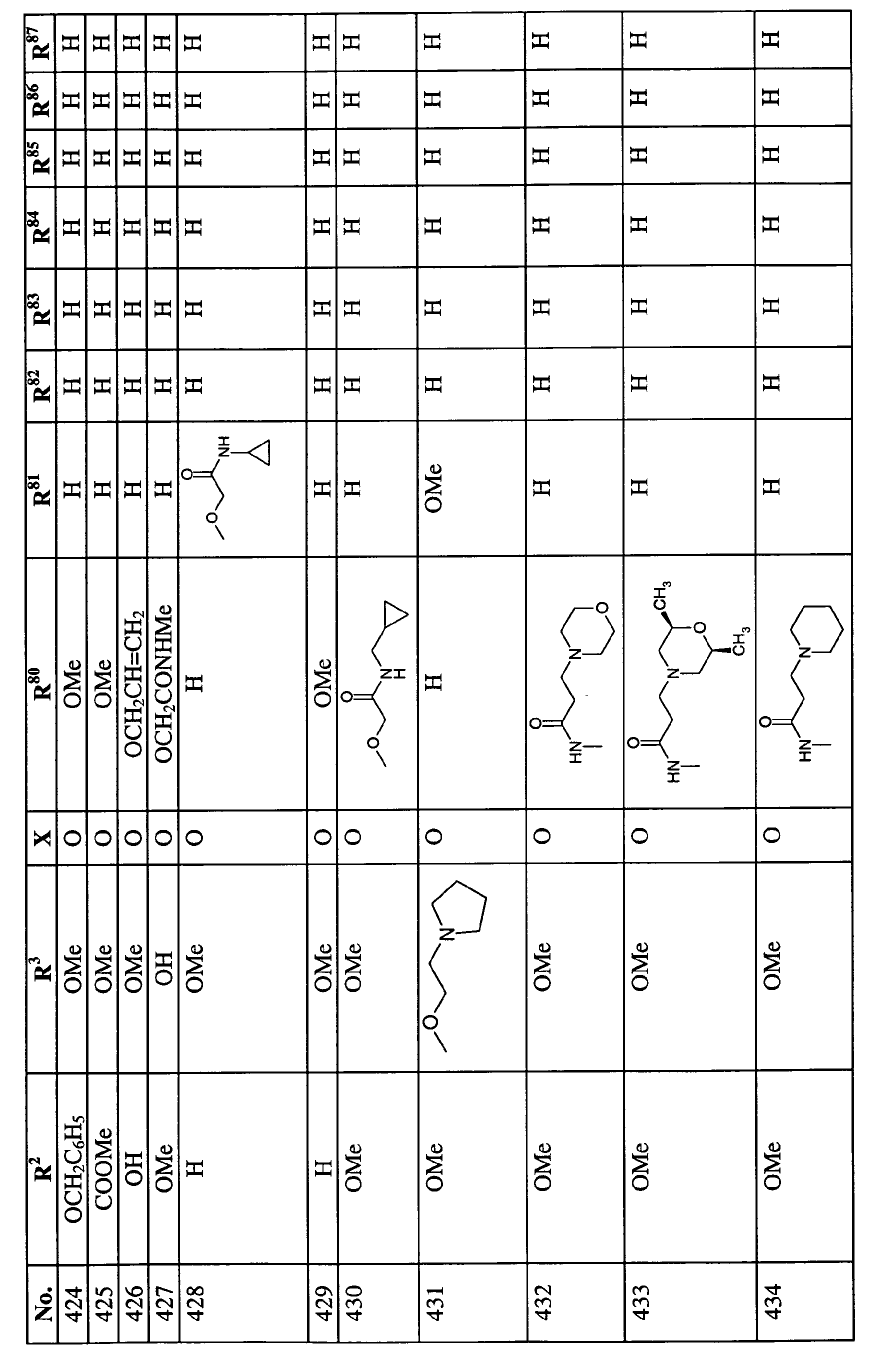

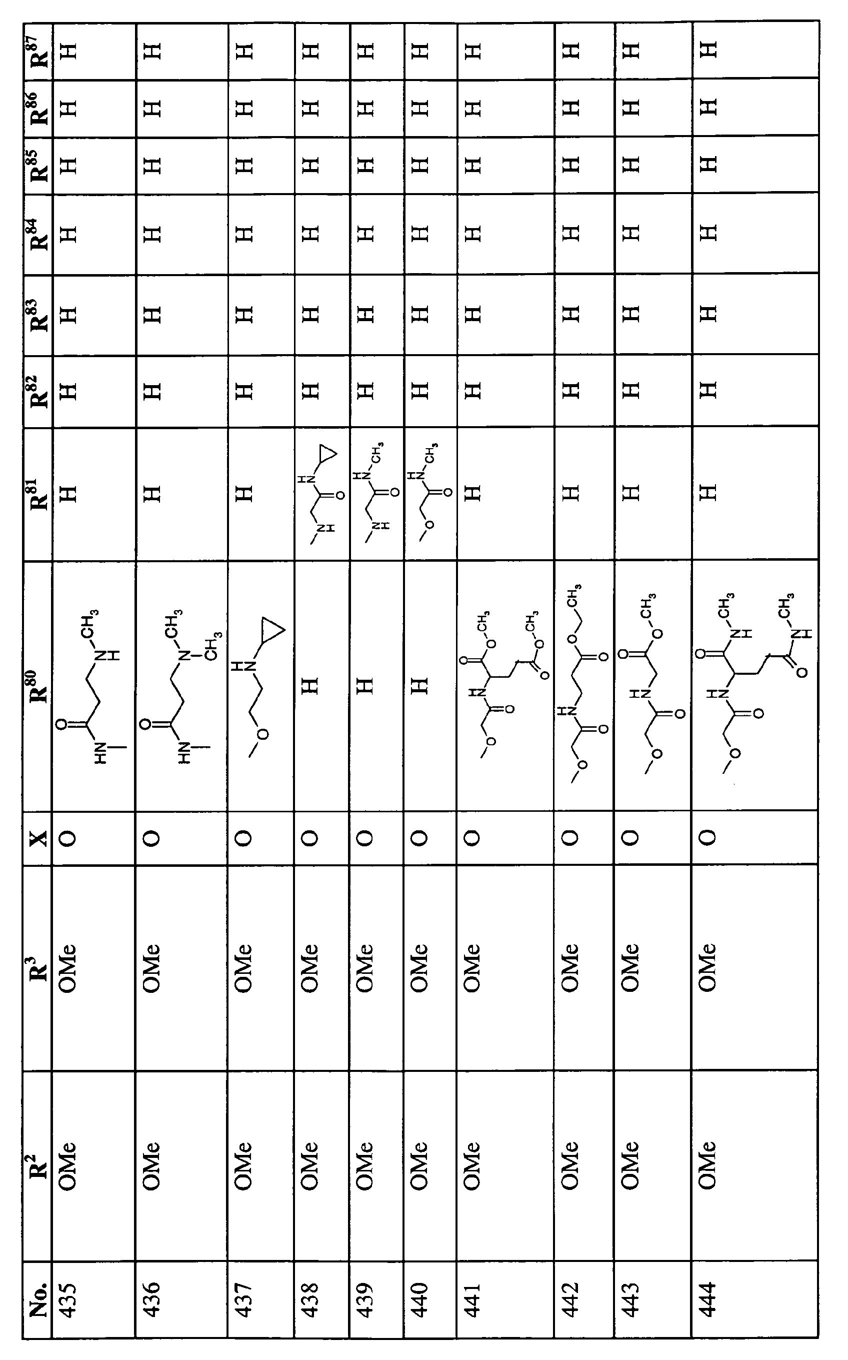

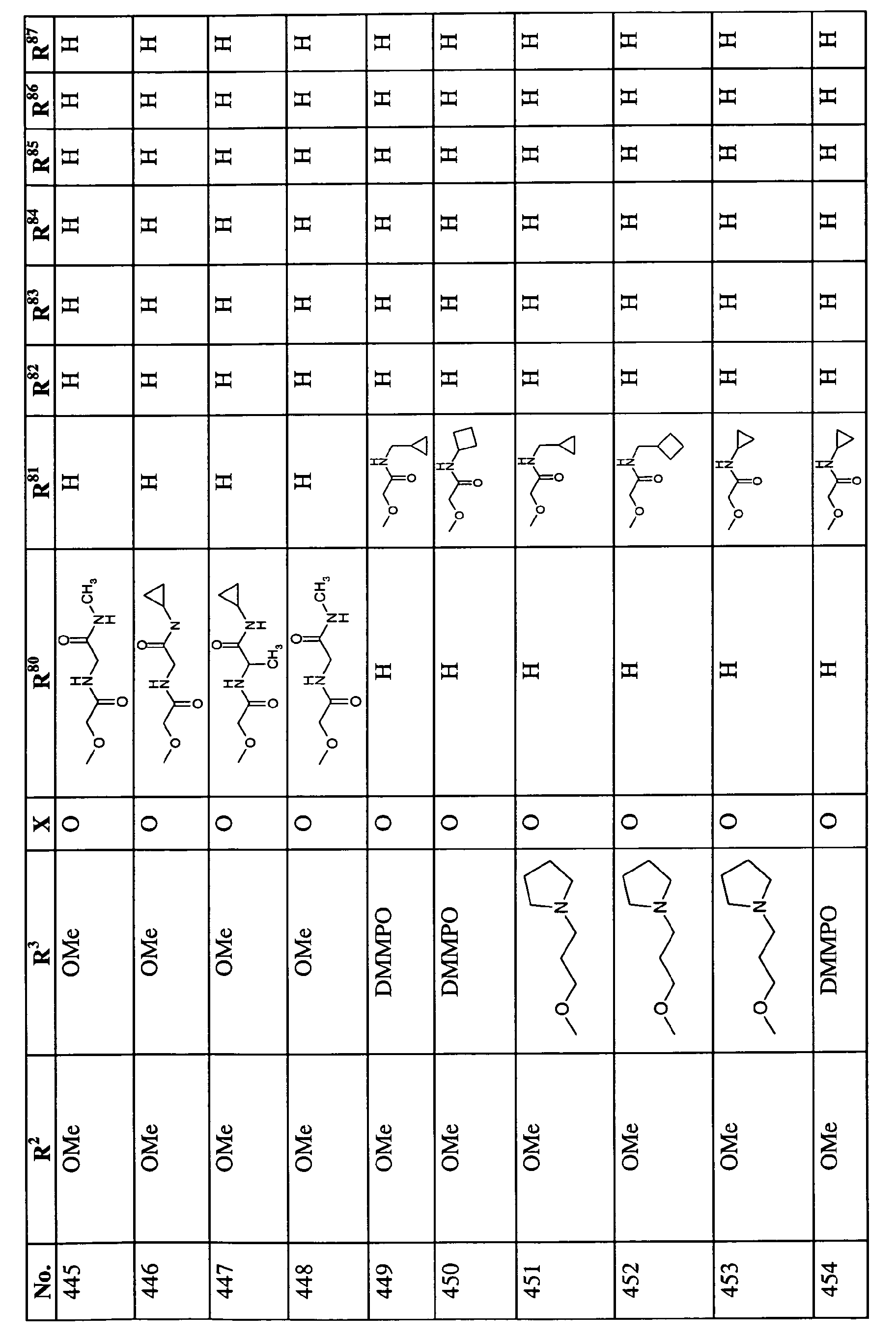

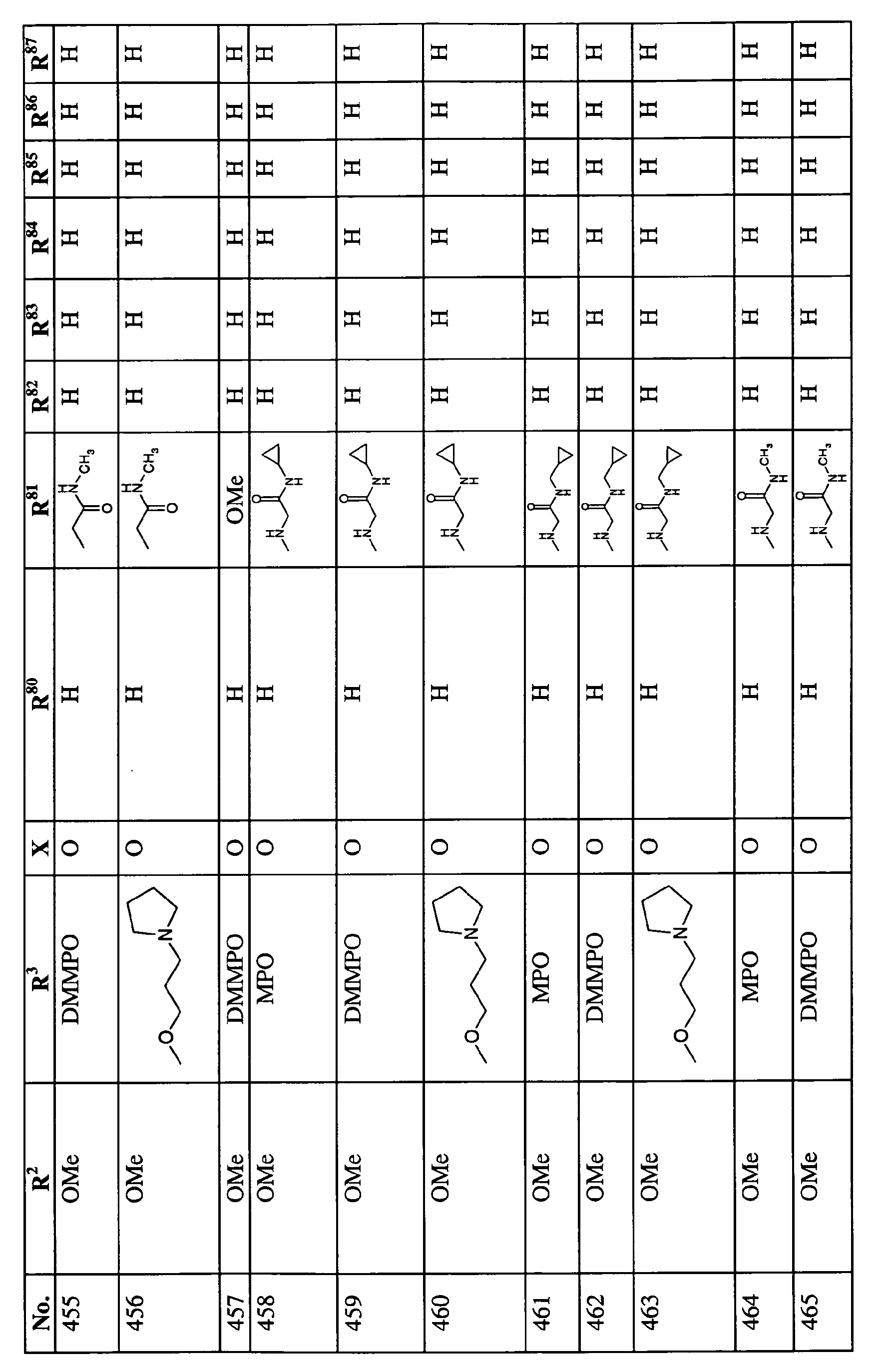

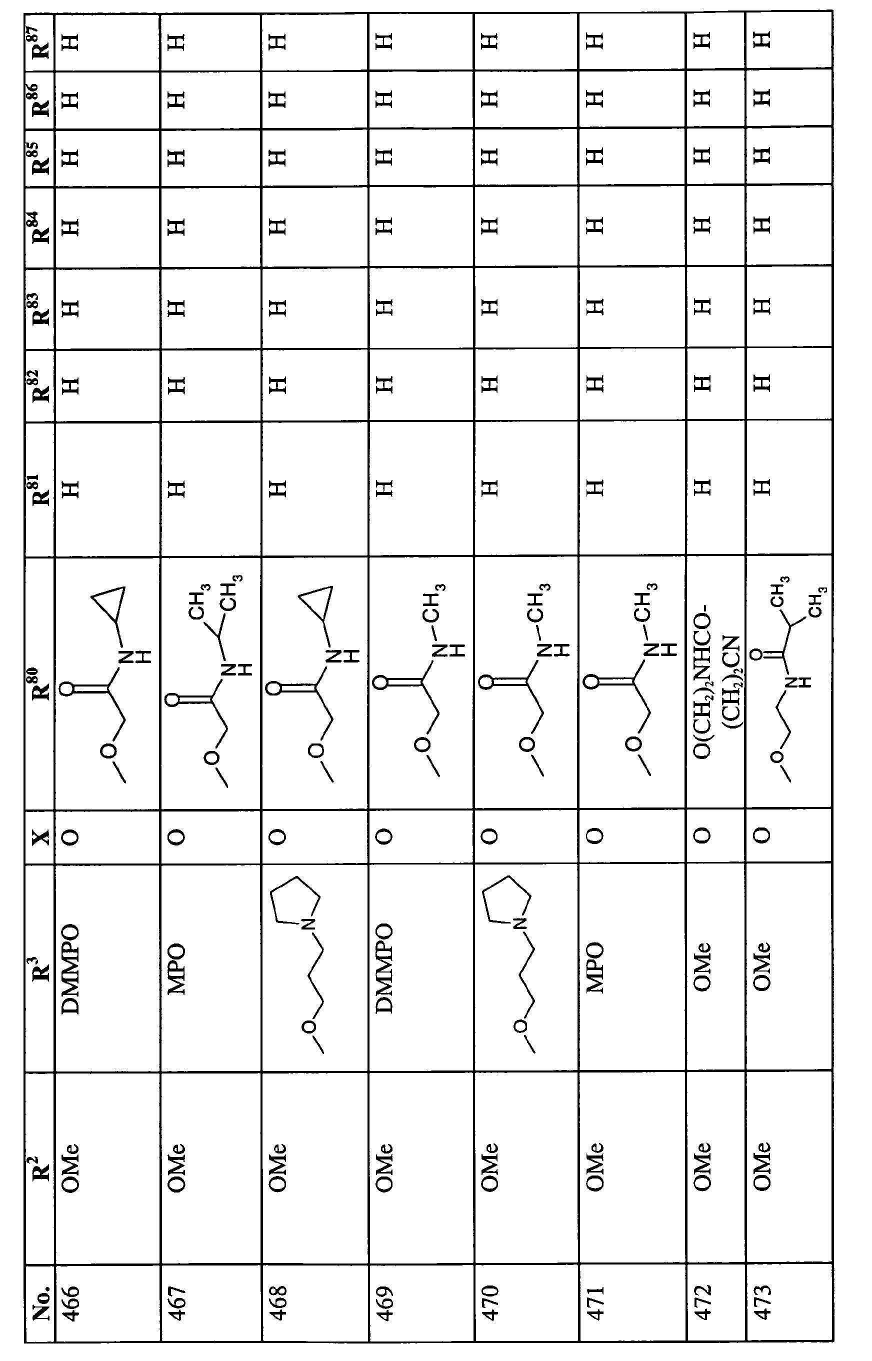

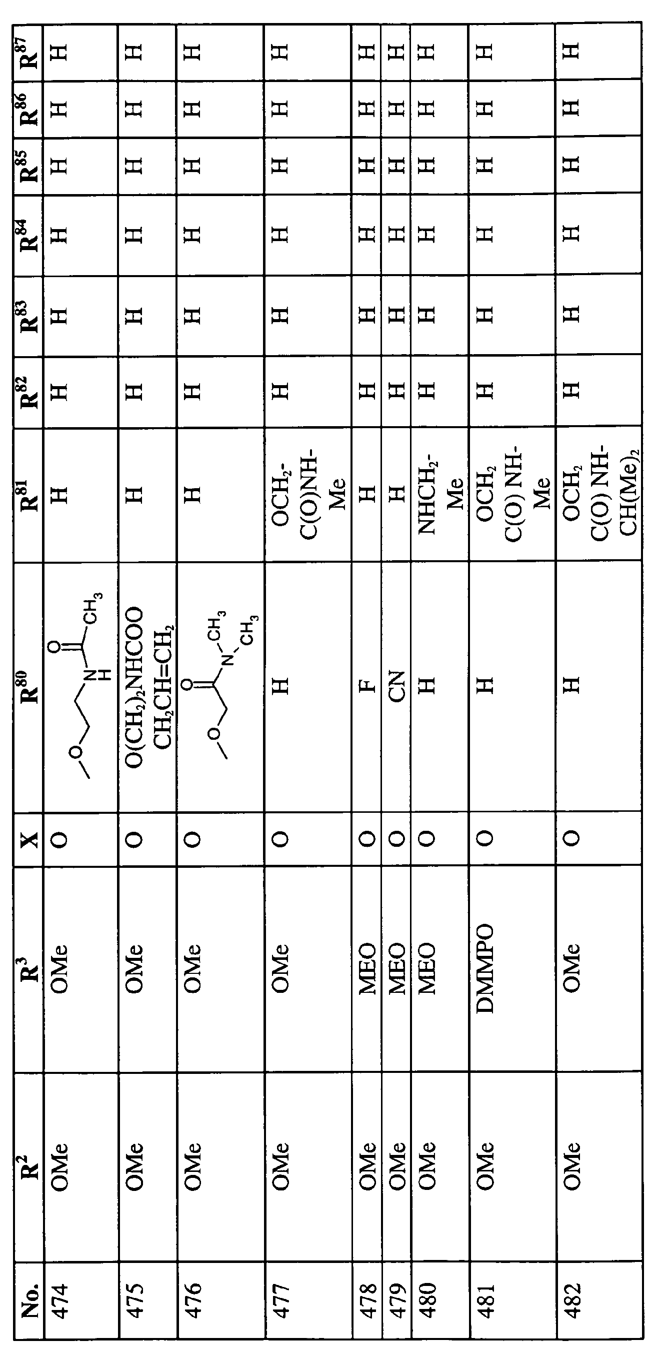

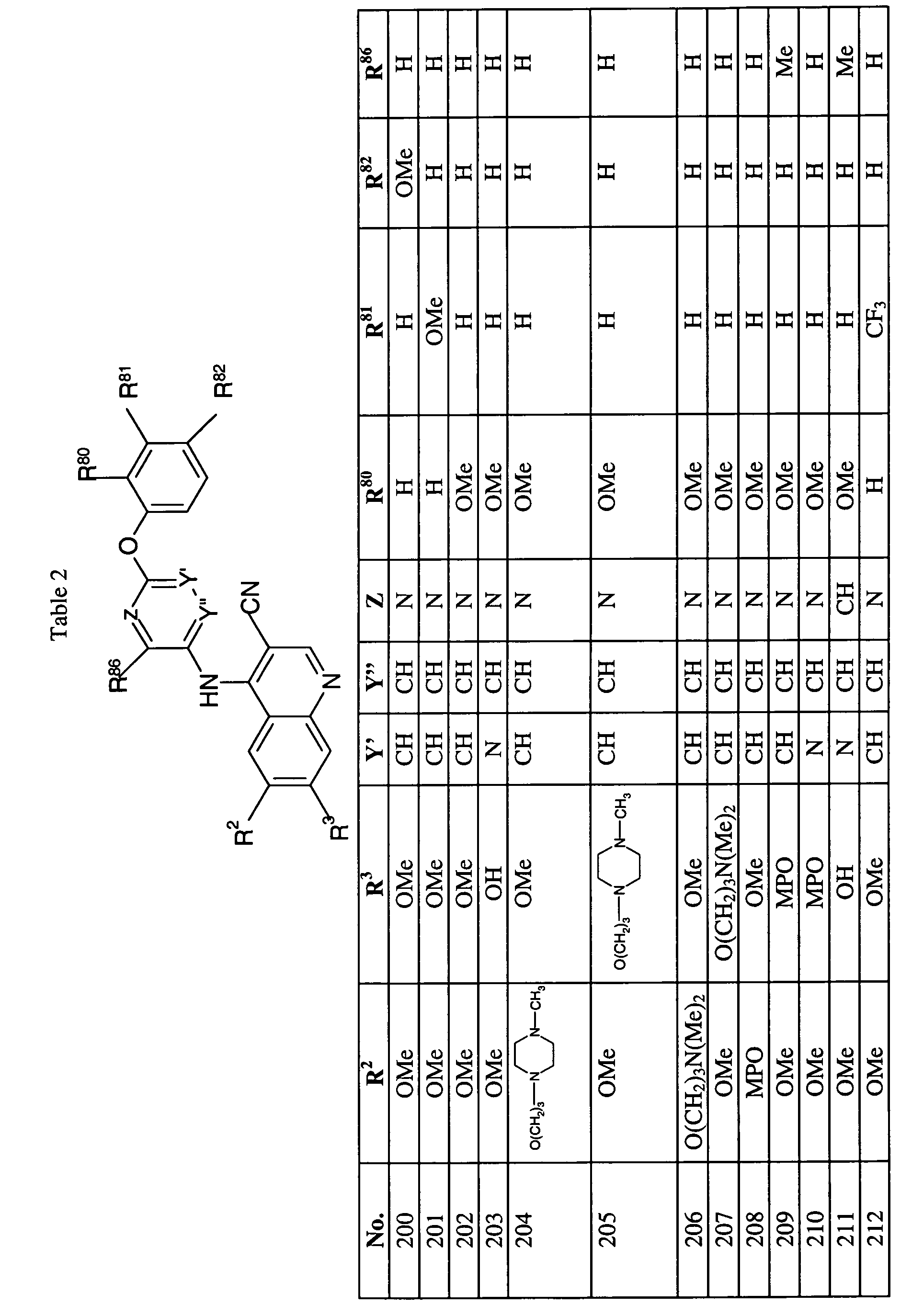

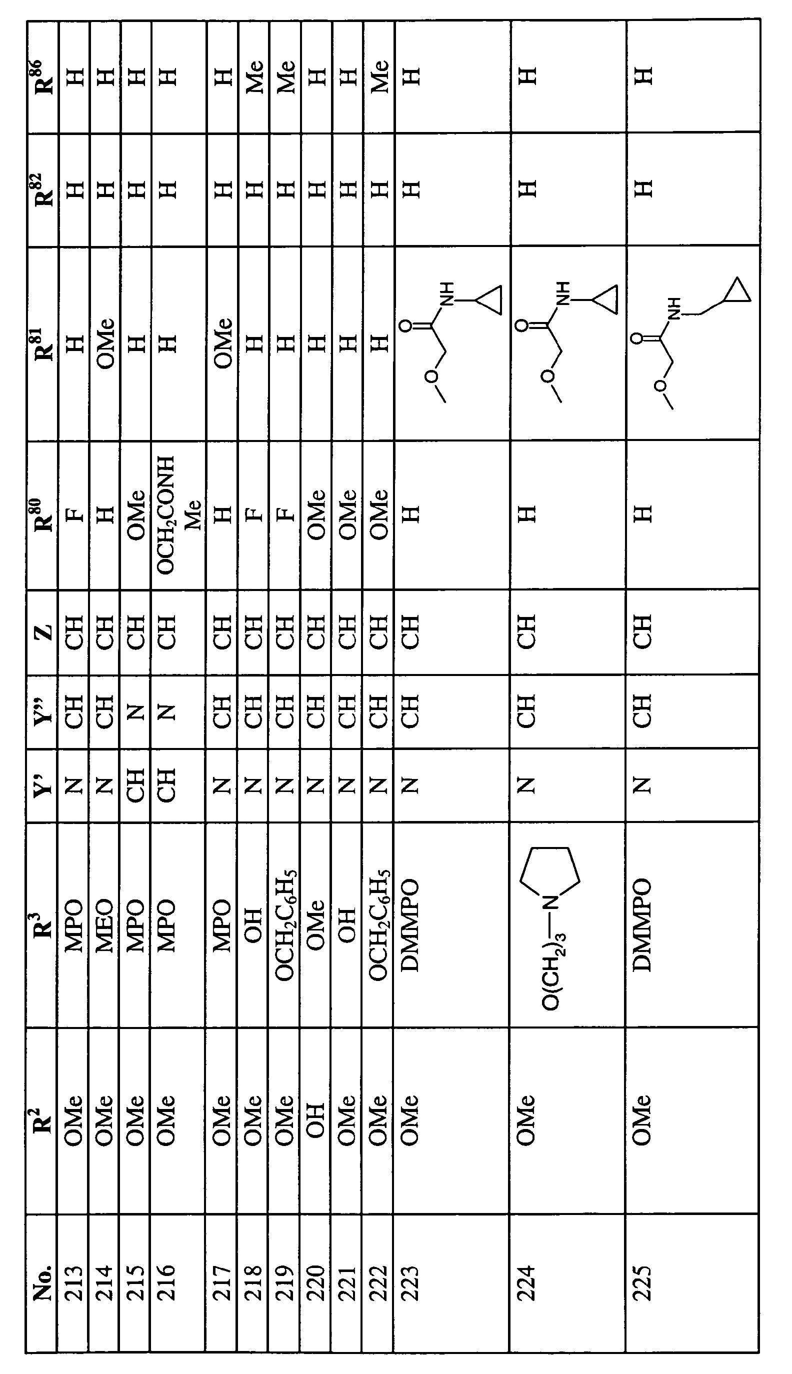

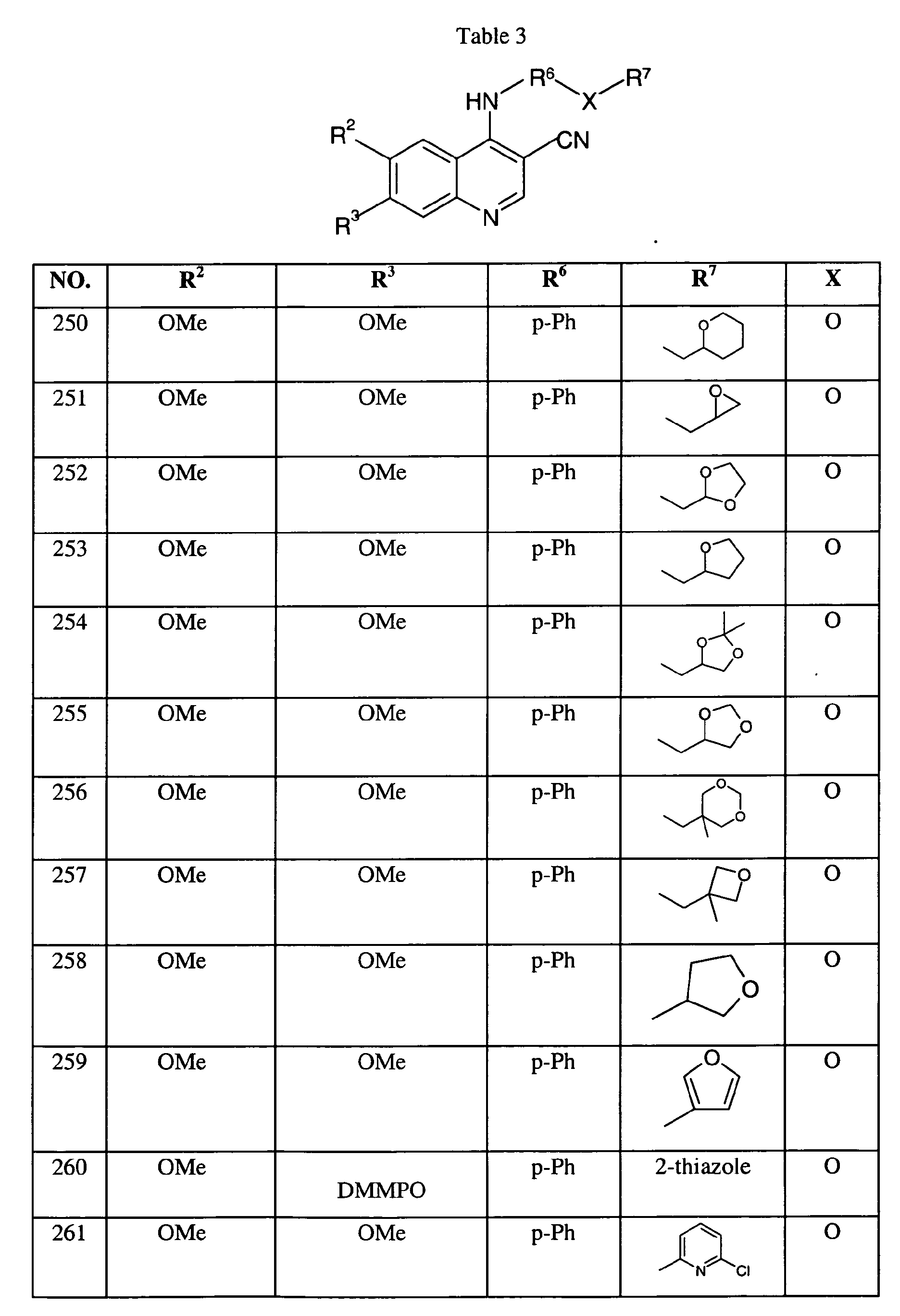

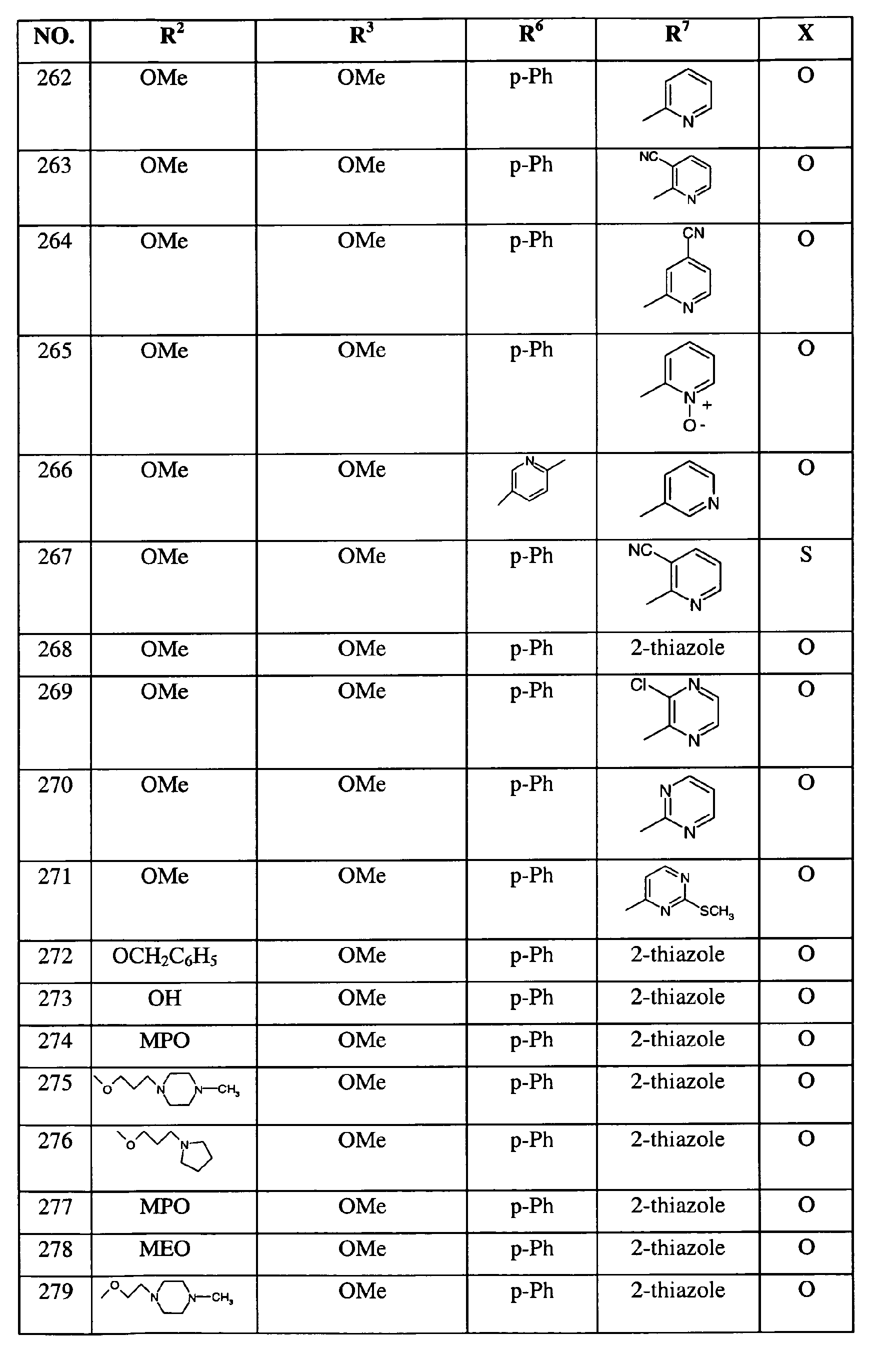

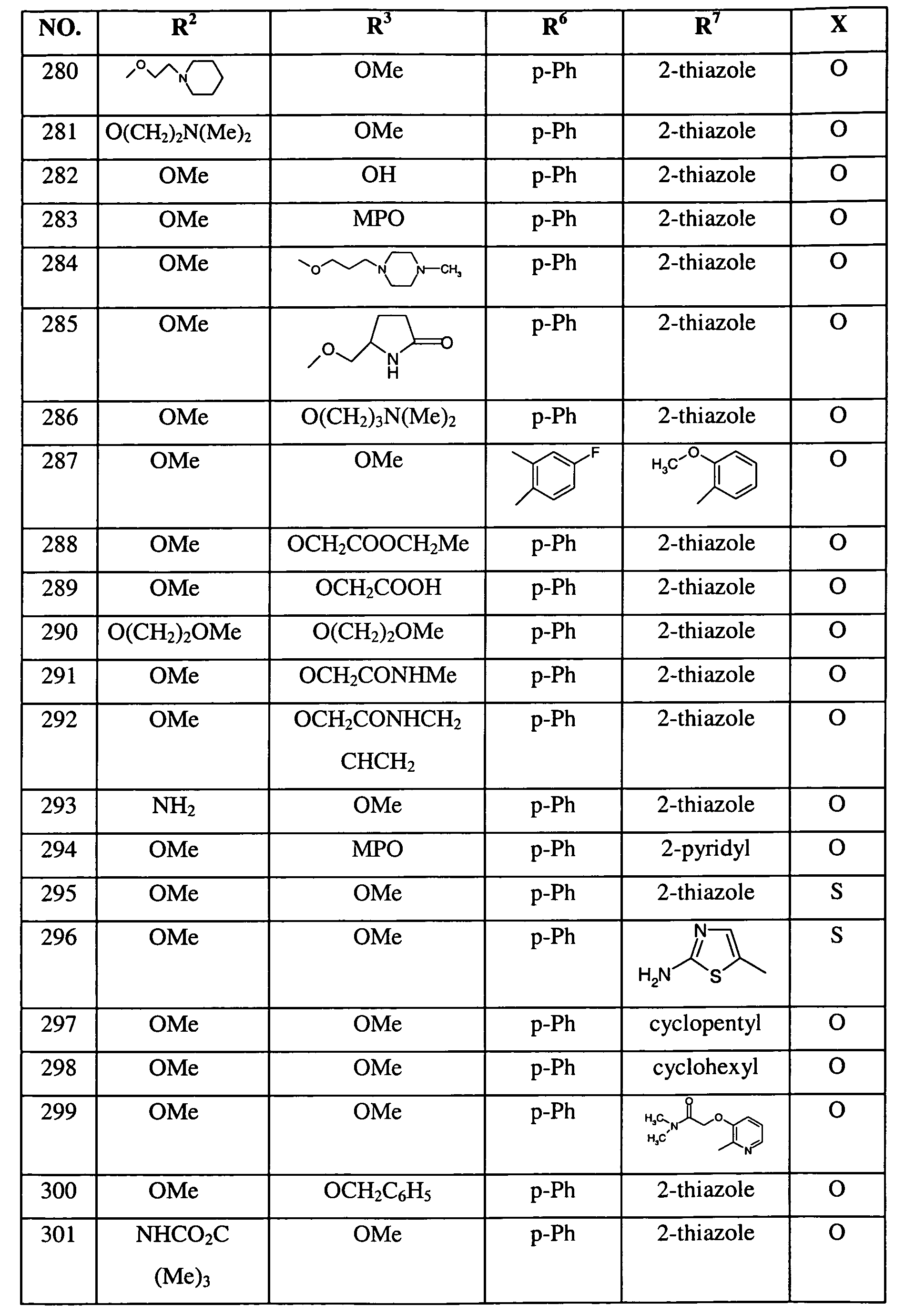

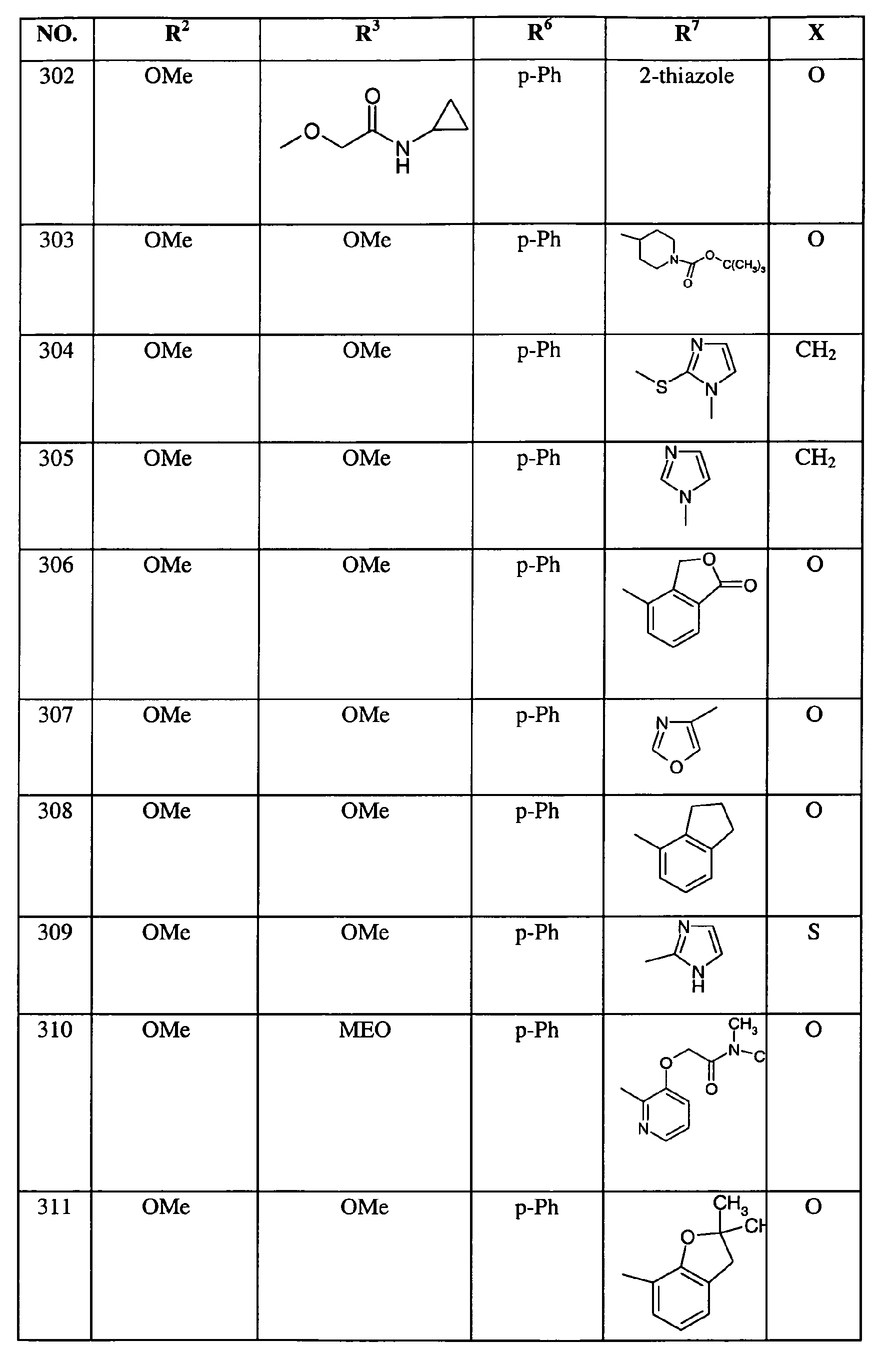

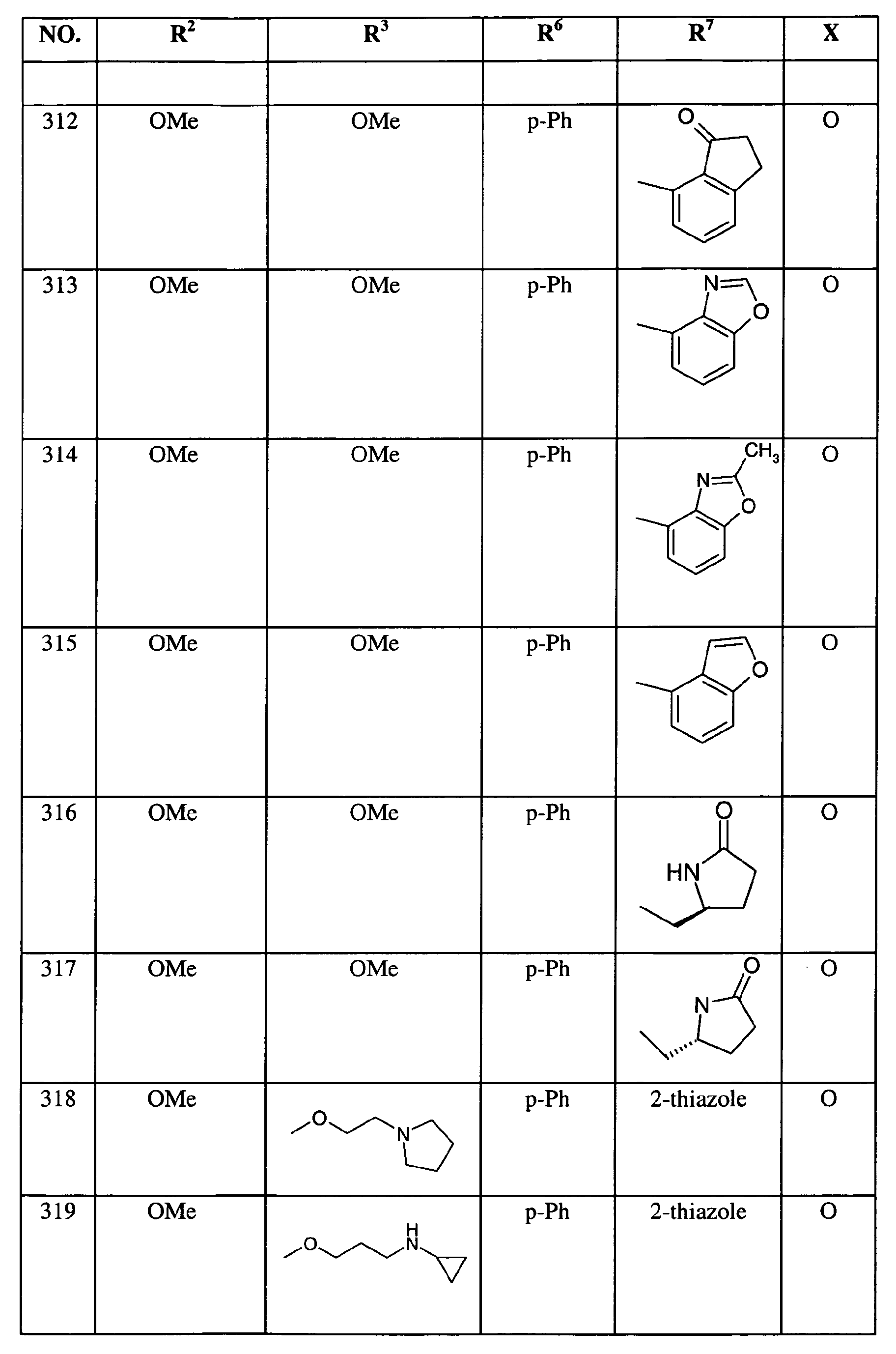

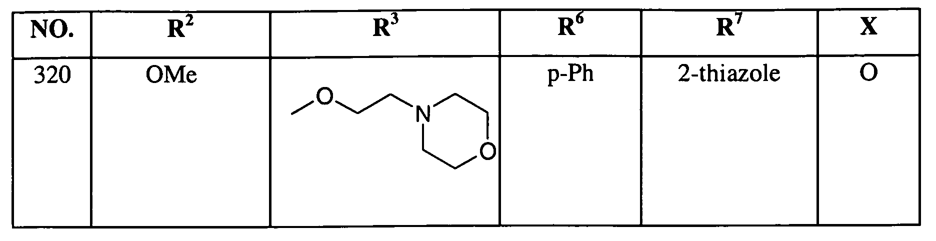

where p-Ph represents a para-phenylene group of formula

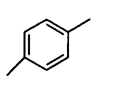

Compounds of formula (I) are suitably prepared by reacting a compound of formula (III) 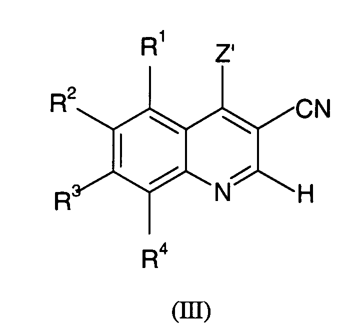 where R^{1'}, R^{2'}, R^{3'}, R^{4'} represent R¹, R², R³ and R⁴ respectively as defined in relation to formula (I) or a precursor thereof, and Z' is a leaving group, with a compound of formula (IV)

H- Y(CH₂)ₙR⁶XR^{7'} (IV)

where R⁶, Y, X, and n are as defined in relation to formula (I), and R^{7'} is a group R⁷ or a precursor thereof; and thereafter if necessary or desired converting precursor groups R^{1'}, R^{2'}, R^{3'}, R^{4'} and R^{7'} to groups of formula R¹, R², R³, R⁴ and R⁷ respectively, or converting a group R¹, R², R³, R⁴ and R⁷ to a different such group.

Suitable leaving groups for Z' include halogen such as bromo or chloro, or a mesylate or tosylate group or a substituted phenoxy group.

The reaction is suitably carried out in an organic solvent such as an alcohol for example propanol or cyclohexanol at elevated temperatures, for example of from 50 to 150°C, for example at about 105°C.

Conversion reactions in which precursor groups R^{1'}, R^{2'}, R^{3'}, R^{4'} are converted to groups of formula R¹, R², R³ and R⁴ respectively, or groups R¹, R², R³ and R⁴ are converted to different such group can be carried out using conventional chemistry as outlined hereinafter. Particular precursor groups R^{1'}, R^{2'}, R^{3'}, R^{4'} are groups of formula R¹³'-X¹-(CH₂)ₓ wherein x and X¹ are as defined hereinafter, and R^{13'} is C₁₋₅alkyl which is substituted with halo other than fluoro, and in particular chloro or bromo. The chloro or bromo group may readily be converted into many other groups R¹³ as defined in relation to claim 1. Such compounds are novel and form a further aspect of the invention. They may have activity similar to that of compounds of formula (I) in their own right and therefore may be used in place of a compound of formula (I).

Thus the invention further provides a compound of formula (IB) 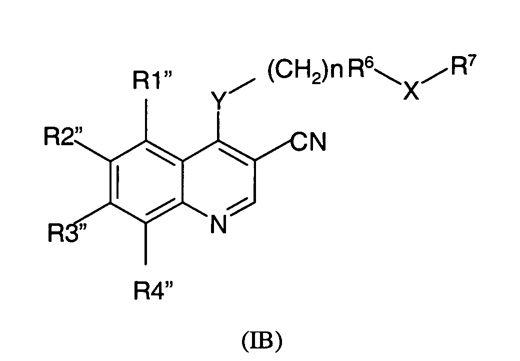 where Y, n, R⁶, X and R⁷ are as defined in claim 1 and at least one of R^{1"}, R^{2"}, R^{3"} or R^{4"} is a group R^{13'}-X¹-(CH₂)ₓ wherein X¹ and x are as defined in claim 1 and R^{13'} is alkyl substituted by chloro or bromo; and the remainder are groups R¹, R², R³ and R⁴ respectively.

Similarly conversion reactions involving groups R⁷ may be effected using conventional chemistry. For example substitutent groups on a group R⁹ within the group R⁷ may be changed, for example by changing acids to esters or amides etc. Alternatively, compounds of formula (I) are prepared by reacting a compound of formula (V) 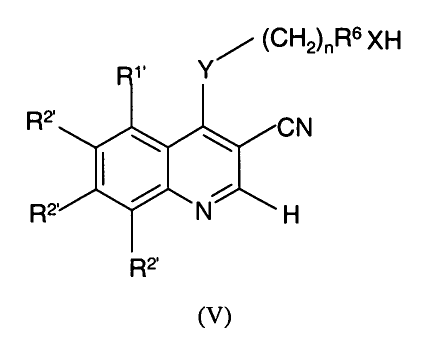 where R^{1'}, R^{2'}, R^{3'}, R^{4'} are as defined in relation to fomula (III) R⁶, X, Y and n are as defined in relation to formula (I), with a compound of formula (VI)

R^{7'}-Z" (VI)

where R⁷ is as defined in relation to formula (IV) and Z" is a leaving group; and thereafter if necessary or desired converting precursor groups R^{1'}, R^{2'}, R^{3'}, R^{4'} and R^{7'} to groups of formula R¹, R², R³, R⁴ and R⁷ respectively, or converting a group R¹, R², R³, R⁴ and R⁷ to a different such group. Suitable leaving groups for Z" include halogen such a bromo or chloro, or a mesylate or tosylate group. Conversion reactions are as described above.

The reaction is suitably carried out in an organic solvent such as DMF at elevated temperatures, for example of from 40 to 120°C, for example at about 80°C.

Compounds of formula (III) and (V) are either known compounds or they can be prepared from known compounds by conventional methods, for example as described in WO 98/43960, WO 98/13350. Exemplary preparations of compounds of formula (III) are included hereinafter.

Compounds of formula (IV) are also known compounds (see for example Rev. Chim. (Bucharest) (1988), 39(6), 477-82 and DD 110651: 74.01.05) or they can be prepared from known compounds using conventional methods. For example, where Y is NH, compounds of formula (IV) are suitably prepared by reduction of a compound of formula (VII)

O₂N(CH₂)ₙR⁶XR^{7'} (VII)

where X, R⁶, R^{7'} and n are as defined above. It may be convenient to convert precursor groups R^{7'} to groups R⁷ or groups R⁷ to other such groups at the level of compound of formula (VII) or (IV) using conventional chemistry.

Compounds of formula (VI) are also known compounds or they can be prepared from known compounds by conventional methods.

Compounds of the invention are useful in the inhibition of MEK enzyme activity and can be used in the treatment of proliferative disease. They will suitably be in the form of a pharmaceutical composition, in combination with a pharmaceutically acceptable carrier. Such compositions form a further aspect of the invention.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

Suitable pharmaceutically acceptable excipients for a tablet formulation include, for example, inert diluents such as lactose, sodium carbonate, calcium phosphate or calcium carbonate, granulating and disintegrating agents such as corn starch or algenic acid; binding agents such as starch; lubricating agents such as magnesium stearate, stearic acid or talc; preservative agents such as ethyl or propyl p-hydroxybenzoate, and anti-oxidants, such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin, or olive oil.

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxyethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives (such as ethyl or propyl p-hydroxybenzoate, anti-oxidants (such as ascorbic acid), colouring agents, flavouring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffin or cetyl alcohol. Sweetening agents such as those set out above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavouring and colouring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurnng gums such as gum acacia or gum tragacanth, naturally-occurring phosphatides such as soya bean, lecithin, and esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in 1,3-butanediol.

Suppository formulations may be prepared by mixing the active ingredient with a suitable non-irritating excipient which is solid at ordinary temperatures but liquid at the rectal temperature and will therefore melt in the rectum to release the drug. Suitable excipients include, for example, cocoa butter and polyethylene glycols.

Topical formulations, such as creams, ointments, gels and aqueous or oily solutions or suspensions, may generally be obtained by formulating an active ingredient with a conventional, topically acceptable, vehicle or diluent using conventional procedure well known in the art.

Compositions for administration by insufflation may be in the form of a finely divided powder containing particles of average diameter of, for example, 30µ or much less, the powder itself comprising either active ingredient alone or diluted with one or more physiologically acceptable carriers such as lactose. The powder for insufflation is then conveniently retained in a capsule containing, for example, 1 to 50mg of active ingredient for use with a turbo-inhaler device, such as is used for insufflation of the known agent sodium cromoglycate.

Compositions for administration by inhalation may be in the form of a conventional pressurised aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

For further information on Formulation the reader is referred to Chapter 25.2 in Volume 5 of Comprehensive Medicinal Chemistry (Corwin Hansch; Chairman of Editorial Board), Pergamon Press 1990.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 2 g of active agent compounded with an appropriate and convenient amount of excipients which may vary from about 5 to about 98 percent by weight of the total composition. Dosage unit forms will generally contain about 1 mg to about 500 mg of an active ingredient. For further information on Routes of Administration and Dosage Regimes the reader is referred to Chapter 25.3 in Volume 5 of Comprehensive Medicinal Chemistry (Corwin Hansch; Chairman of Editorial Board), Pergamon Press 1990.

The size of the dose for therapeutic or prophylactic purposes of a compound of the Formula I will naturally vary according to the nature and severity of the conditions, the age and sex of the animal or patient and the route of administration, according to well known principles of medicine. As mentioned above, compounds of the Formula I are useful in treating diseases or medical conditions which are due alone or in part to the effects MEK enzymes.

In using a compound of the Formula I for therapeutic or prophylactic purposes it will generally be administered so that a daily dose in the range, for example, 0.5 mg to 75 mg per kg body weight is received, given if required in divided doses. In general lower doses will be administered when a parenteral route is employed. Thus, for example, for intravenous administration, a dose in the range, for example, 0.5 mg to 30 mg per kg body weight will generally be used. Similarly, for administration by inhalation, a dose in the range, for example, 0.5 mg to 25 mg per kg body weight will be used. Oral administration is however preferred.

In a further aspect, the invention provides a method of treating proliferative disease by administering a compound of formula (I) as described above, or a pharmaceutical composition as described above.

Yet a further aspect of the invention provides the use of a compound of formula (I) as defined above, in the preparation of a medicament for use in the inhibition of MEK enzyme activitiy and in particular for the treatment of proliferative disease such as cancer.

The invention will now be particularly described by way of Example. The preparation of various intermediates used in the Examples is described in the Preparations.

### Preparation 1

### Chloroquinoline intermediates

These can be prepared for example using the following scheme where "Bz" represents benzyl.

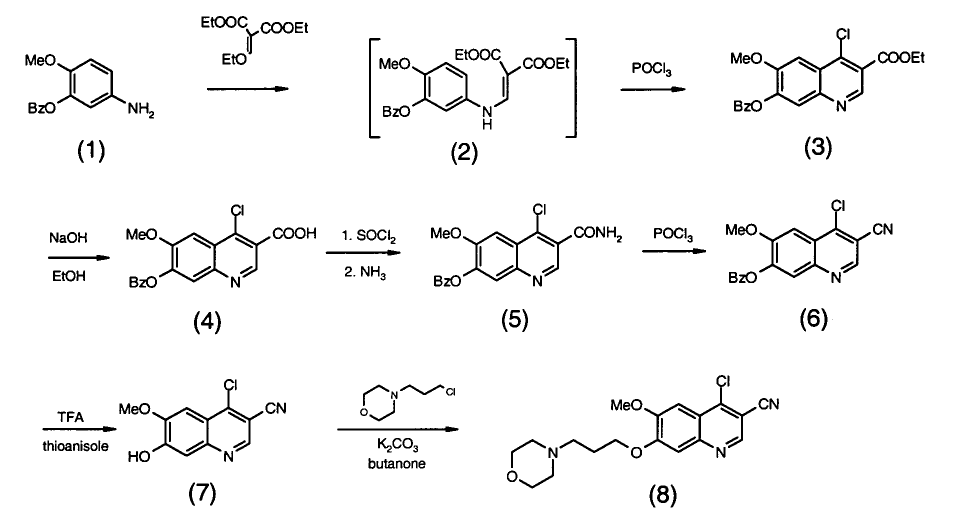

A mixture of **(1)** (10.36g., 45.3 mmole) and diethylethoxymethylene malonate (9mL, 45.3 mmole) was heated at 110°C for 1 hour and then allowed to cool overnight. The mixture was evaporated and the product **(2)** used in the next step without further purification.
Mass Spectrum m/e 400 (M⁺+H).

### Preparation of (3)

A mixture of **(2)** (assumed 45.3 mmole) and phosphoryl chloride (83.3mL, 906 mmole) was heated at 115°C for 18 hours. After cooling, the solution was evaporated to remove excess phosphoryl chloride. The residue was treated with ice and aqueous ammonia to hydrolyse the remaining phosphoryl chloride. The solid product was filtered off and dried in a vacuum oven to give a cream coloured solid, 9.0g (53% yield).
Mass Spectrum m/e 372 (M⁺+H).

### Preparation of (4)

A mixture of **(3)** (9.0g, 24.2 mmole) was stirred in ethanol (48.3mL) for 15 minutes at ambient temperature to give a smooth suspension. Aqueous sodium hydroxide solution (2.0M, 48.3mL, 96.7 mmole) was added and the mixture stirred for 18 hours at ambient temperature. The ethanol was removed by rotary evaporation and the resulting solution was acidified to pH 2 with hydrochloric acid while stirring. The precipitate was filtered off and dried in a vacuum oven to give an orange solid, 7.19g (86% yield).
Mass Spectrum m/e 344 (M⁺+H).

### Preparation of (5)

A mixture of **(4)** (7.18g, 20.9 mmole) and thionyl chloride (90 mL) was refluxed for 2 hours. After cooling the excess thionyl chloride was removed by rotary evaporation and the residue was suspended in acetone (175mL) and the resulting suspension cooled in an ice-bath. Aqueous ammonia (S.G. 0.880, 20mL) was added gradually, keeping the temperature below 10°C. The resulting suspension was filtered off, washed with water and air-dried to give a solid, 5.15g (75% yield).
Mass Spectrum m/e 343 (M⁺+H).

### Preparation of (6)

A mixture of **(5)** (20.55g, 60 mmole) and phosphoryl chloride (250mL) was heated and stirred at 120°C for 4 hours when the starting material had dissolved. Heating and stirring was continued at 110°C for 18 hours. After cooling, the solution was evaporated to remove excess phosphoryl chloride. Last traces of phosphoryl chloride were removed by azeotroping with toluene. The residue was treated with ice and aqueous ammonia to remove acidity. The solid product was filtered off and dried in a vacuum oven to give a grey solid, 19.23g (99% yield).
(This may also be prepared as described in WO 9843960)
Mass Spectrum m/e 325 (M⁺+H).

### Preparation of (7)

A mixture of **(6)** (19.23g, 60.0 mmole) and trifluoroacetic acid (300 mL) and thioanisole (35mL) was refluxed in a nitrogen atmosphere for 3 hours. After cooling the trifluoroacetic acid was removed by rotary evaporation and the oily residue was stirred with ice and water and basified with aqueous ammonia (S.G. 0.880). The resulting suspension was filtered and the solid was washed successively with water, ethyl acetate and diethyl ether and then dried to give a khaki solid, 13.74g (97% yield).
Mass Spectrum m/e 235 (M⁺+H).

### Preparation of (8)

### (4-chloro-6-methoxy-7-[3-(1-morpholino)propoxy]-3-quinolinecarbonitrile)

A mixture of **(7)** (2.34g, 10.0 mmole) and 1-(3-chloropropyl)morpholine (2.45g, 15.0 mmole) and anhydrous potassium carbonate (2.07g, 15.0 mmole) suspended in butanone (150mL) was stirred in a oil-bath at 88°C for 96 hours. The suspension was filtered hot to remove inorganics and the filtrate was allowed to cool and then evaporated to ca. 100mL. A solid precipitated on standing for 72 hours. The solid was filtered off and washed with a little acetone and then dried to give a white solid, 0.54g (15% yield).
Mass Spectrum m/e 362 (M⁺+H).

### Preparation 2

By similar processes the following analogues were also prepared:-

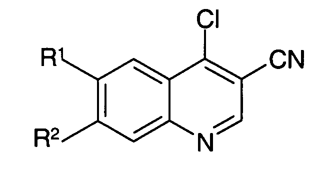

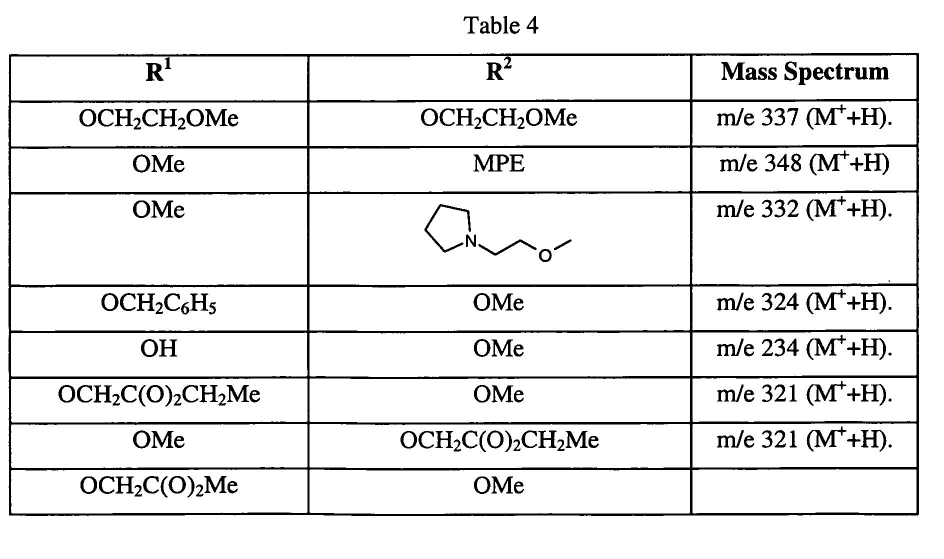

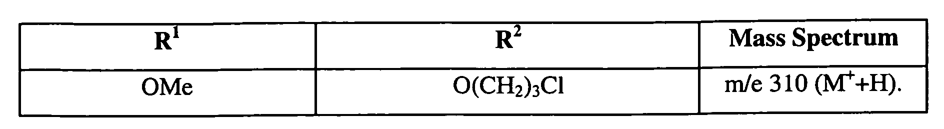

### Example 1

A mixture of 4-chloro-3-cyano-6,7-dimethoxyquinoline (1.5 g), prepared as described in WO 9843960, and 4-(2-methoxyphenoxy)-aniline (2.58 g), prepared as described in Rev. Chim. (Bucharest) (1988), 39(6),477-82, in 1-propanol (90 ml) was stirred and heated at 105°C for 6 hours. The mixture was cooled to ambient temperature and then filtered. The crystals were washed with a small volume of 1-propanol and then dried to give 4-(2-methoxyphenoxy)-anilino-3-cyano-6,7-dimethoxyquinoline (Compound 1 in Table 1) (2.19 g, 85%).
Mass Spectrum m/e 428 (M⁺+H).
NMR Spectrum (d-6-DMSO, d values) 3.75 (s, 3H), 4.00 (s, 6H), 6.95 (m, 3H), 7.05 (m, 1H), 7.20 (m, 2H), 7.40 (d, 2H), 7.50 (s, 1H), 8.20 (s, 1H), 8.85 (s, 1H), 11.10 (broad, 1H).

### Example 2

### Preparation of Compound 253 in Table 3

### Step 1

A mixture of 4-chloro-3-cyano-6,7-dimethoxy-quinoline (2.49 g) and 4-aminophenol (2.4 g) in n-propanol (150 ml) was stirred and heated at 110°C for 4 hours. The mixture was cooled to ambient temperature and then filtered. The crystals were washed with a small volume of diethyl ether and then dried to give 3-cyano-6,7-dimethoxy-4-(4-hydroxy)-anilino-quinoline (2.68 g, 83%).
Mass Spectrum m/e 322 (M⁺+H).
NMR Spectrum (d-6-DMSO, d values) 3.85 (s, 3H), 3.9 (s, 3H), 6.8 (d, 2H), 7.1 (d, 2H), 7.25 (s, 1H), 7.8 (s, 1H), 8.3 (s, 1H), 9.3 (broad s, 1H).

### Step 2

3-Cyano-6,7-dimethoxy-4-(4-hydroxy)-anilino-quinoline (160.5 mg) was dissolved in DMF (5 ml) and potassium carbonate (138 mg) was added. The mixture was stirred under an atmosphere of nitrogen for 5 minutes and then 2-bromomethyl-tetrahydrofuran (180 ml) was added. The mixture was stirred and heated at 80°C for 18 hours. The mixture was cooled to ambient temperature and then diluted with ethyl acetate and then extracted with water. The aqueous phase was re-extracted with ethyl acetate and the combined organic extracts were washed with brine, dried (Na₂SO₄) and evaporated. The residue was then purified by column chromatography using 2-3% methanol/dichloromethane mixtures as eluent. There was thus obtained 3-cyano-6,7-dimethoxy-4-(2-tetrahyrofuranyl-methoxy)-anilino-quinoline (70 mg, 34%).
Mass Spectrum m/e 406 (M⁺+H).
NMR Spectrum (CDCl₃, d values) 1.8 (m, 1H), 1.95 (m, 2H), 2.05 (m, 1H), 3.6 (s, 3H), 3.85 (dd, 1H), 3.9 (m, 1H), 3.95 (m, 1H), 4.0 (s, 3H), 4.25 (m, 1H), 6.8 (broad s, 1H), 6.85 (s, 1H), 6.95 (d, 2H), 7.1 (d, 2H), 7.35 (s, 1H), 8.6 (s, 1H).

### Example 3

By an analogous procedure to that described for Example 2, step 2, but using an alternative bromide, the compounds listed in Table 5 were prepared:

**Table 5**

| **No** | **bromide** | **mass spec** | **nmr** | **Notes** |
|---|---|---|---|---|
| 250 | 2-bromomethyltetrahydropyran | m/e 420 (M⁺+H) | (d-6-DMSO, d values) 1.2-1.7 (m, 6H), 3.40 (m, 1H), 3.60 (m, 1H), 3.90 (s, 3H), 3.90 (s, 3H), 3.9 (m, 3H), 6.95 (d, 2H), 7.20 (d, 2H), 7.25 (d, 1H), 7.75 (d, 1H), 8.30 (d, 1H), 9.35 (broad s, 1H). | |
| 251 | epibromohydr in | m/e 378 (M⁺+H) | (d-6-DMSO, d values) 2.70 (dd, 1H), 2.83 (dd, 1H), 3.35 (m, 1H), 3.85 (dd, 1H), 3.90 (s, 3H), 3.95 (s, 3H), 4.35 (dd, 1H), 7.00 (d, 2H), 7.20 (d, 2H), 7.26 (s, 1H), 7.75 (s, 1H), 8.30 (s, 1H), 9.35 (broad s, 1H). | RT/ 48hrs/ DMF/ K₂CO₃ |
| 252 | 2-bromomethyl-1,3-dioxolane | m/e 408 (M⁺+H) | (CDCl₃, d values) 3.60 (s, 3H), 3.95 (m, 3H), 4.00 (s, 3H), 4.05 (m, 3H), 5.30 (t, 1H), 6.80 (broad s, 1H), 6.85 (s, 1H), - 6.95 (d, 2H), 7.15 (d, 2H), 7.35 (s, 1H), 8.60 (s, 1H). | |

### Example 4

By an analogous procedure to that described for Example 2, step 2, but using a tosylate instead of a bromide, the following compounds were prepared.

**Table 6**

| No | intermediate | mass | nmr |
|---|---|---|---|
| 254 | 2,2-dimethyl-4-(4-toluenesulphonylo xymethyl)-1,3-dioxolane | m/e 436 (M⁺+H) . | (CDCl₃, d values) 1.4 (s, 3H), 1.45 (s, 3H), 3.65 (s, 3H), 3.90 (dd, 1H), 3.95 (m, 1H), 4.00 (s, 3H), 4.05 (m, 1H), 4.15 (dd, 1H), 4.50 (m, 1H), 6.80 (broad s, 1H), 6.90 (s, 1H), 6.95 (d, 2H), 7.10 (d, 2H), 7.35 (s, 1H), 8.60 (s, 1H). |
| 255 | 4-(4-toluenesulphonylo xymethyl)-1,3-dioxolane | m/e 408 (M⁺+H) | (CDCl₃, d values) 3.60 (s, 3H), 3.85 (m, 1H), 3.95 (m, 1H), 4.00 (s, 3H), 4.05 (m, 2H), 4.40 (m, 1H), 4.95 (s, 1H), 5.10 (s, 1H), 6.80 (broad s, 1H), 6.85 (s, 1H), 6.95 (d, 2H), 7.10 (d, 2H), 7.35 (s, 1H), 8.60 (s, 1H). |
| 256 | 5-bromo-5-(4-toluenesulphonylo xymethyl)-1,3-dioxane | m/e 436 (M⁺+H) | (CDCl₃, d values) 0.95 (s, 3H), 3.50 (d, 2H), 3.65 (s, 3H), 4.00 (d, 2H), 4.00 (s, 3H), 4.10 (s, 1H), 4.70 (d, 1H), 5.00 (d, 1H), 6.80 (broad s, 1H), 6.85 (s, 1H), 6.95 (d, 2H), 7.15 (d, 2H), 7.35 (s, 1H), 8.60 (s, 1H). |

### Example 5

Using a method analogous to that described in Example 1 (except that in some instances, intermediates (1) and (2) were modified prior to further reaction as described in Examples 14 and 15 hereinafter) i.e. as set out in the following scheme: 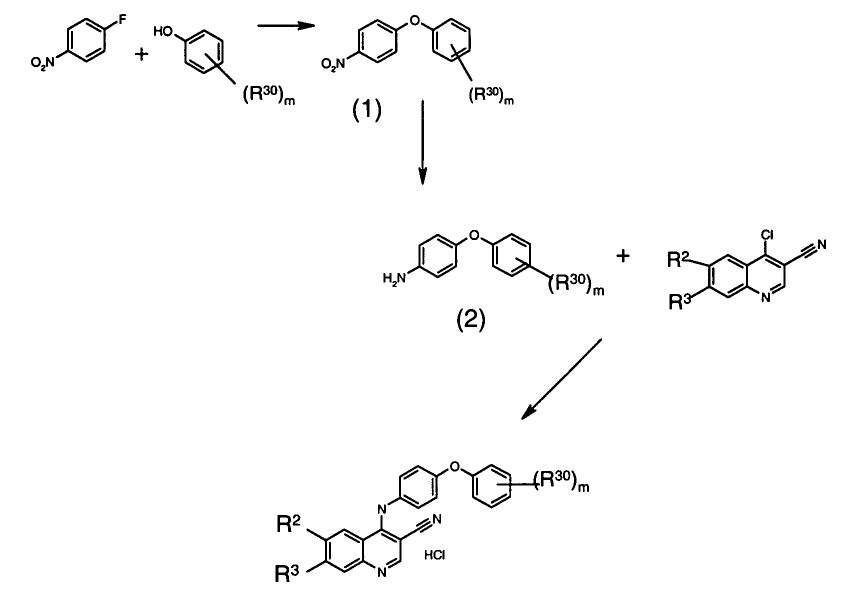 but with the appropriate aniline intermediate (2) (where (R³⁰)ₘ are substitutents R²⁰, R²¹, R²², R²³ and R²⁴ are as set out in Table 1) and quinoline where R² and R³ are as defined in Table 1, the following compounds set out in Table 7 were prepared.

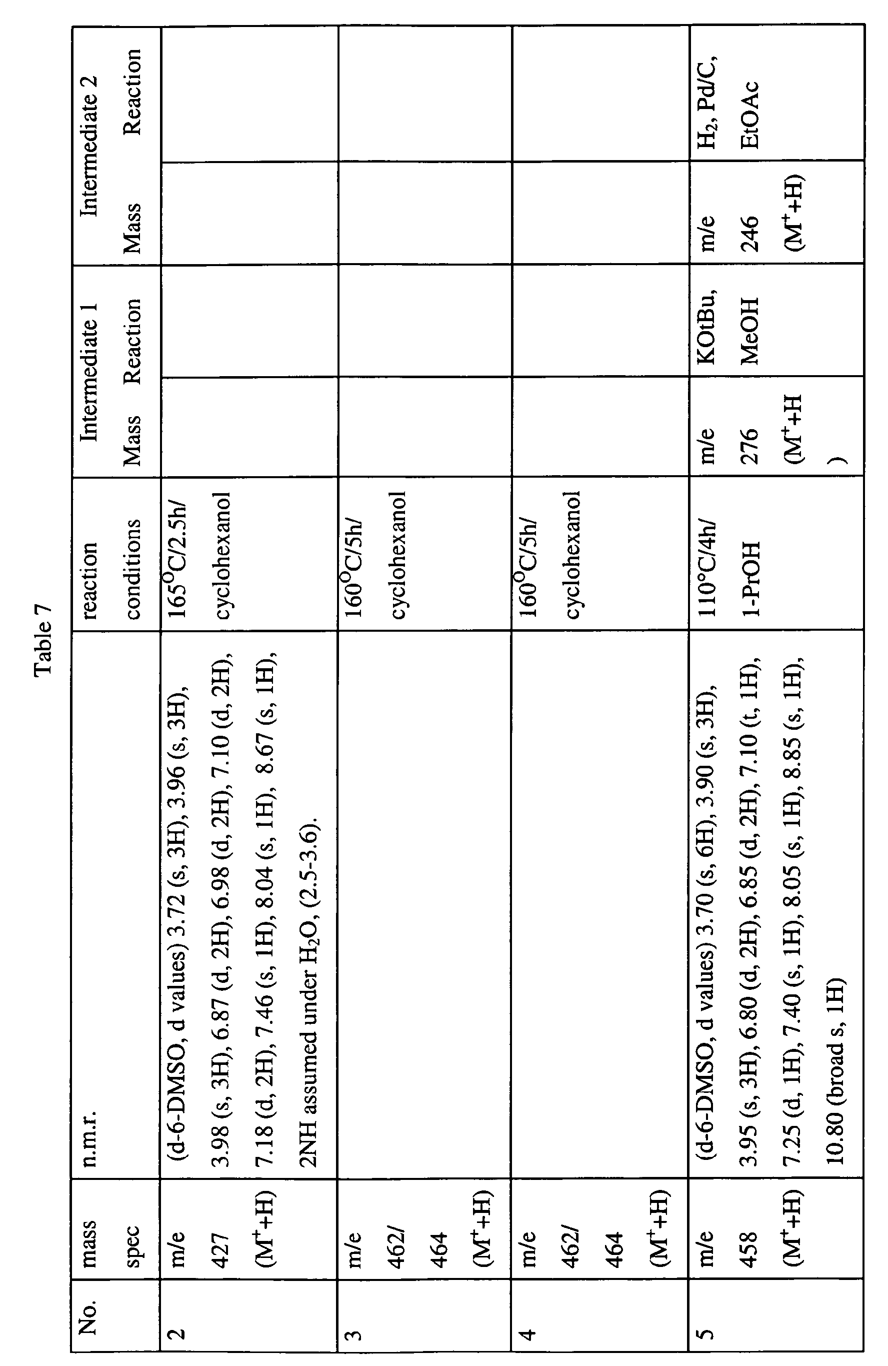

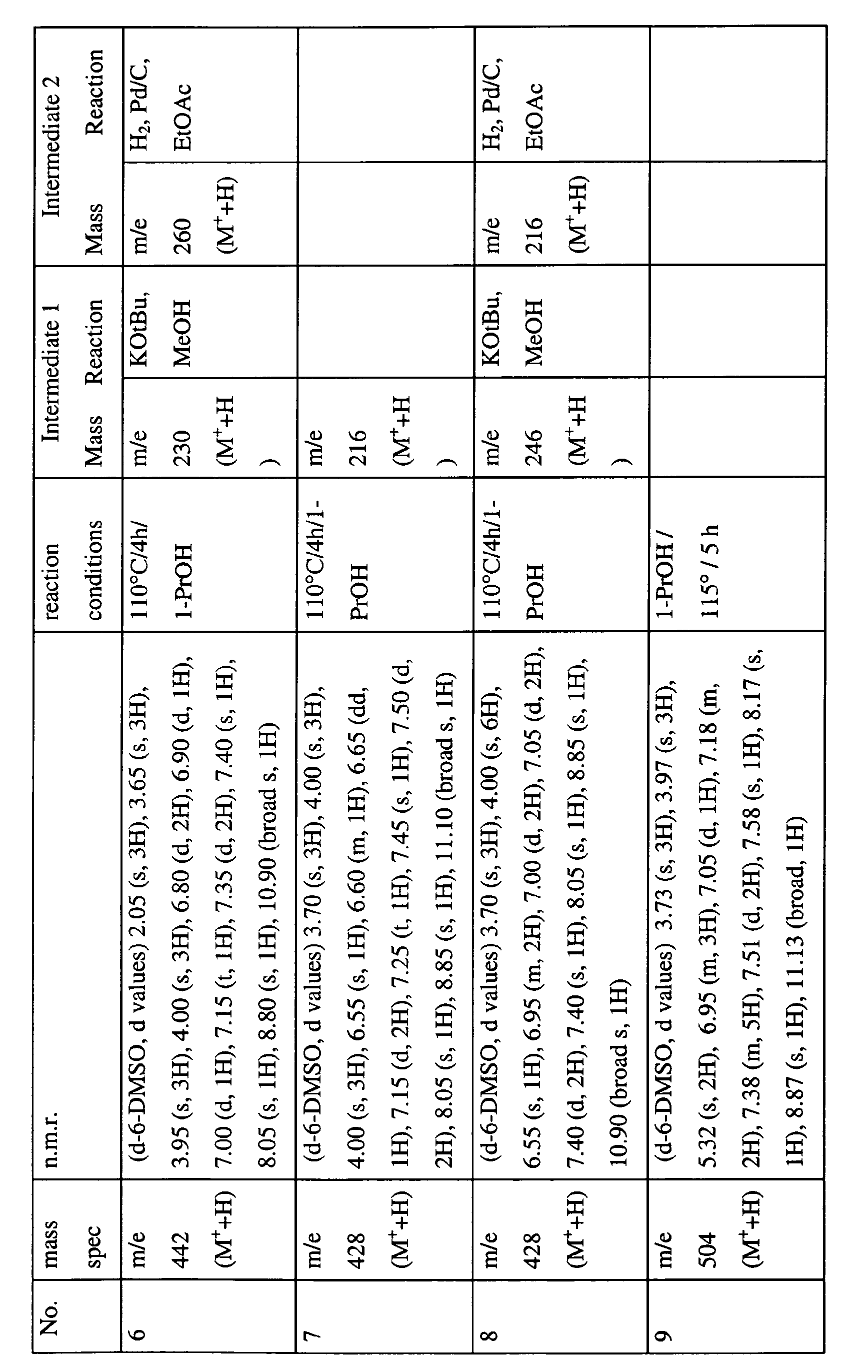

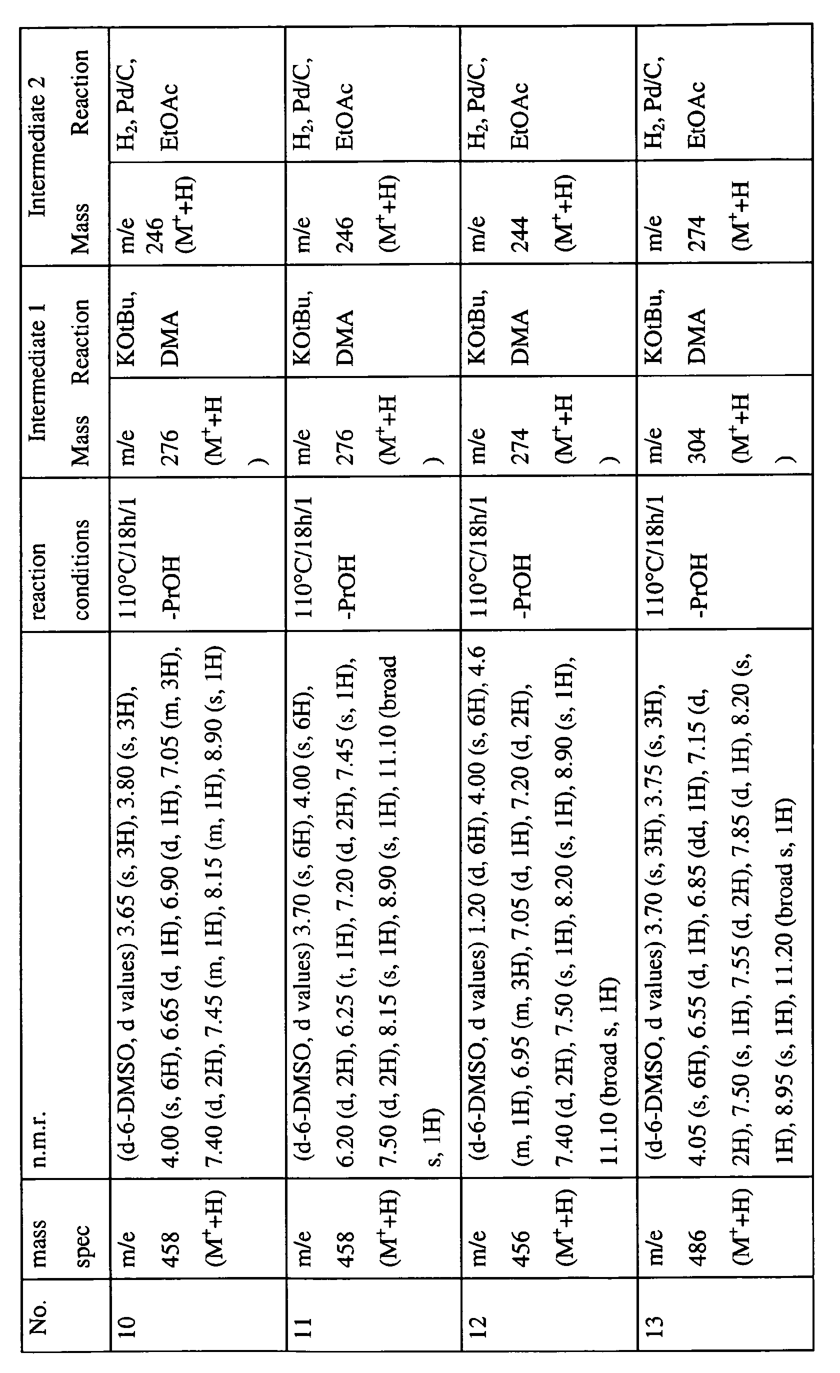

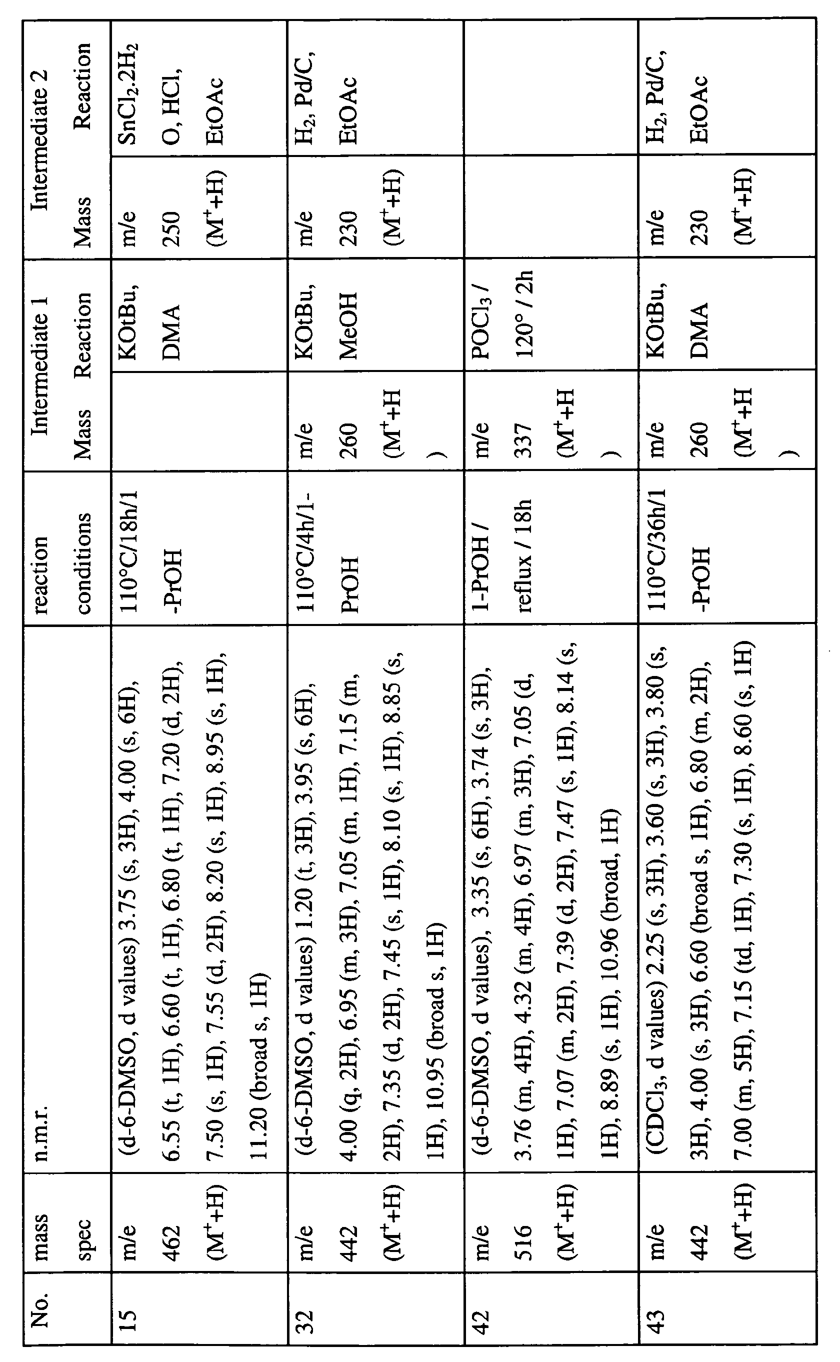

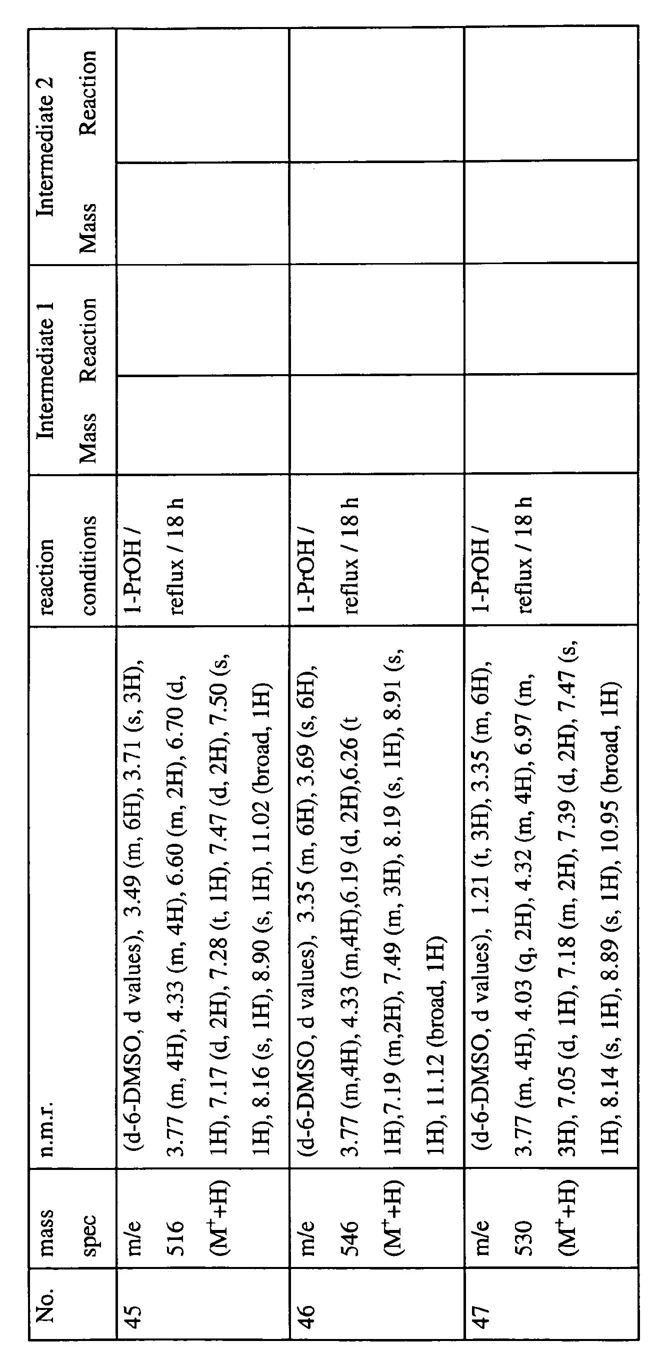

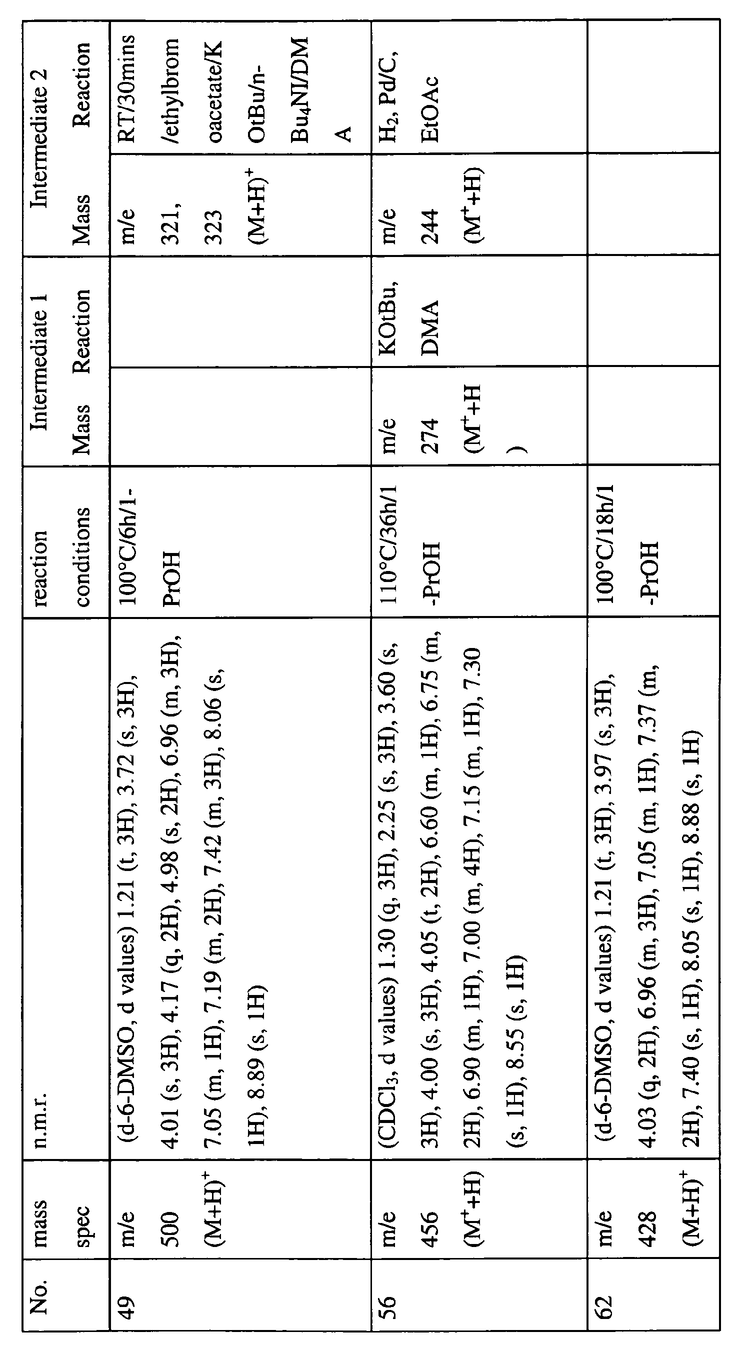

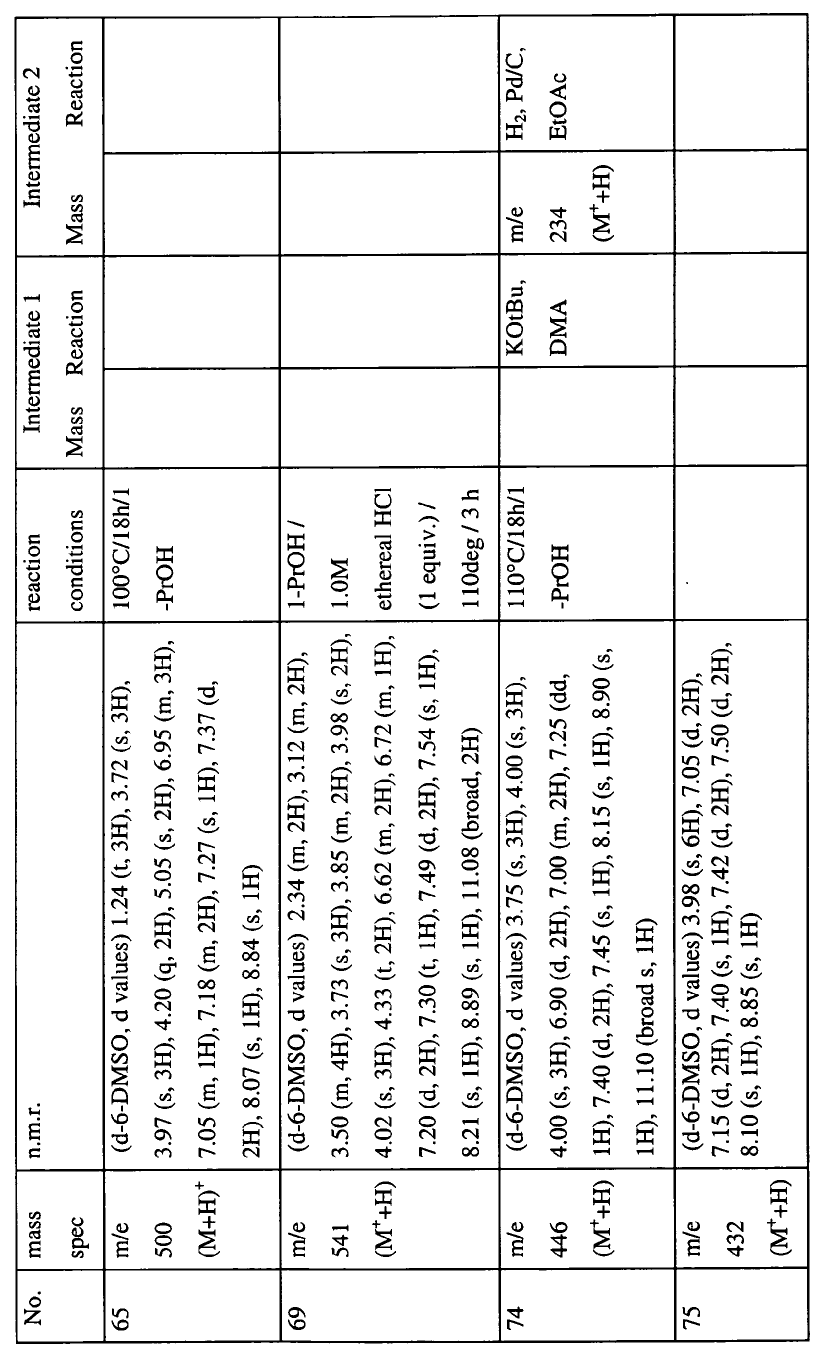

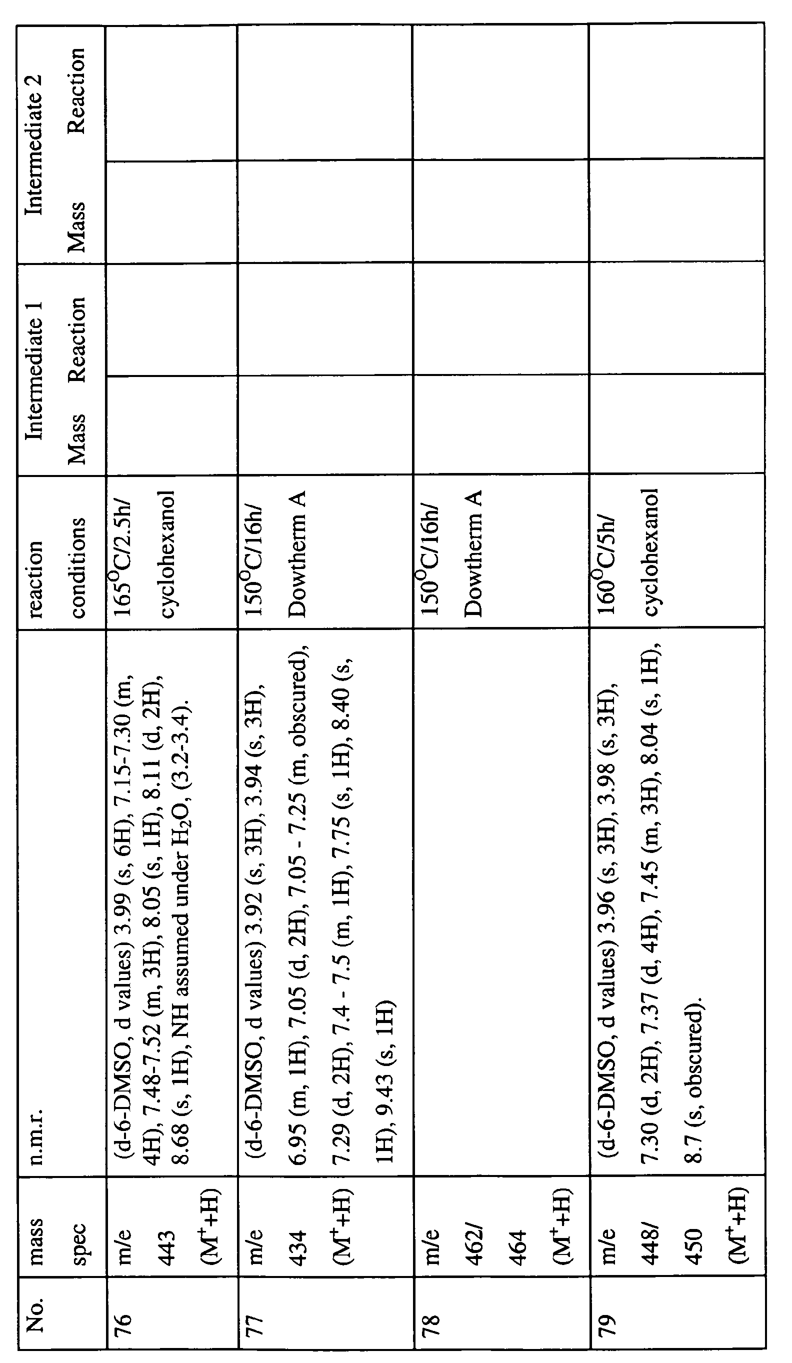

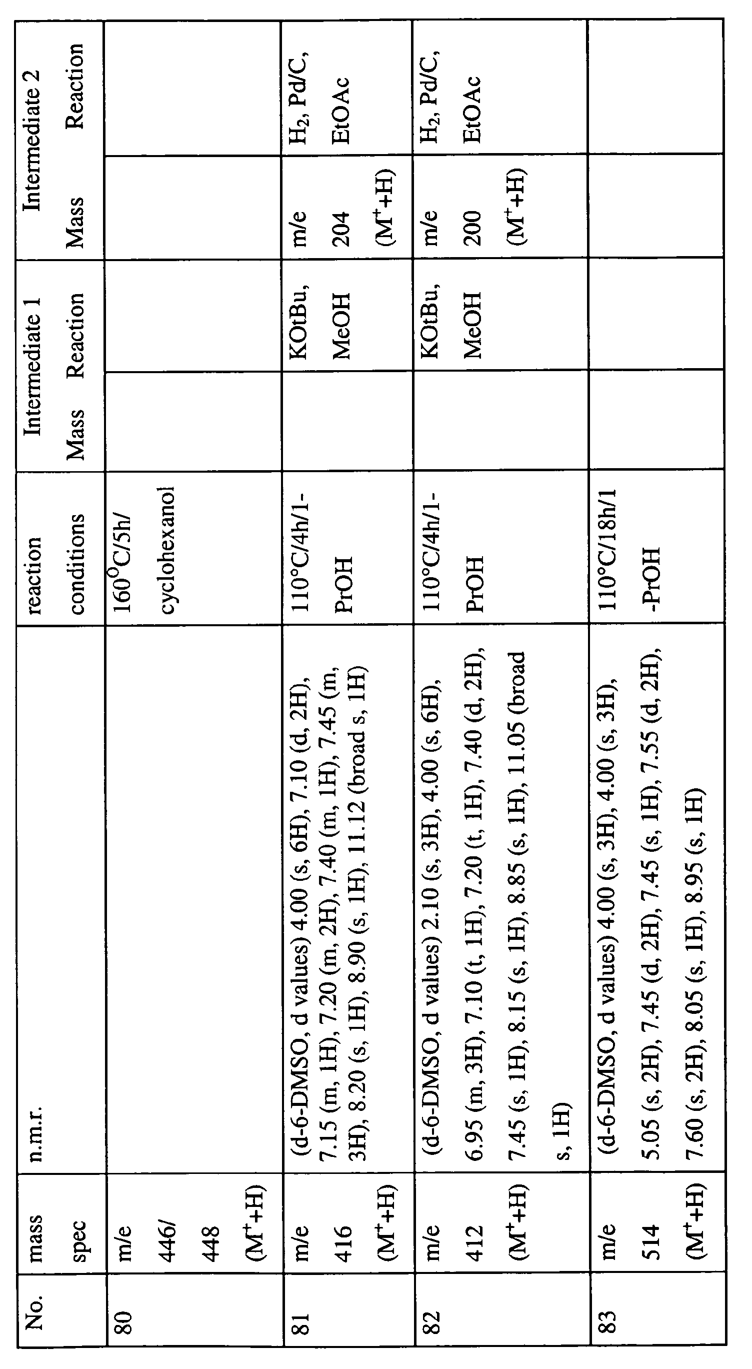

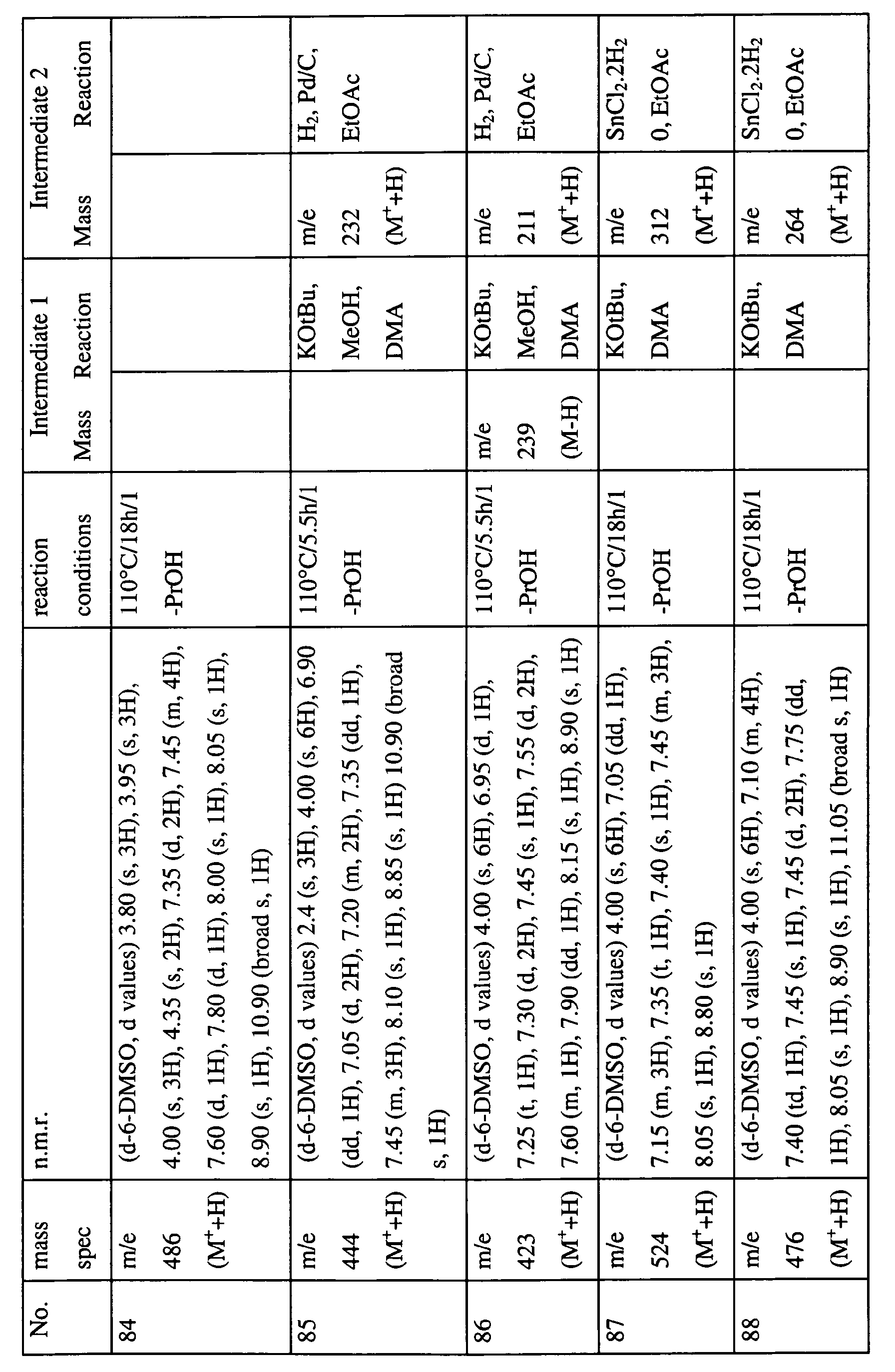

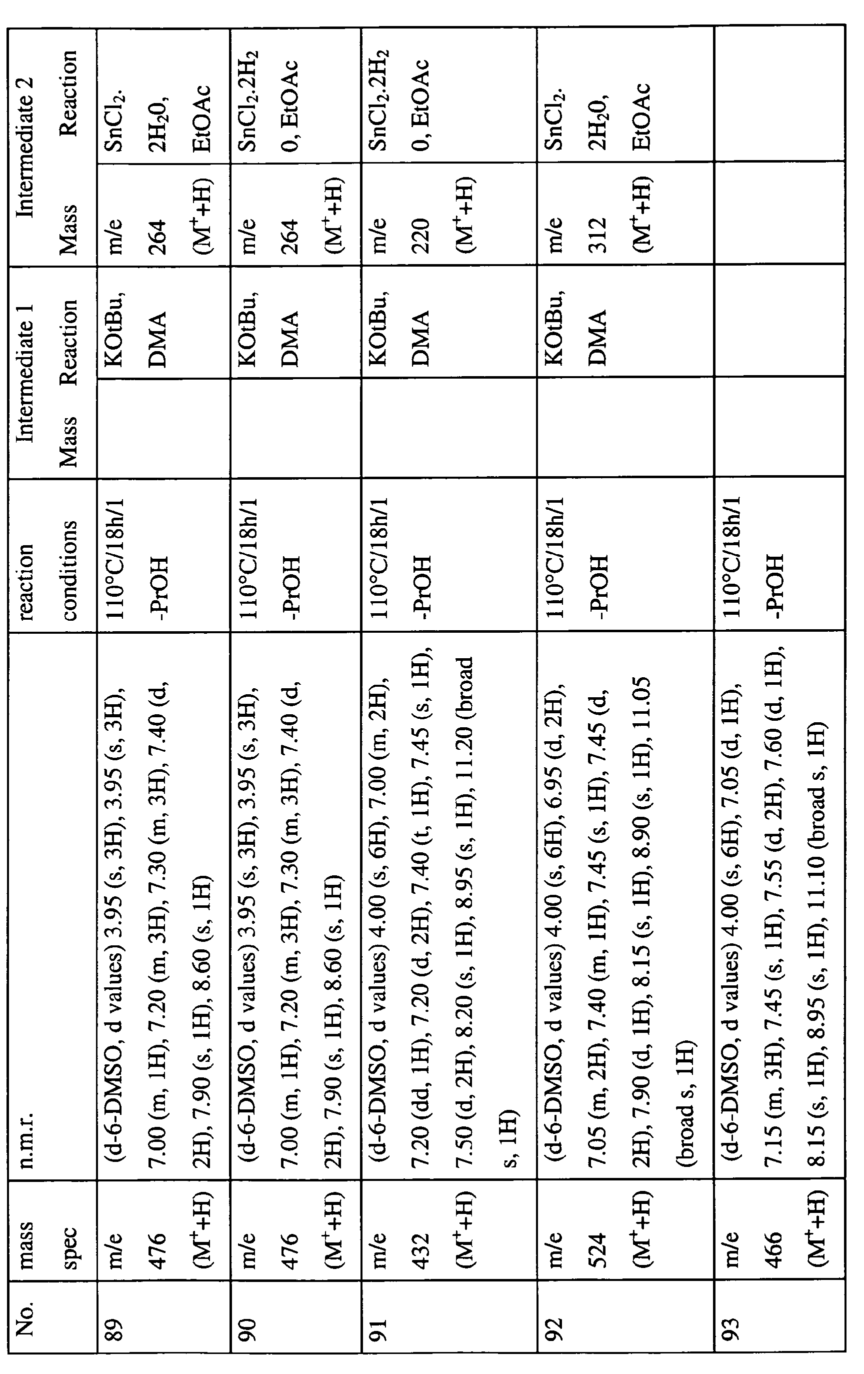

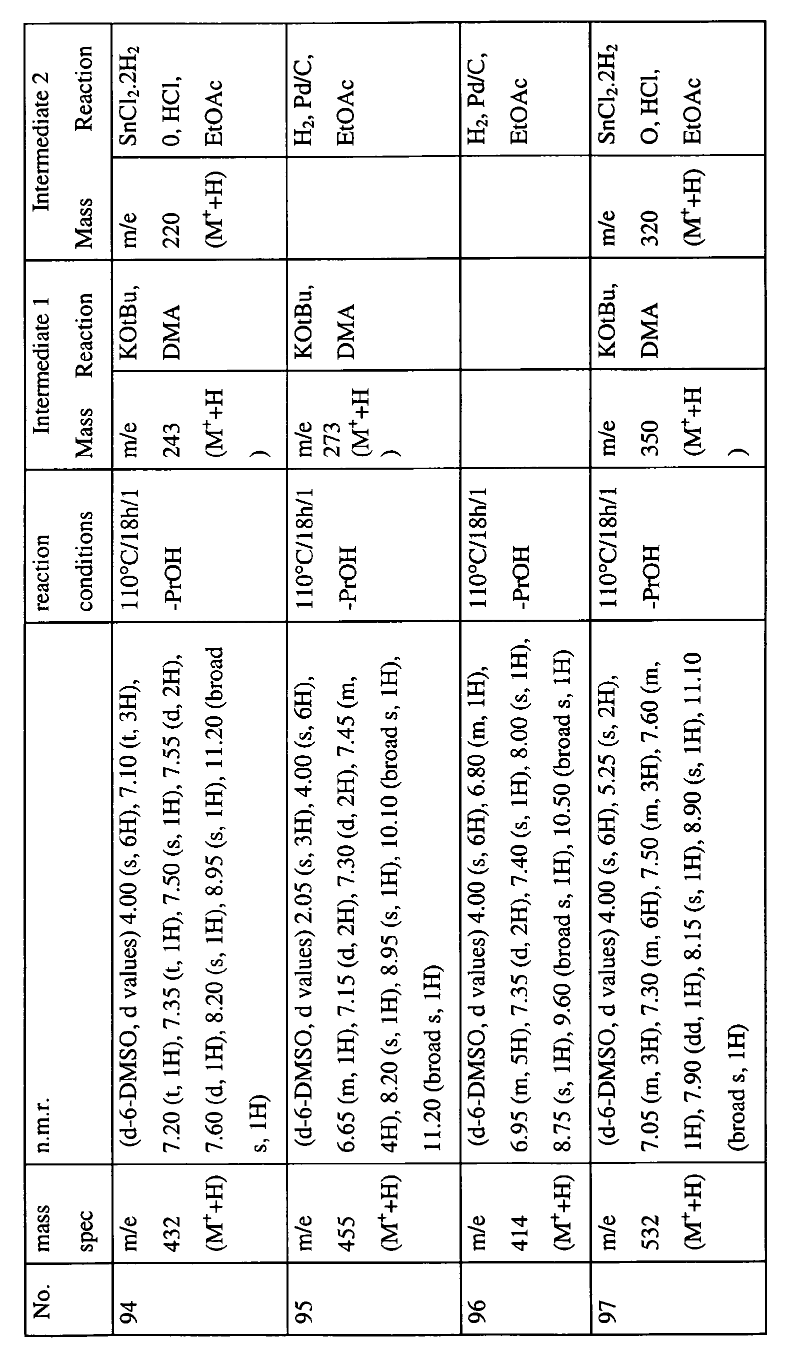

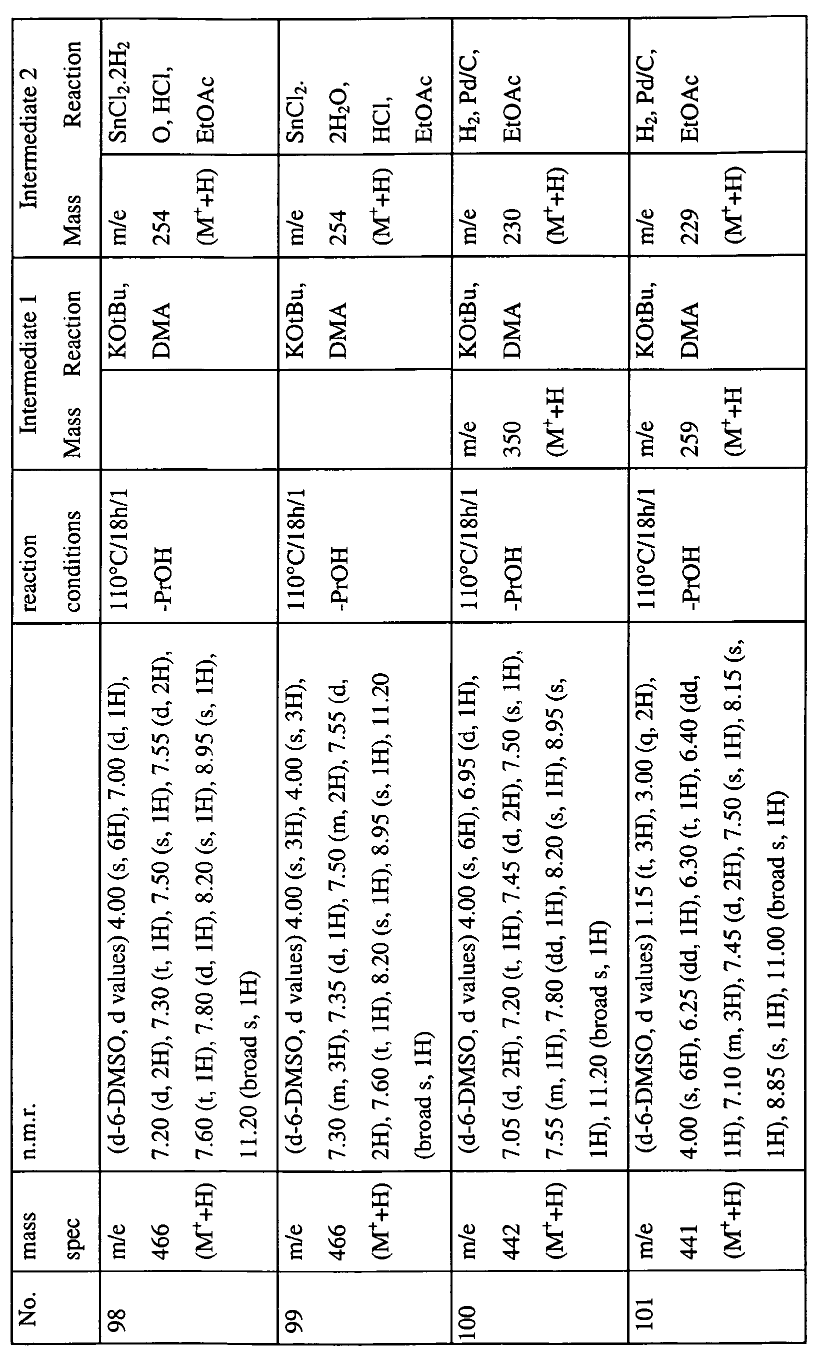

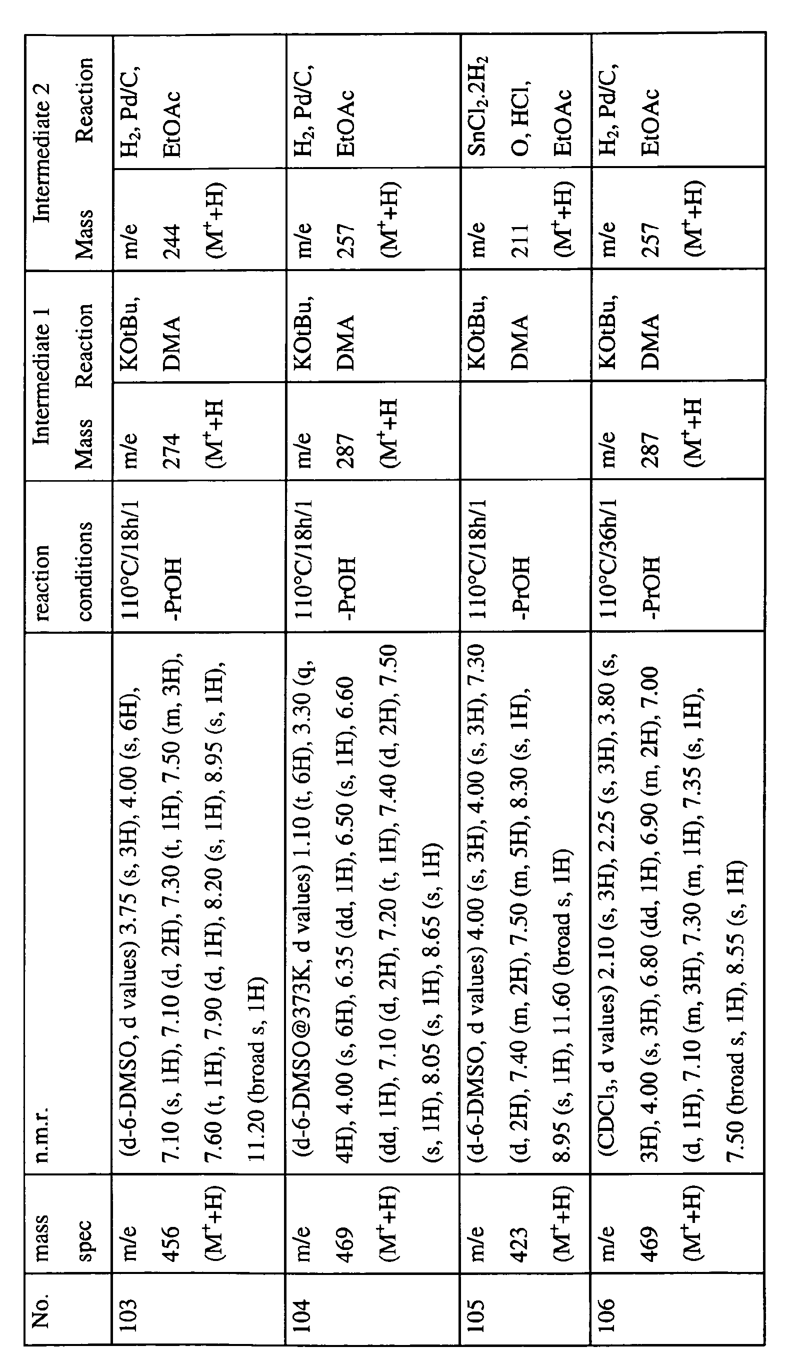

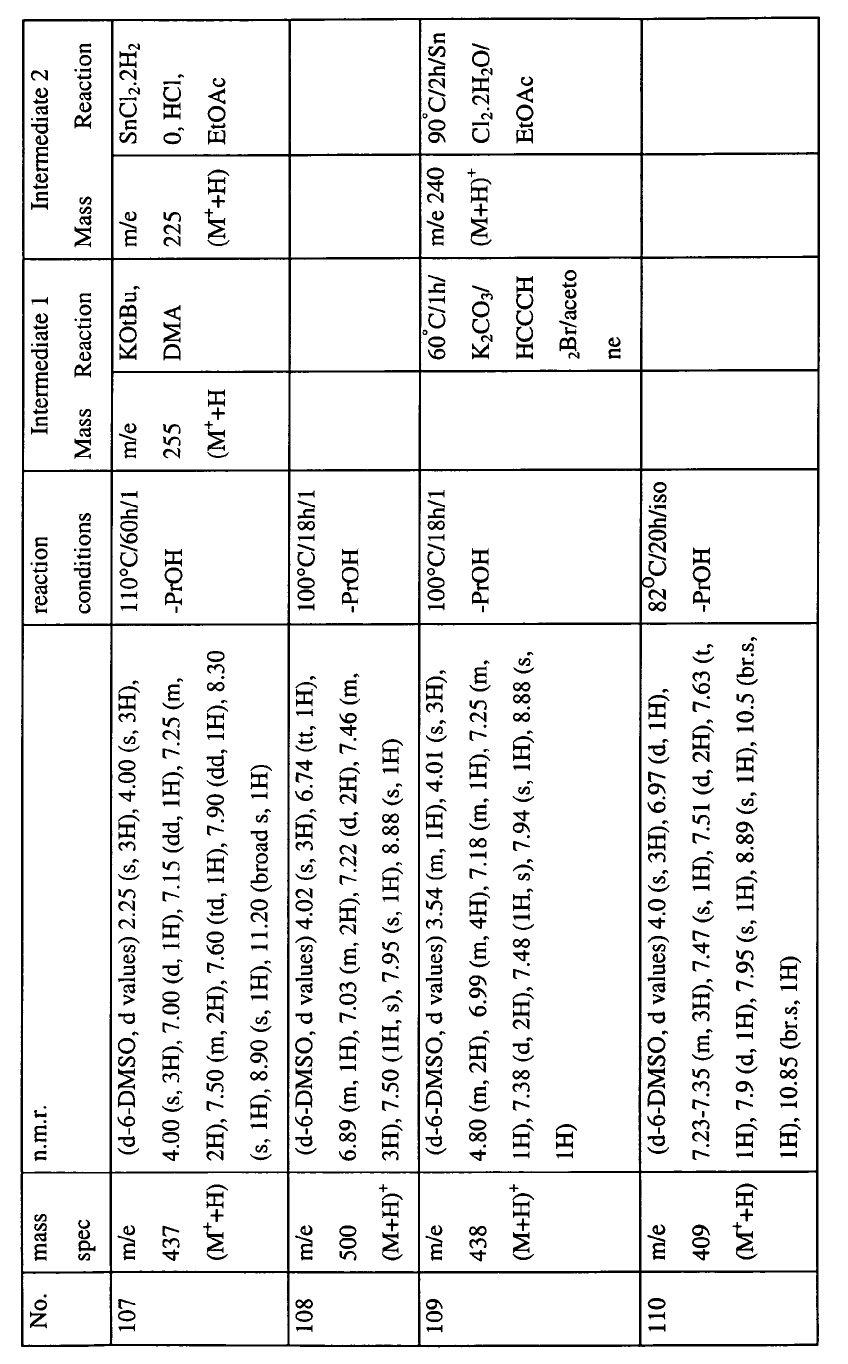

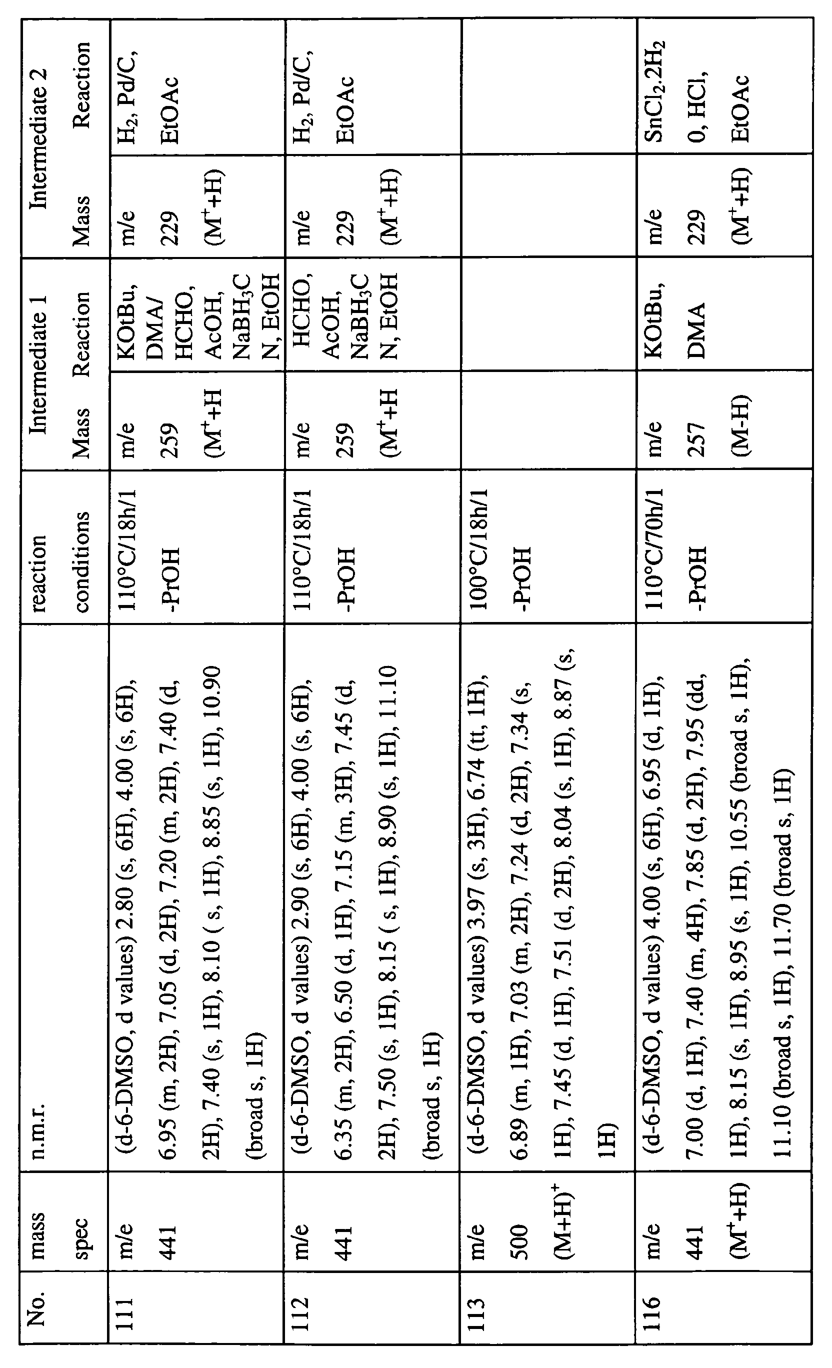

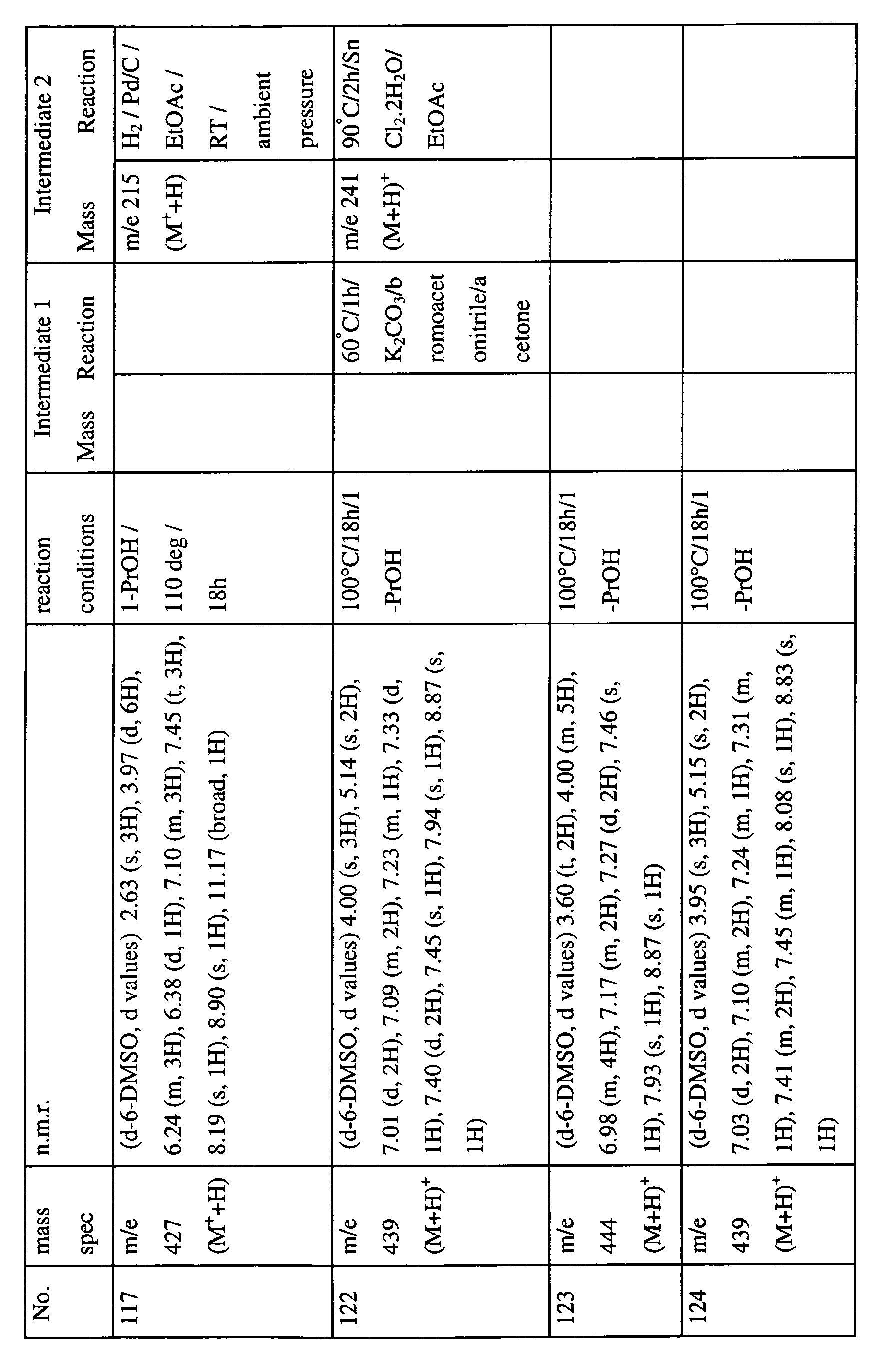

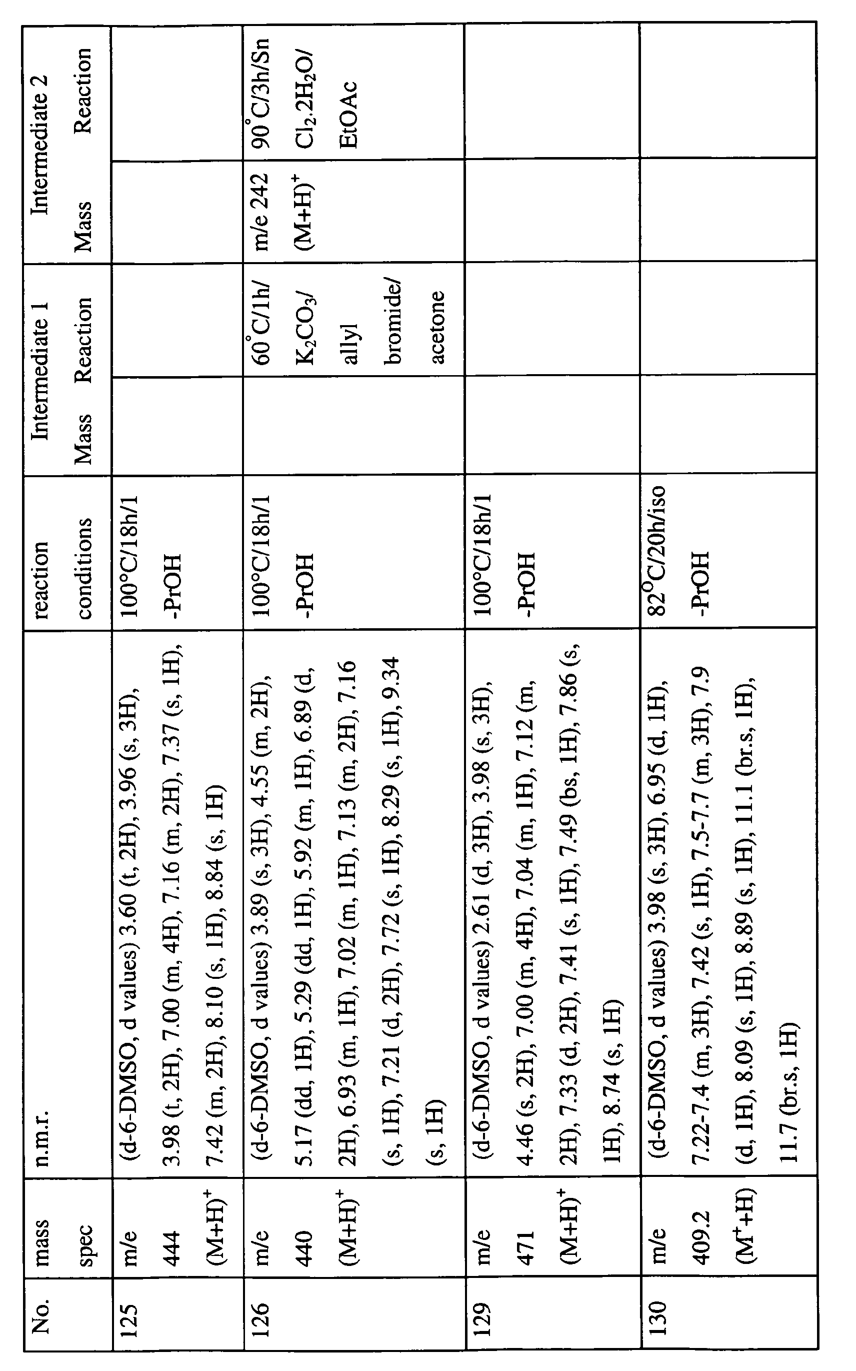

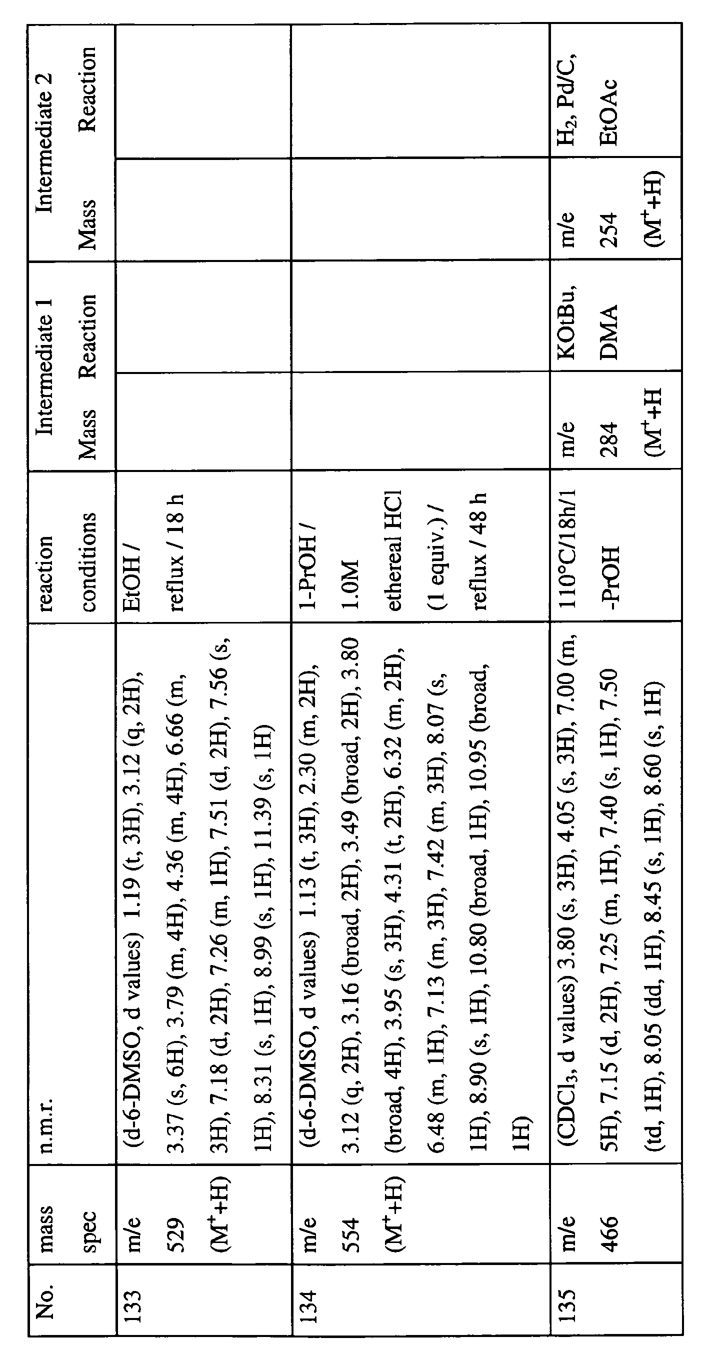

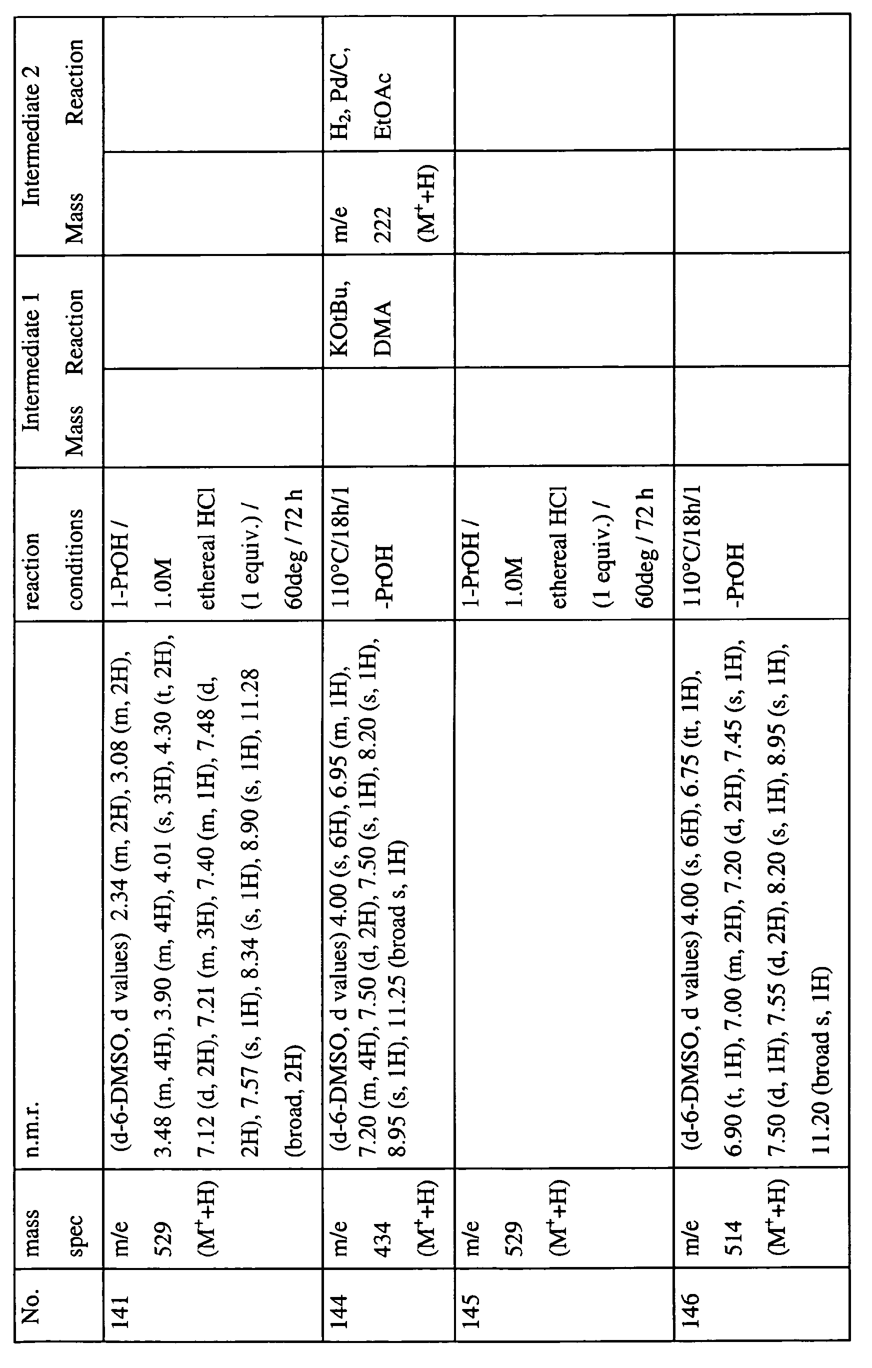

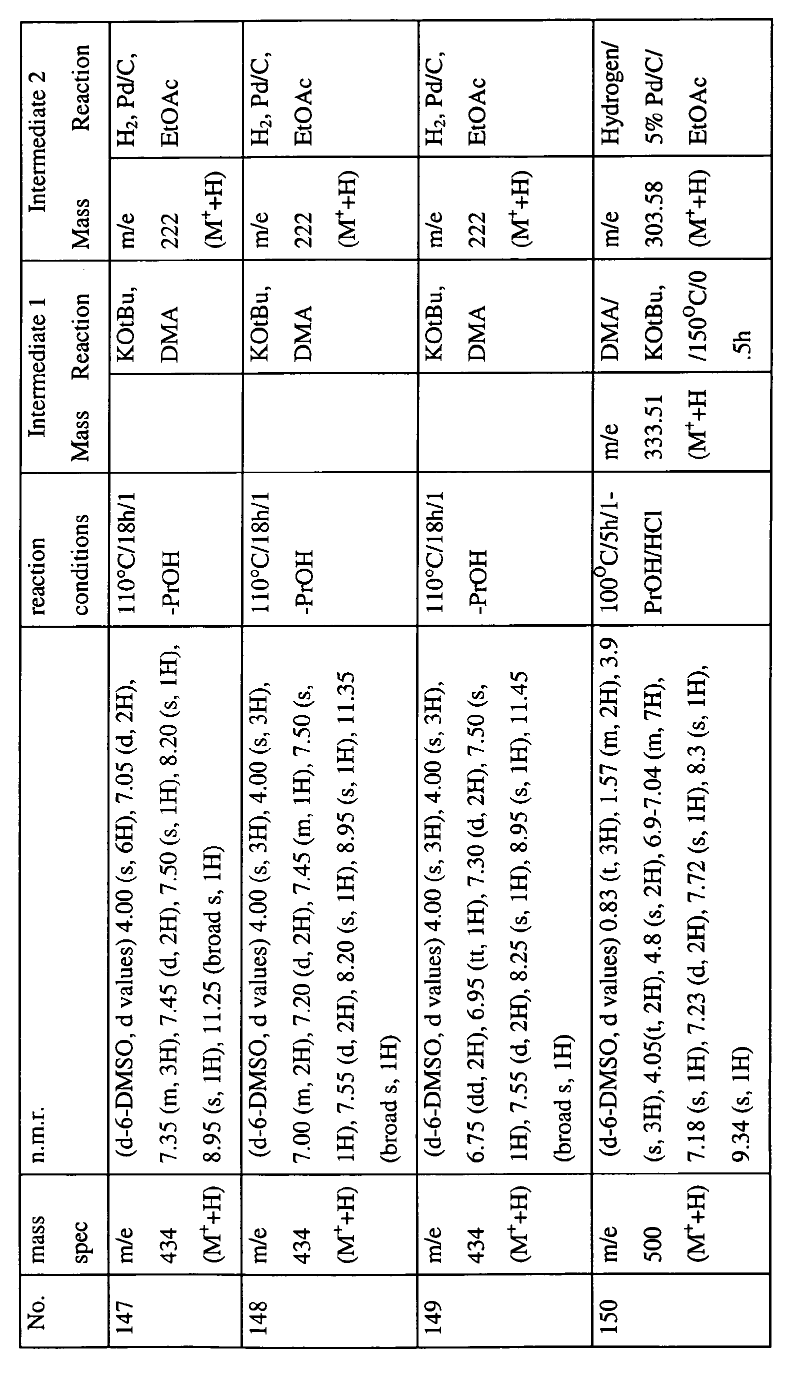

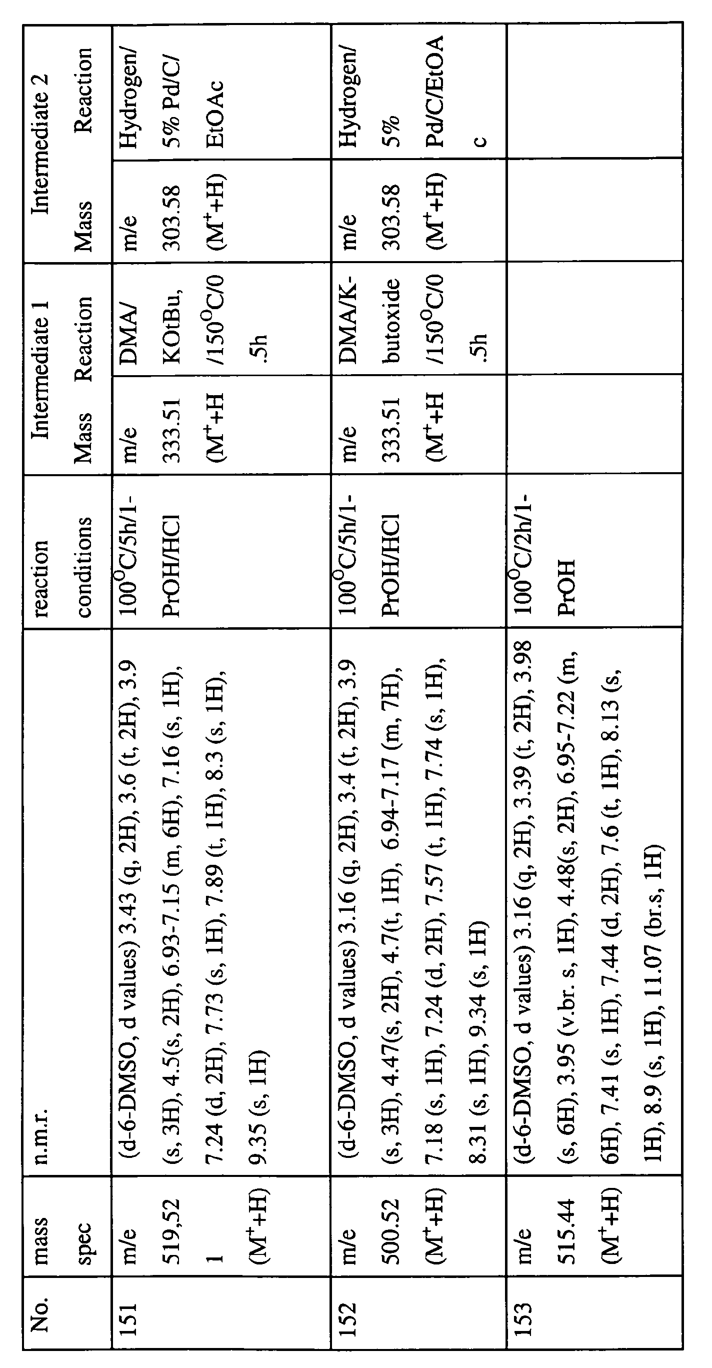

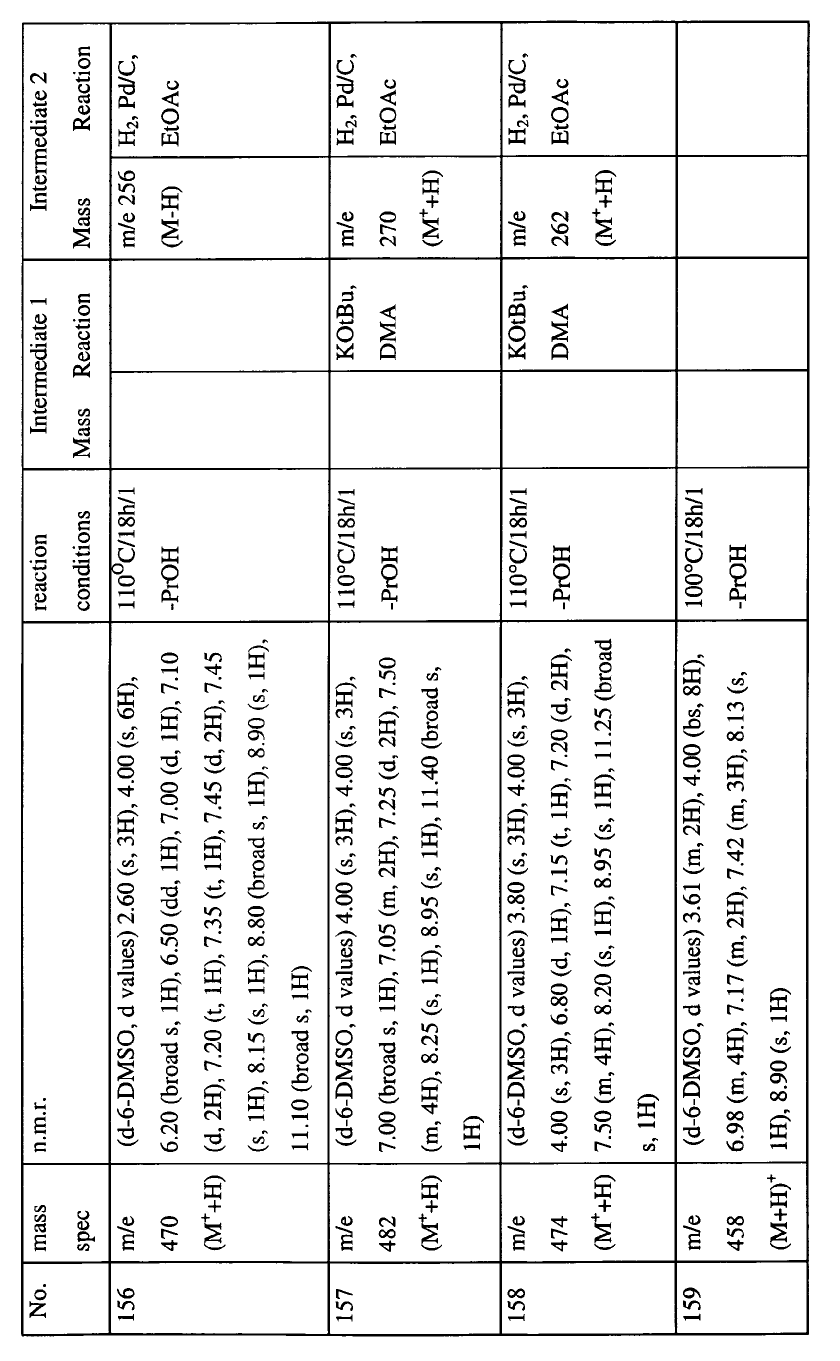

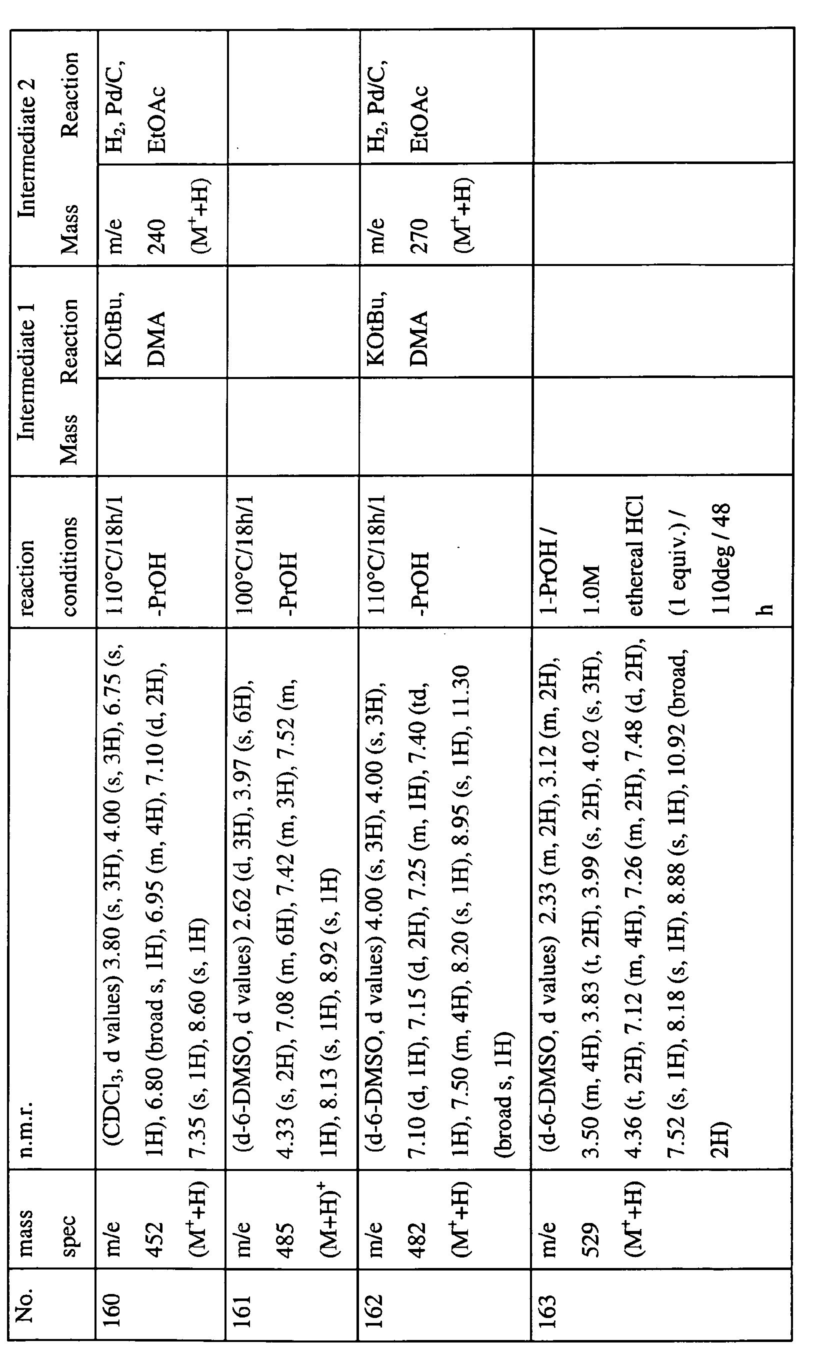

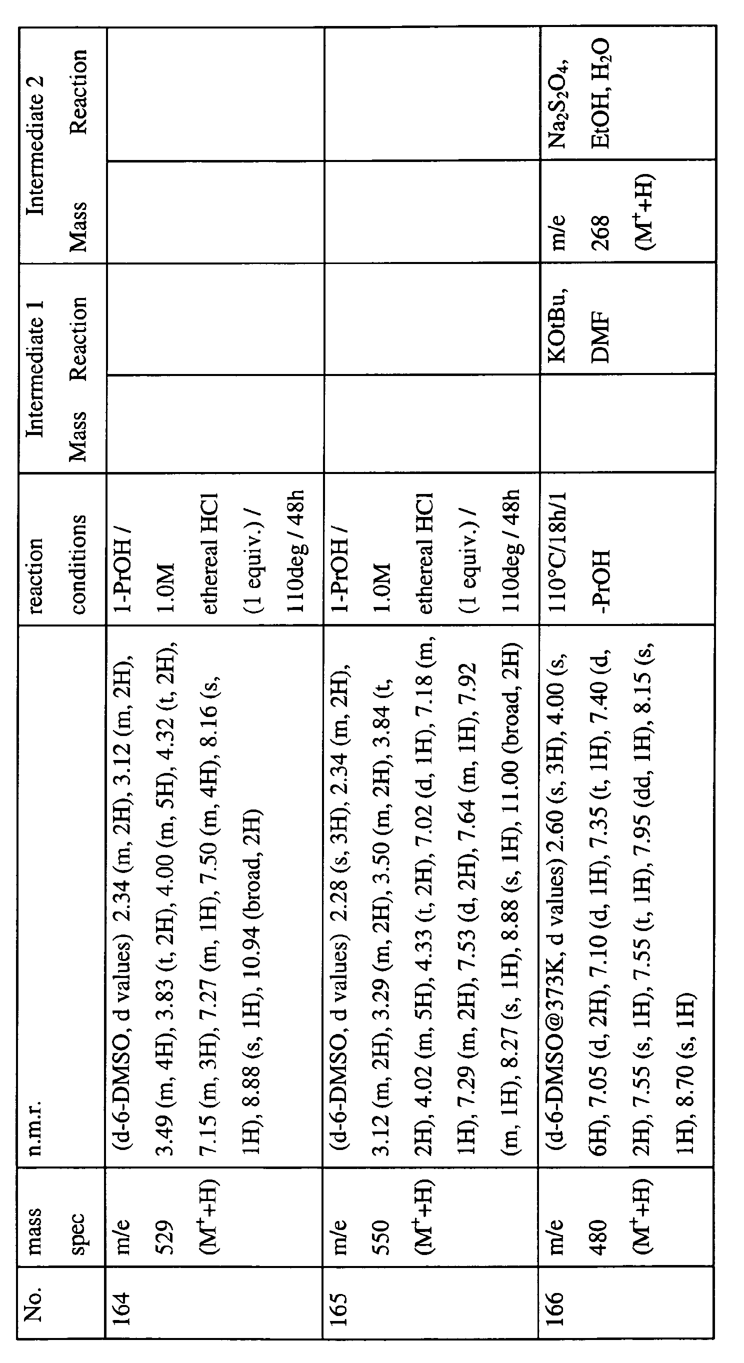

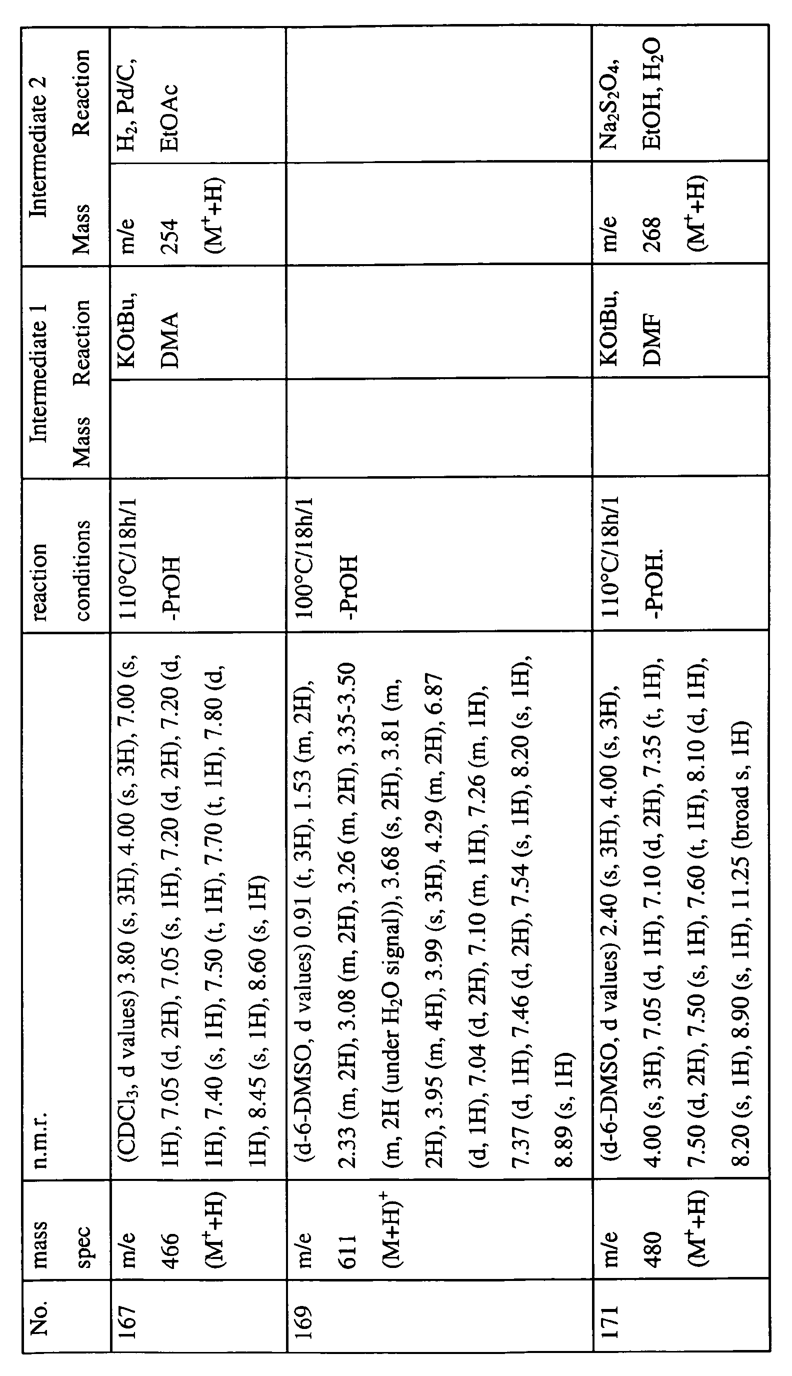

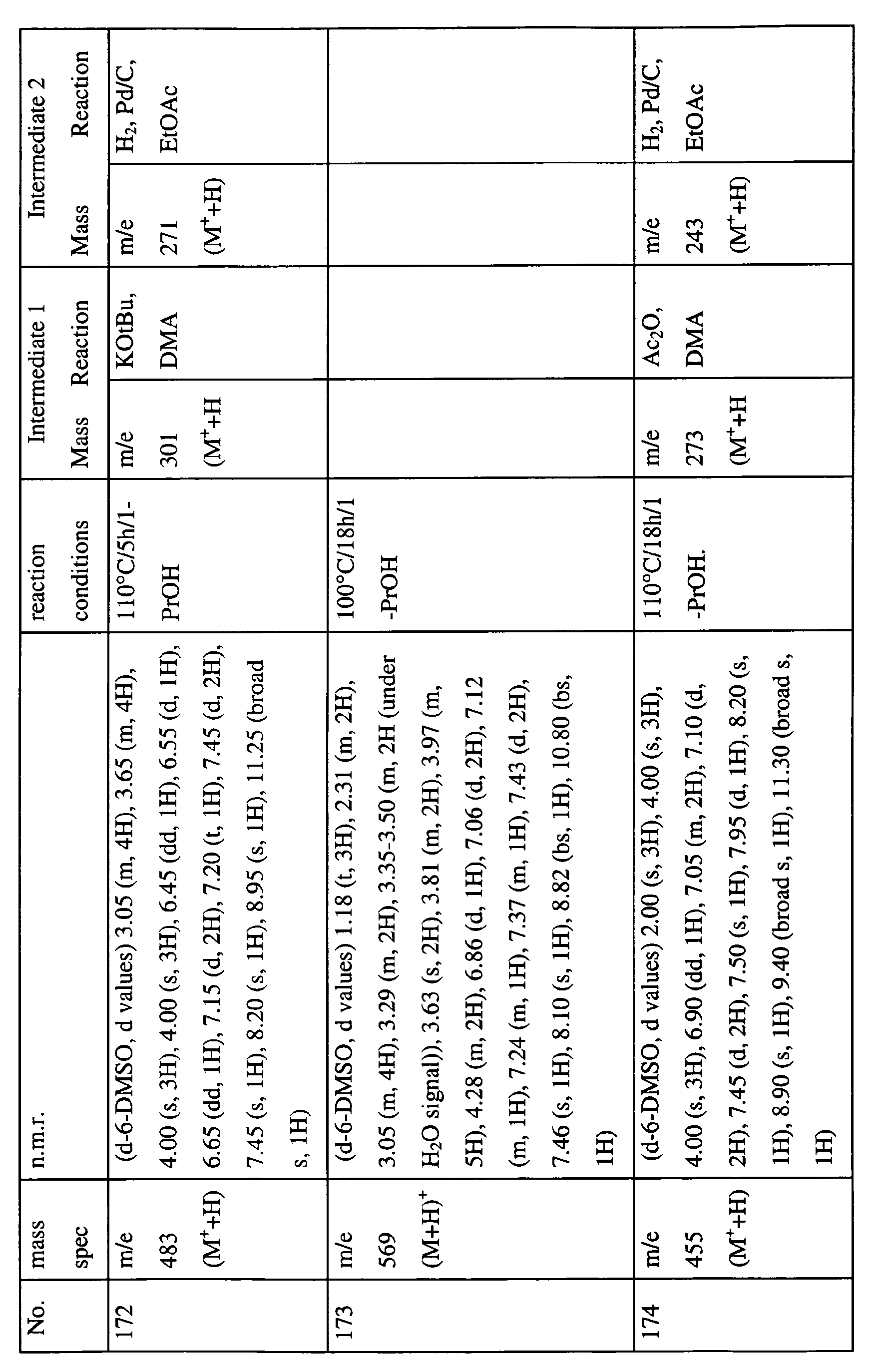

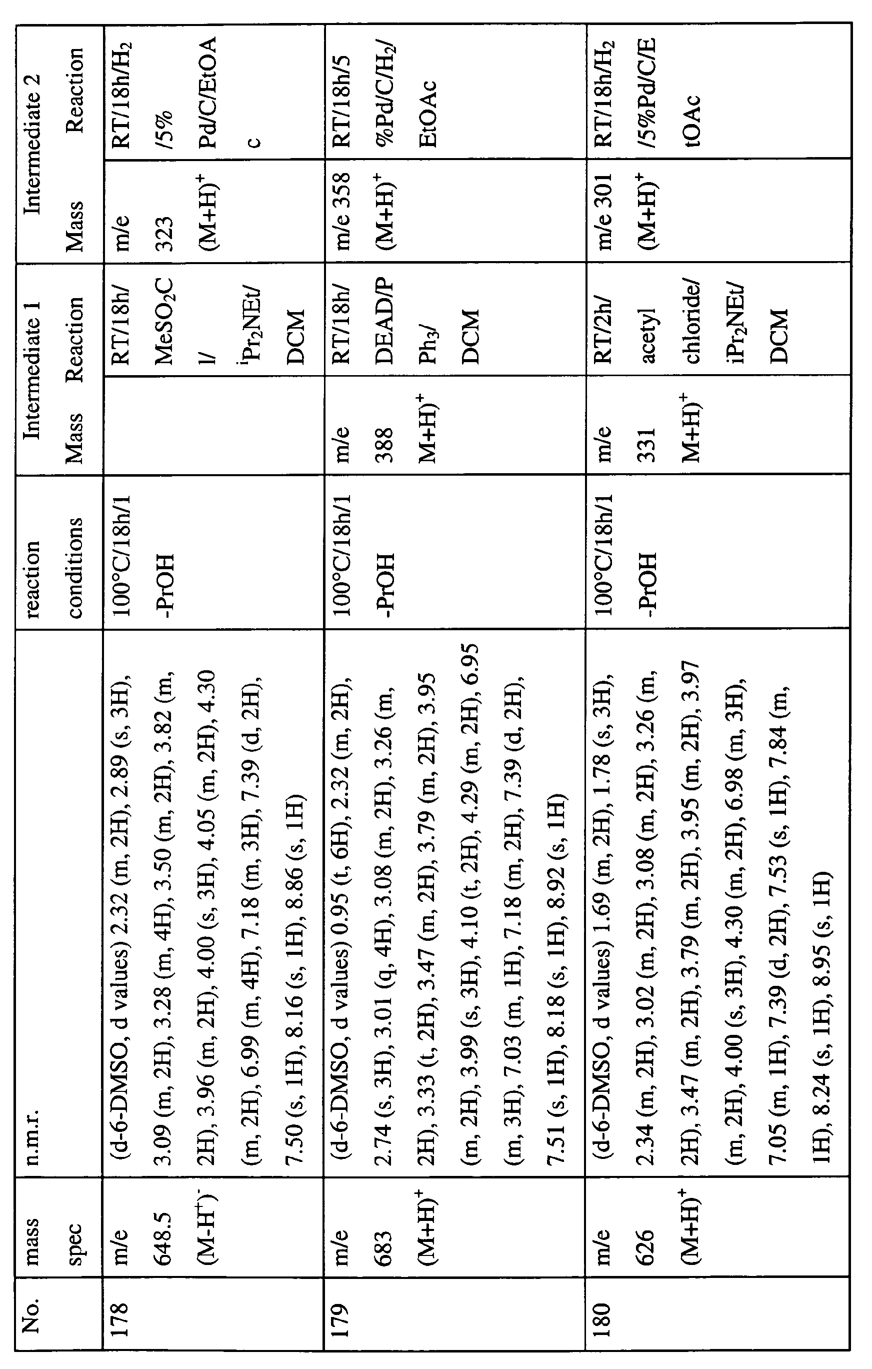

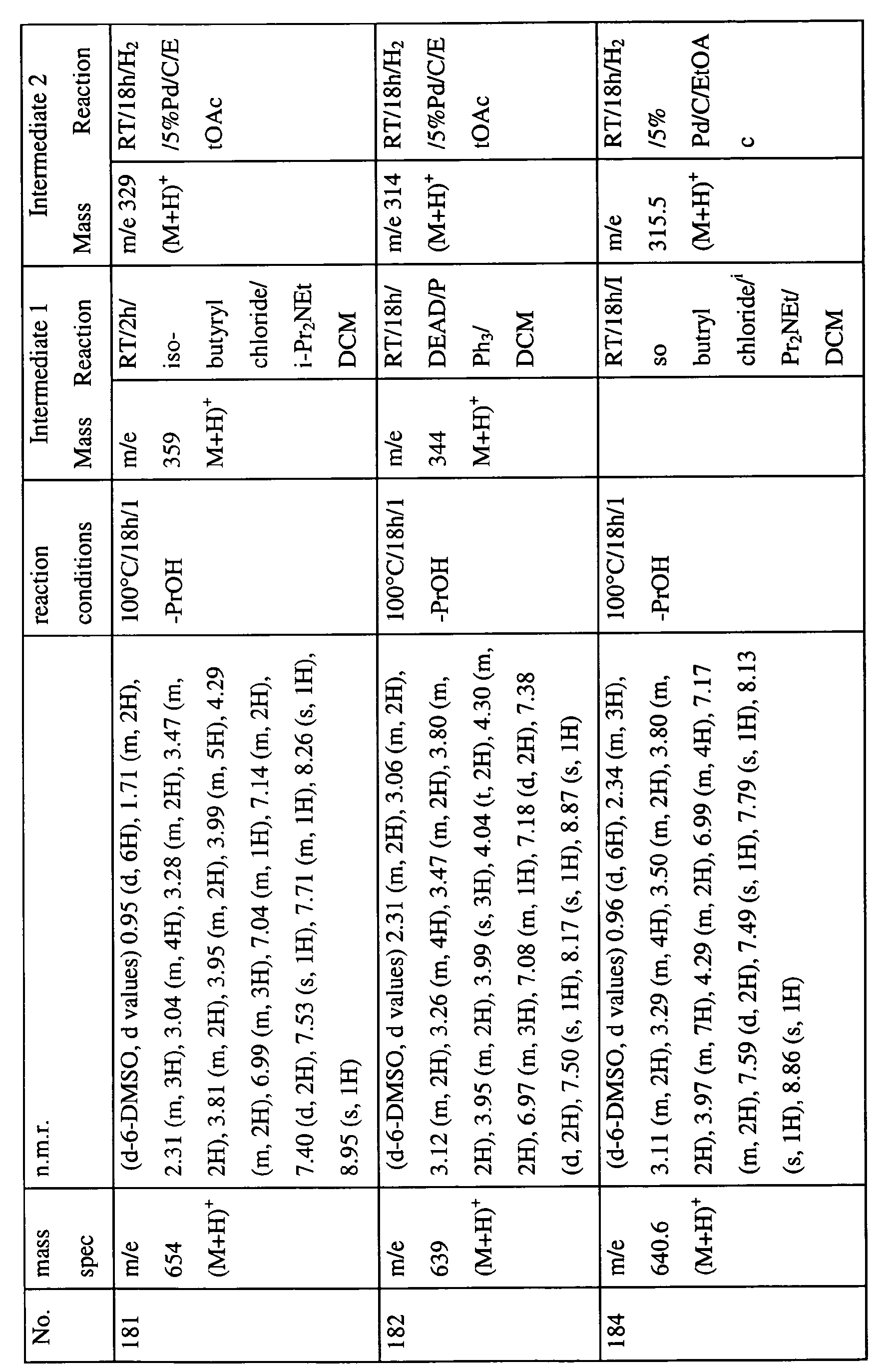

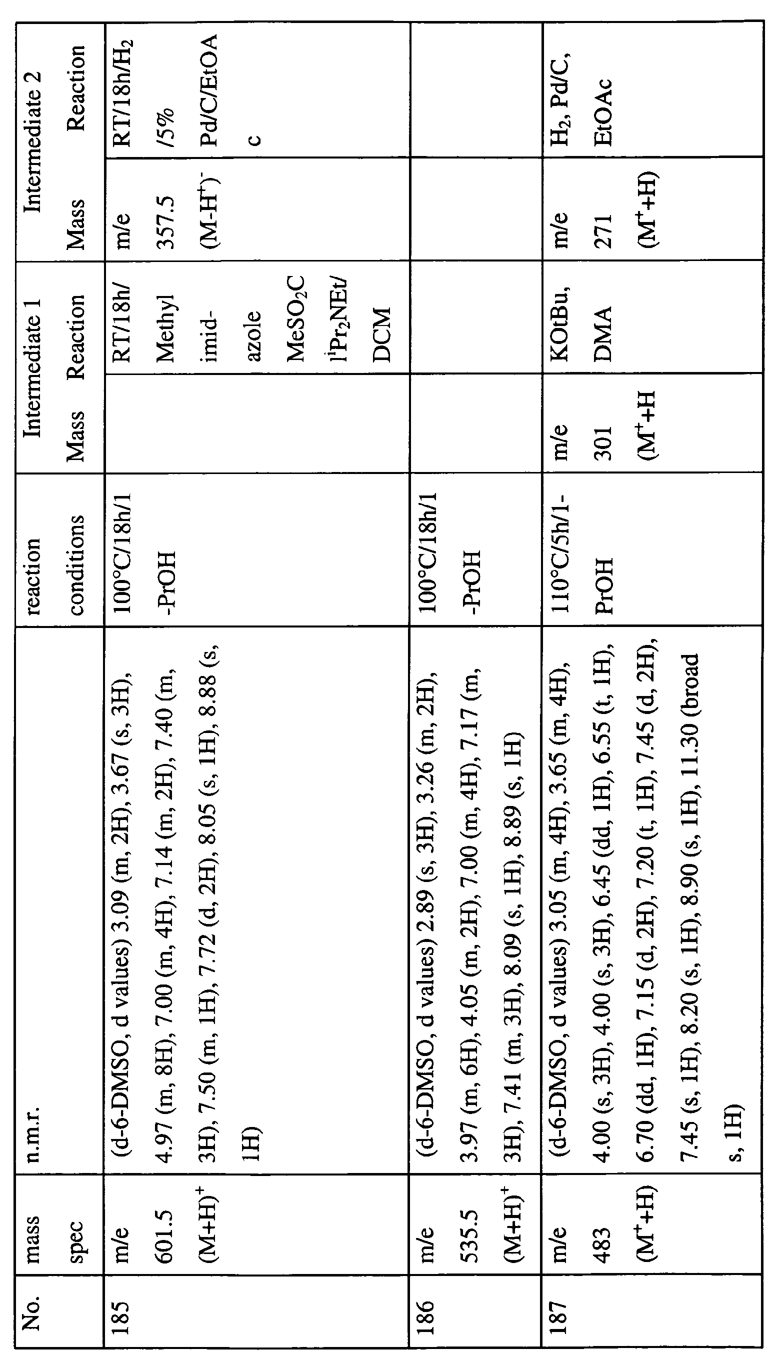

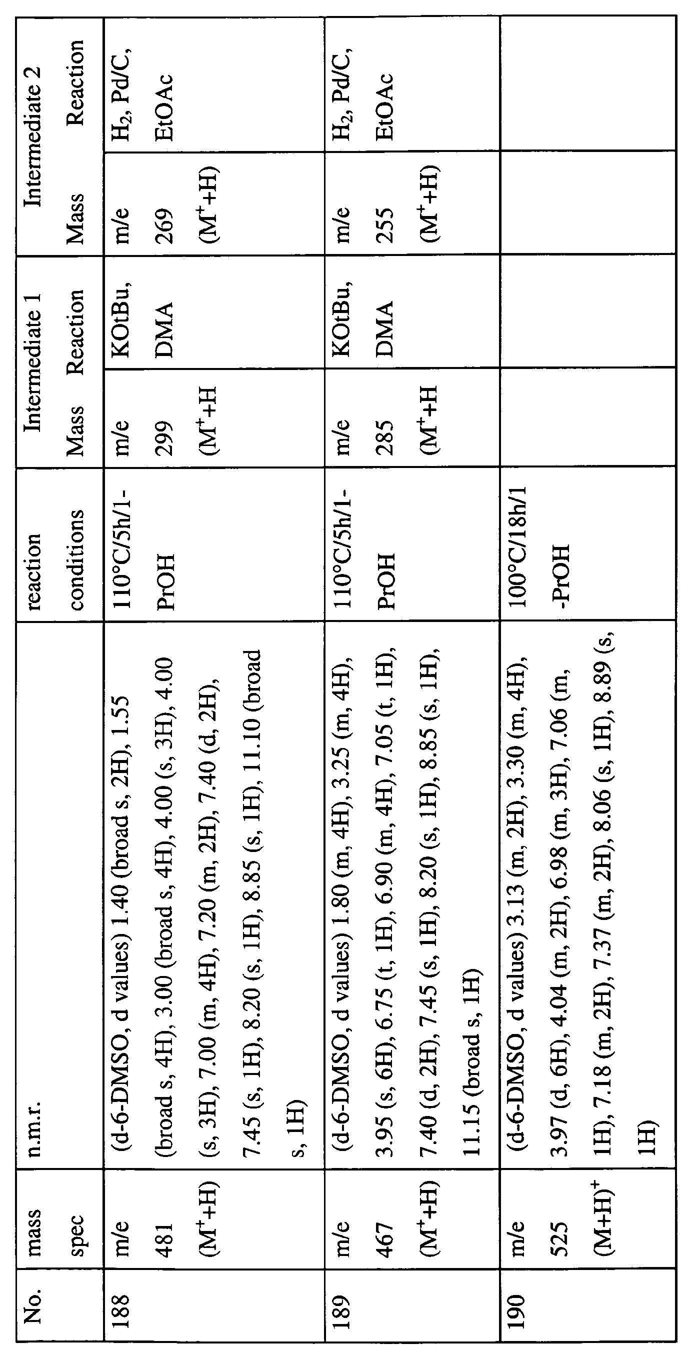

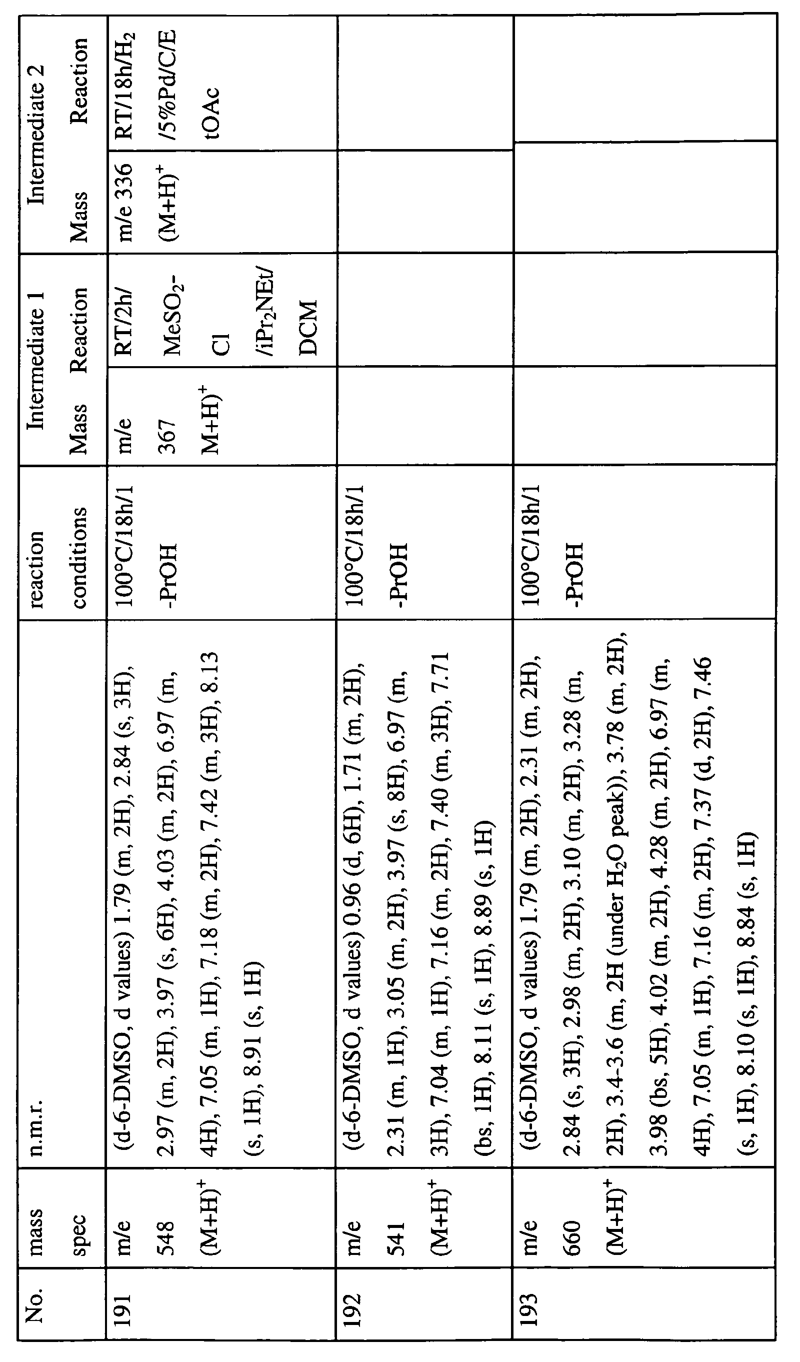

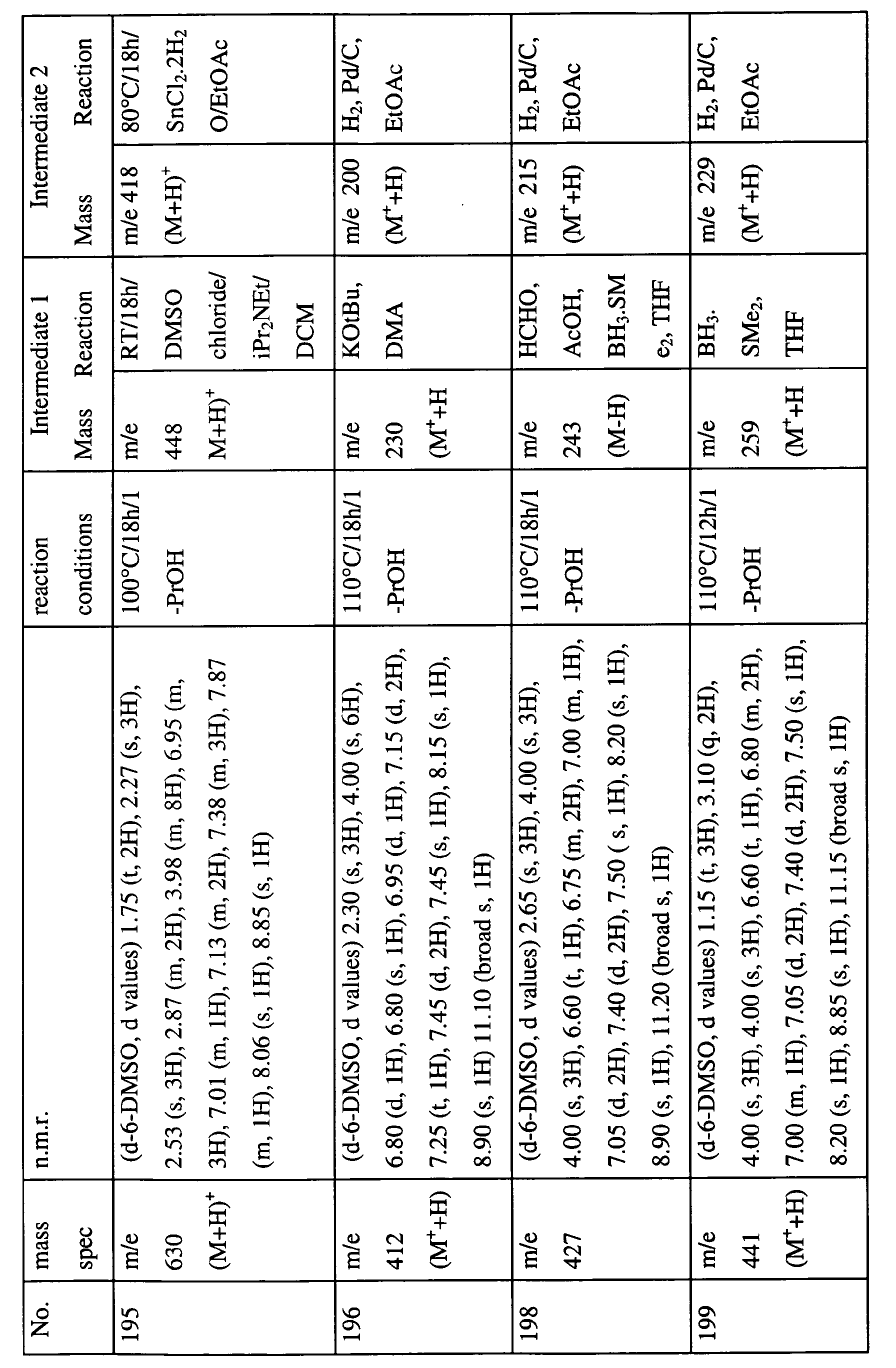

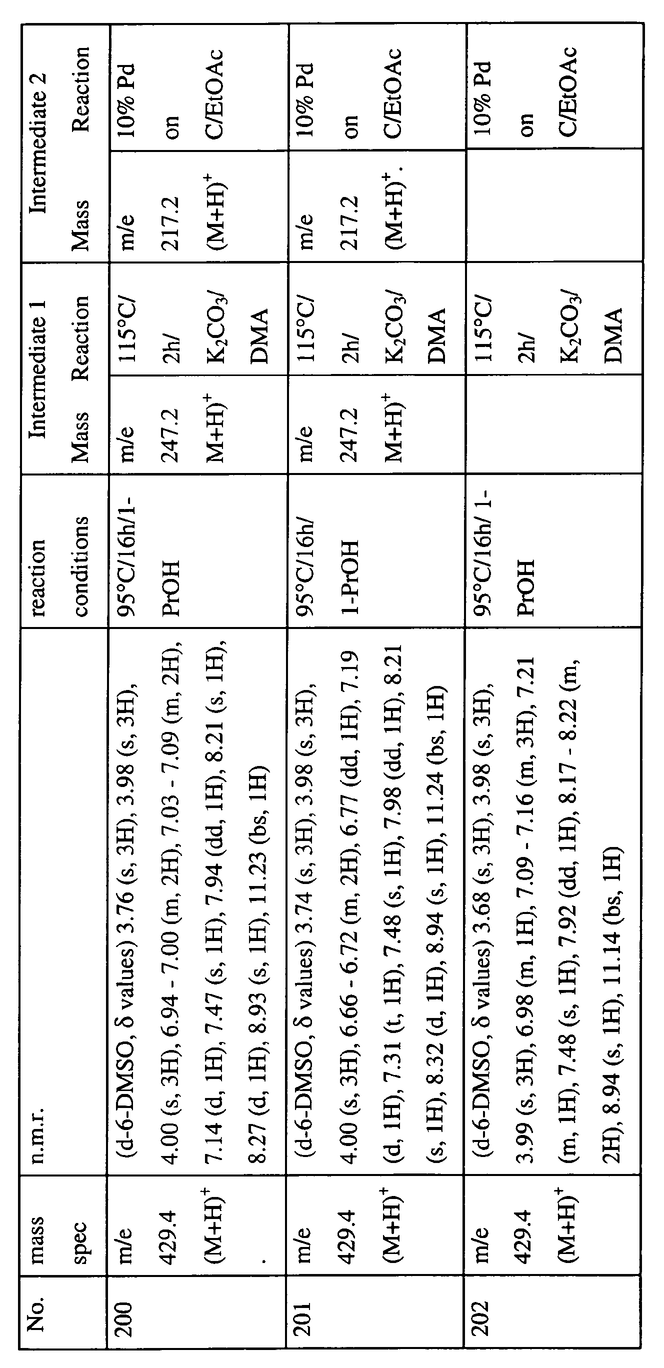

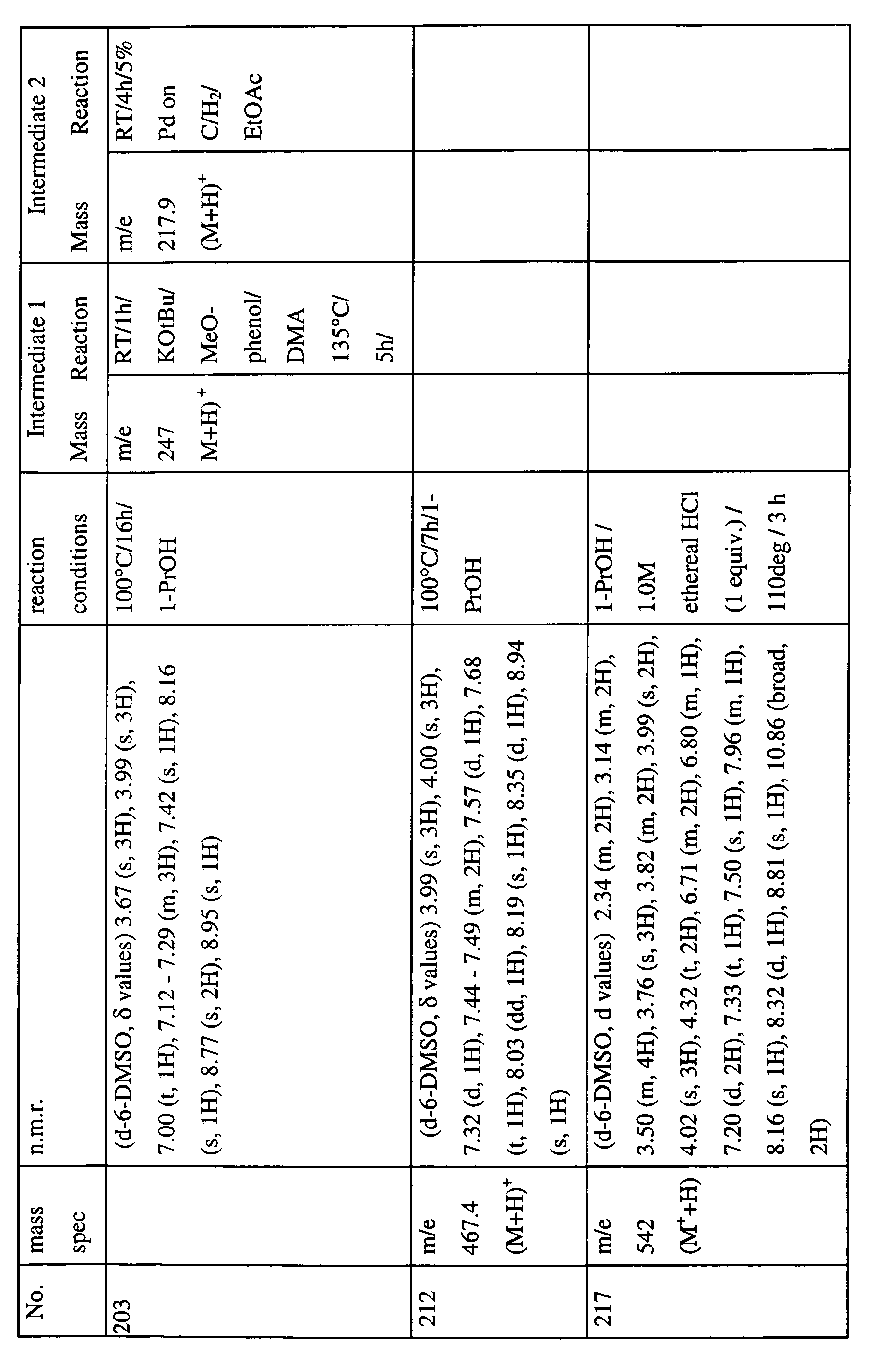

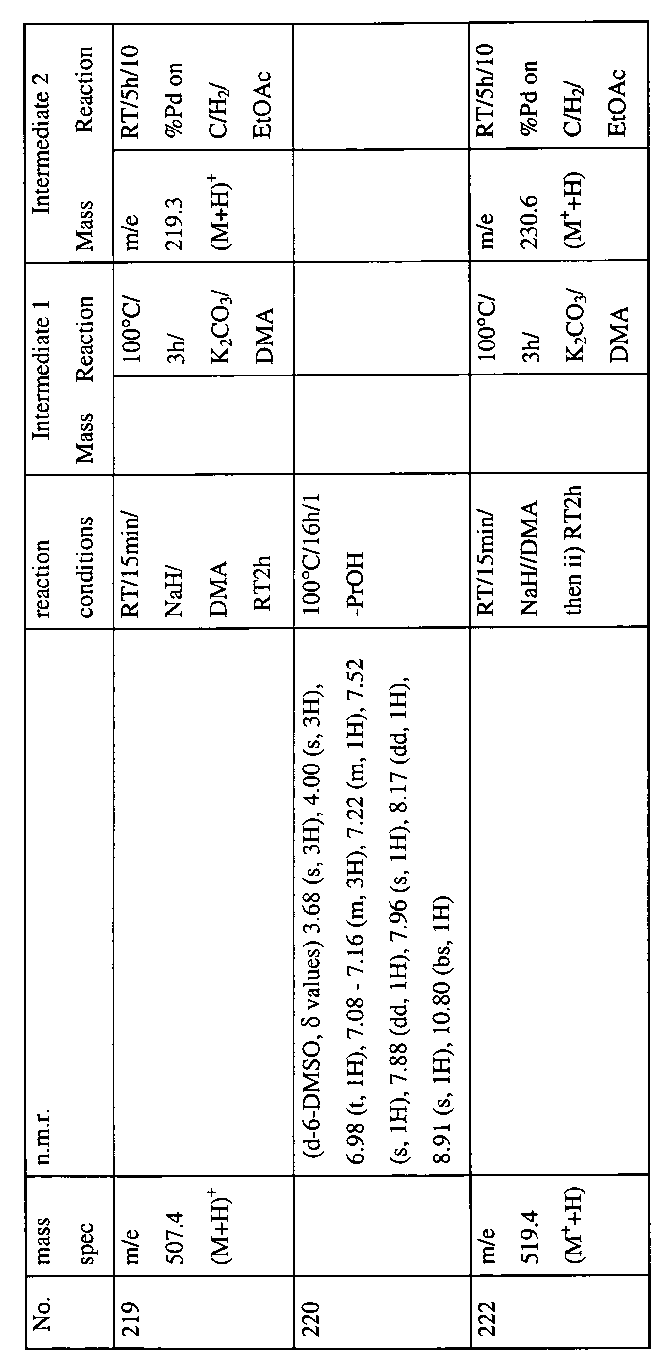

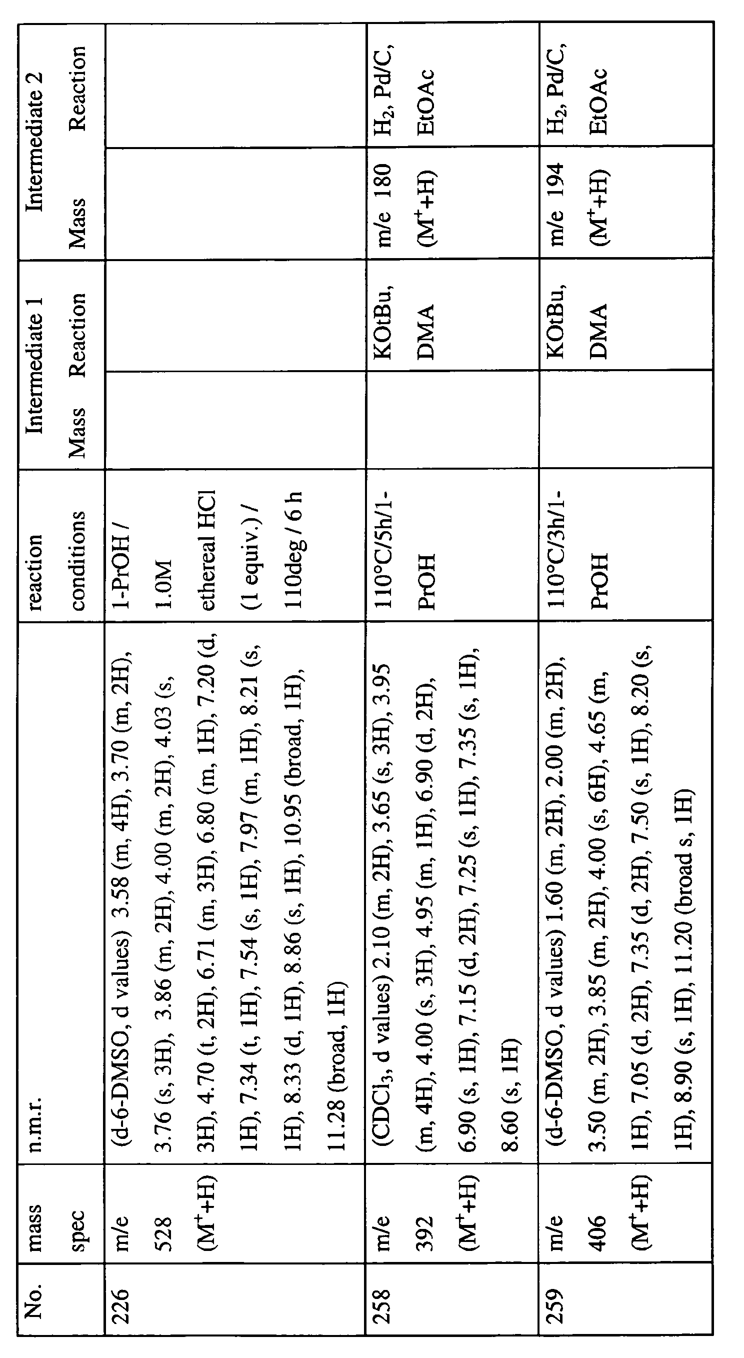

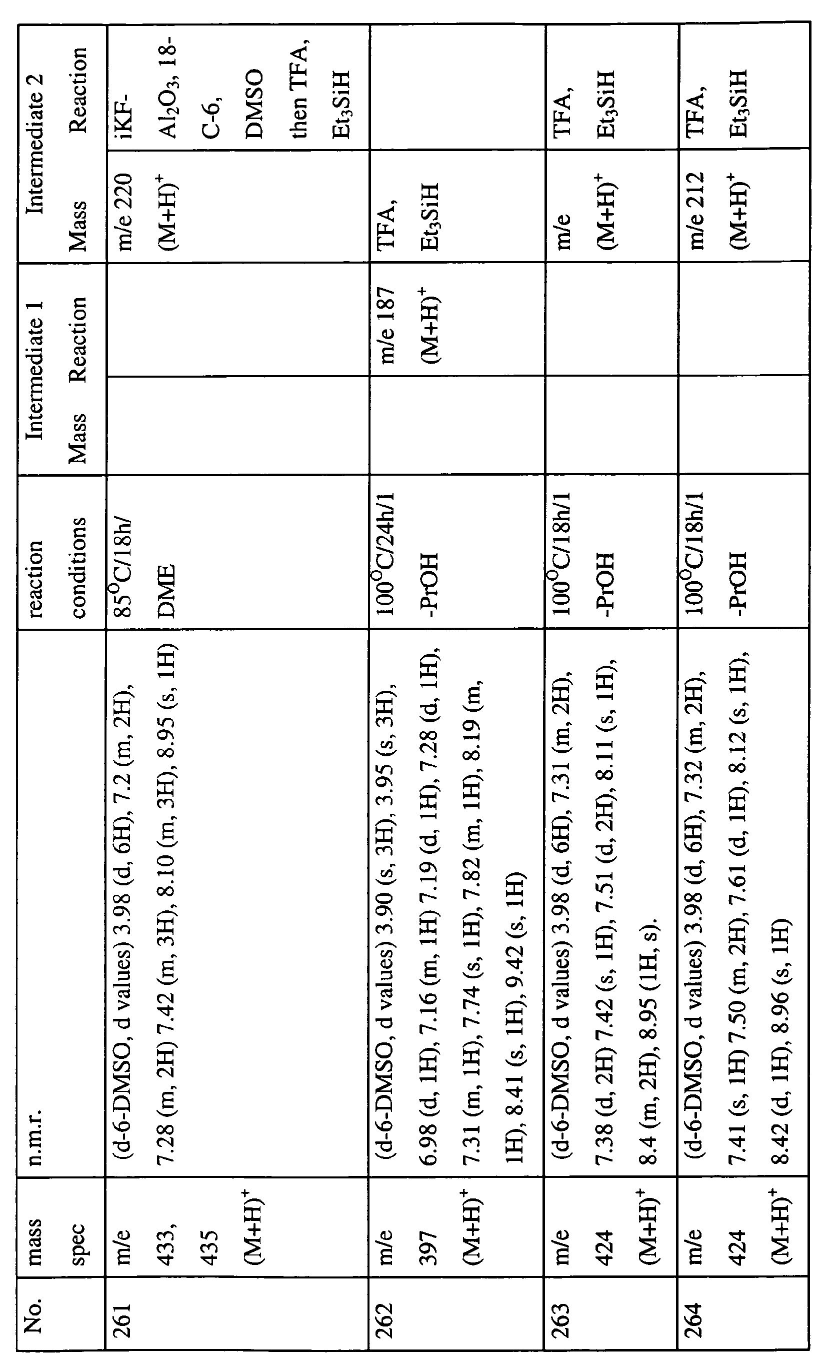

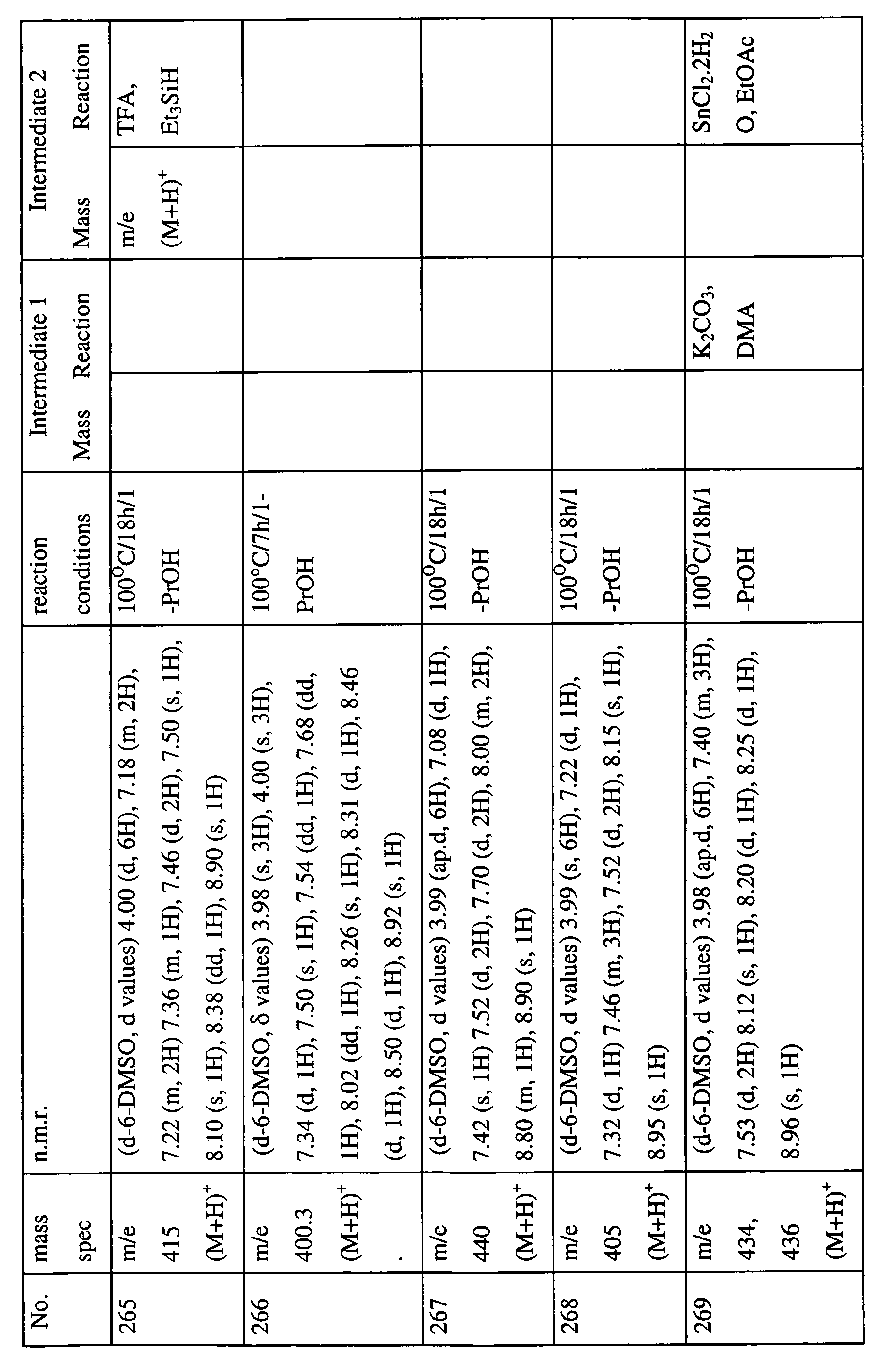

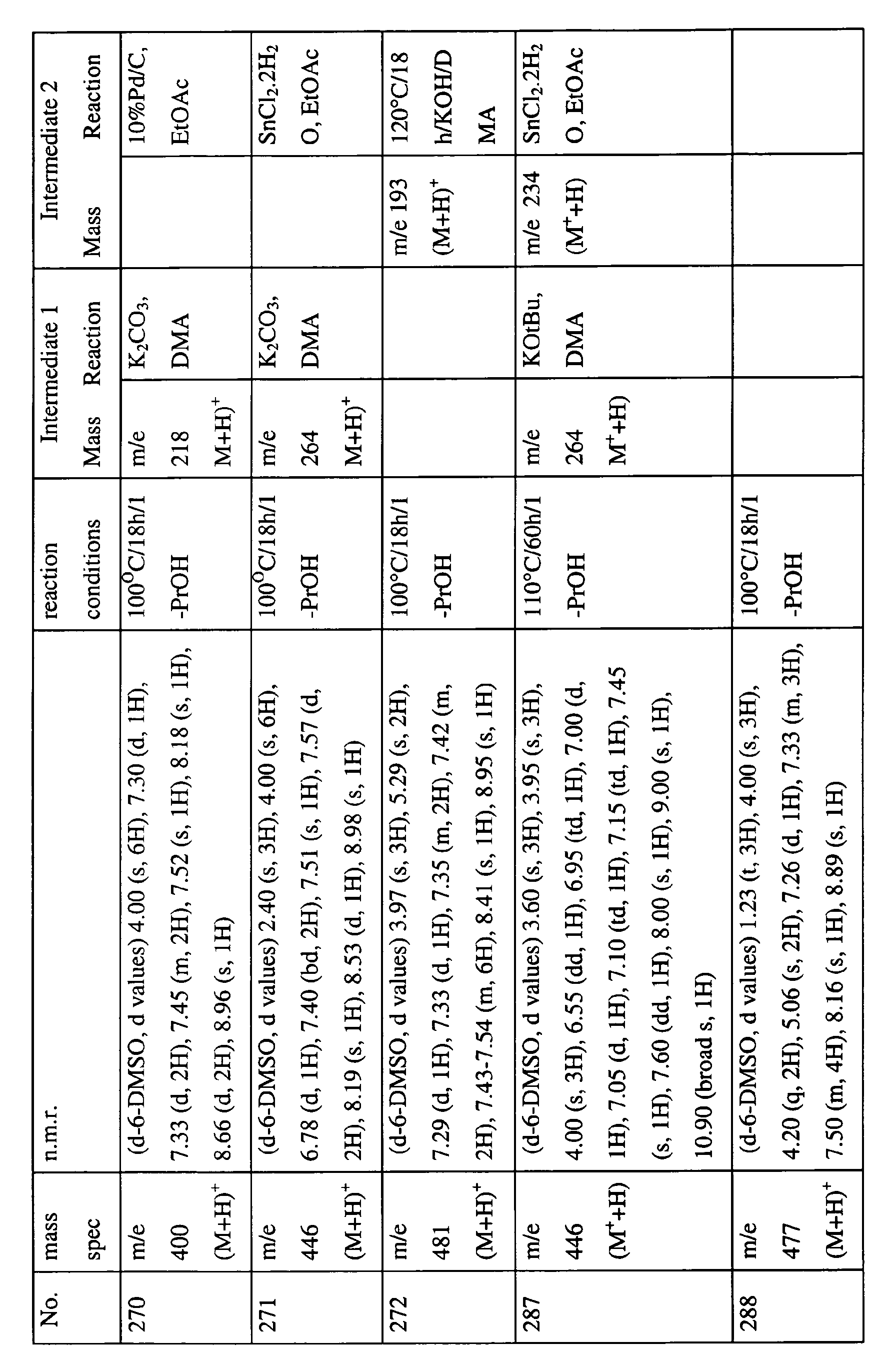

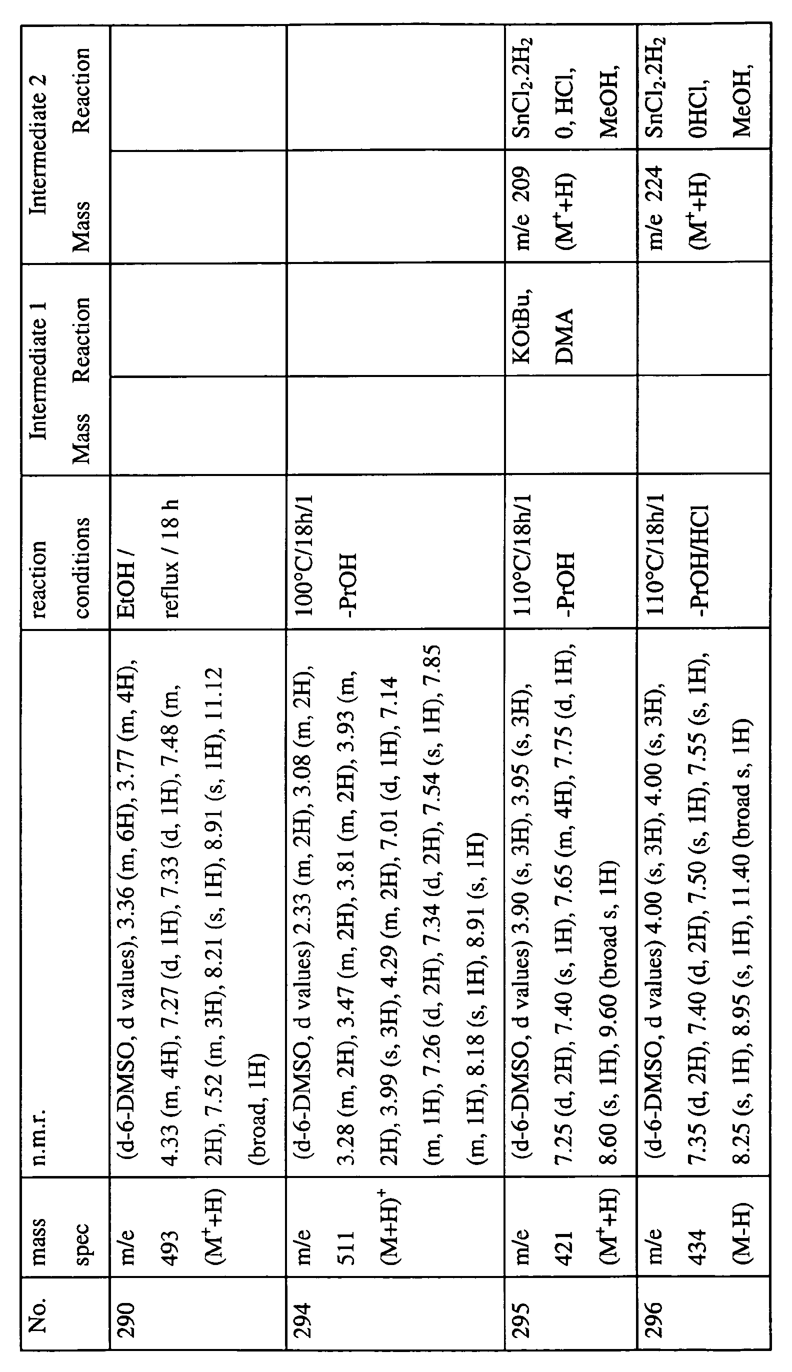

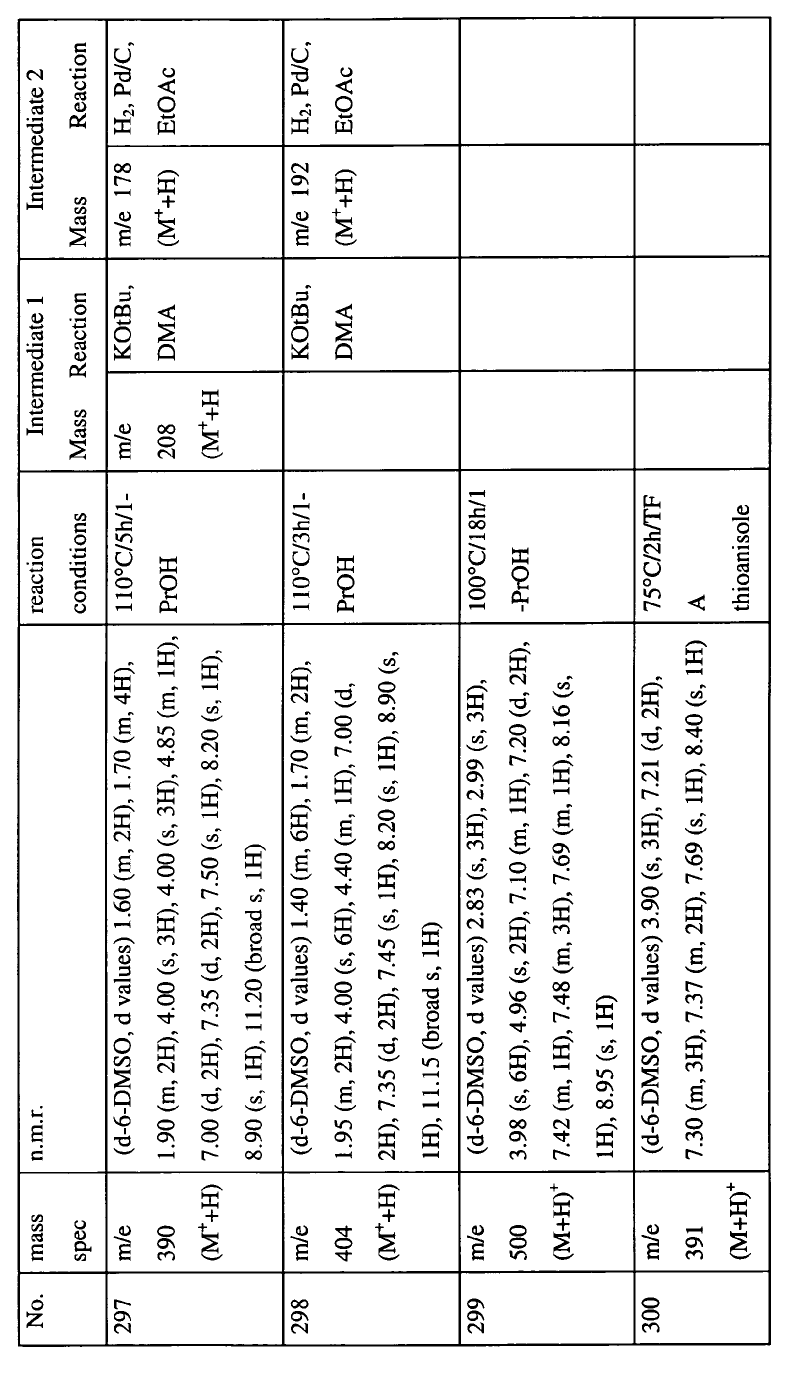

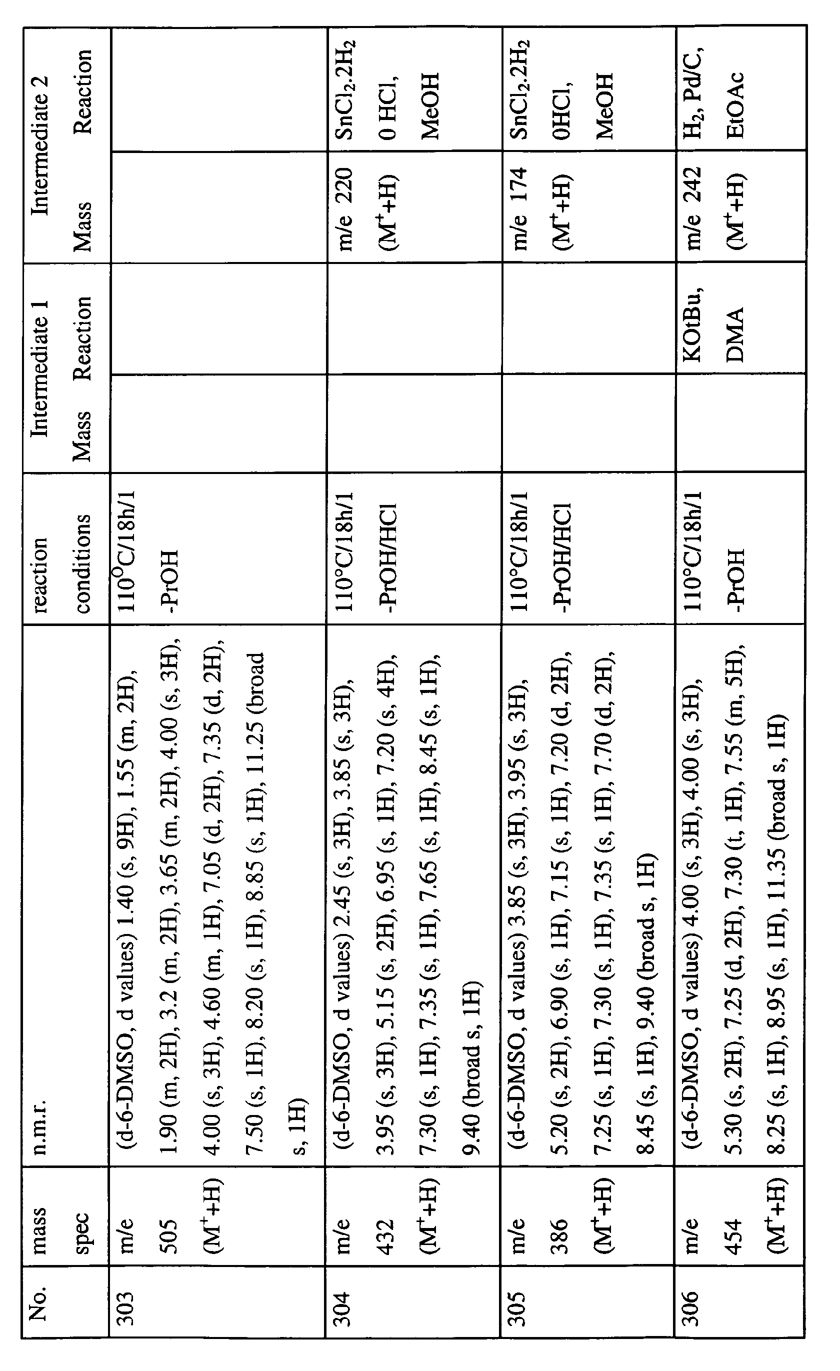

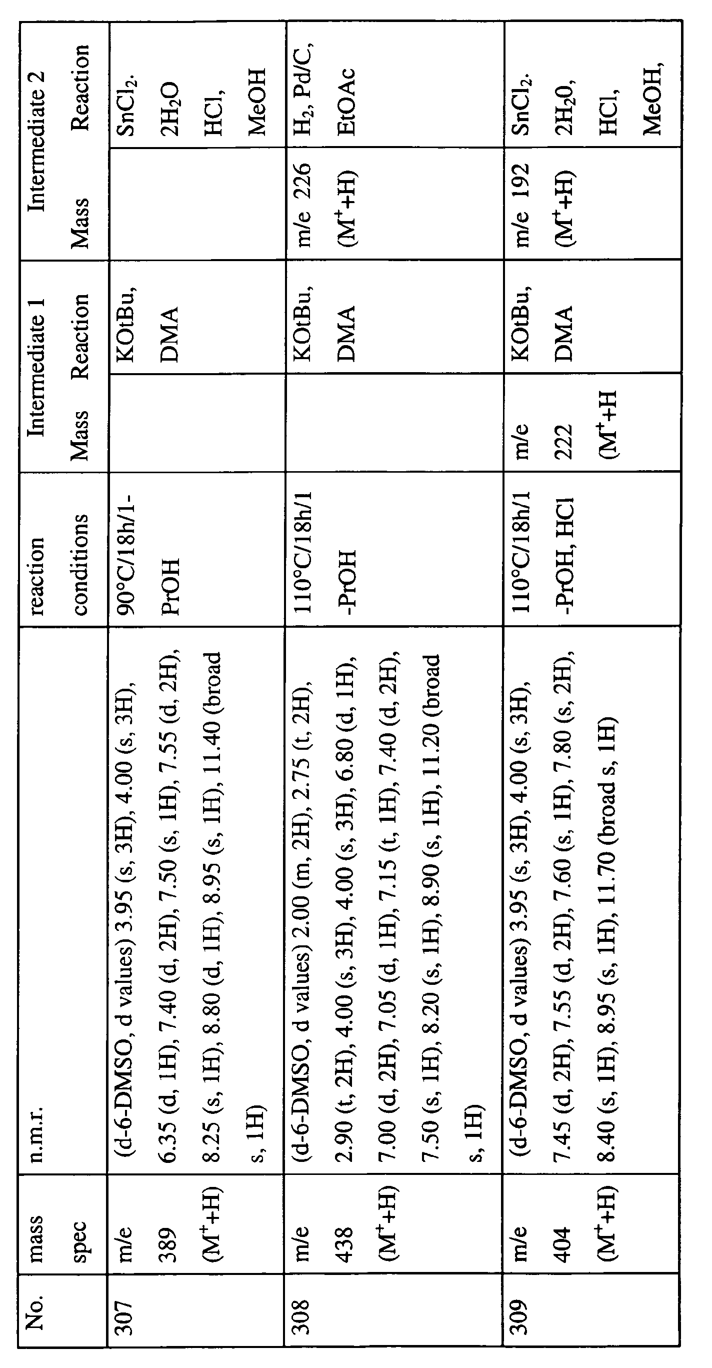

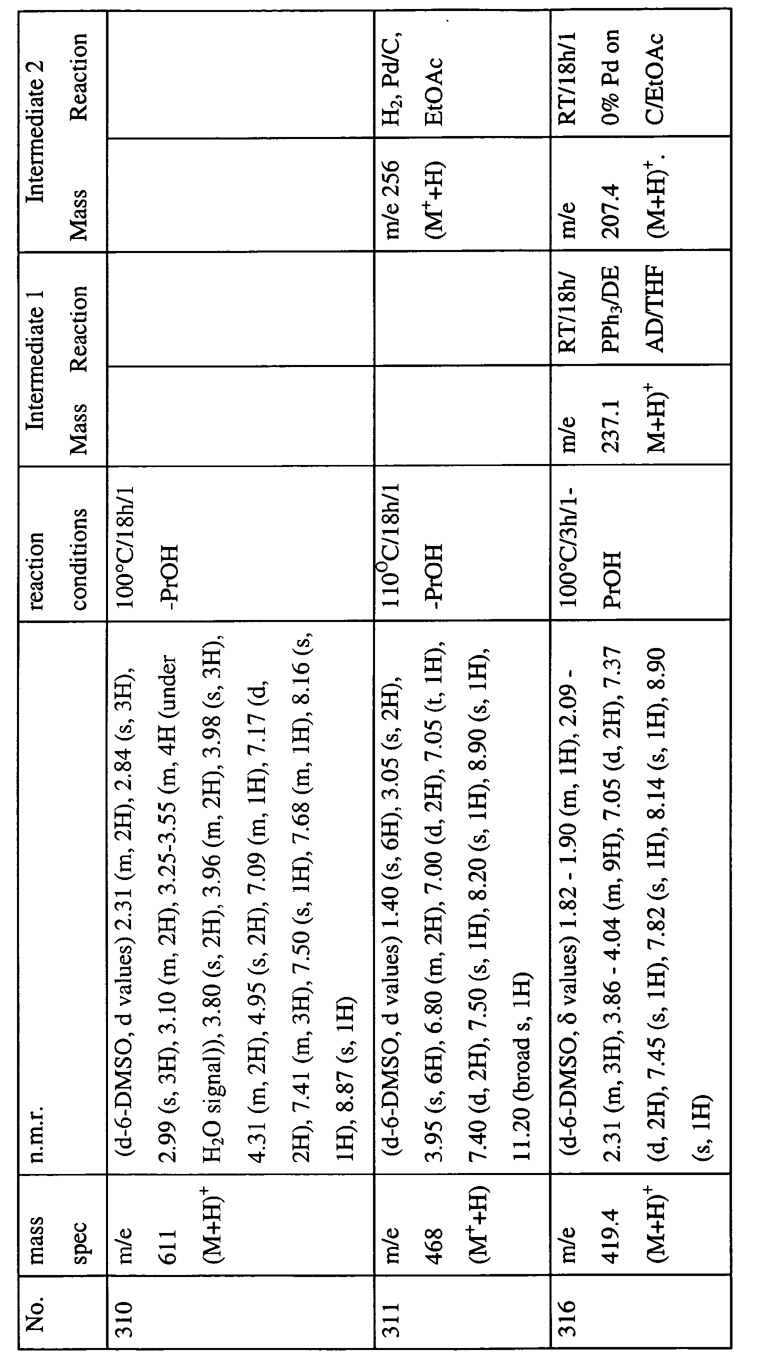

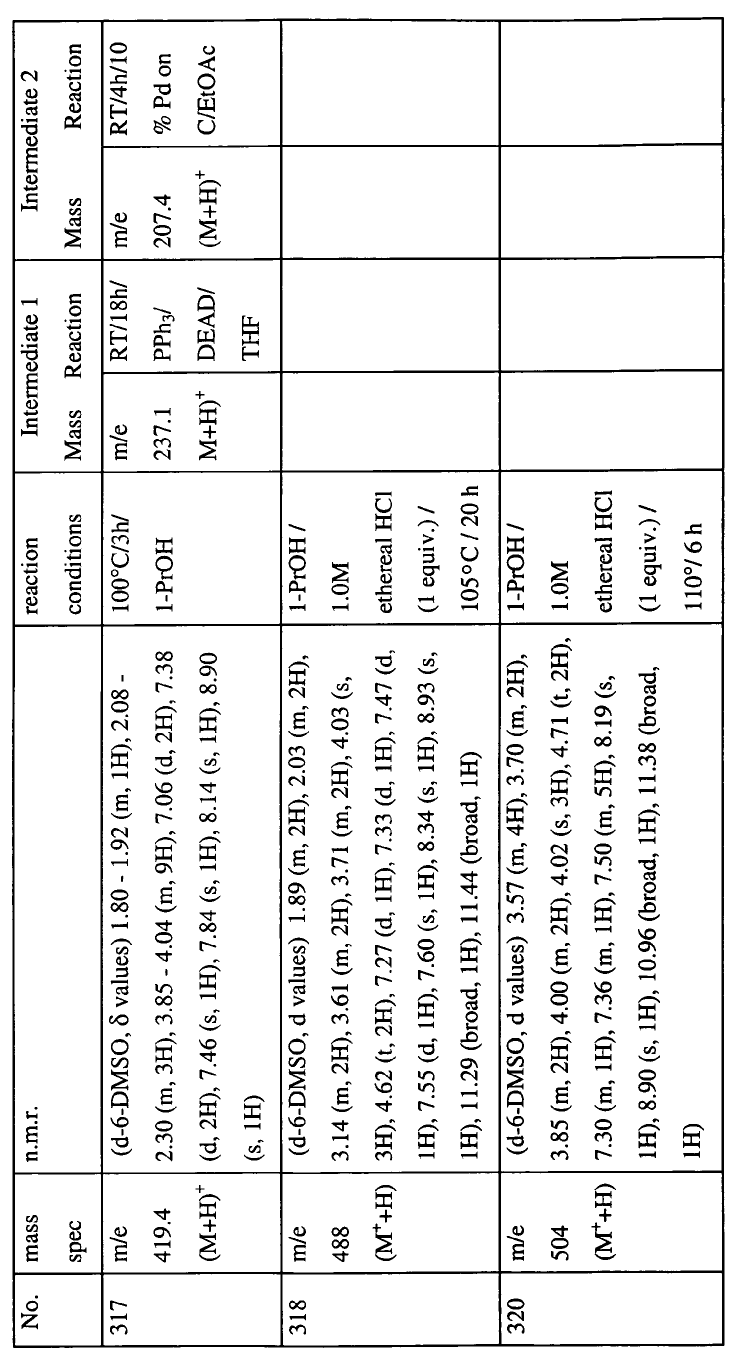

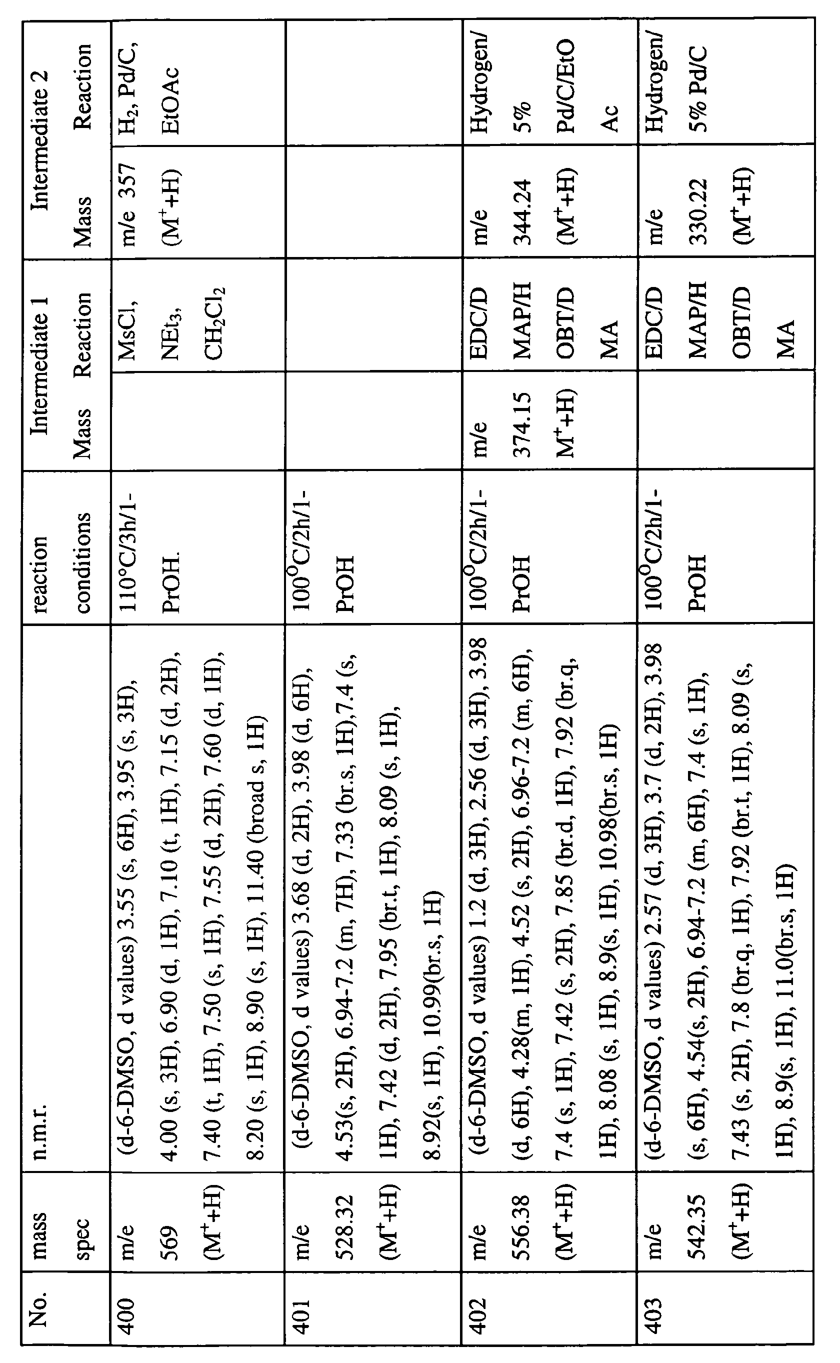

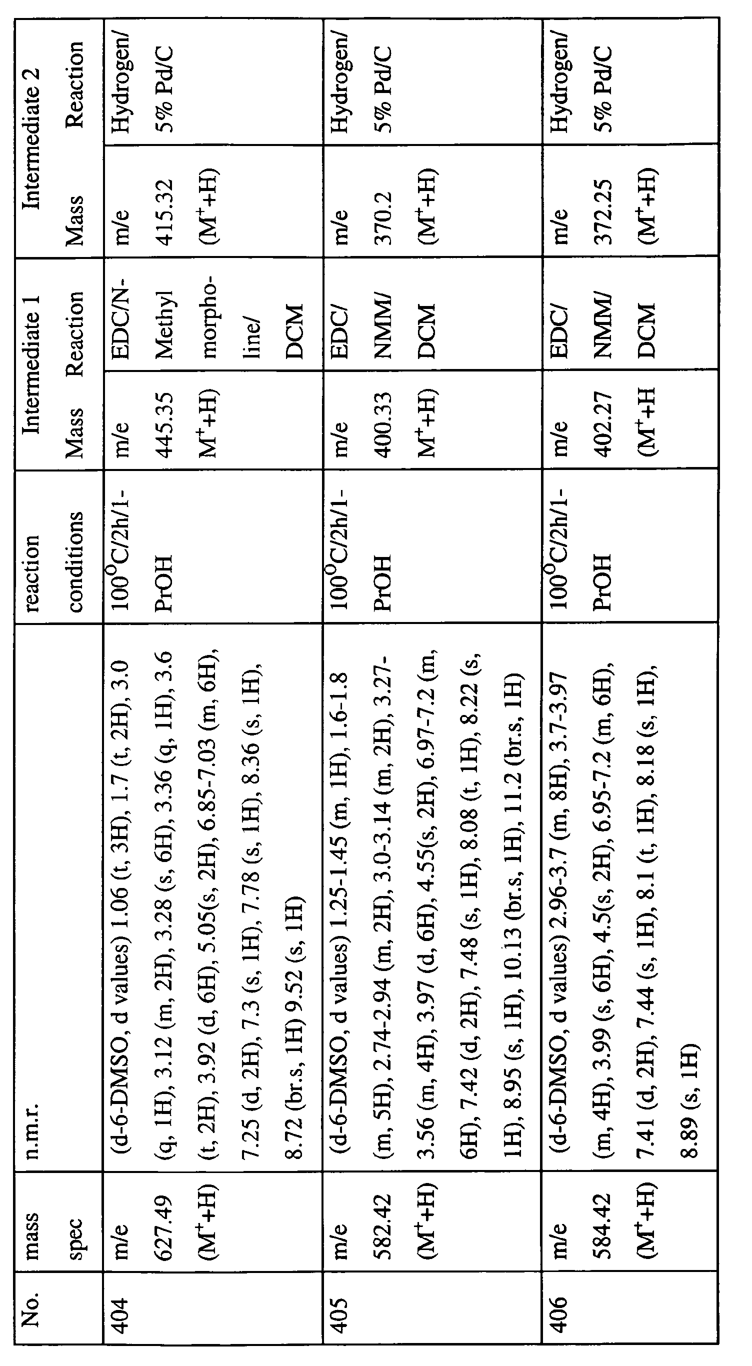

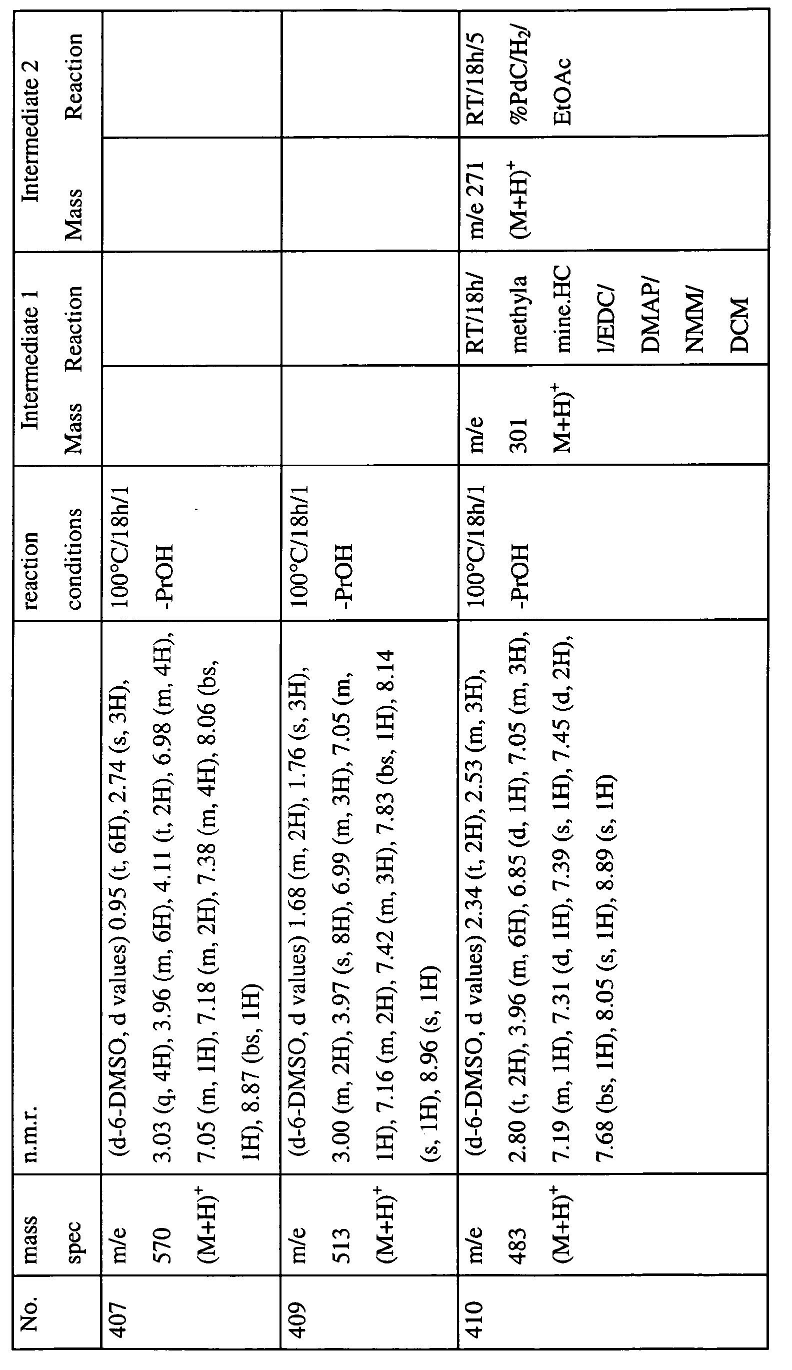

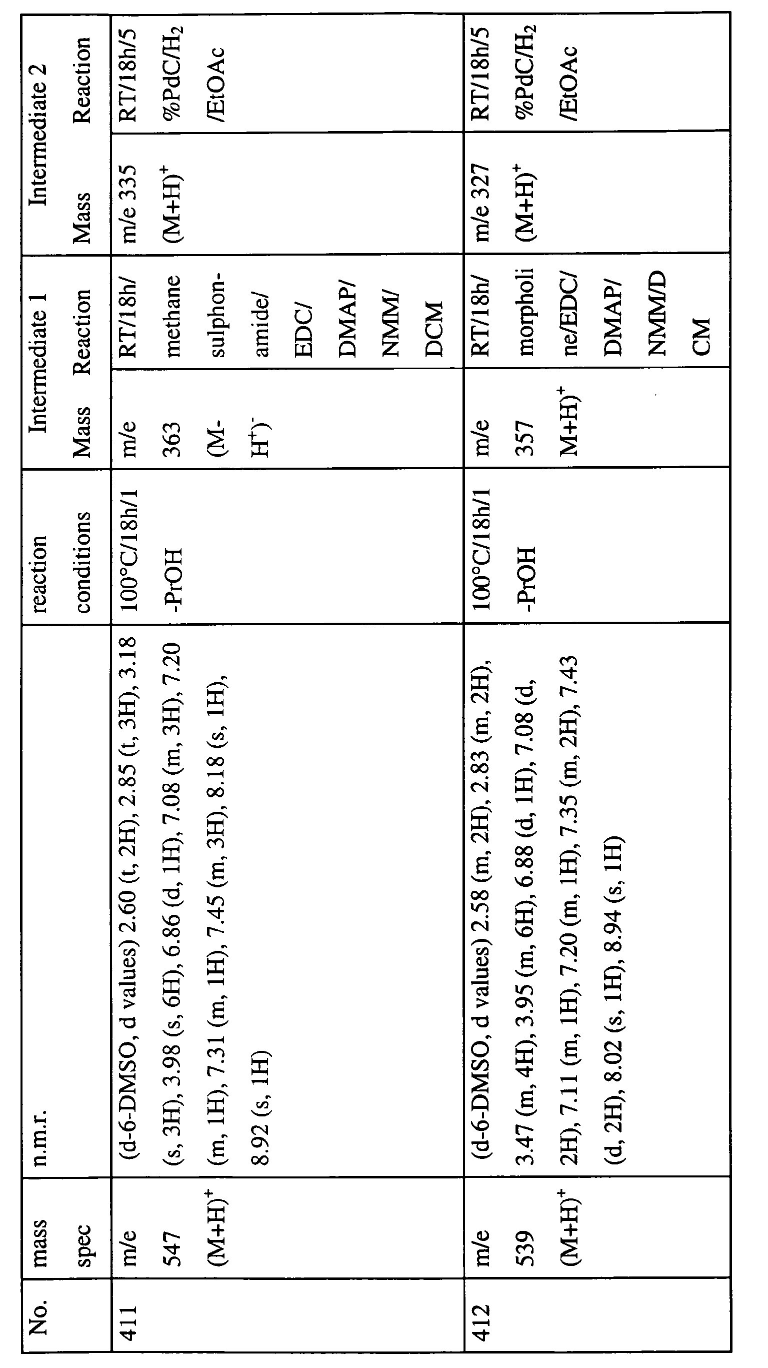

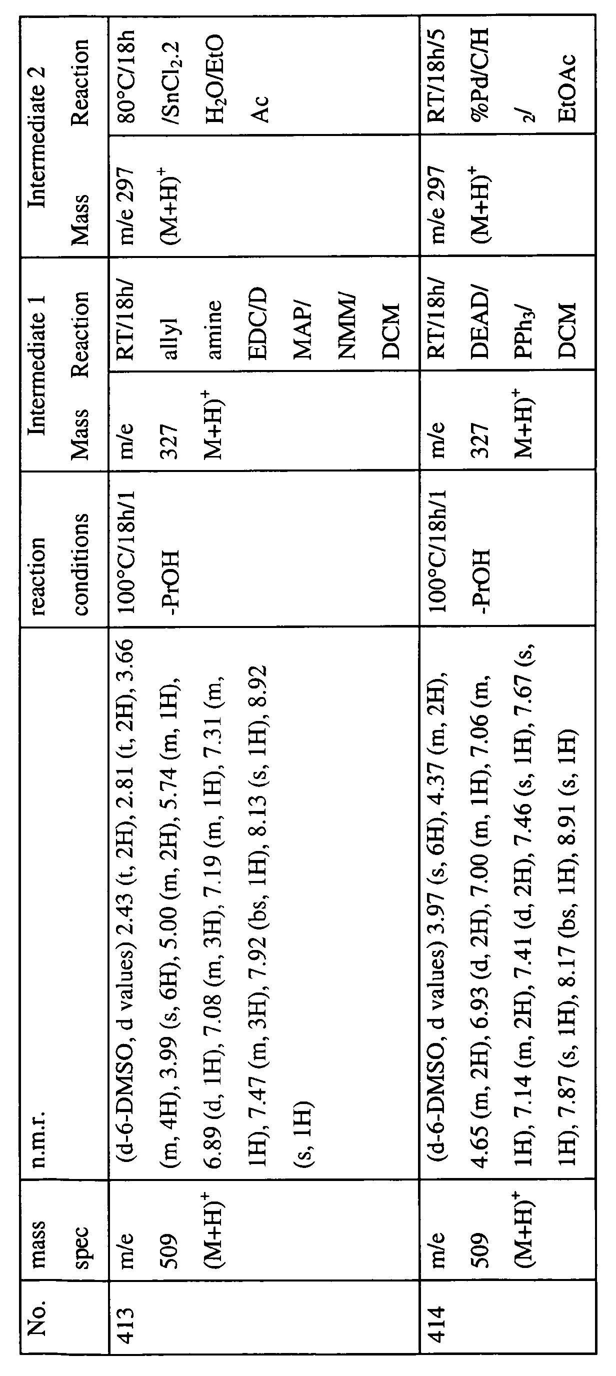

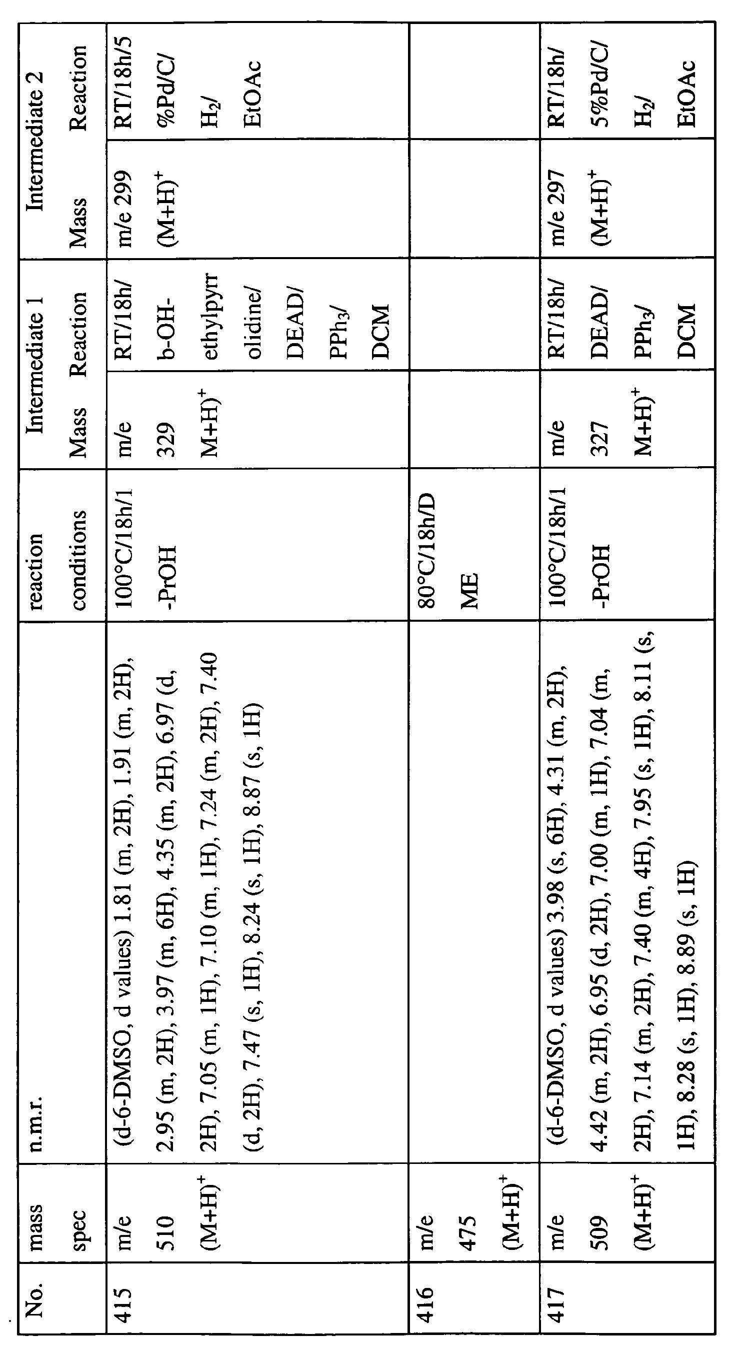

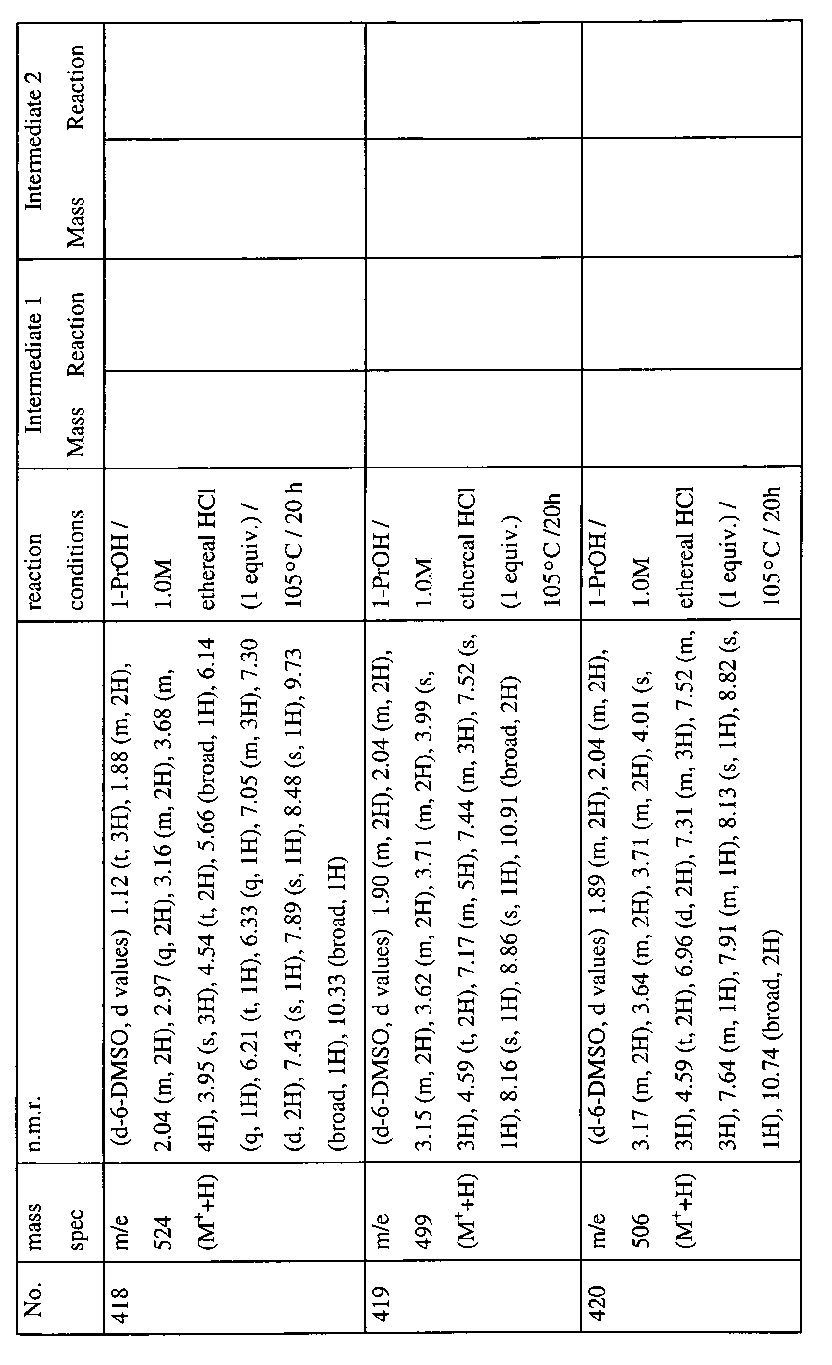

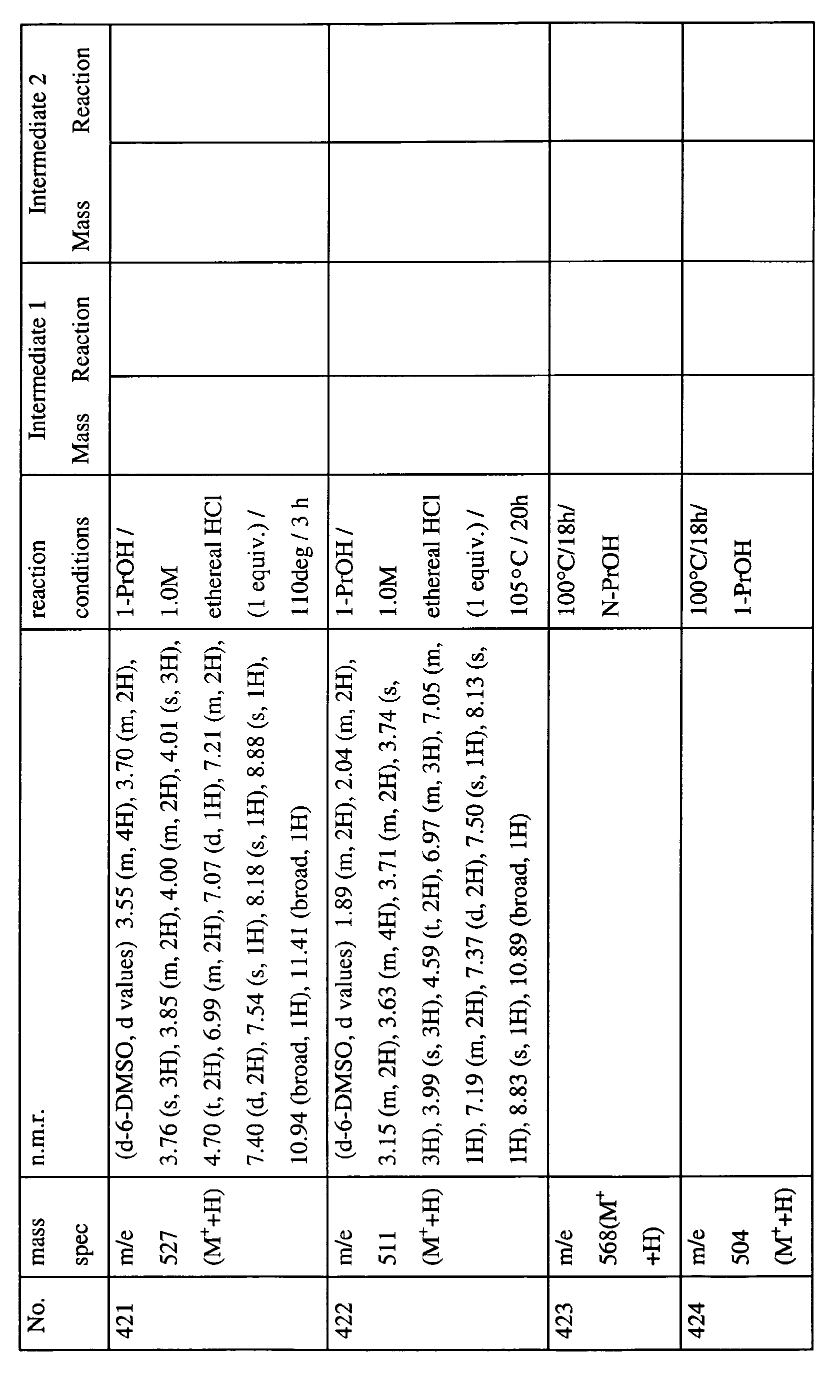

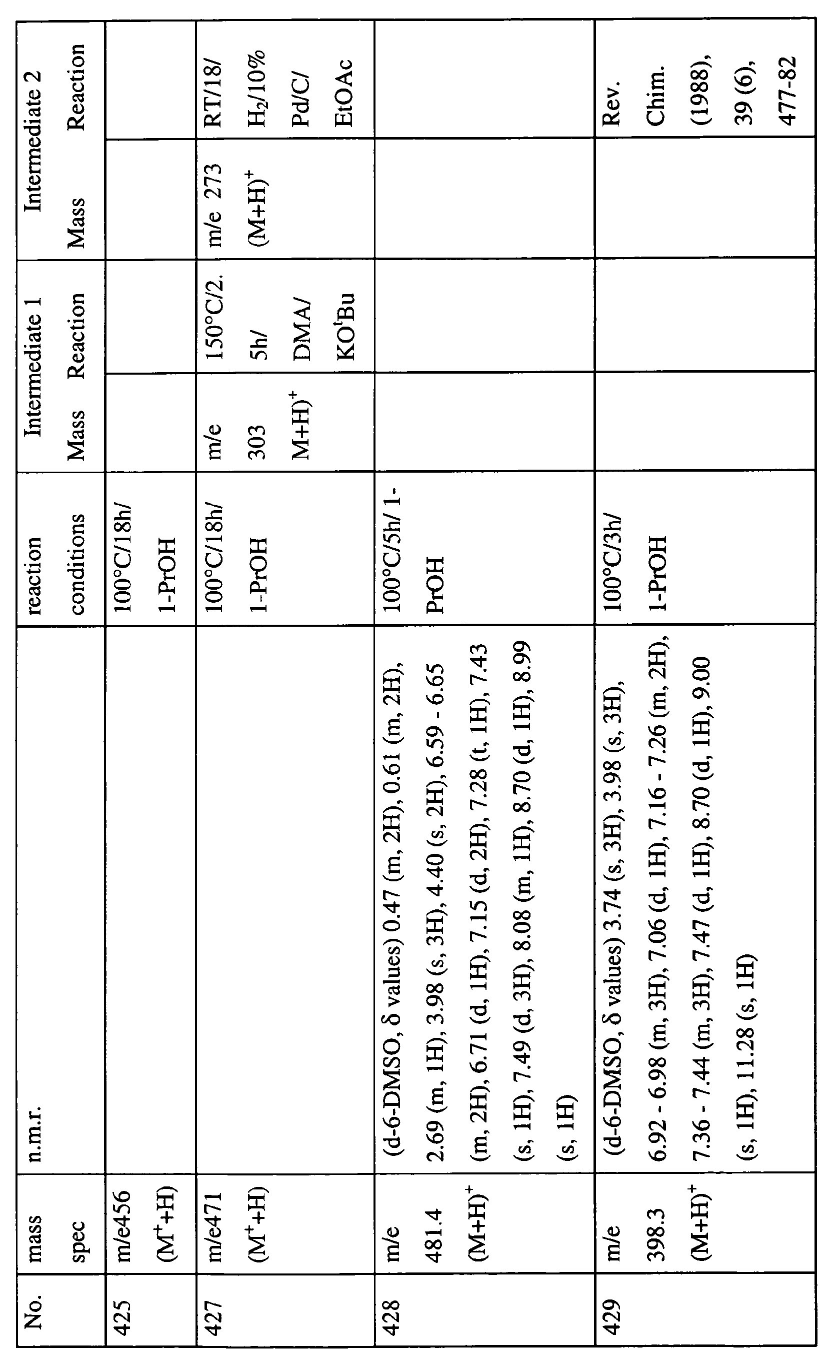

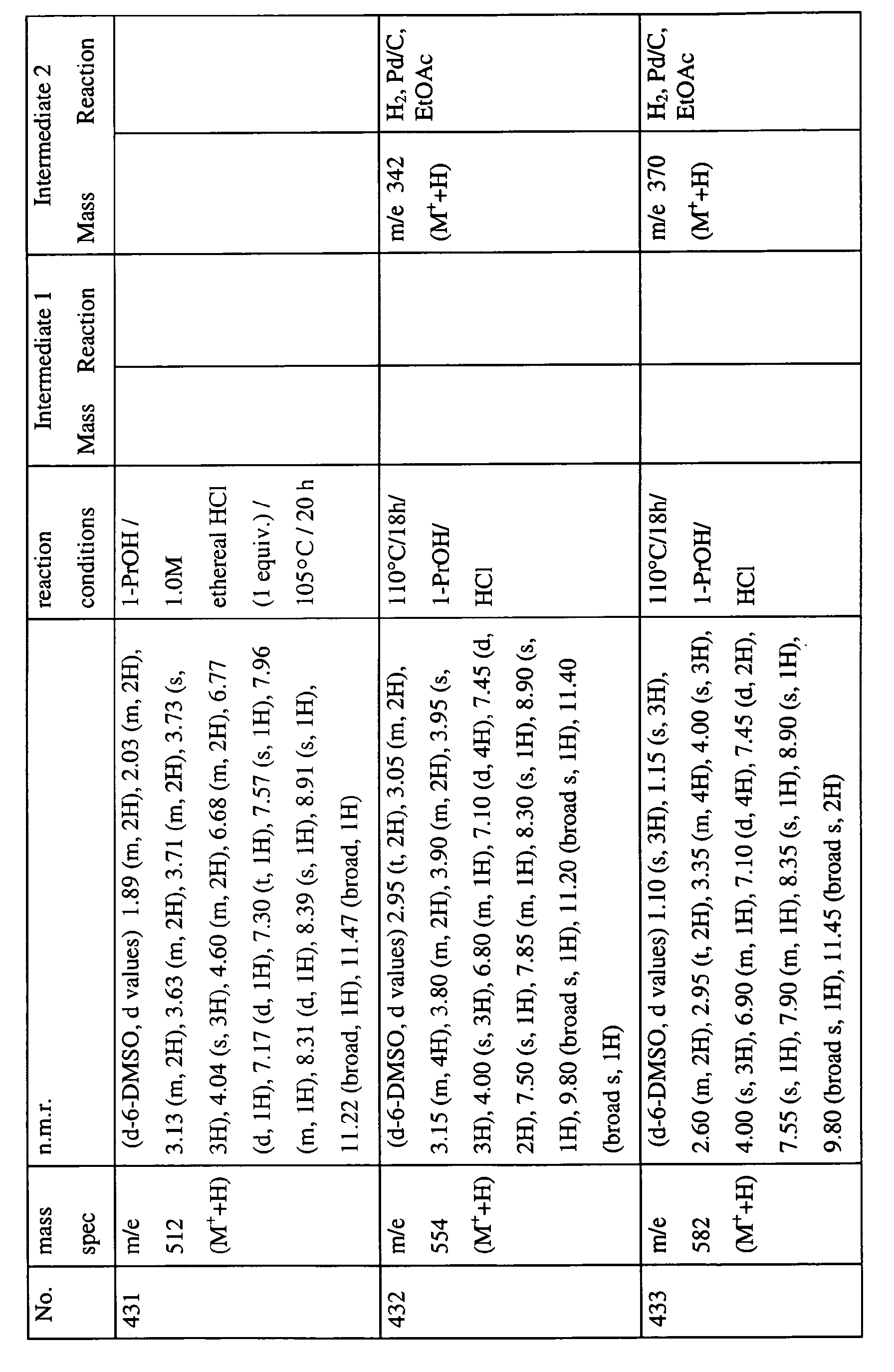

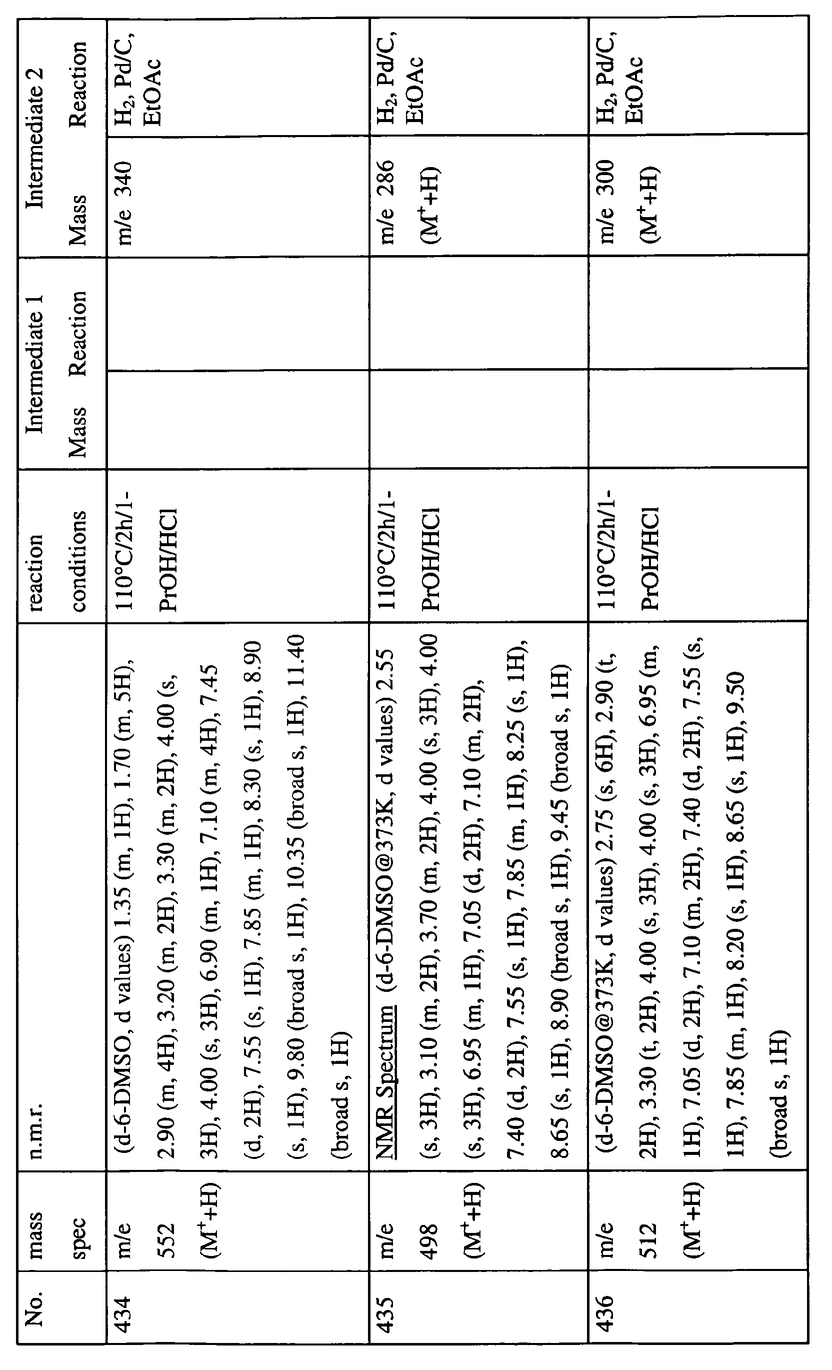

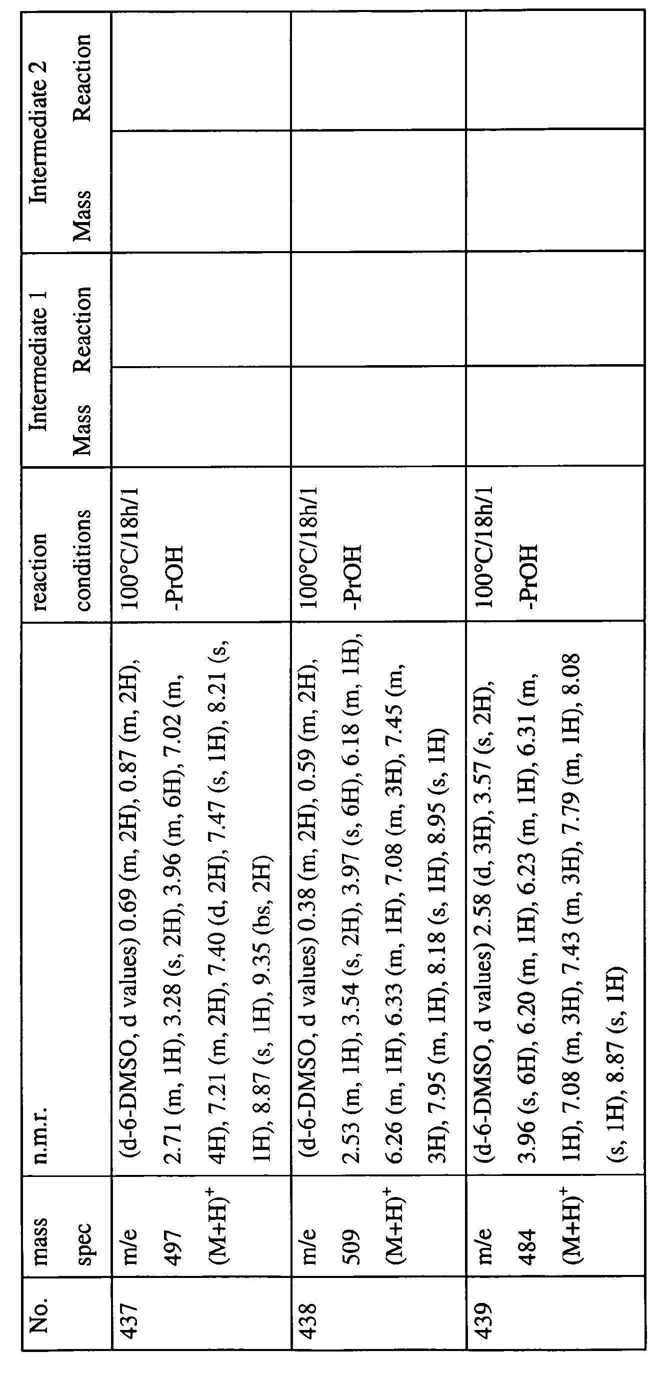

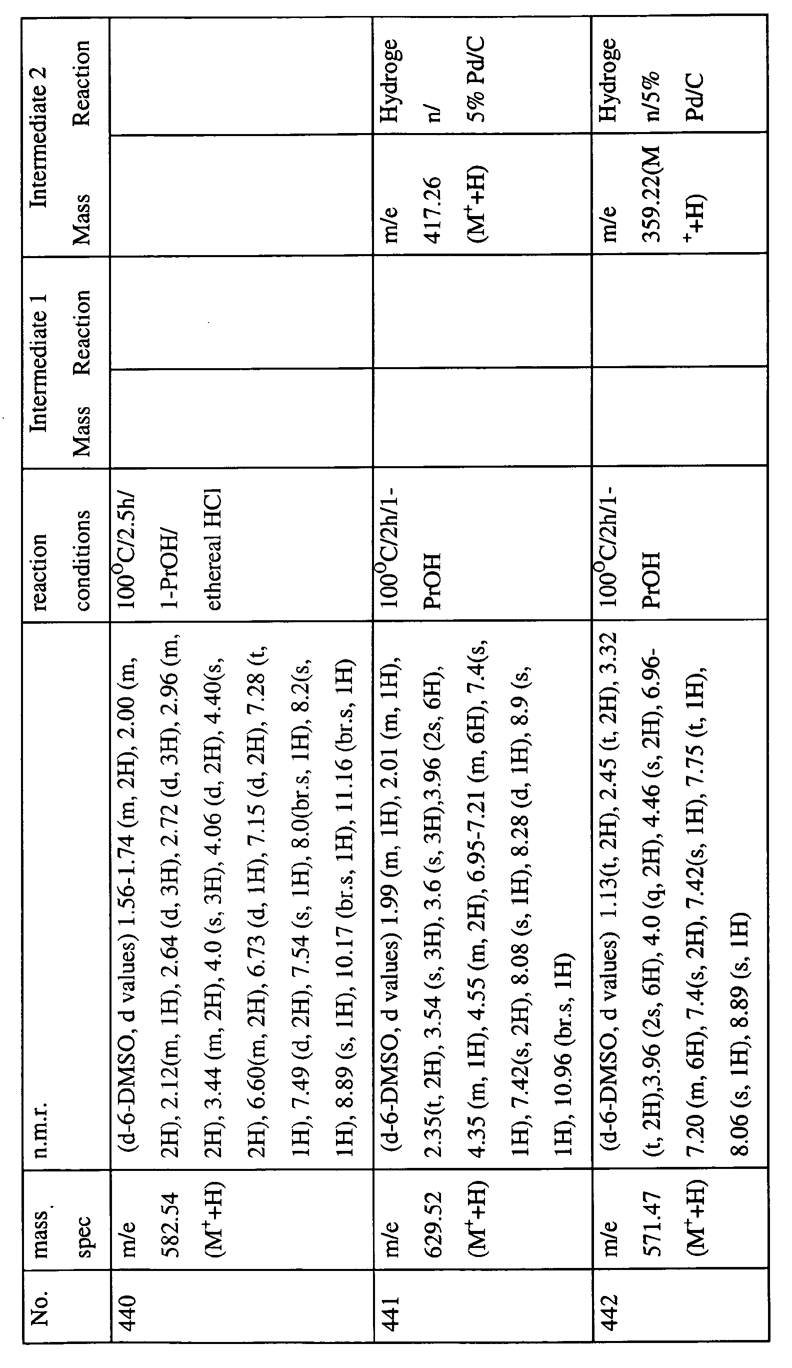

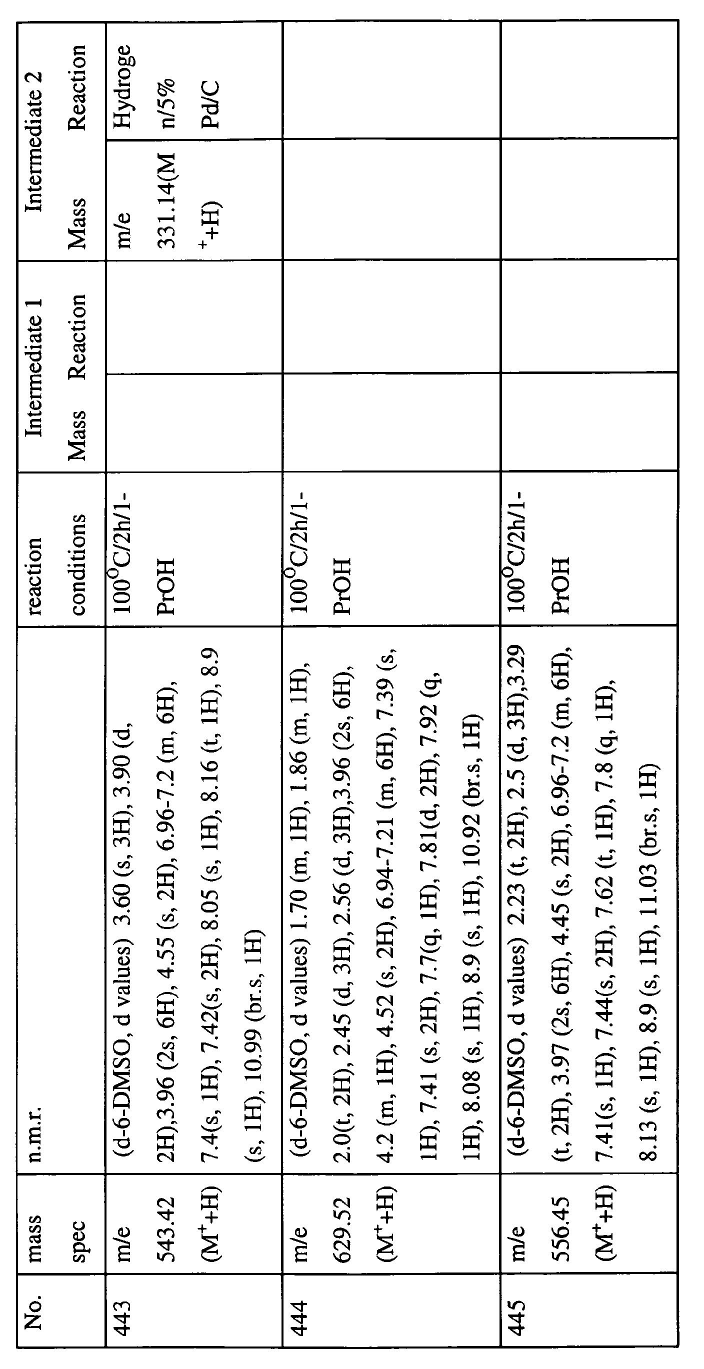

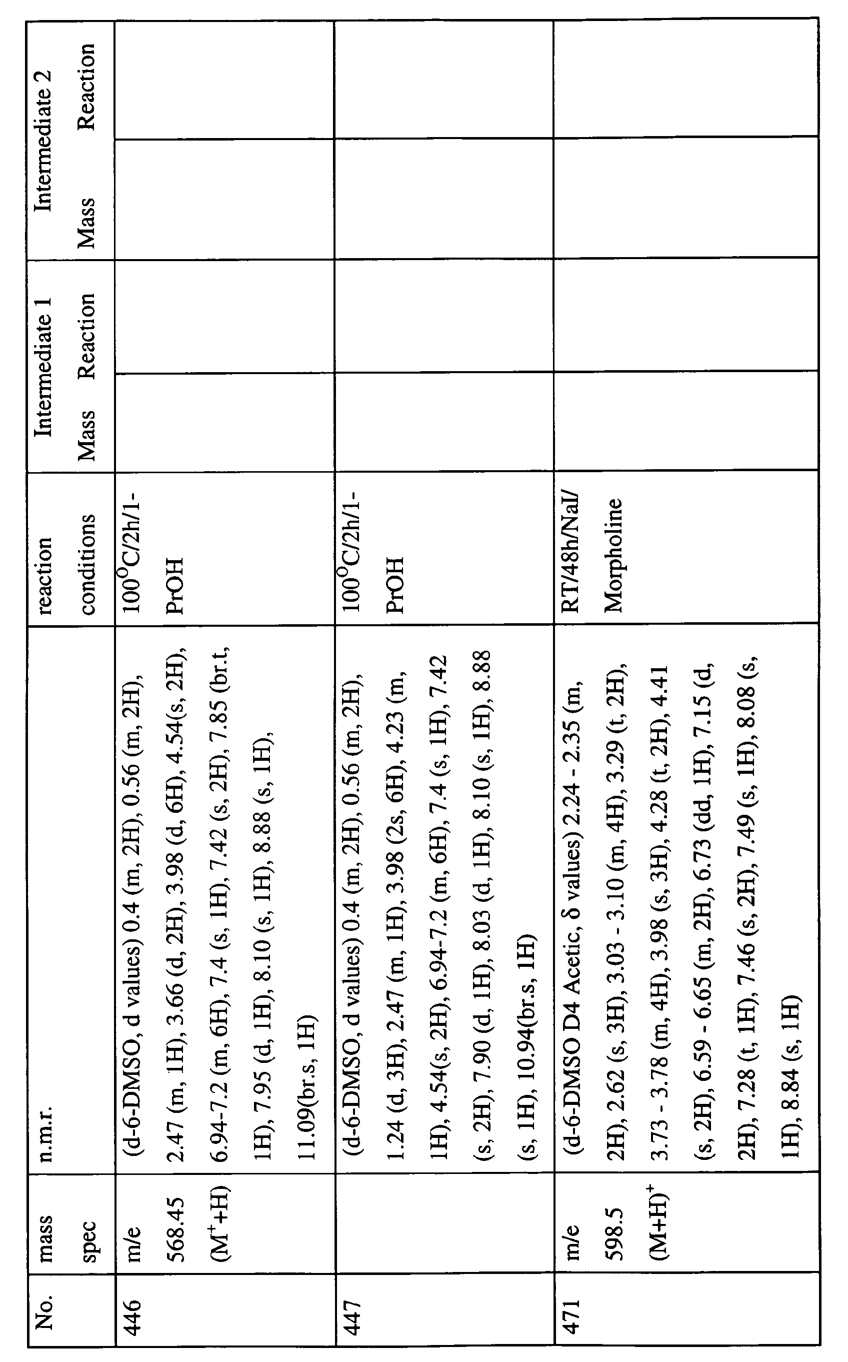

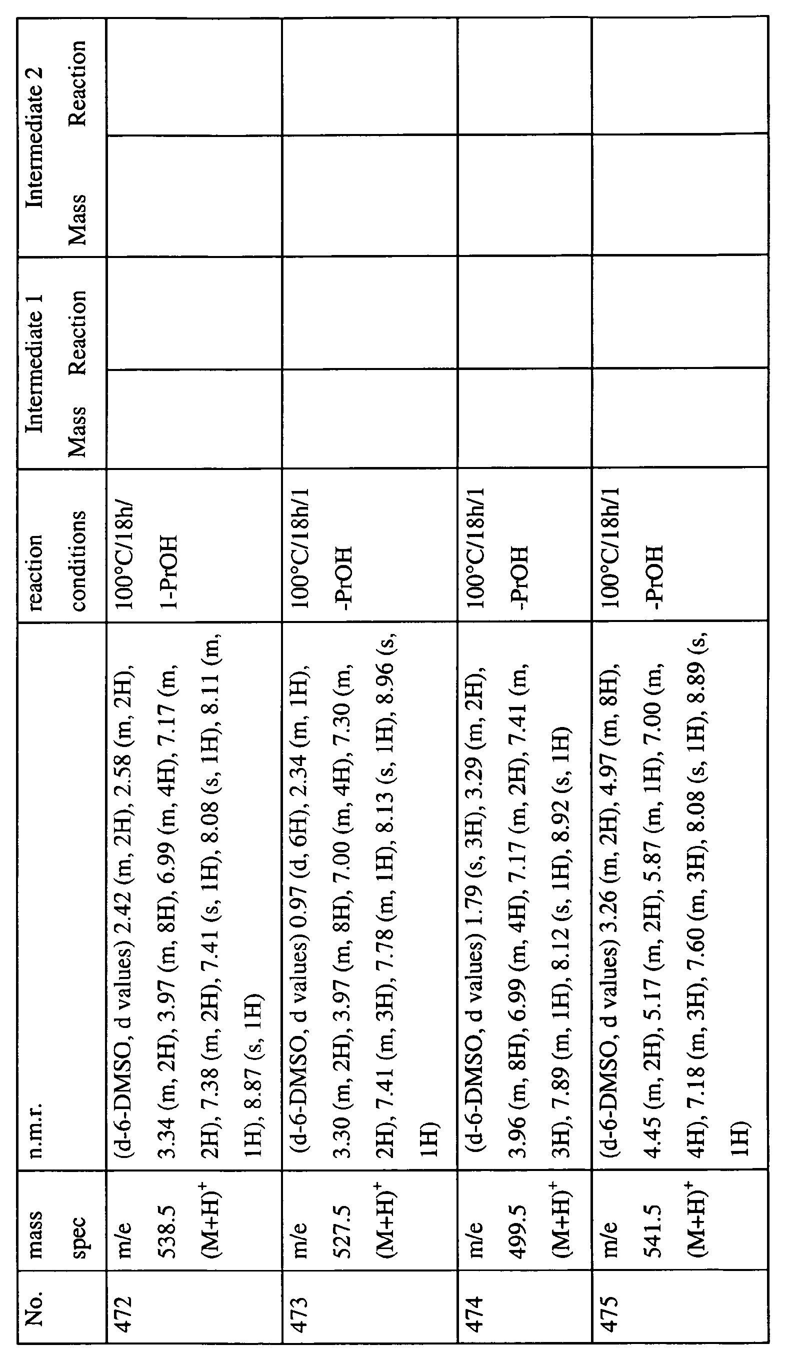

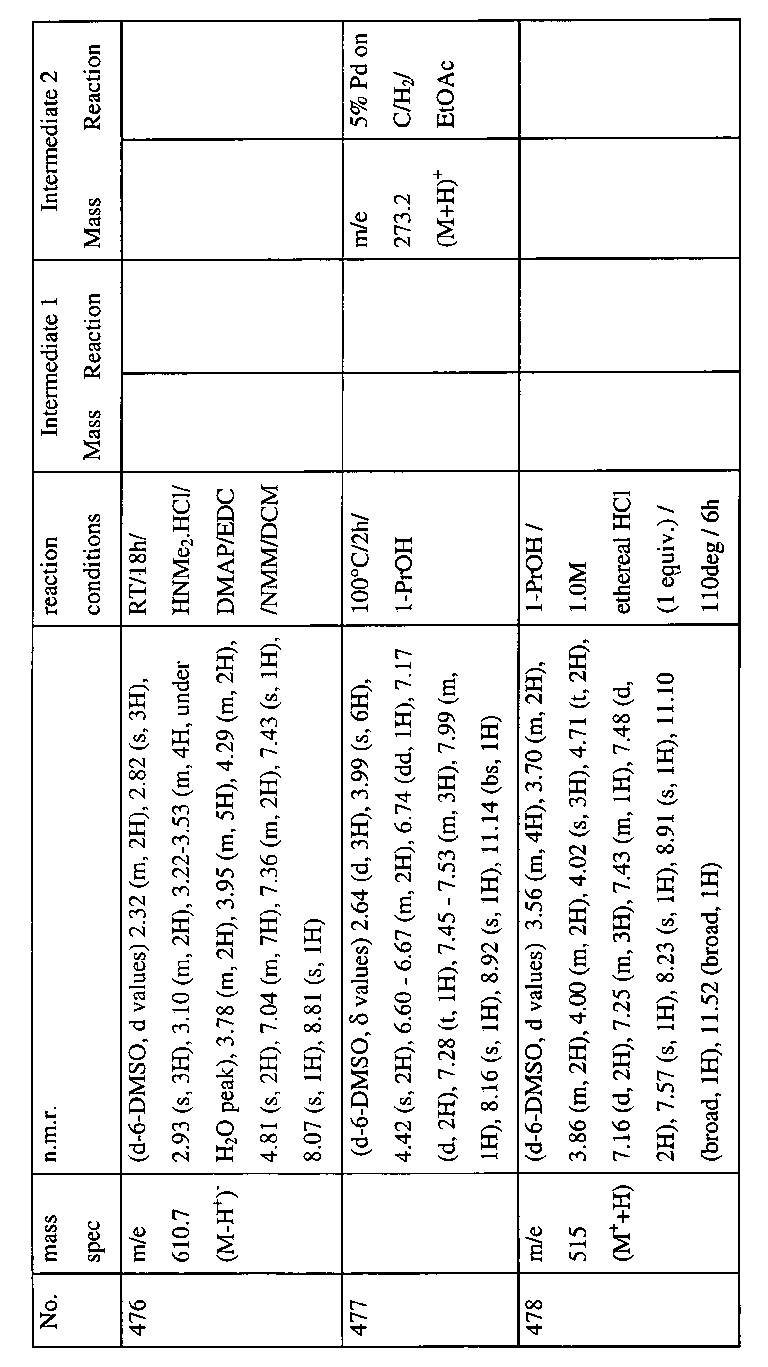

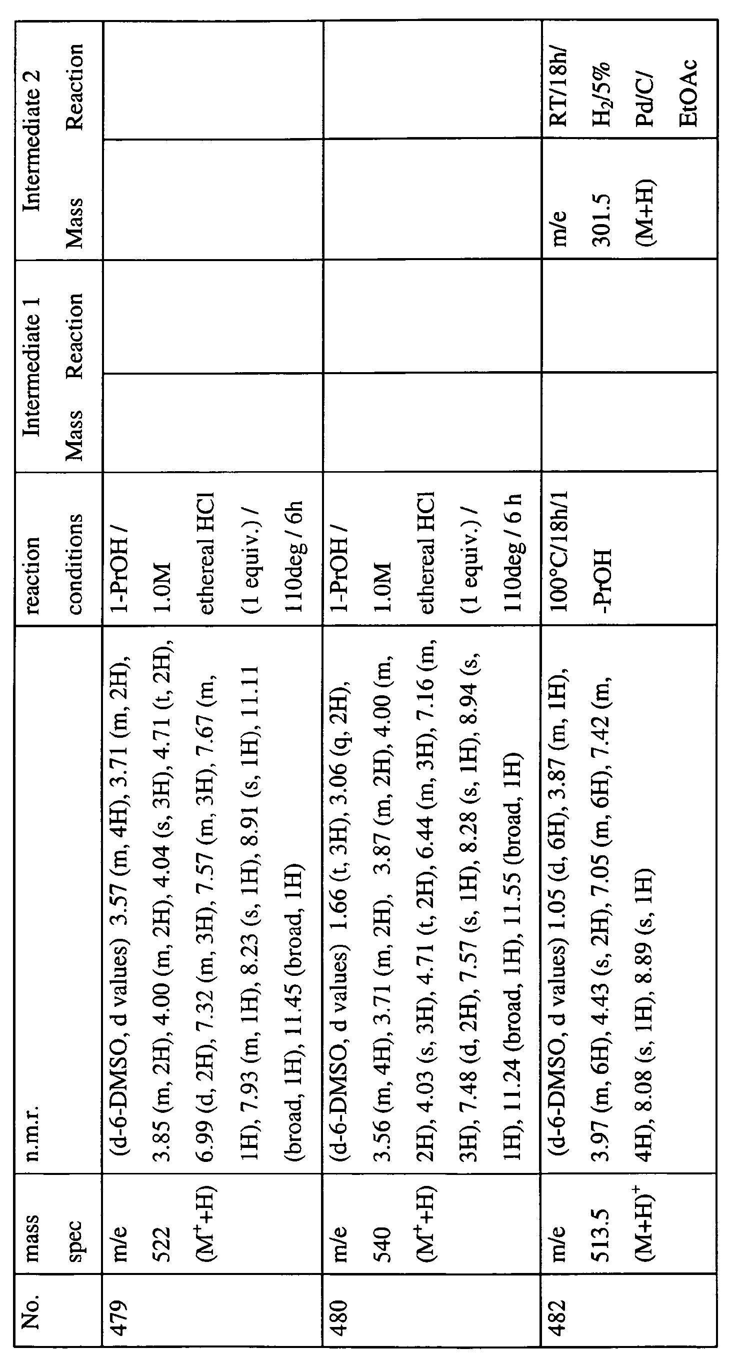

In the above and other Examples, the following abbreviations have been been used:
- ¹H NMR data is quoted and is in the form of delta values for major diagnostic protons, given in parts per million (ppm) relative to tetramethylsilane (TMS) as an internal standard;
- nitrogen atoms which are shown as less than trivalent are H substituted to complete the trivalency;
- the following abbreviations are used:
   - DMSO: dimethyl sulphoxide;
   - DMF: *N,N*-dimethylformamide;
   - DCM: dichloromethane;
   - EtOAc: ethyl acetate;
   - HOBT: N-hydroxybenzotriazole hydrate ;
   - NMM: N-Methylmorpholine;
   - TFA: Trifluoroacetic acid;
   - 1-Pr-OH: propan-1-ol;
   - MeOH: methanol;
   - EtOH: ethanol;
   - KOtBu: potassium tert-butoxide;
   - RT: room/ambient temperature.

### Example 6

Compounds of formula (I) were also converted to different such compounds by reacting appropriate derivatisation reactions, either directly or by way of certain chloro substituted intermediates. These can be summarised in the following Table 8 with the Intermediates listed in the Intermediate Table 9 below.

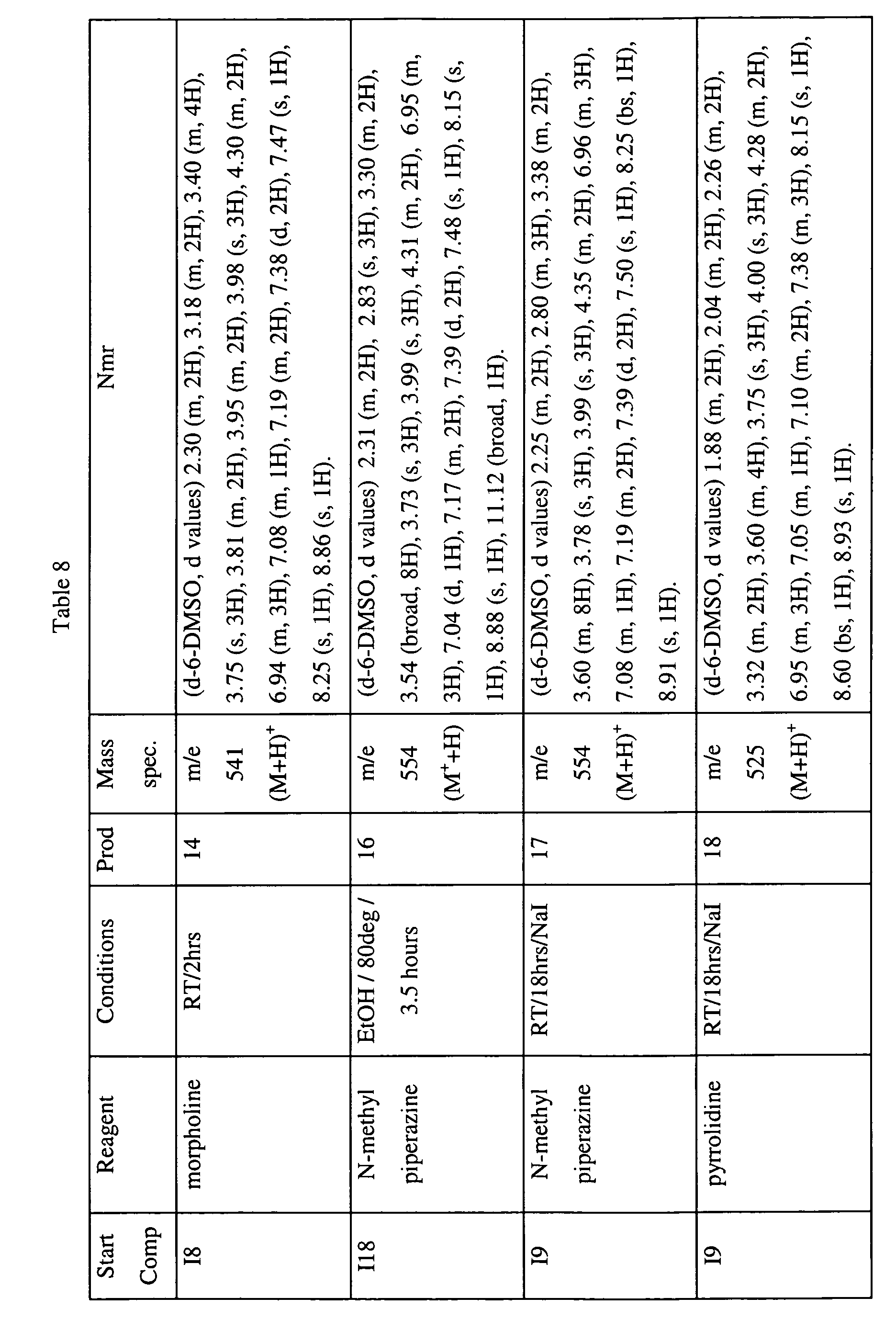

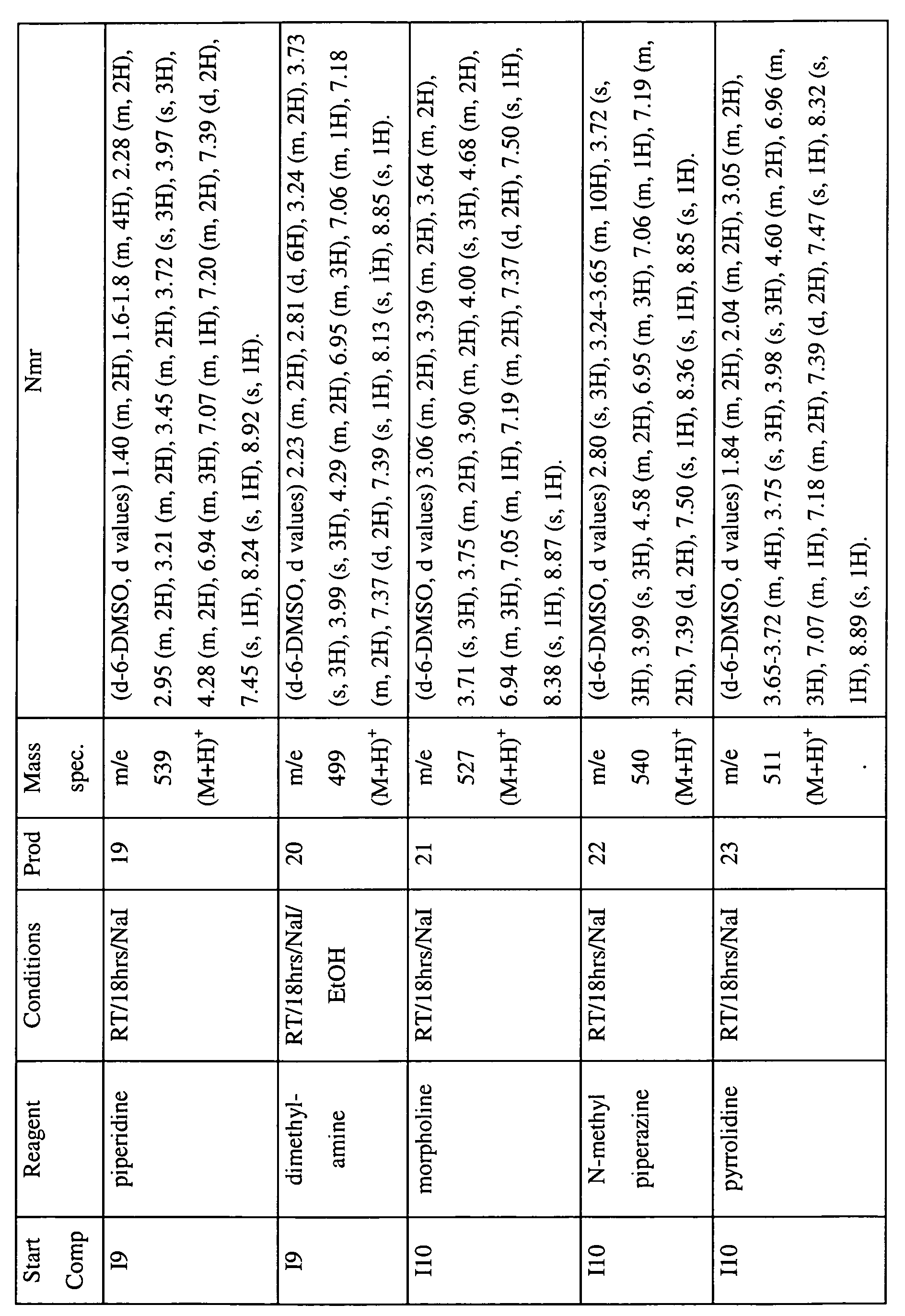

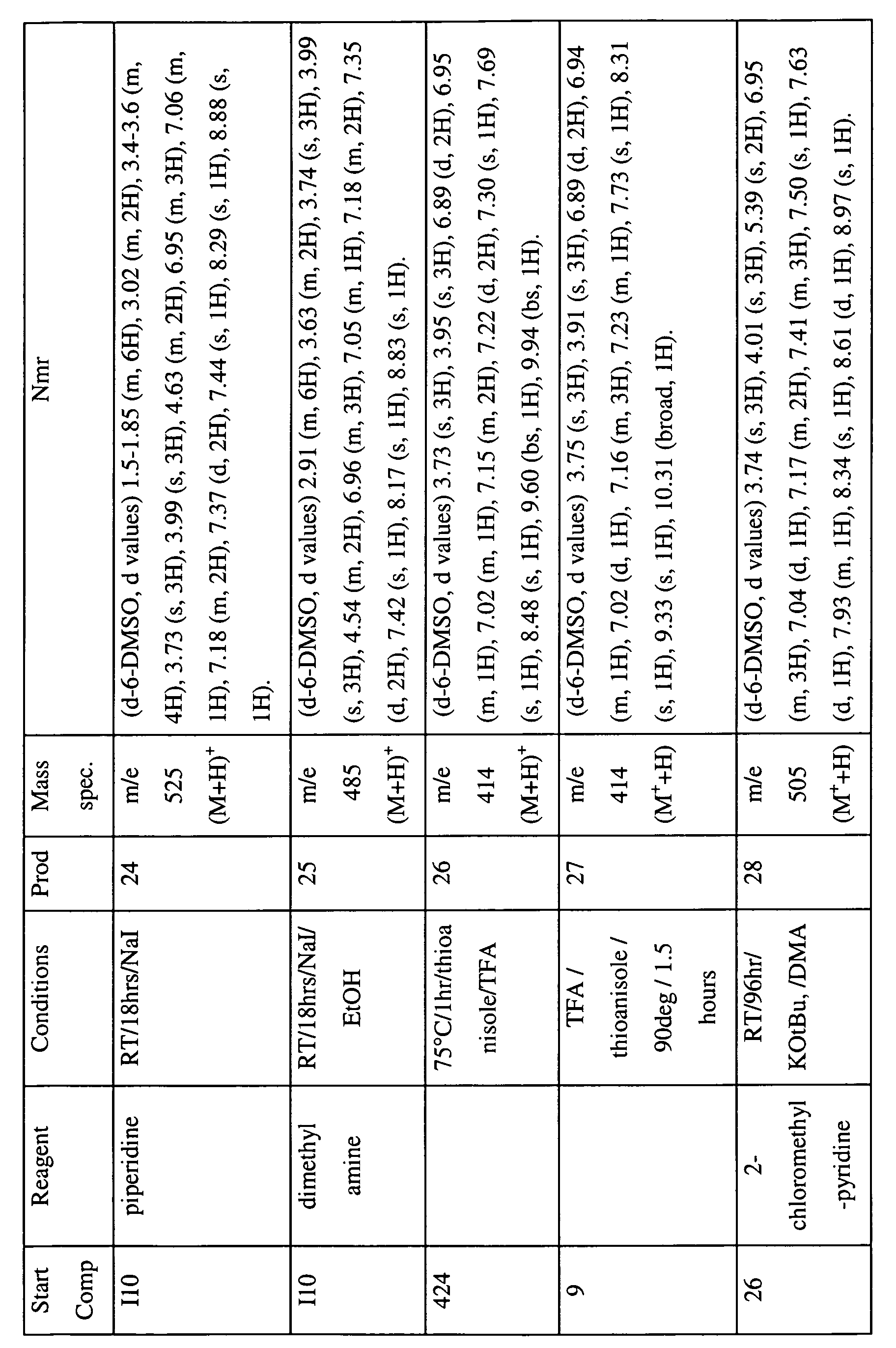

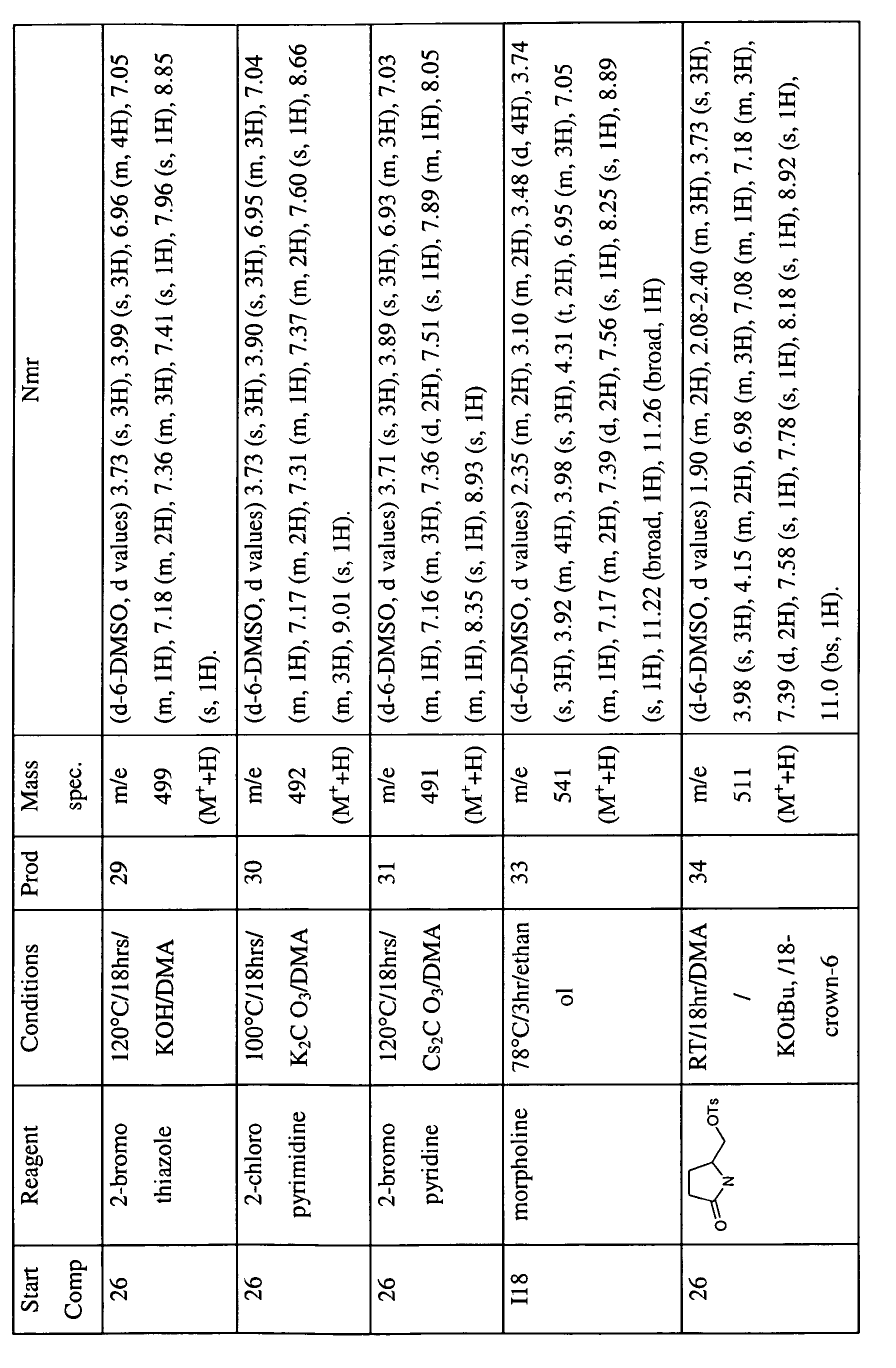

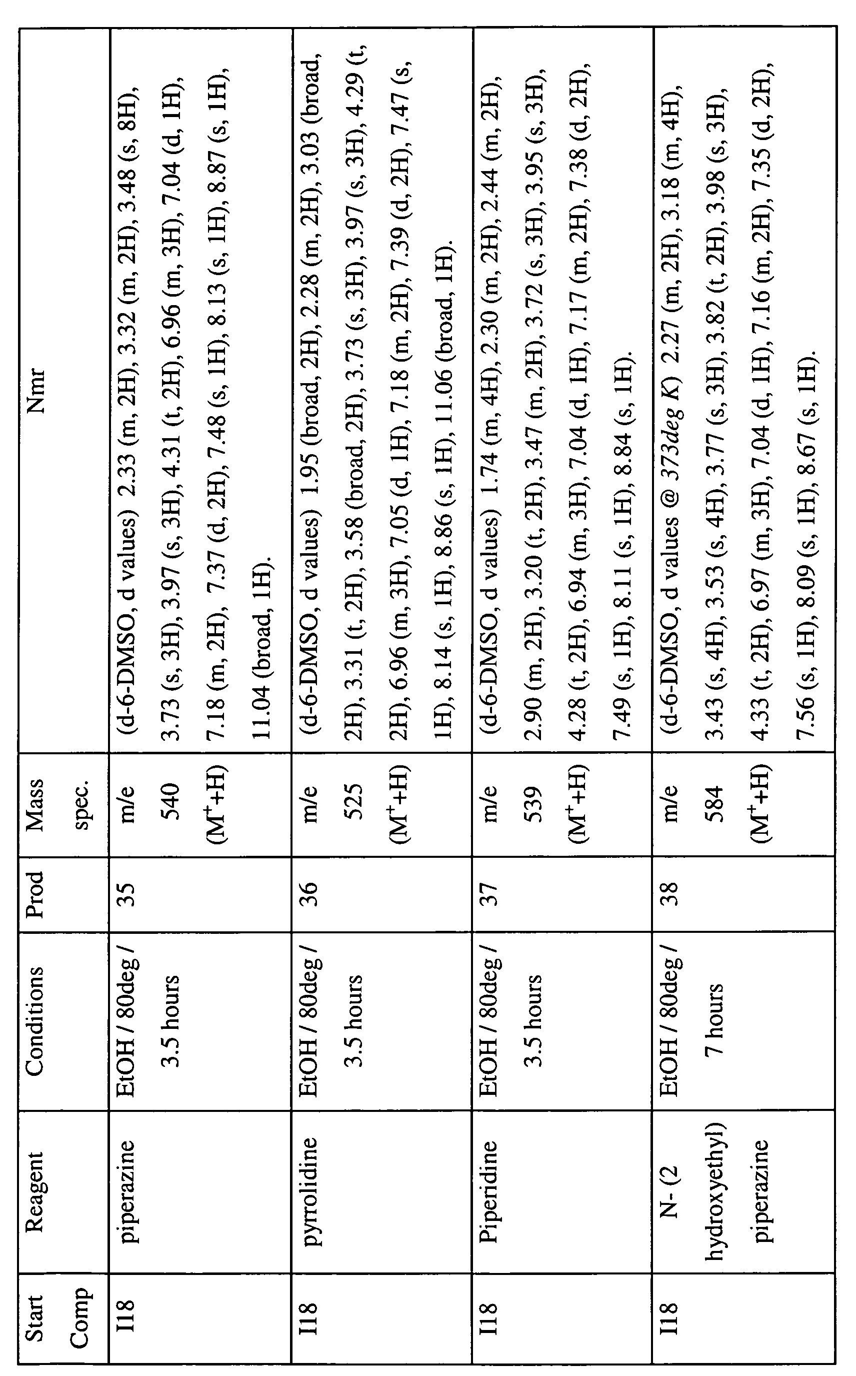

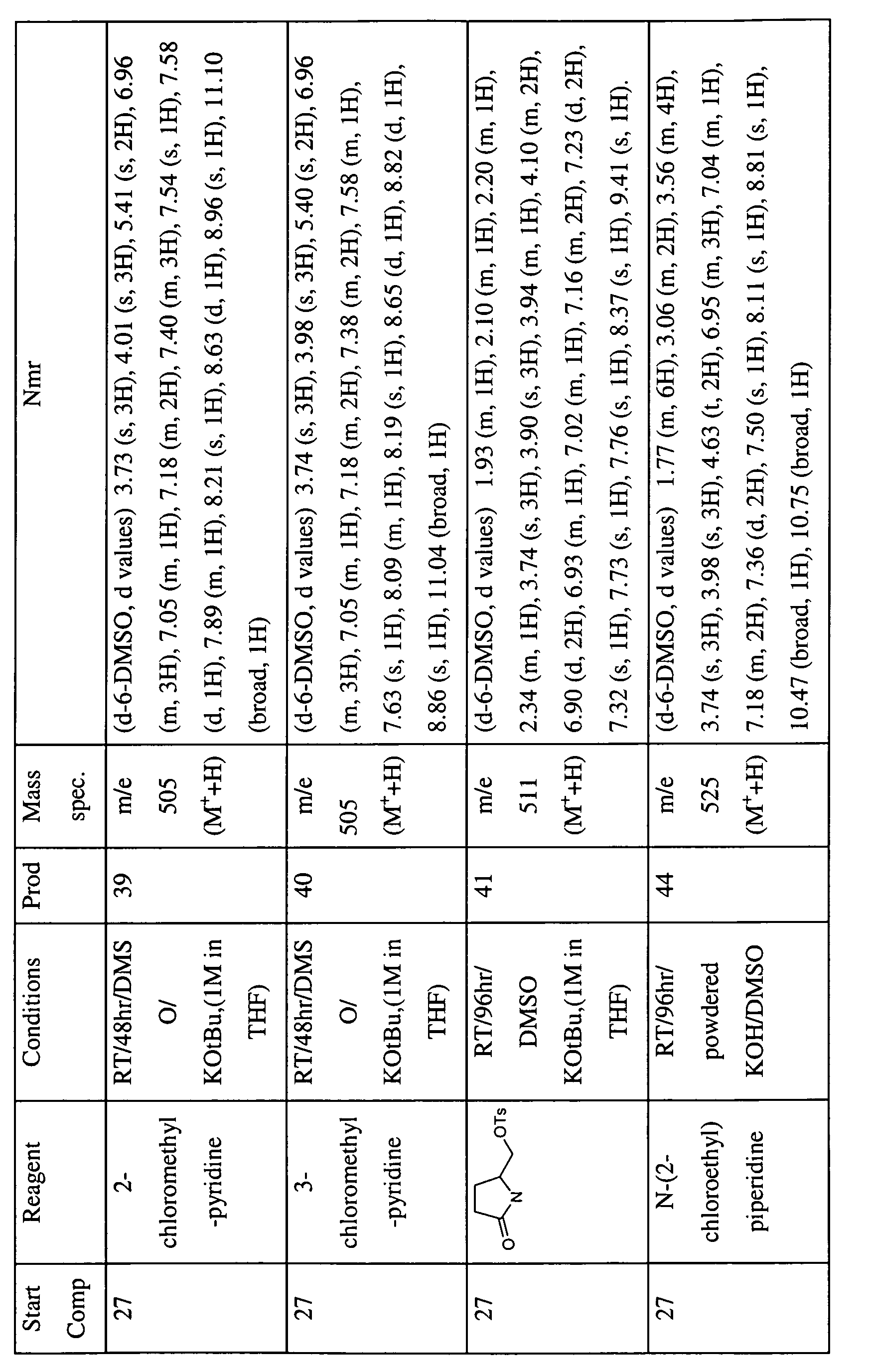

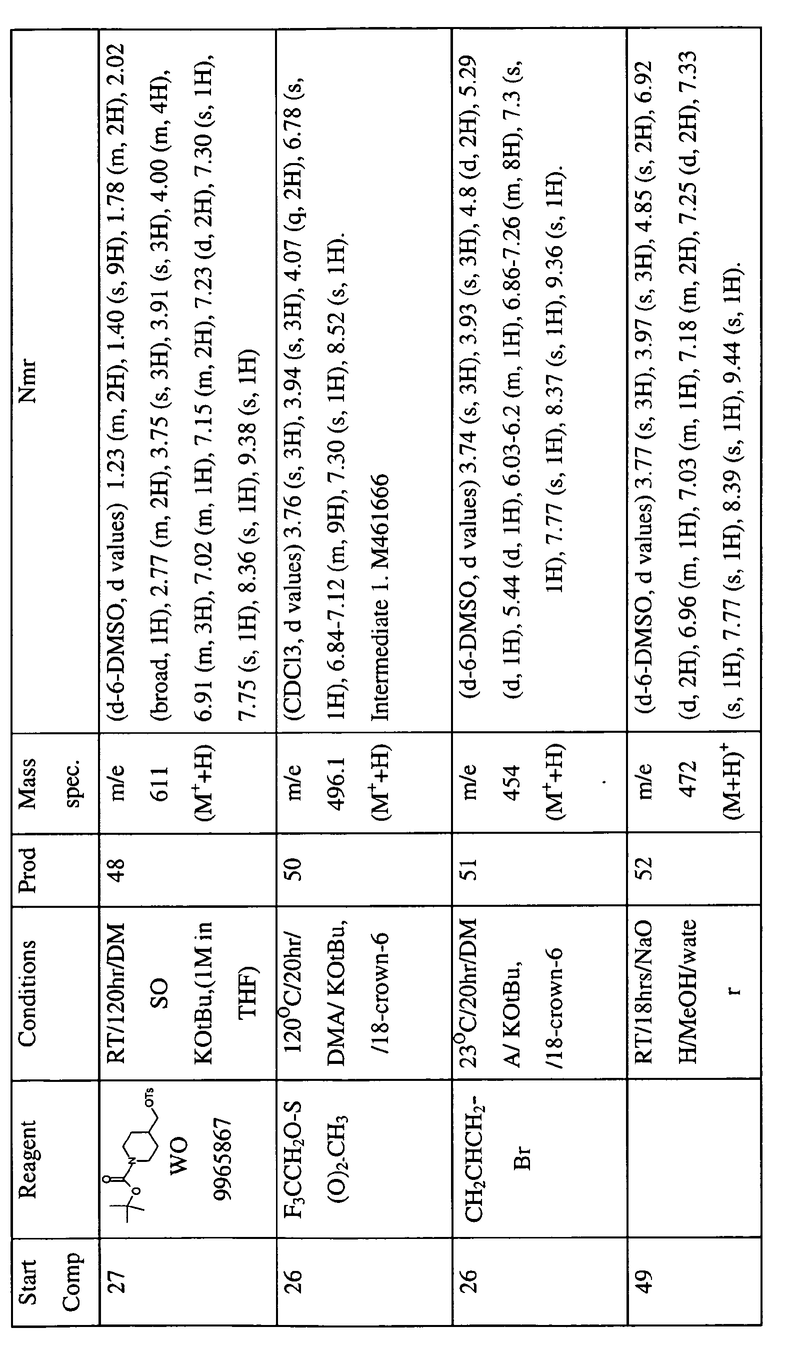

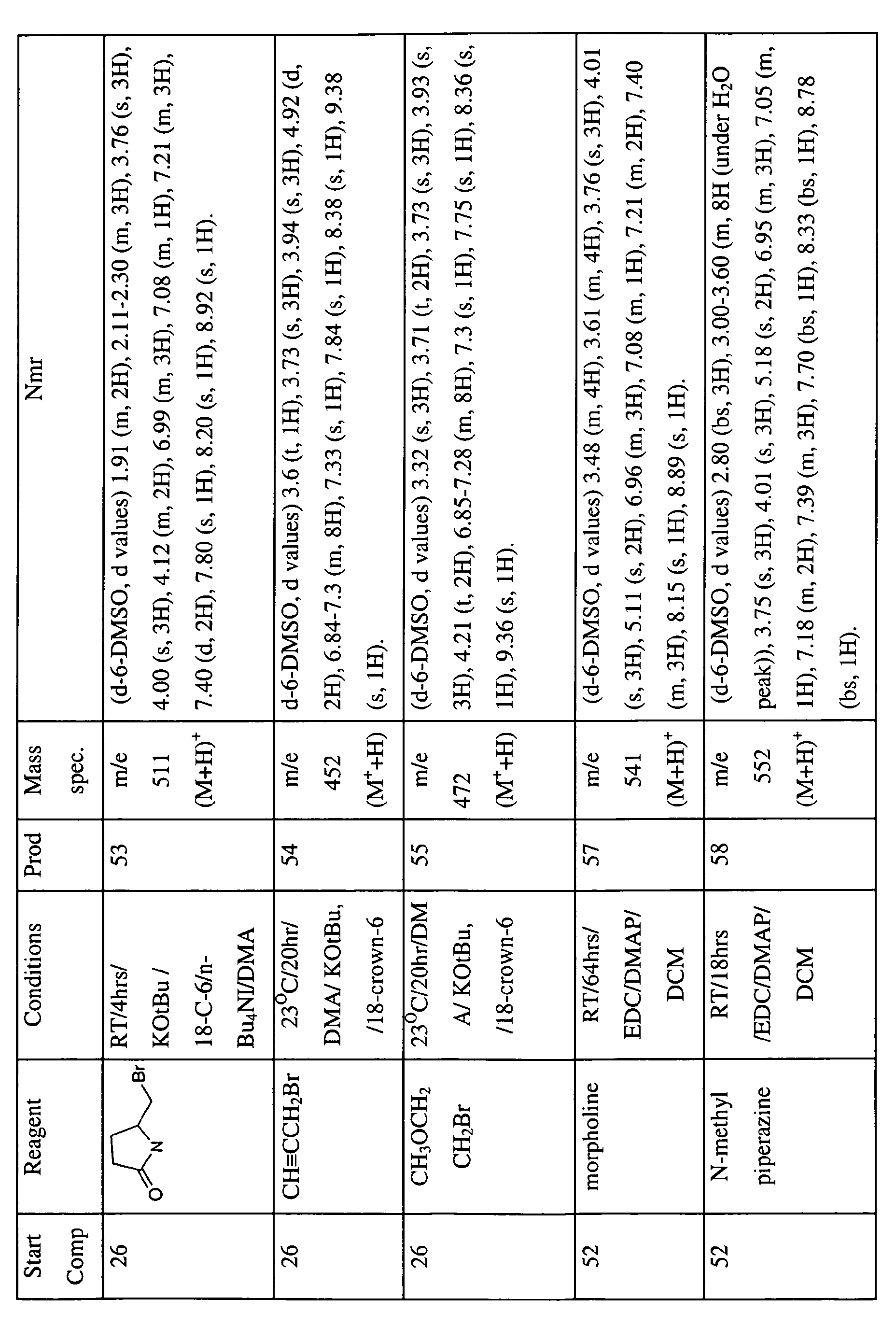

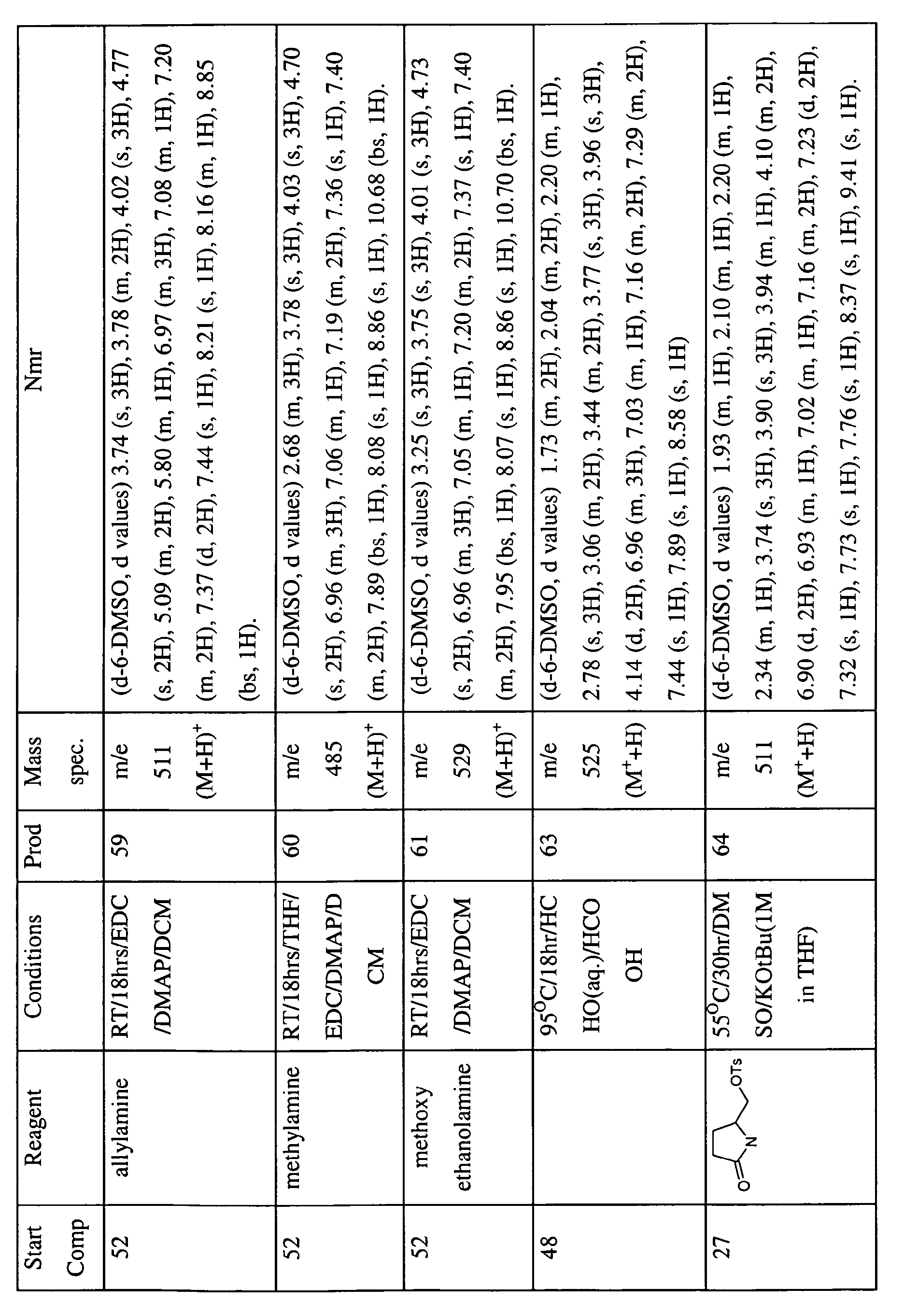

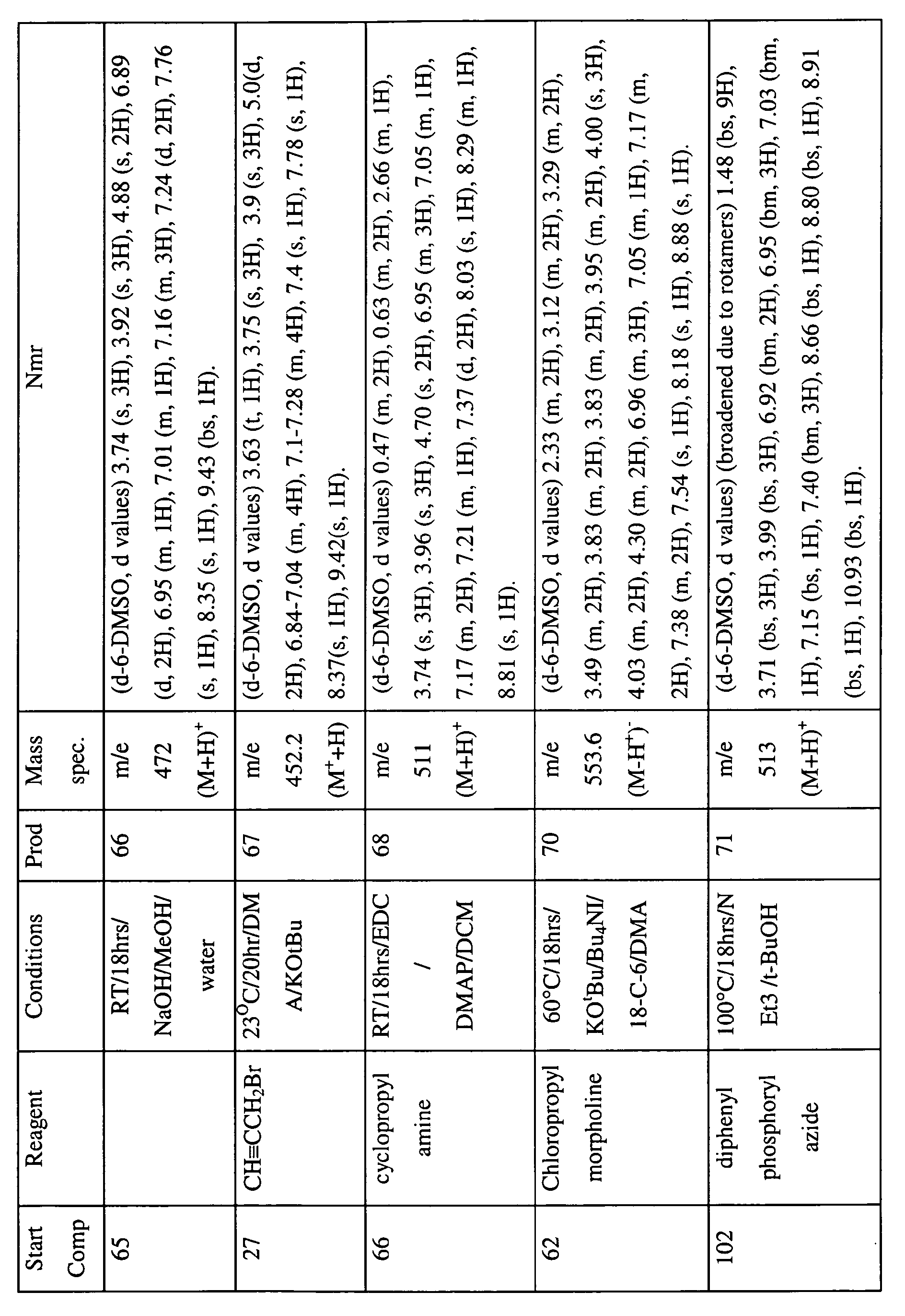

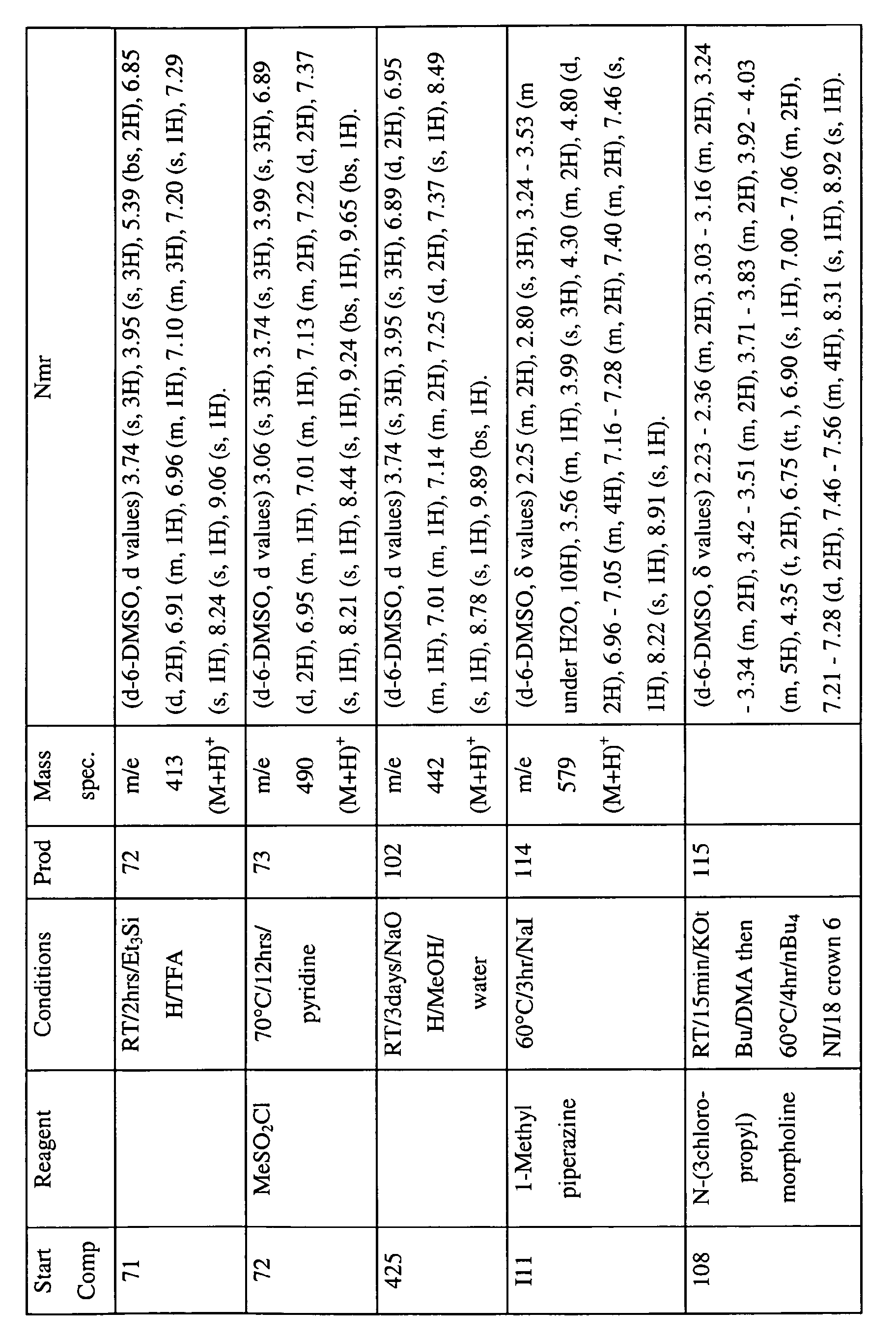

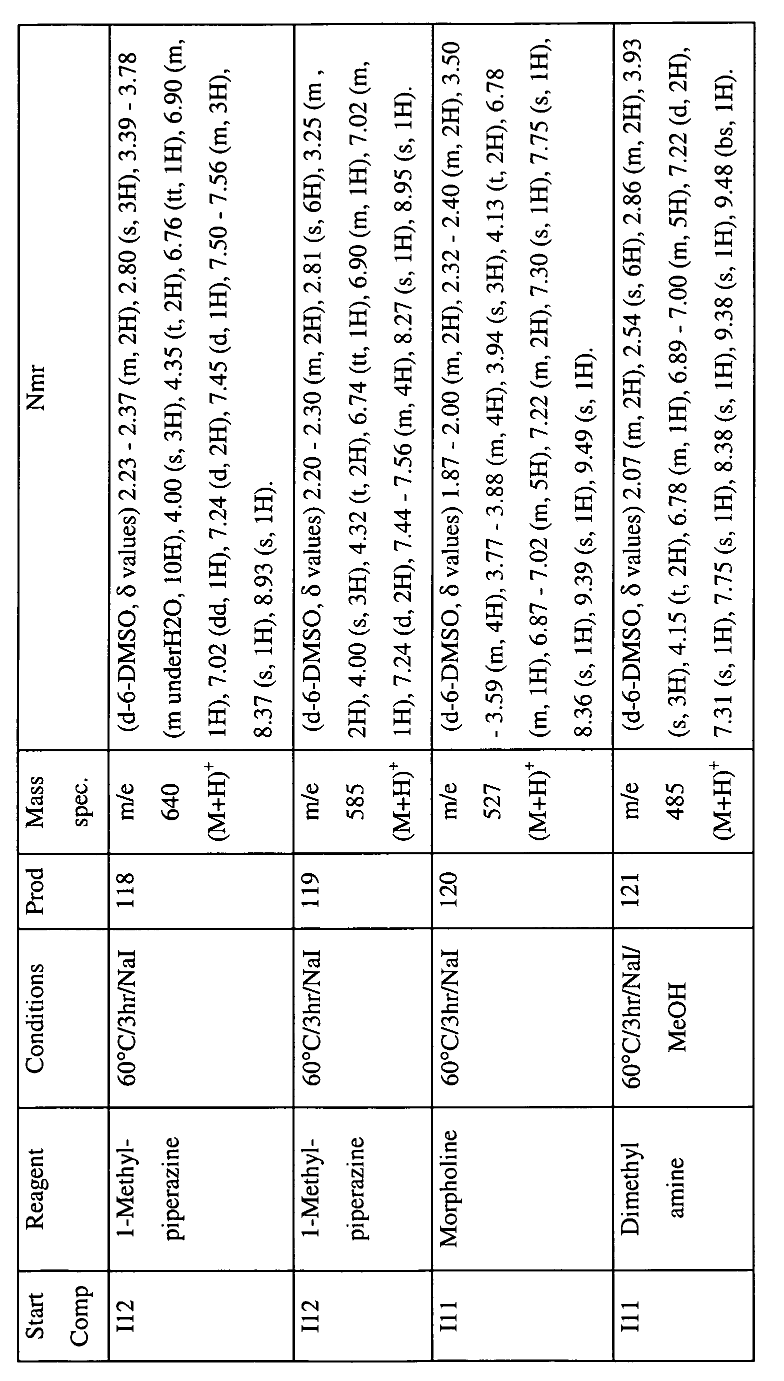

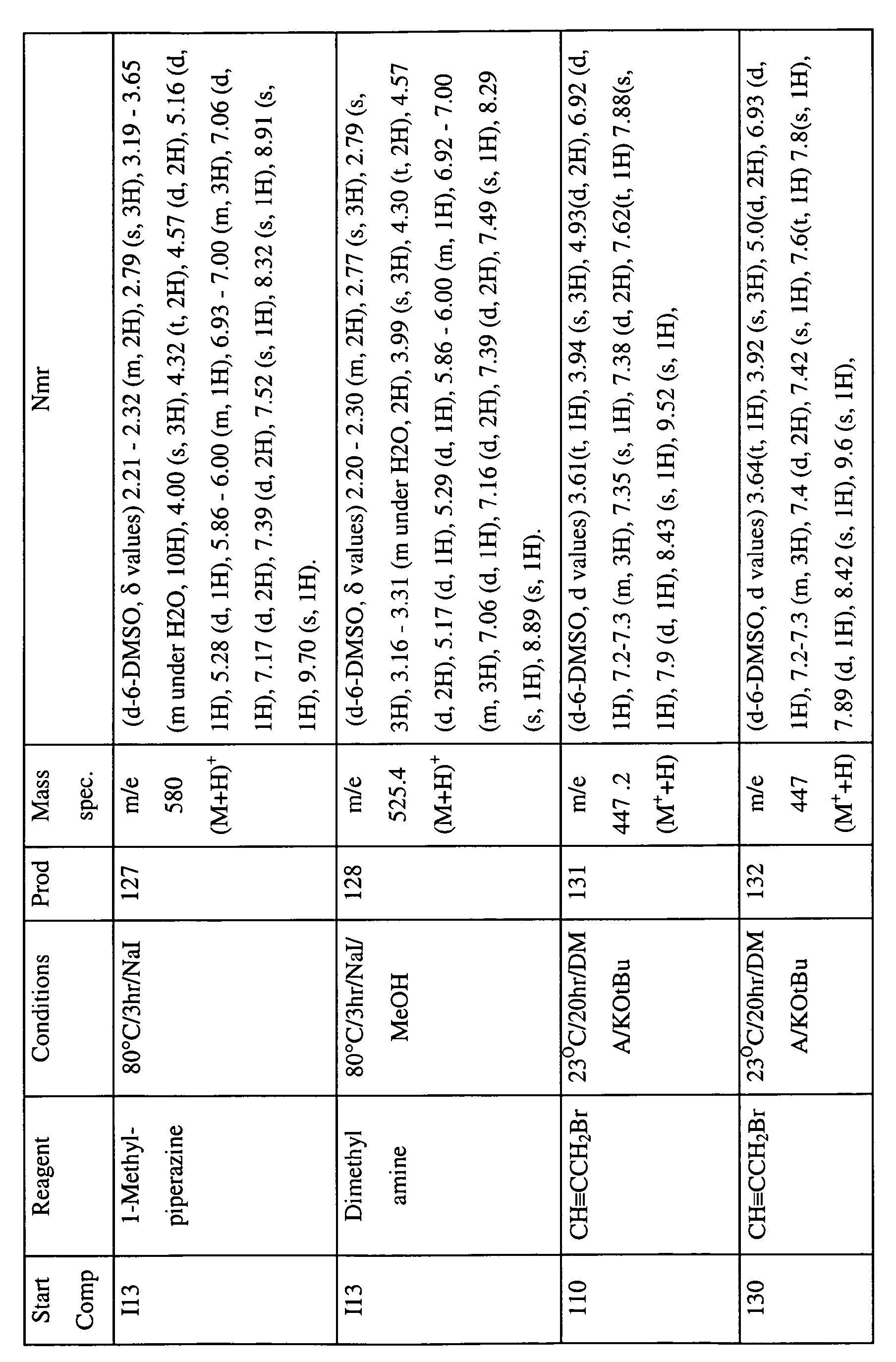

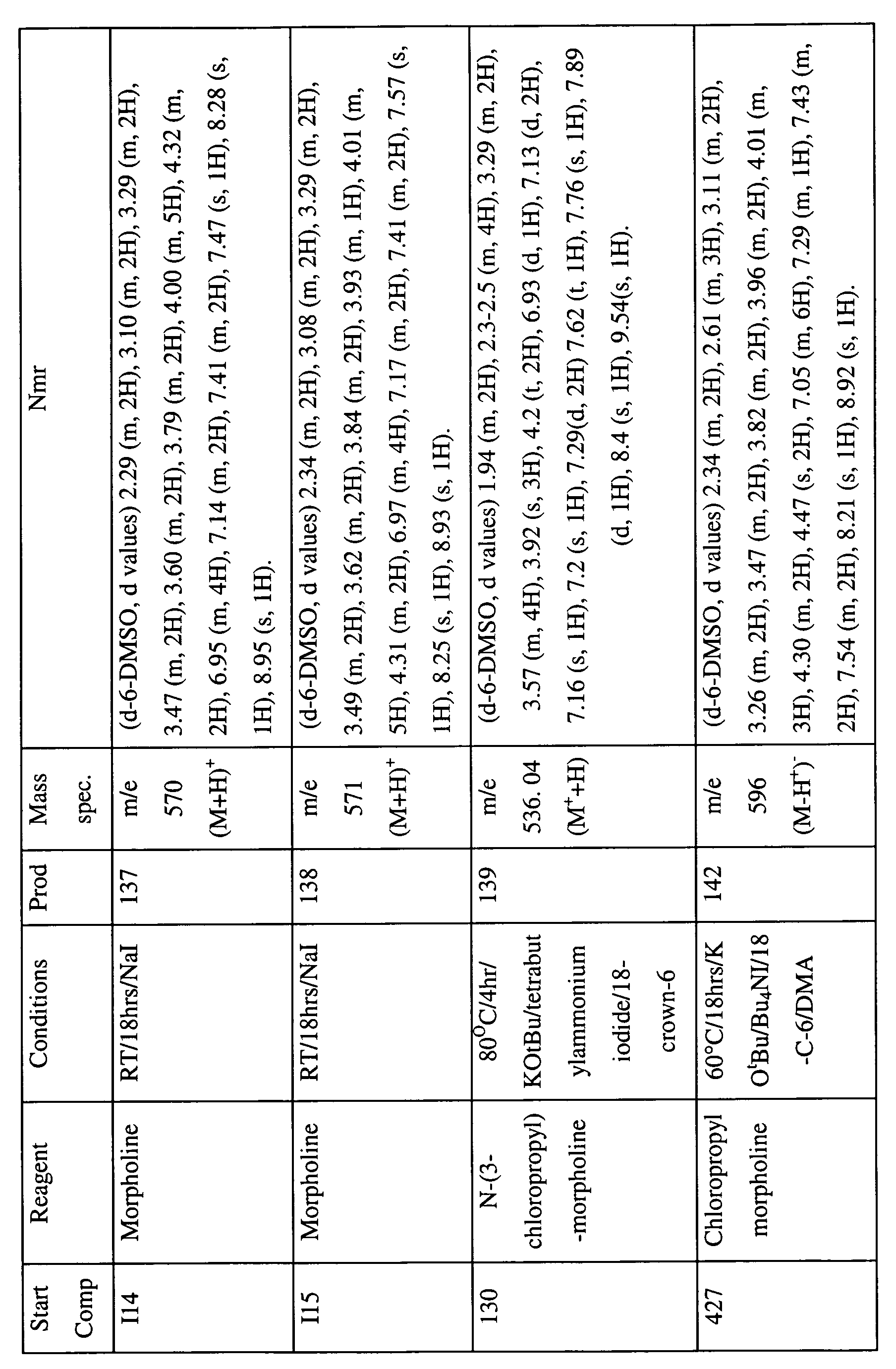

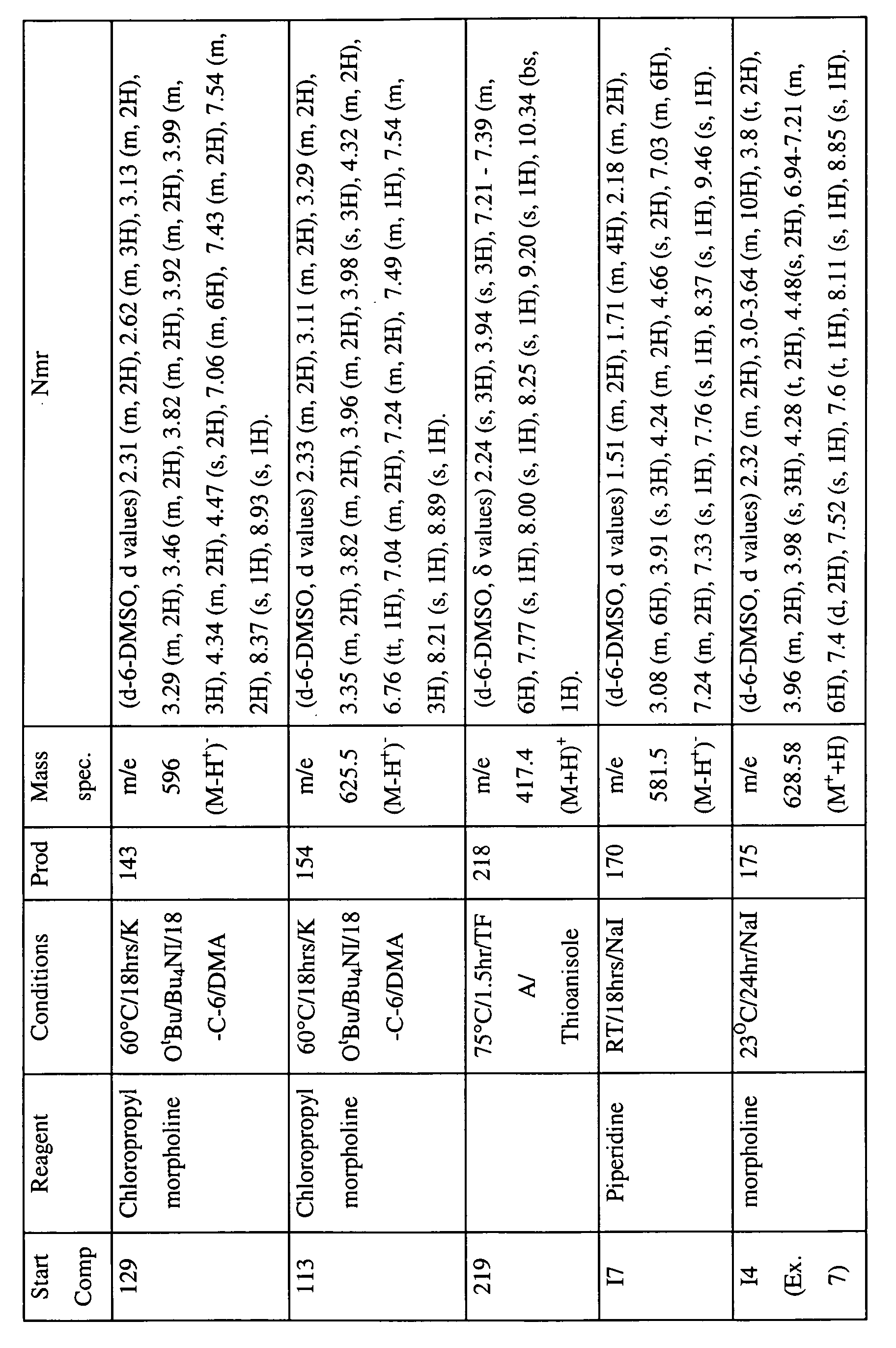

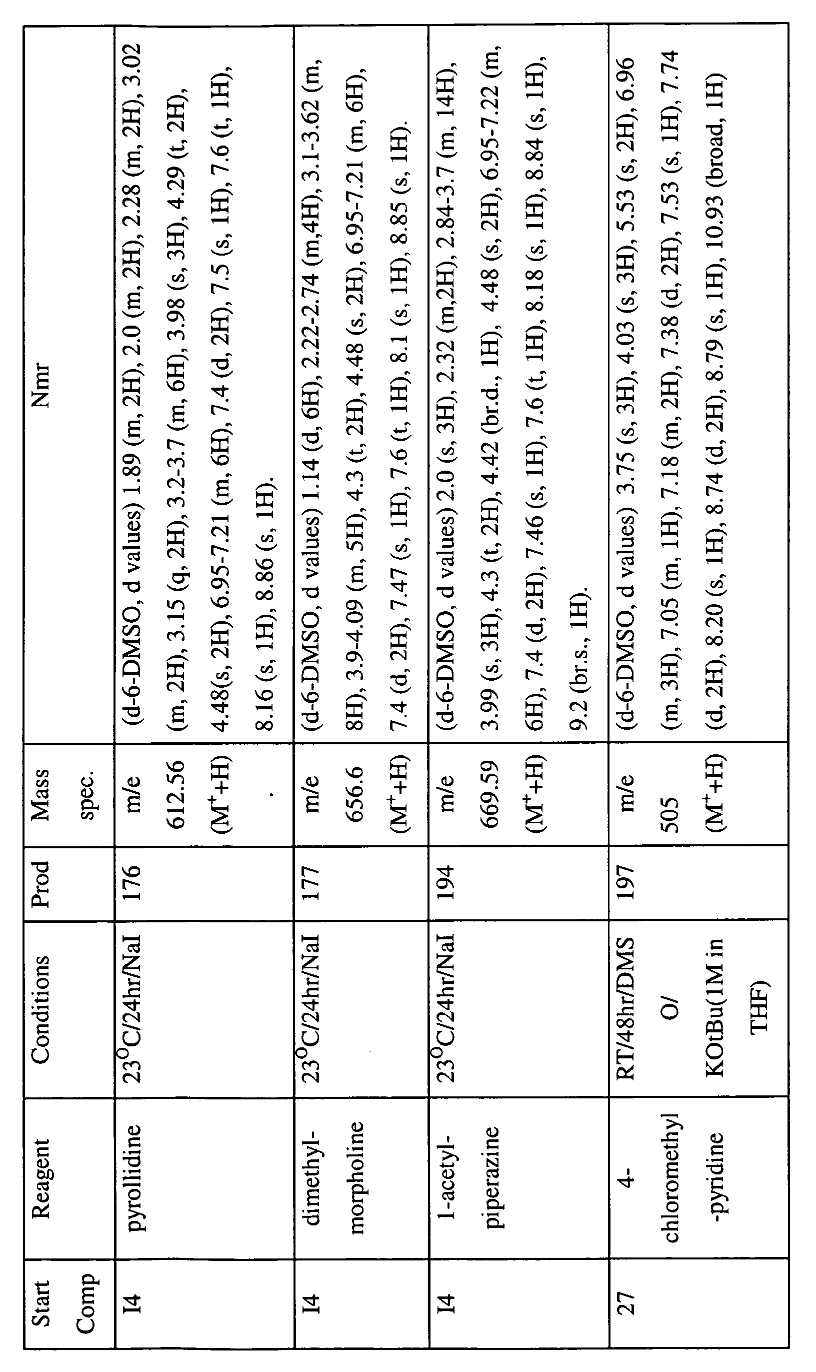

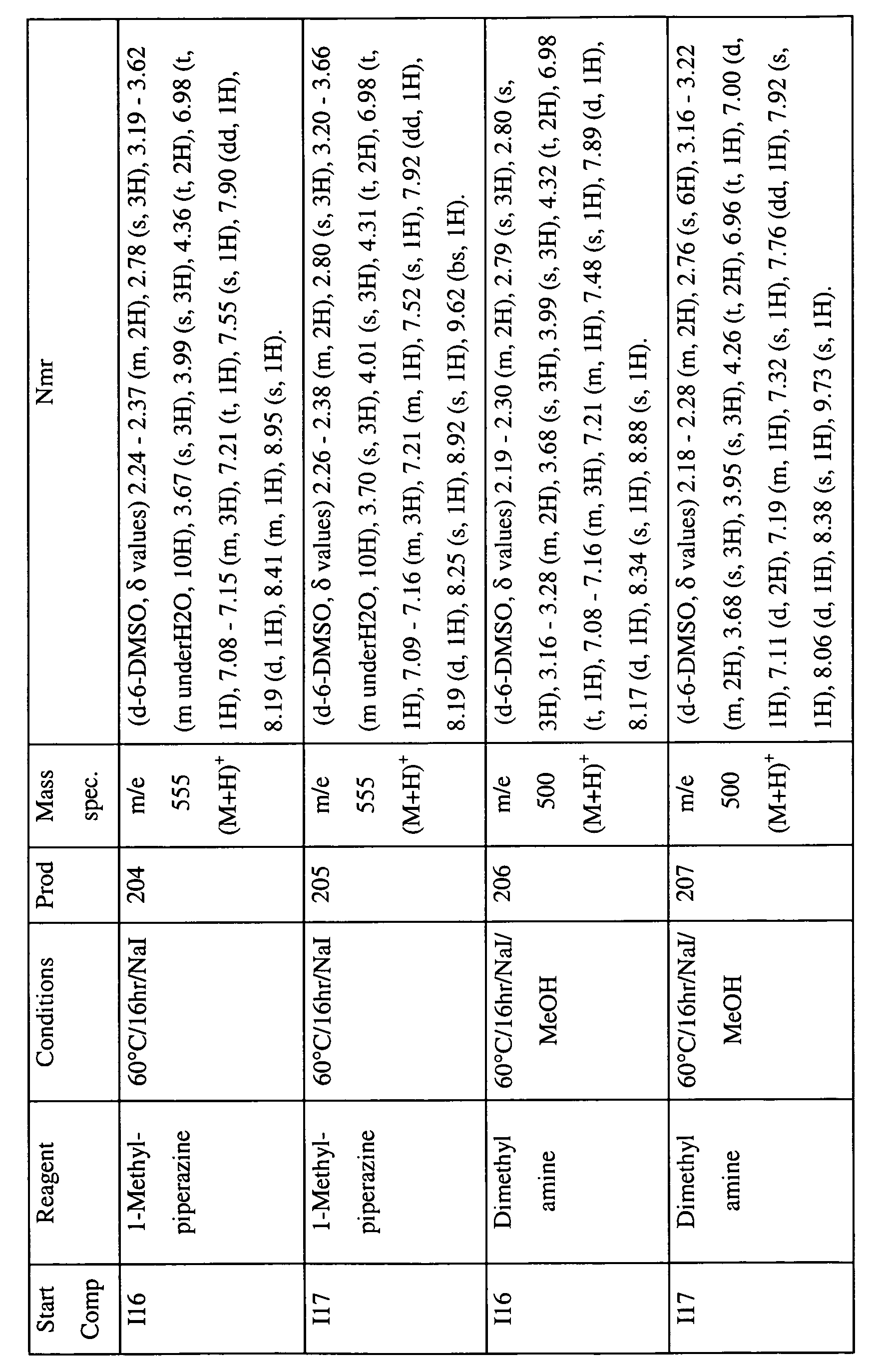

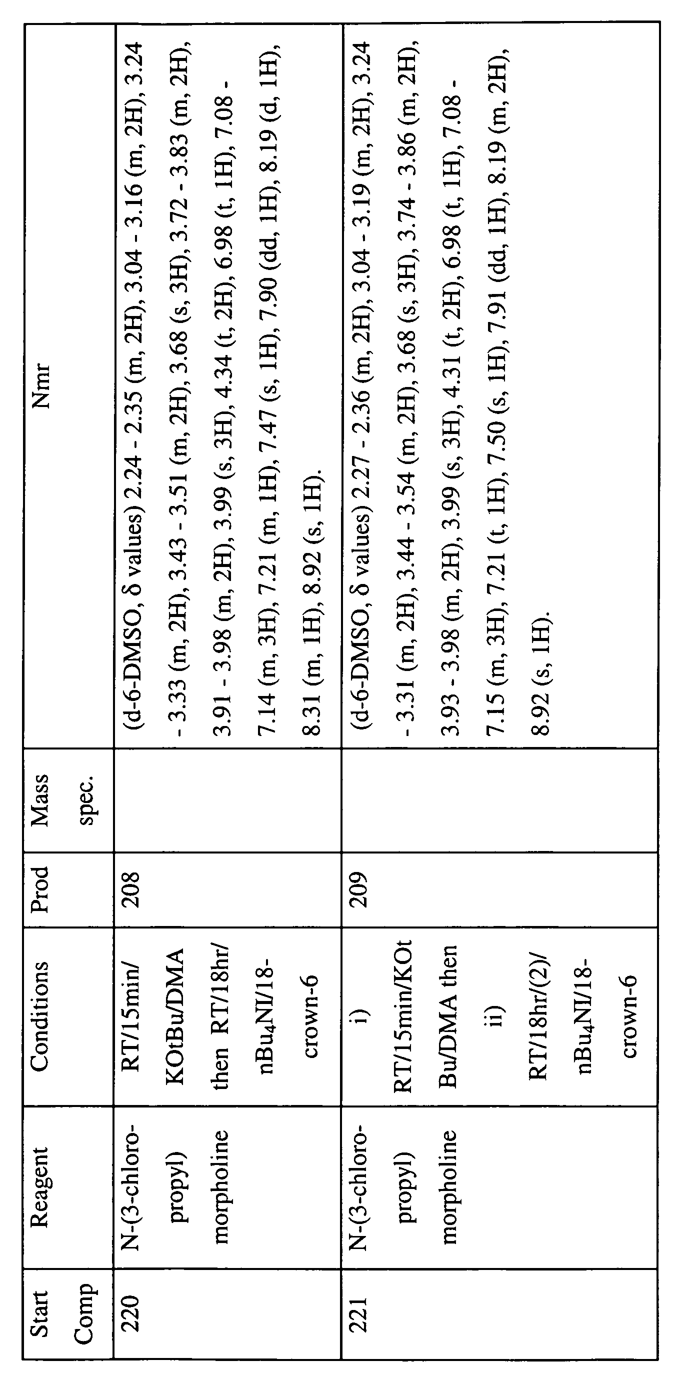

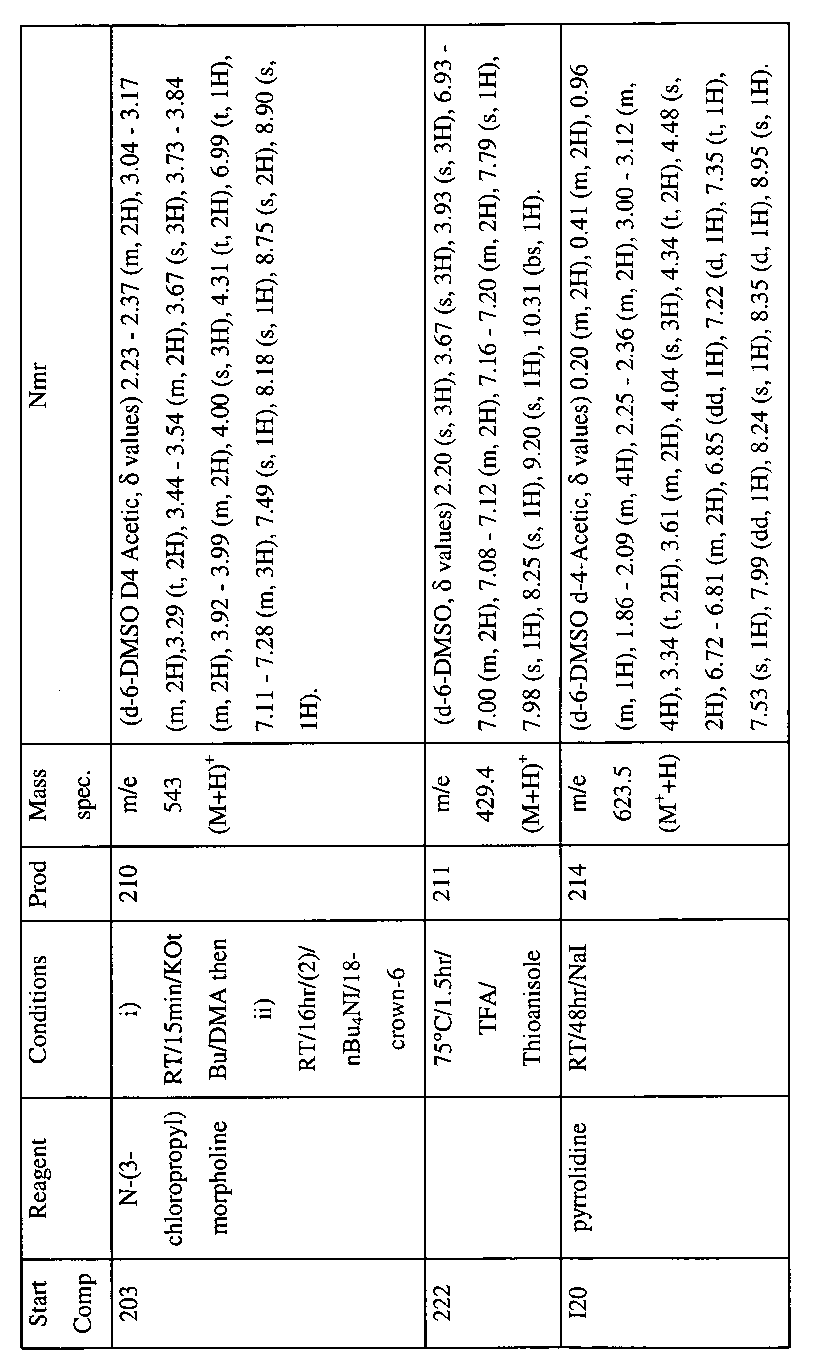

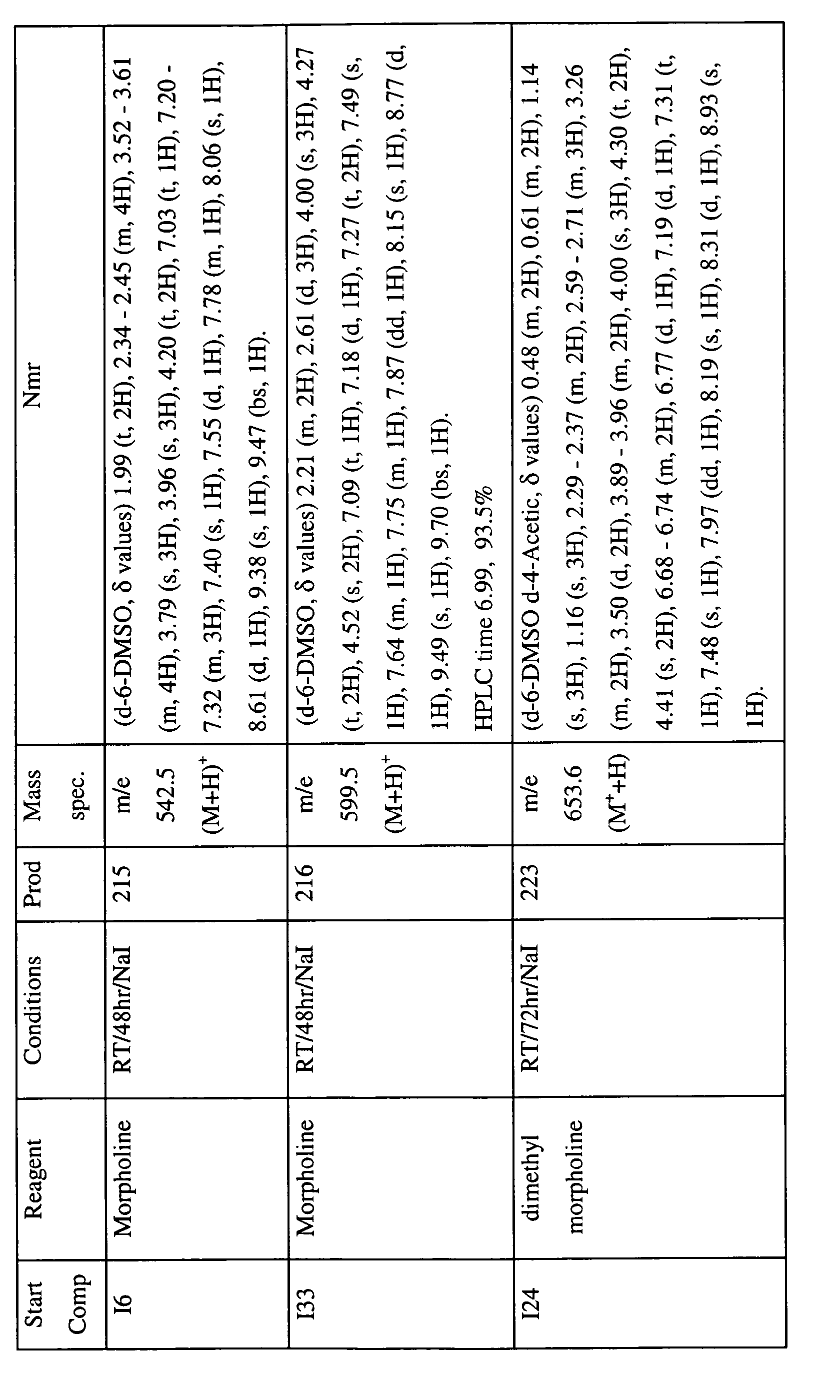

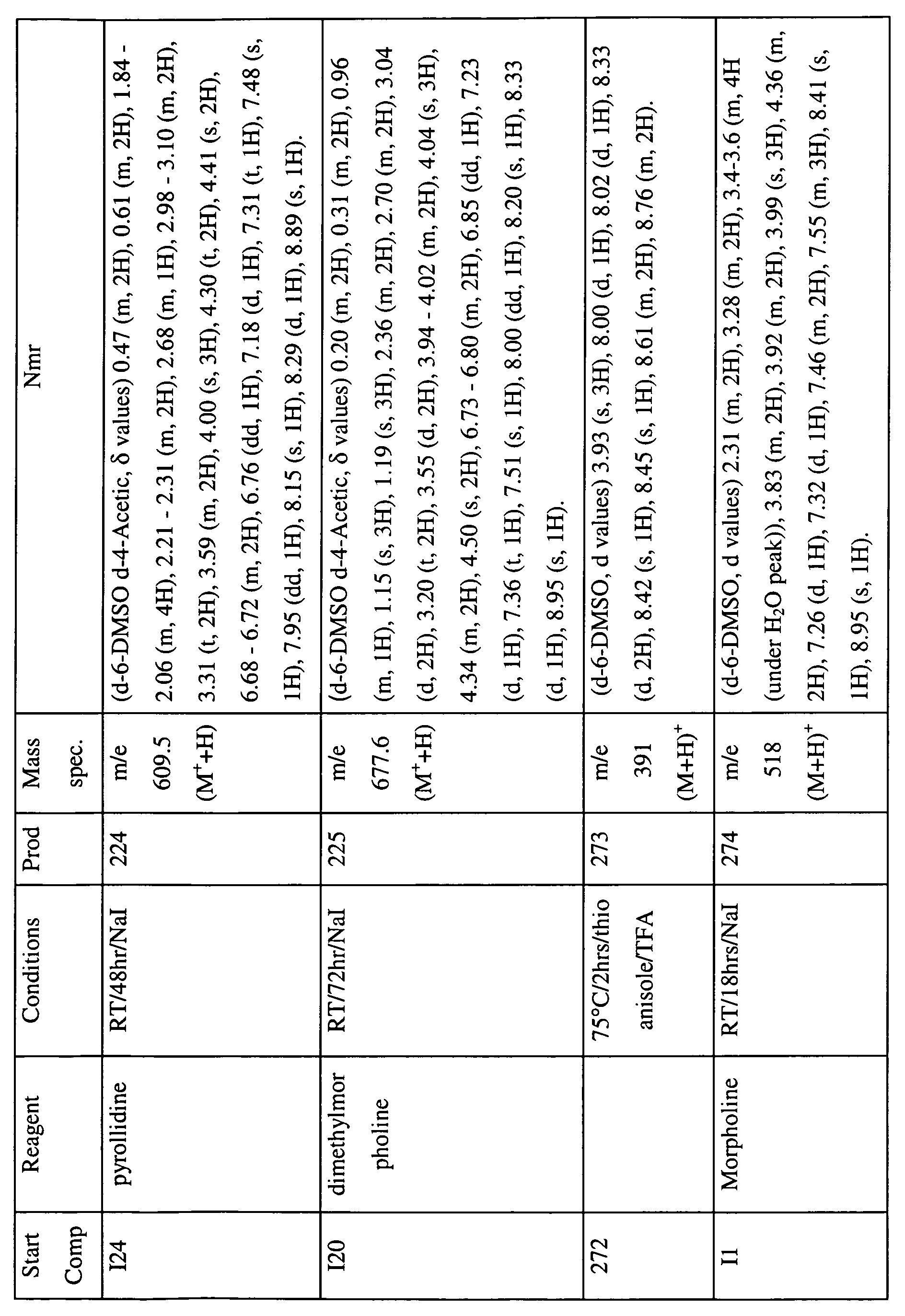

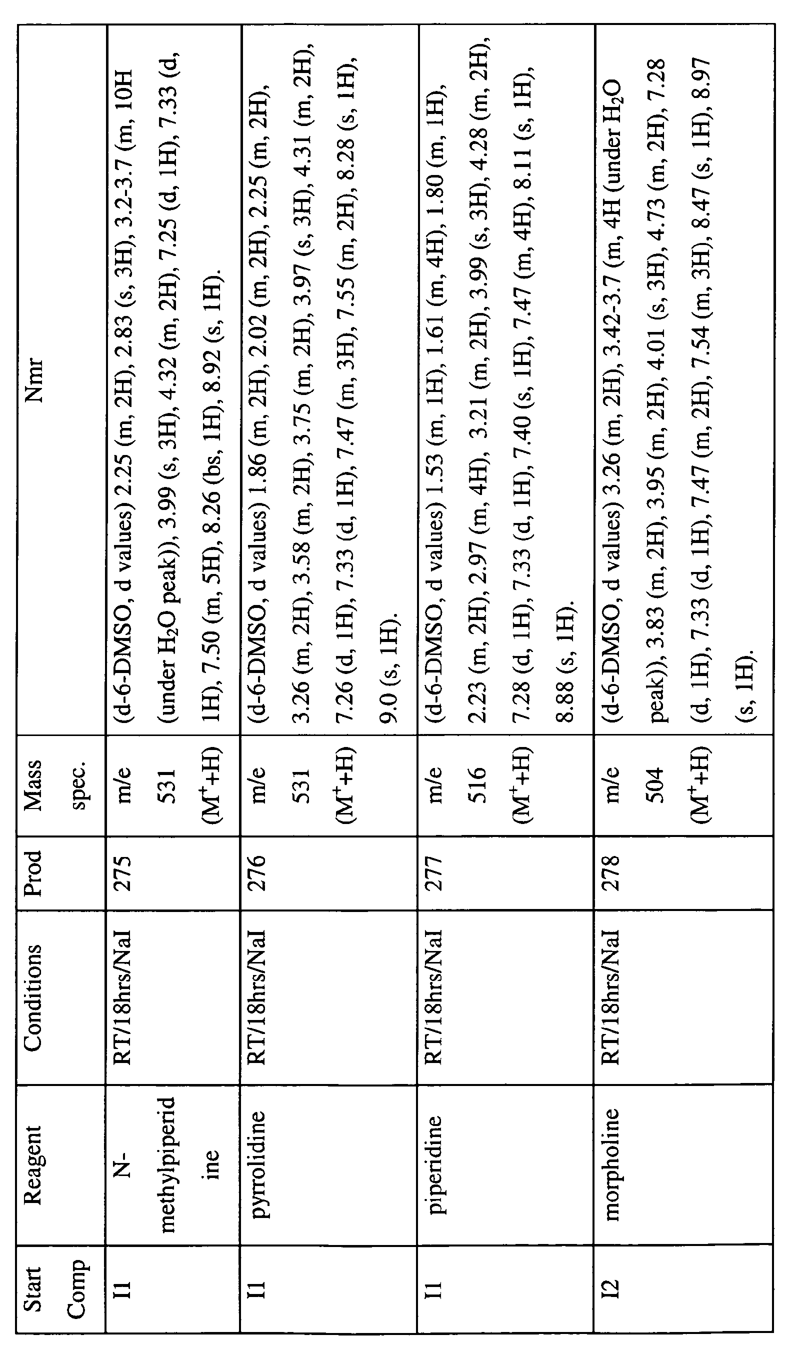

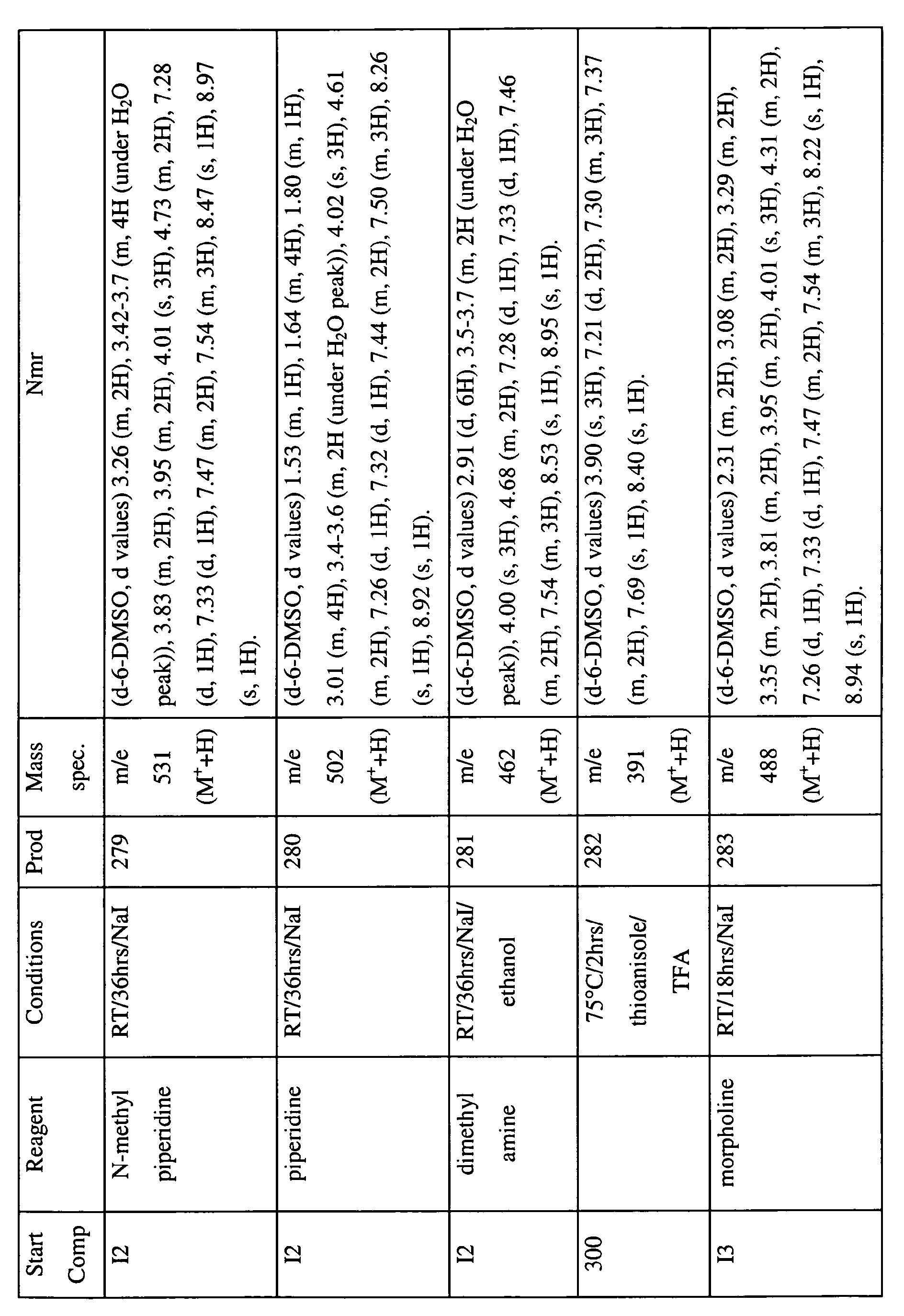

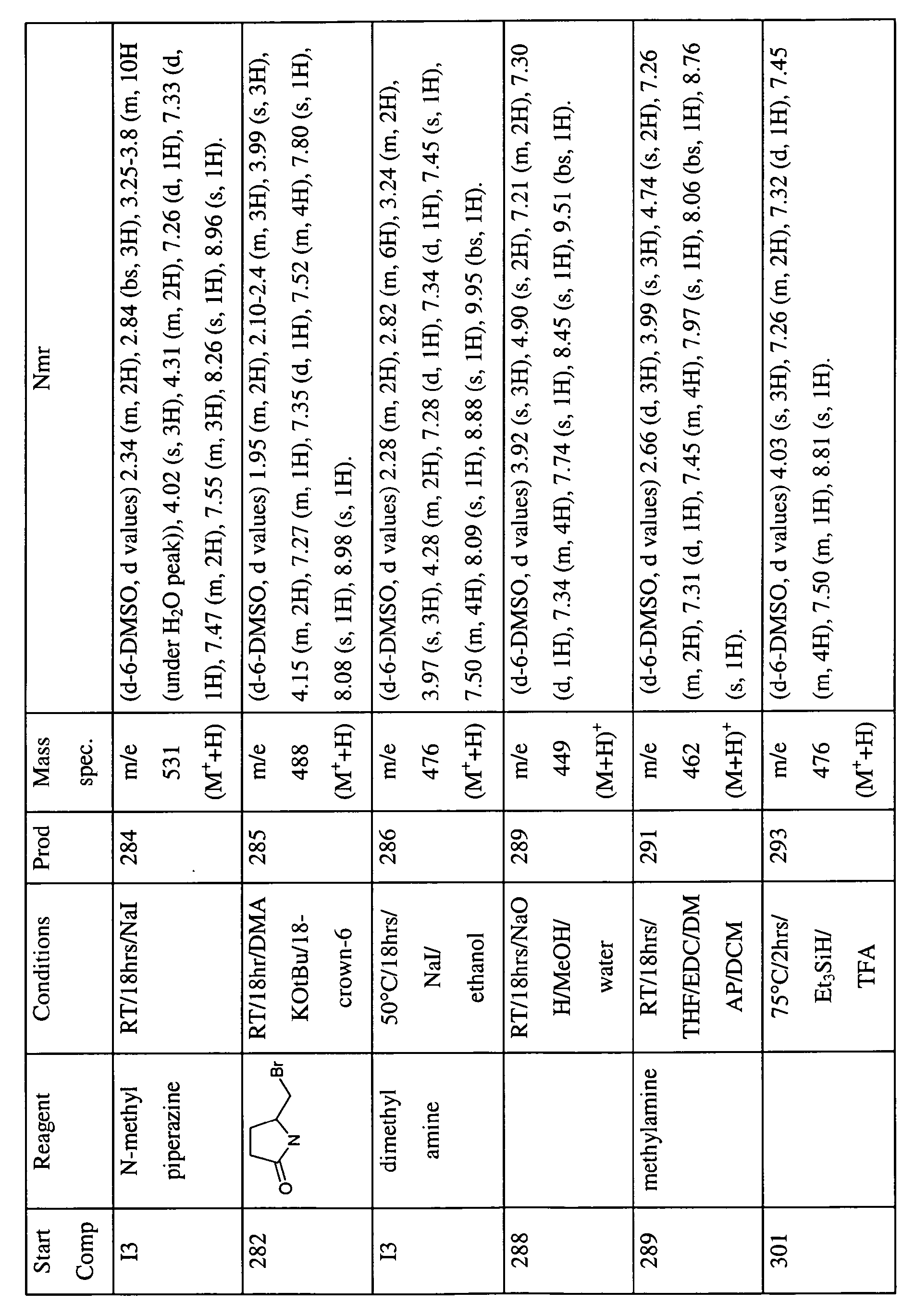

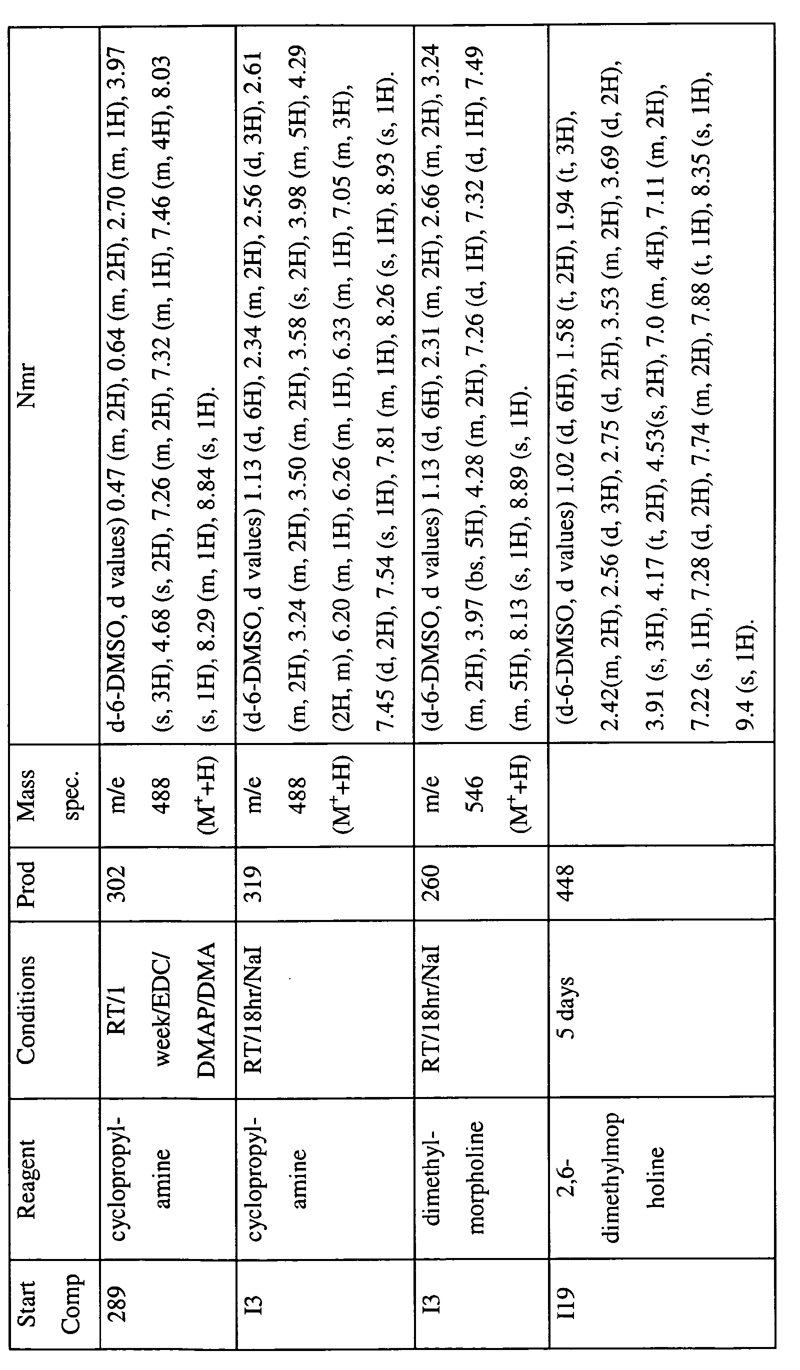

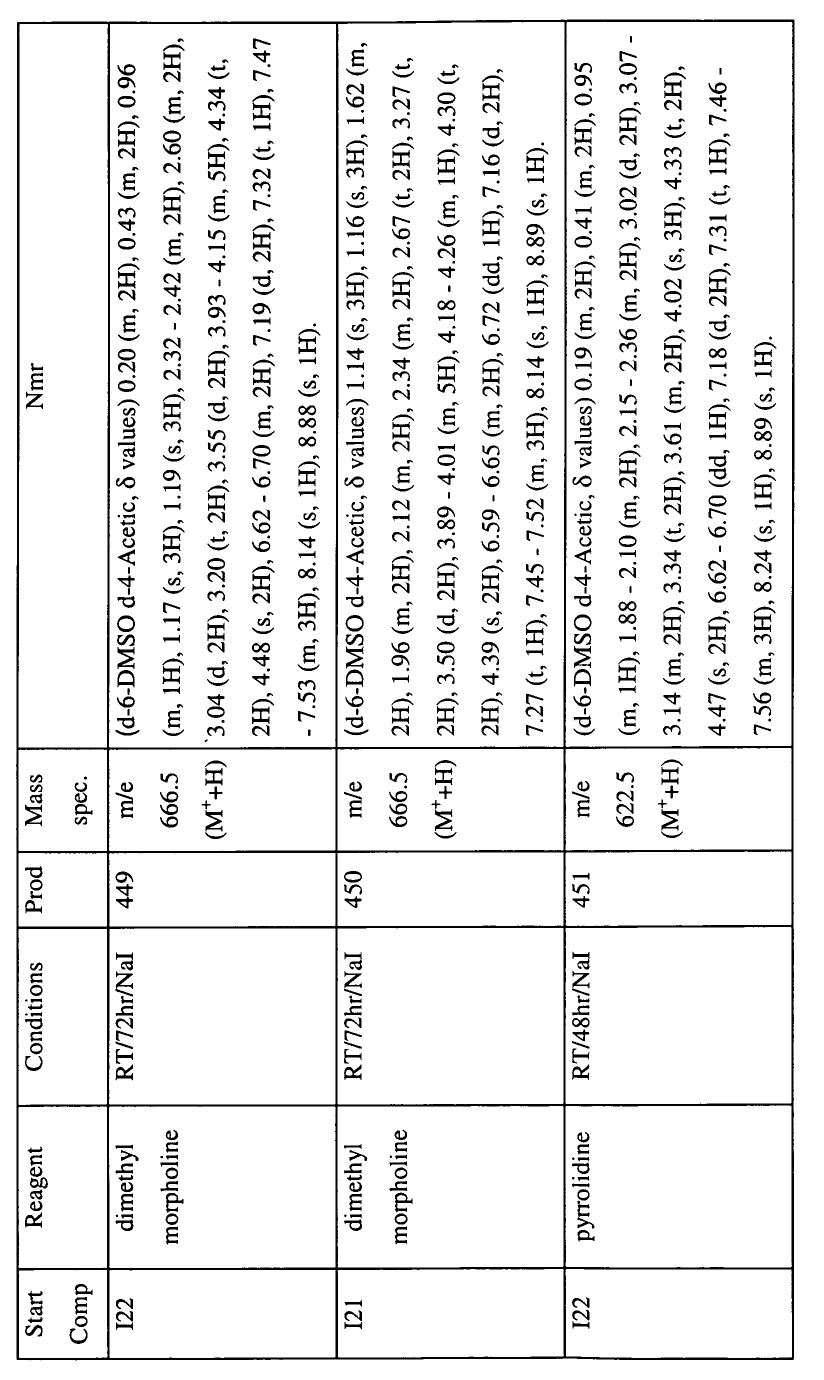

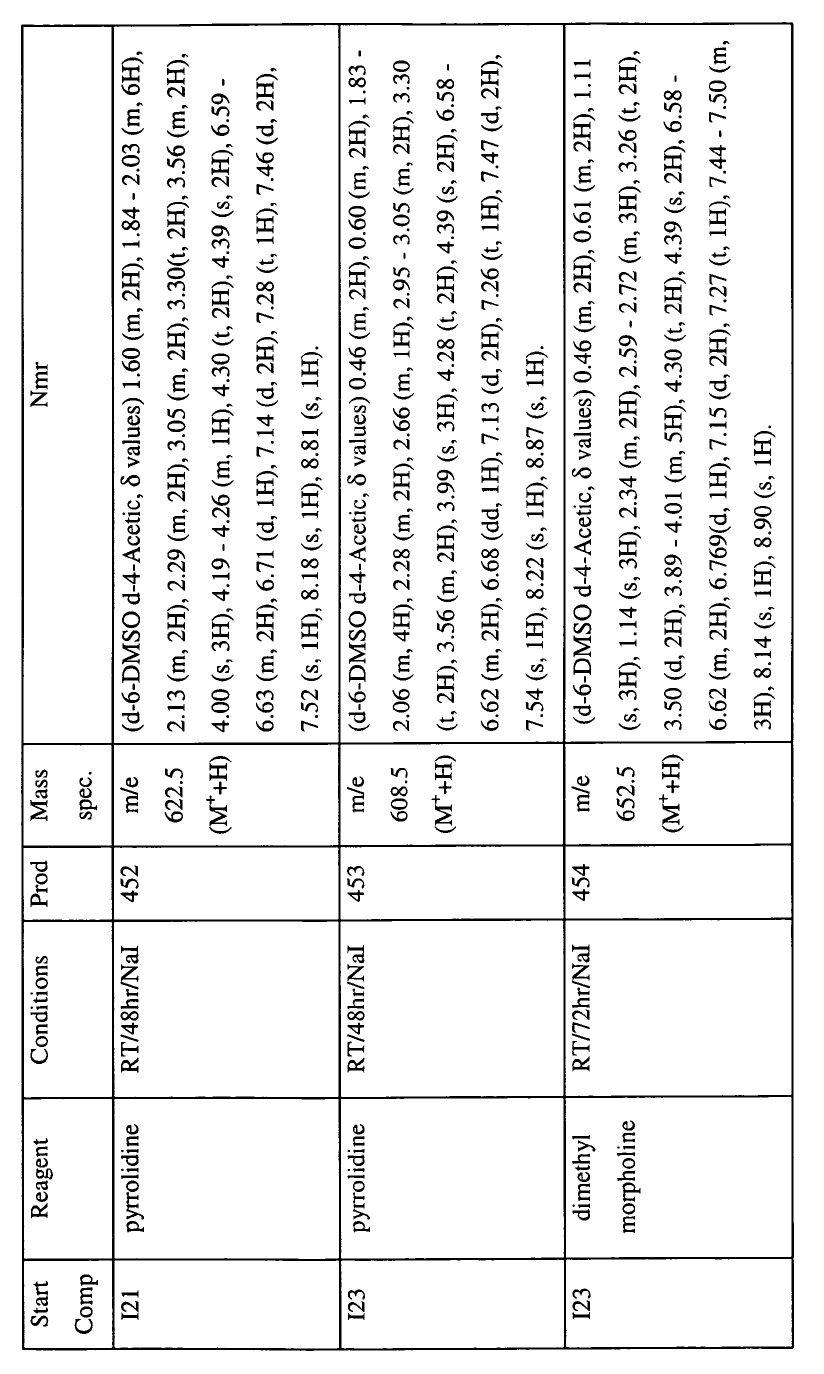

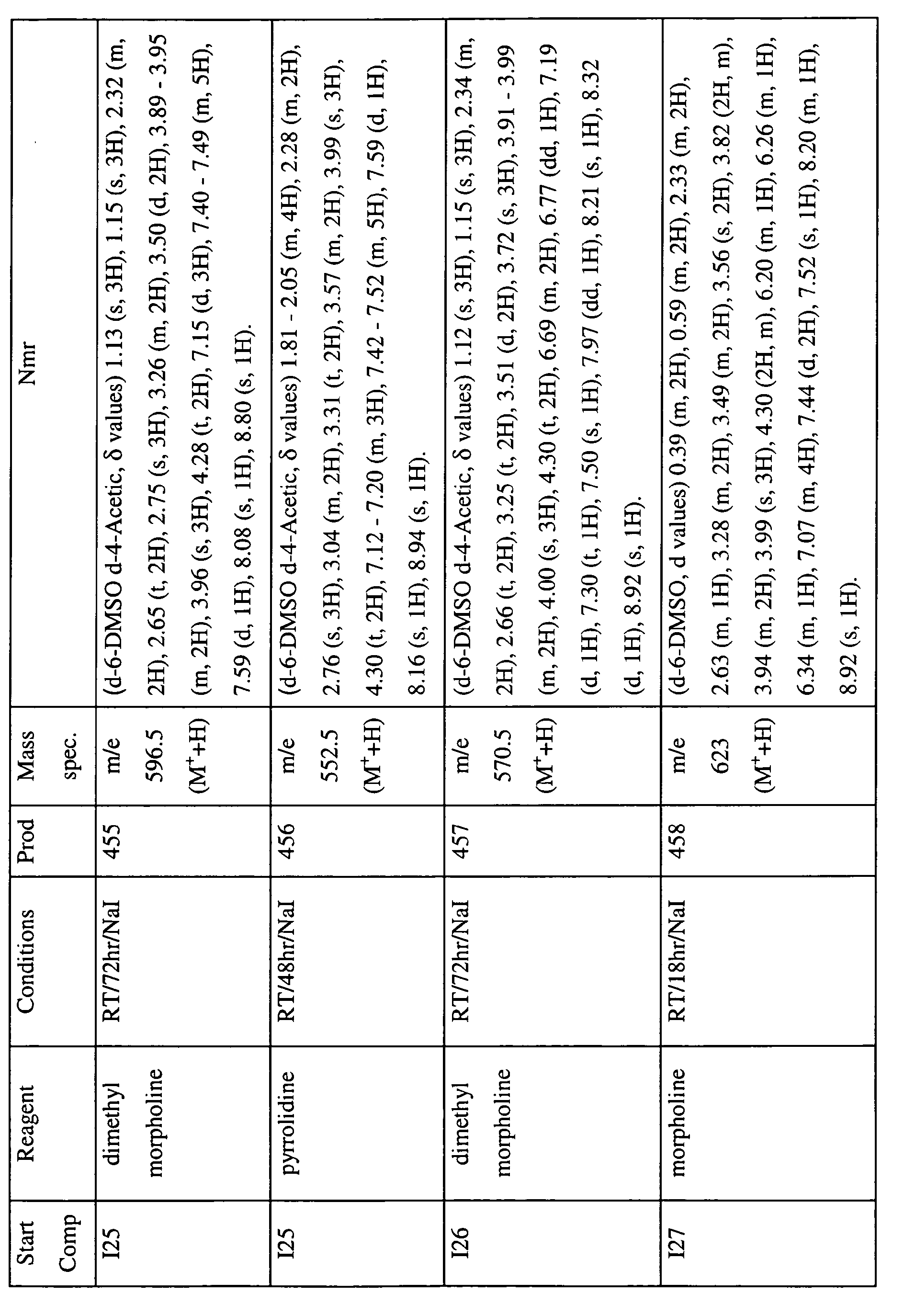

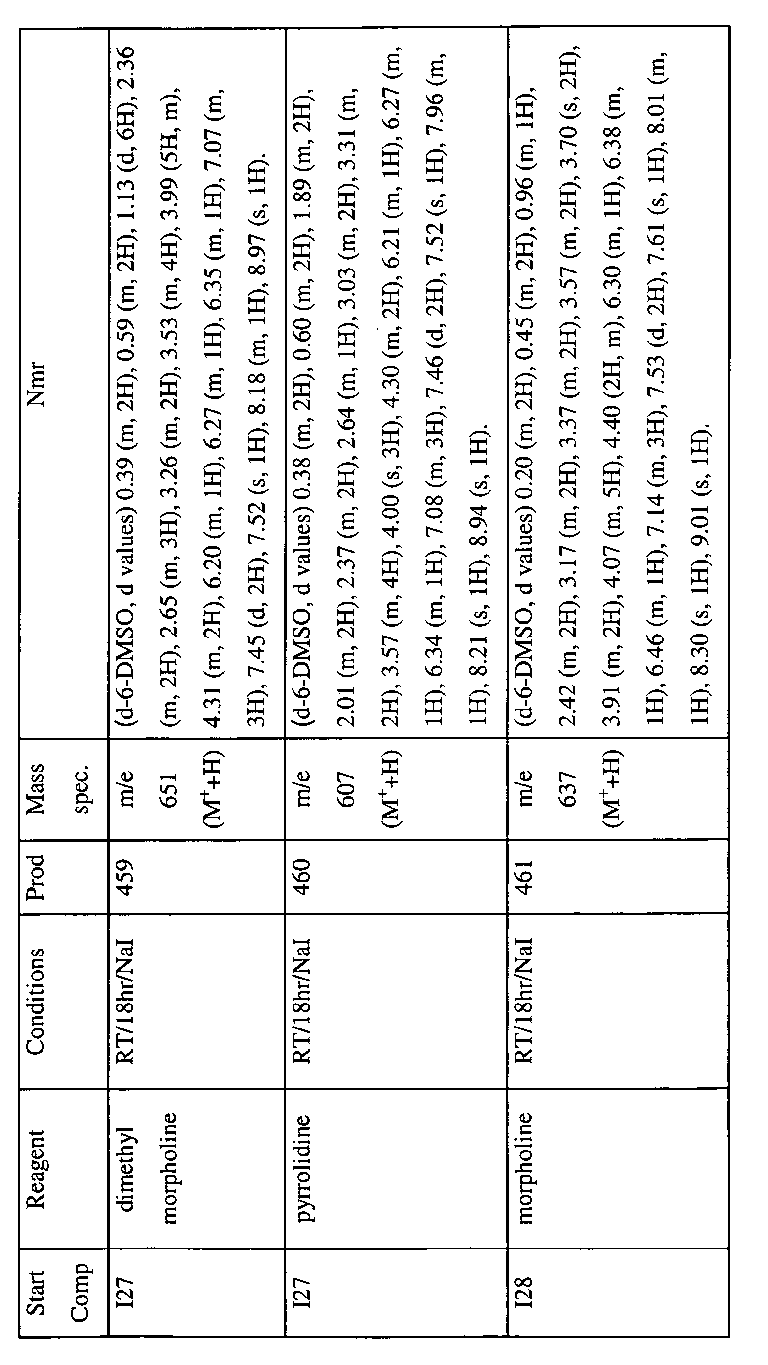

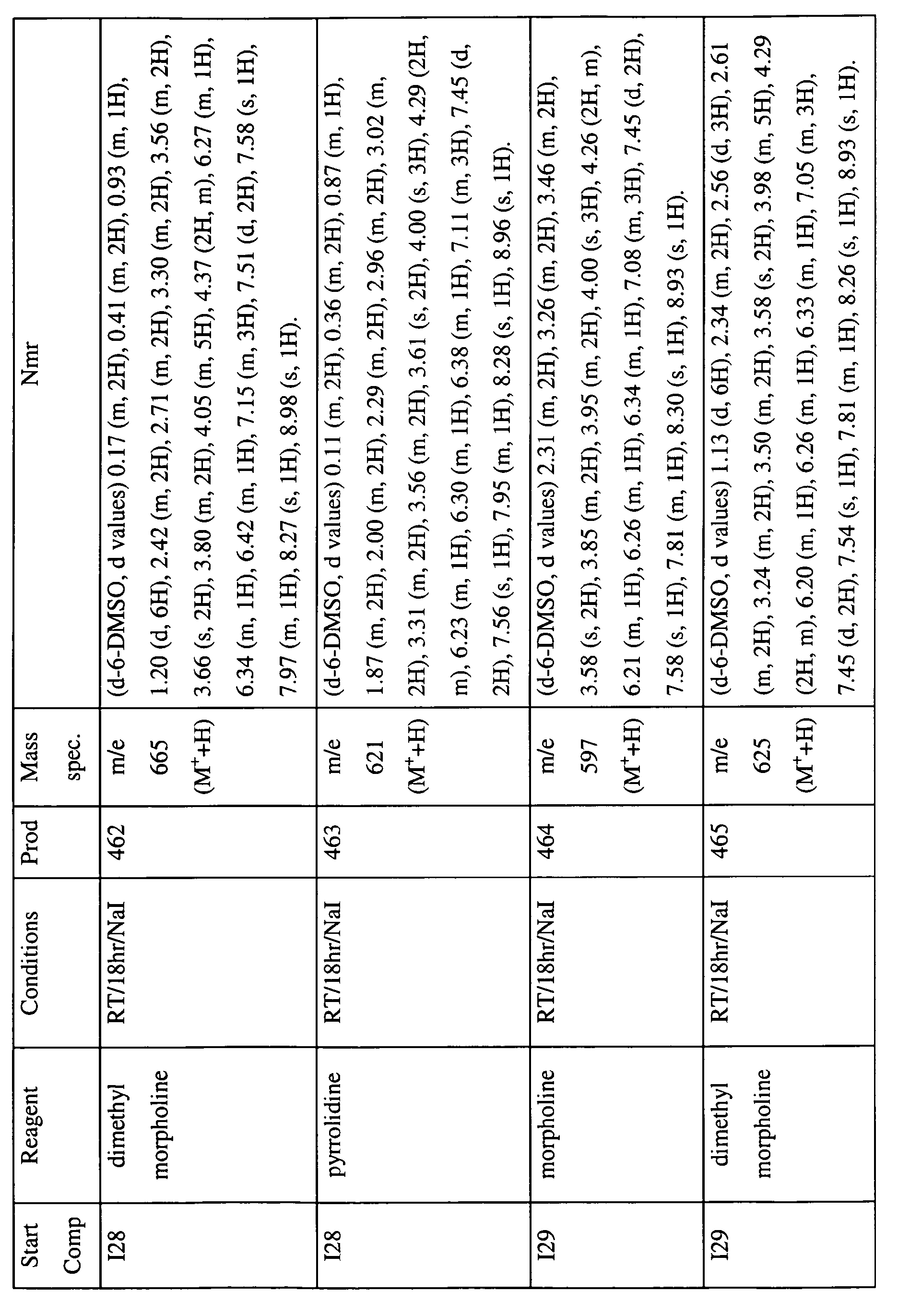

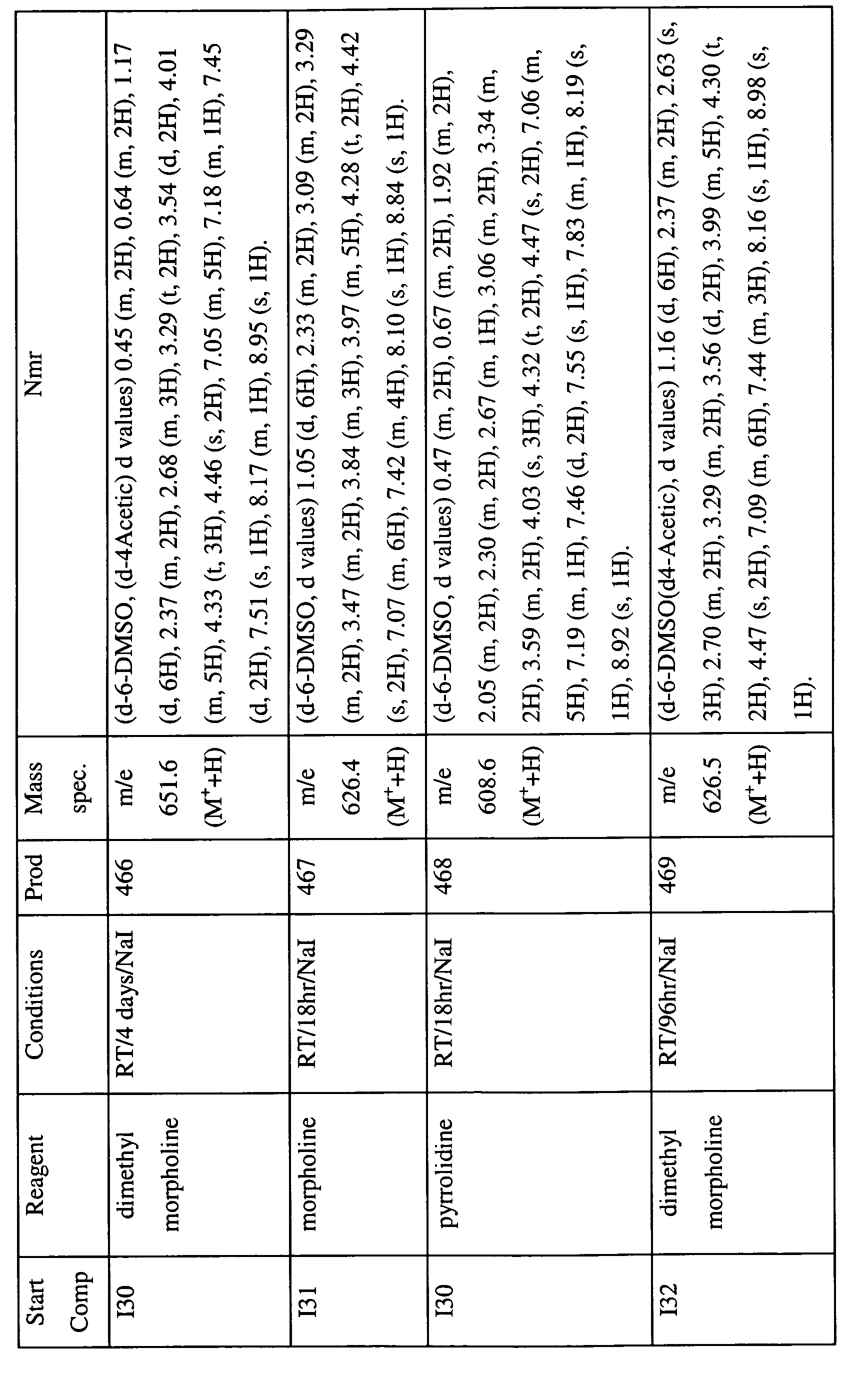

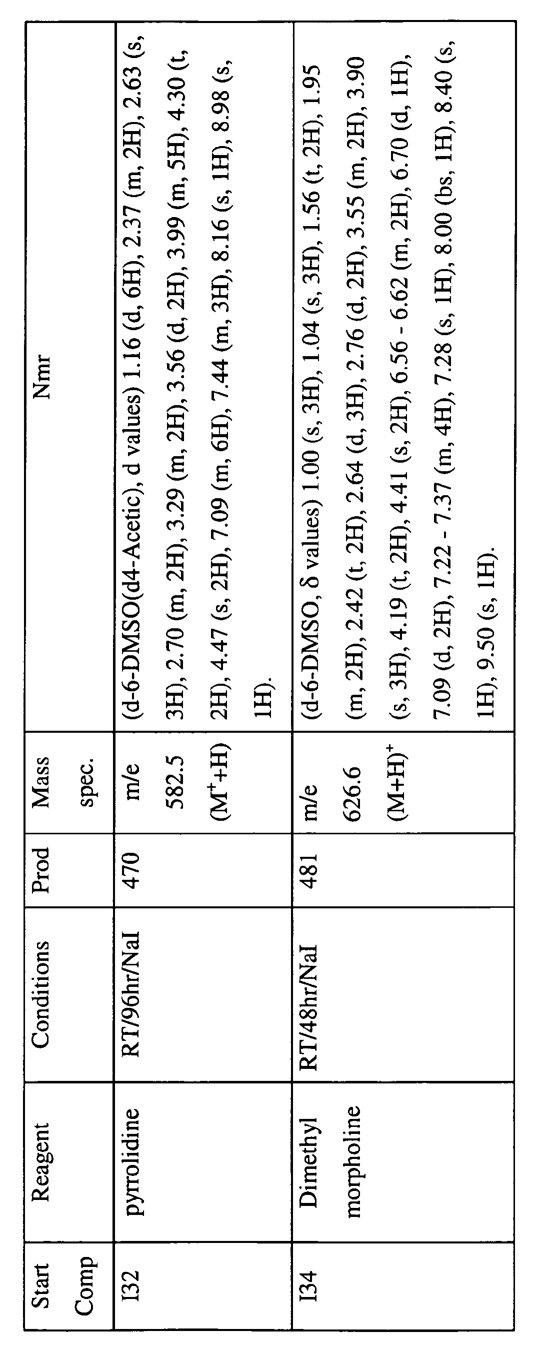

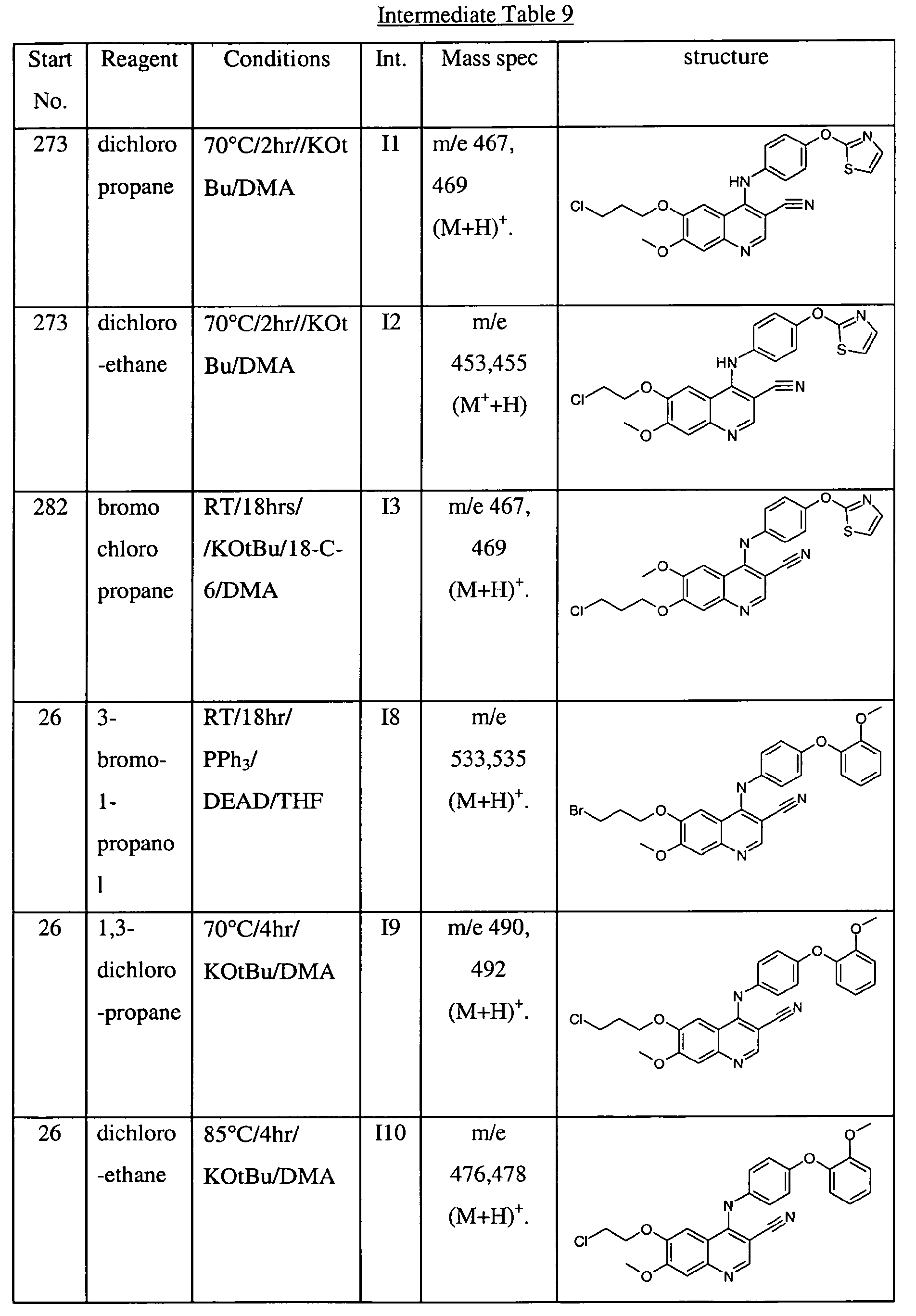

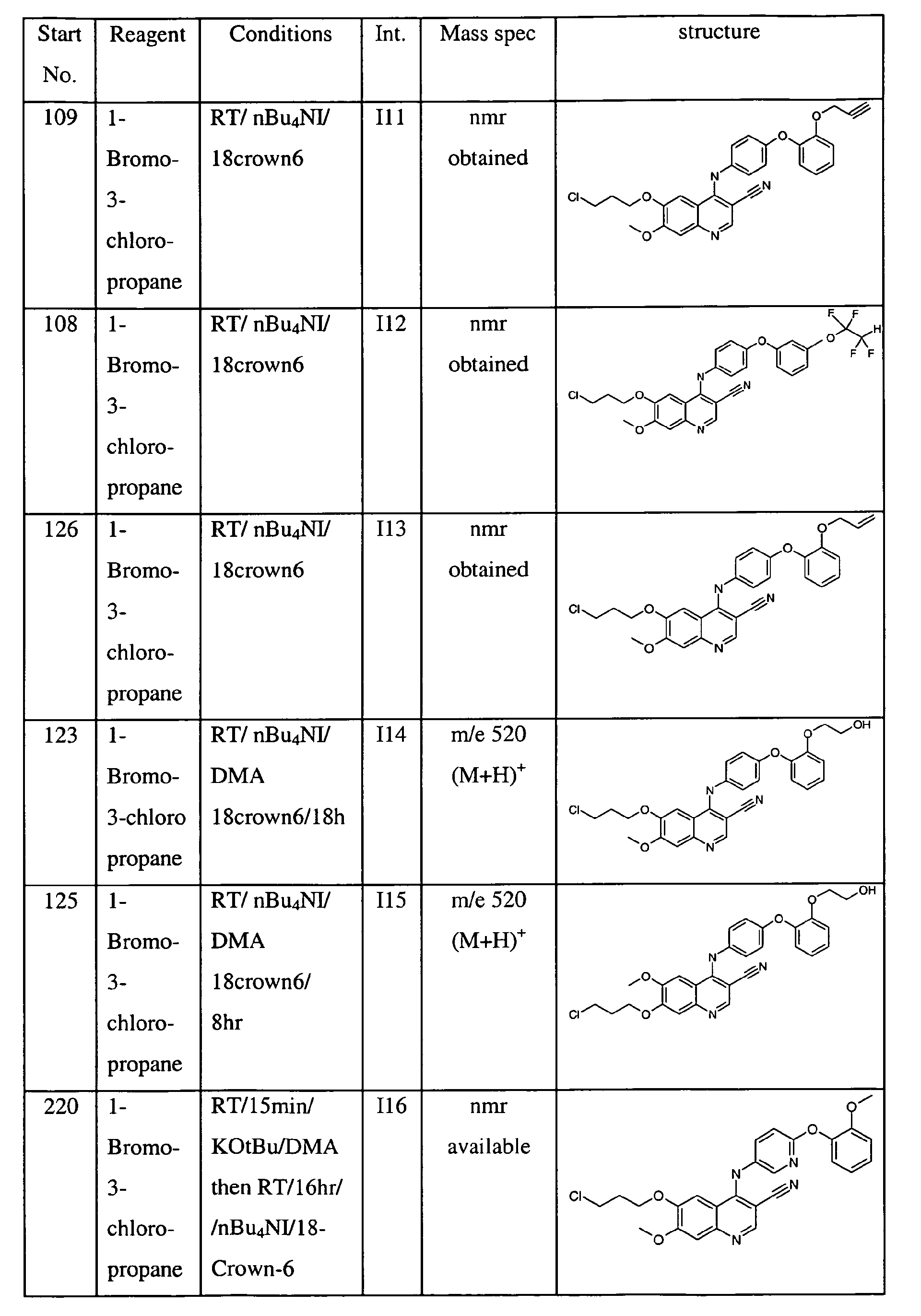

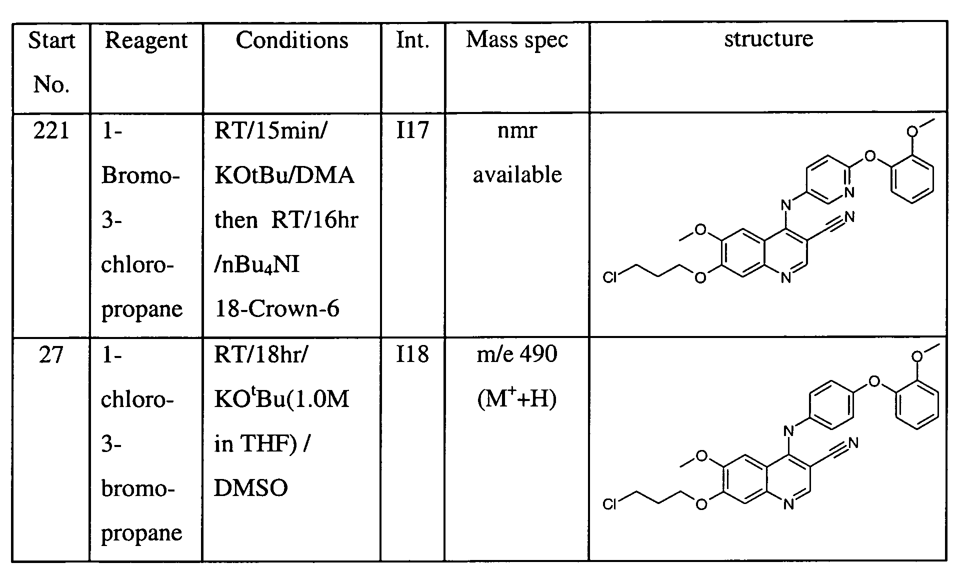

### Example 7

In the above Table I4 is a compound of structure 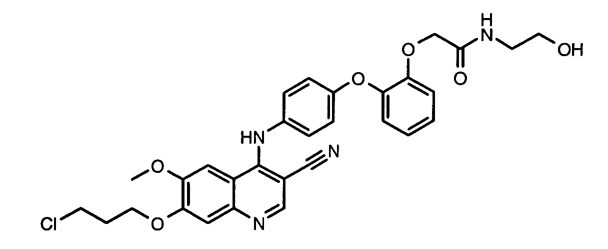 which had been prepared by a method analogous to that described in Example 1, but using reaction conditions of 100°C/2hr/1-PrOH.
Mass Spectrum m/e 577.45,579.46 (M⁺+H).
NMR Spectrum (d-6-DMSO, d values) 2.28 (m, 2H), 3.16 (q, 2H), 3.4 (t, 2H), 3.82 (t, 2H), 3.98 (s, 3H), 4.3 (t, 2H), 4.48(s, 2H), 6.95-7.22 (m, 6H), 7.4 (d, 2H), 7.46 (s, 1H), 7.6 (t, 1H), 8.09 (s, 1H), 8.9 (s, 1H), 11.07 (br.s, 1H).

The chloropropoxyquinoline intermediate (Mass Spectrum m/e 311.2 (M+H)⁺) was prepared by reacting the corresponding hydroxy quinoline with 1-bromo-3- chloropropane at room temperature for 16hr in the presence of nBu4NI/18-crown-6

The following haloalkoxy quinolines were prepared by analogous routes:

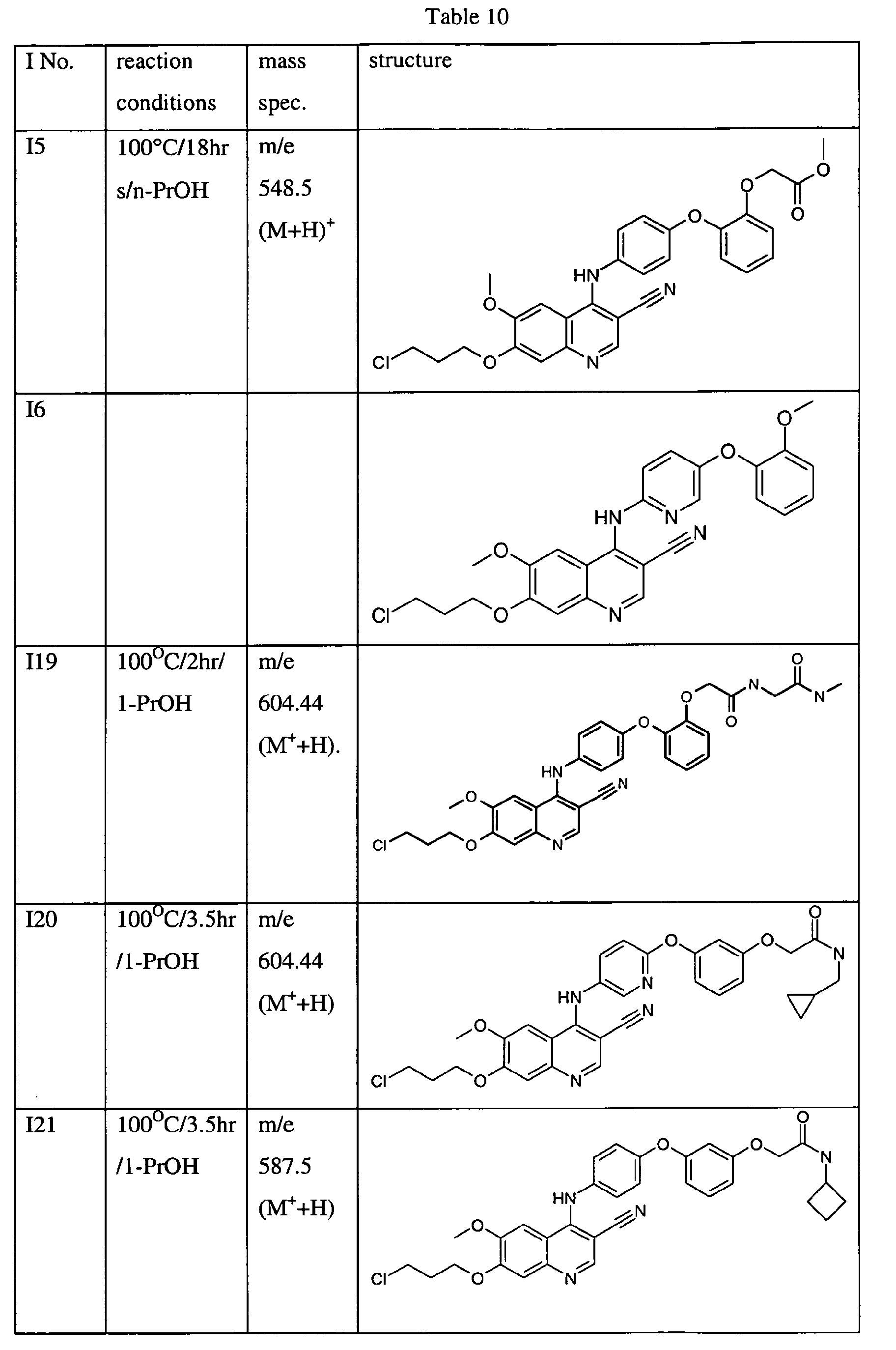

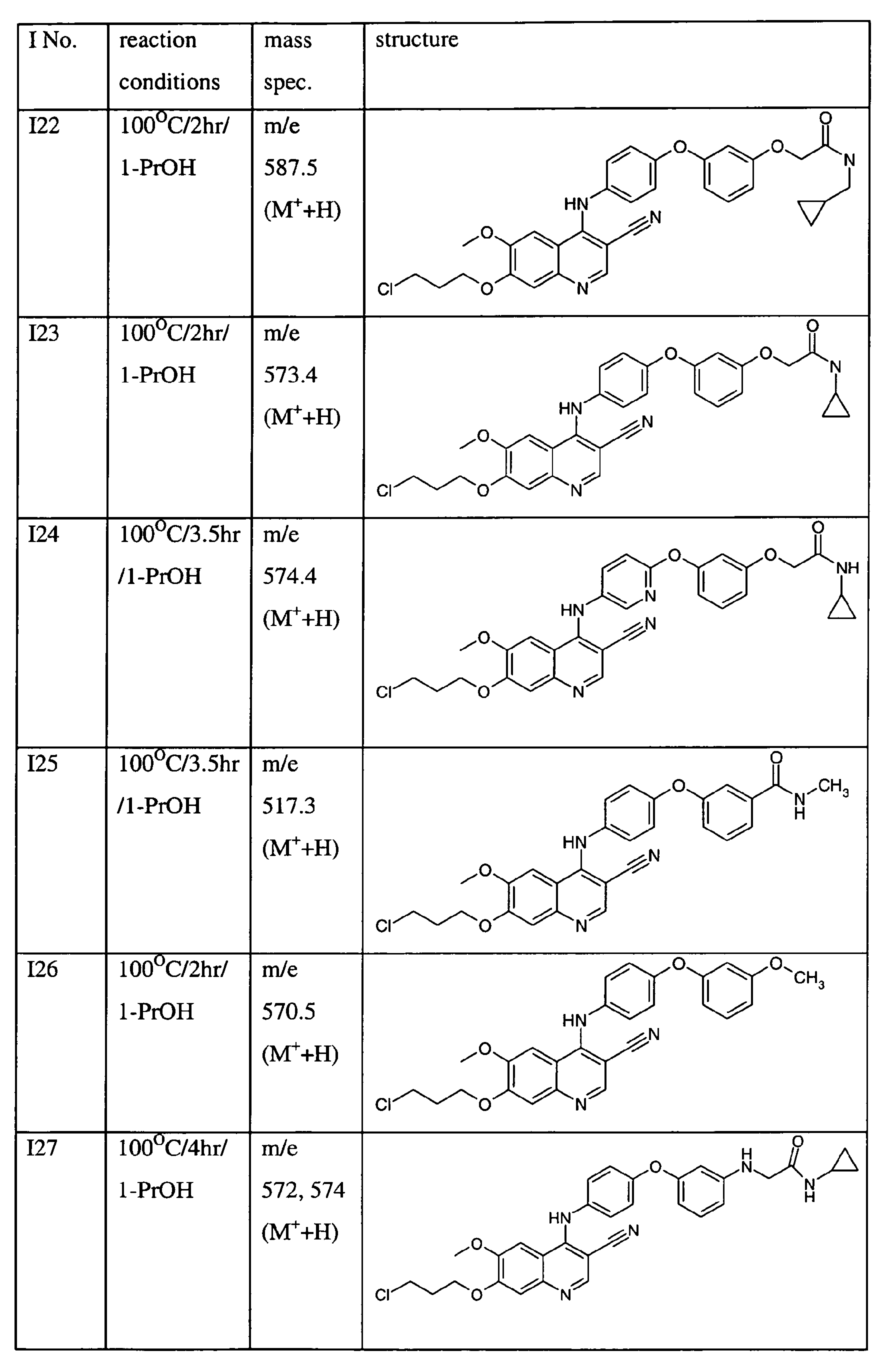

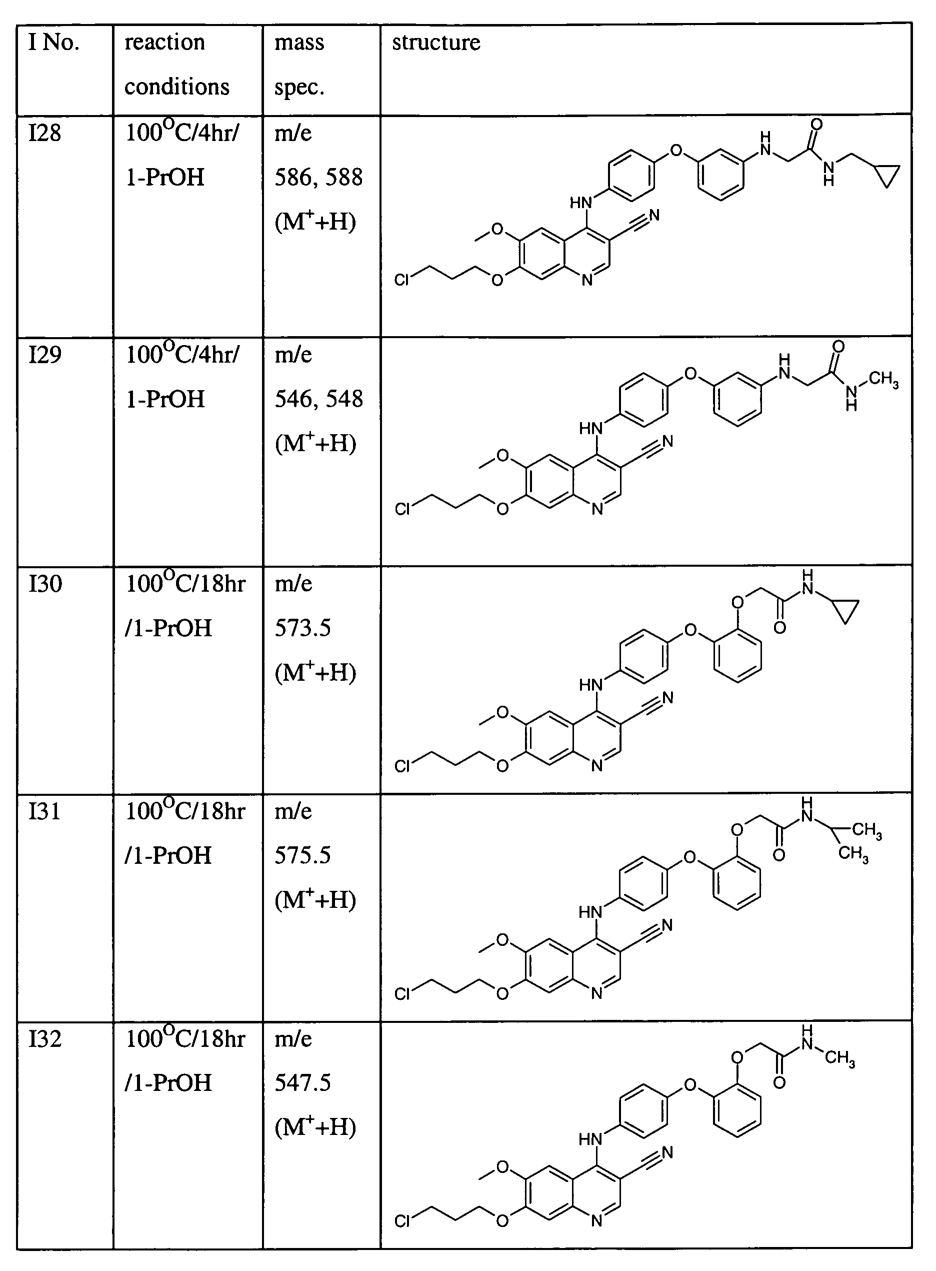

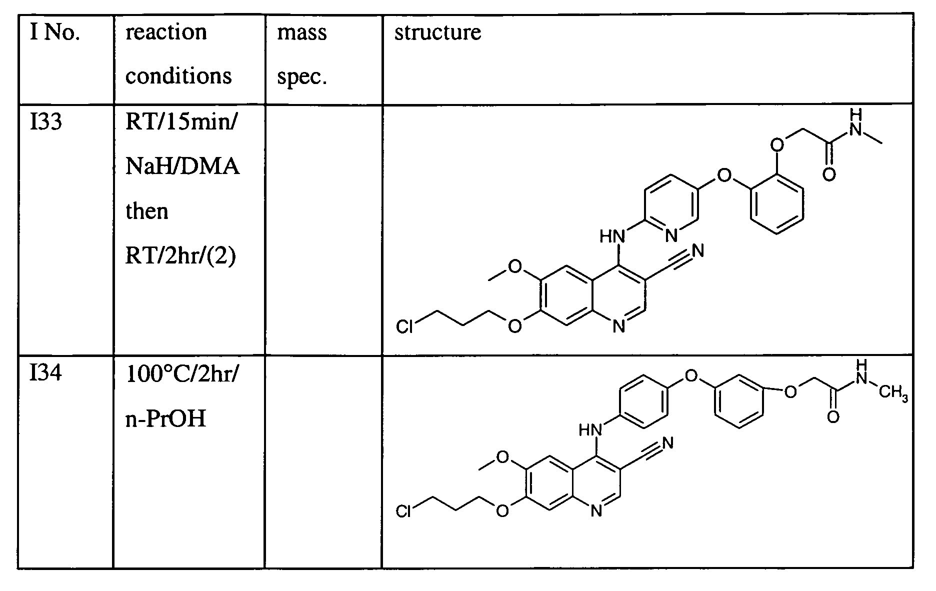

In addition I5 was converted to I7 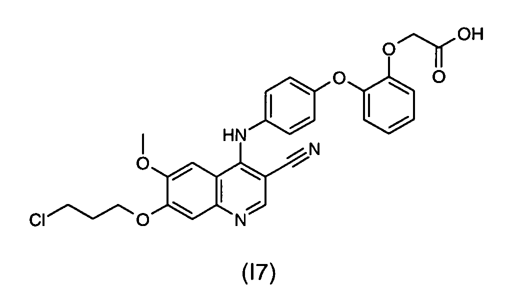 using the following reaction conditions: RT/3hrs/LiOH.H₂O/MeOH/H₂O
Mass Spectrum m/e 534.5 (M+H)⁺
NMR Spectrum (d-6-DMSO, d values) 2.26 (m, 2H), 3.82 (m, 2H), 3.93 (s, 3H), 4.26 (t, 2H), 4.68 (s, 2H), 7.04 (m, 6H), 7.29 (m, 2H), 7.39 (s, 1H), 7.93 (s, 1H), 8.55 (s, 1H).

### Example 8

### Preparation of Compound No. 312

In this example, an intermediate nitro compound of formula (2) was reacted in situ with a chloroquinoline intermediate to produce compound 312, (a compound of formula (I)) directly in accordance with the following scheme:

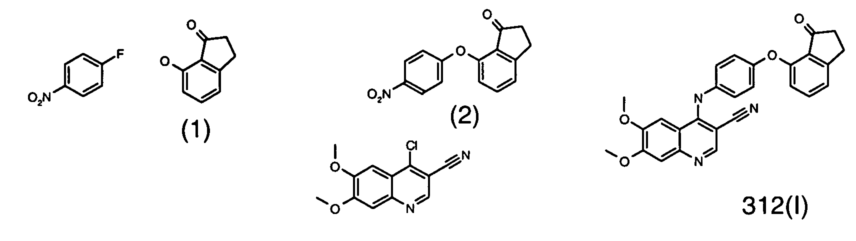

The reaction conditions were: Cyclohexene, 1-propanol, Pd/C, filter then add quinoline to obtain the desired product
Mass Spectrum m/e 452 (M⁺+H)
NMR Spectrum (CDCl₃, d values) 2.70 (m 2H), 3.15 (m 2H), 3.75 (s, 3H), 4.00 (s, 3H), 6.70 (d, 1H), 6.80 (broad s, 1H), 6.95 (s, 1H), 7.05 (d, 2H), 7.15 (d, 2H), 7.15 (m, 1H), 7.35 (s, 1H), 7.45 (t, 1H), 8.60 (s, 1H).
Quinoline SM: WO 9843960

The reaction conditions used to obtain Intermediate labelled (2) was KOtBu, DMA.
Mass Spectrum m/e 270 (M⁺+H)

Using an analogous method, the following compounds were also produced

**Table 11**

| No. | Mass spec | N.M.R |
|---|---|---|
| 313 | m/e 429 (M⁺+H) | (CDCl₃, d values) 3.70 (s, 3H), 4.00 (s, 3H), 6.85 (broad s, 1H), 6.90 (m, 2H), 7.10 (d, 2H), 7.15 (d, 2H), 7.35 (m, 3H), 8.00 (s, 1H), 8.60 (s, 1H). |
| 314 | m/e 453 (M⁺+H) | (d-6-DMSO@373K, d values) 3.60 (s, 3H), 3.95 (s, 3H), 4.00 (s, 3H), 6.90 (d, 1H), 7.15 (d, 2H), 7.25 (t, 1H), 7.40 (m, 3H), 7.45 (s, 1H), 8.00 (s, 1H), 8.70 (s, 1H). |
| 315 | m/e 438 (M⁺+H) | (d-6-DMSO, d values) 4.00 (s, 3H), 4.00 (s, 3H), 6.75 (d, 1H), 6.85 (d, 1H), 7.20 (d, 2H), 7.30 (t, 1H), 7.40 (d, 1H), 7.50 (d, 2H), 7.50 (s, 1H), 7.95 (d, 1H), 8.20 (s, 1H), 8.95 (s, 1H), 11.30 (broad s, 1H). |

### Example 9

### Preparation of Compounds 136 and 140 in Table 1

Compound 85 prepared as described above, was dissolved in trichloromethane and reacted with oxone in the presence of wet alumina to yield the title compounds.
Compound 136
Mass Spectrum m/e 460 (M⁺+H)
NMR Spectrum (d-6-DMSO, d values) 2.80 (s, 3H), 3.90 (s, 3H), 3.95 (s, 3H), 6.85 (d, 1H), 7.20 (d, 2H), 7.35 (m, 4H), 7.45 (m, 1H), 7.75 (m, 2H), 8.40 (s, 1H), 9.55 (broad s, 1H).
Compound 140
Mass spec m/e 476 (M⁺+H)
NMR Spectrum (d-6-DMSO, d values) 3.40 (s, 3H), 3.95 (s, 3H), 4.00 (s, 3H), 6.95 (d, 1H), 7.20 (d, 2H), 7.35 (m, 2H), 7.40 (d, 2H), 7.65 (m, 1H), 7.80 (s, 1H), 7.90 (dd, 1H), 8.45 (s, 1H), 9.65 (broad s, 1H).

### Example 10

### Preparation of Compound 168 in Table 1

Compound 173 in Table 1 was reacted with methylamine for 18 hours at room temperature in the presence of HCl, EDC, NMM and DCM to yield the desired amide.
Mass spec. m/e 582 (M+H)⁺.
NMR Spectrum (d-6-DMSO, d values) 2.33 (m, 2H), 2.55 (d, 3H), 3.12 (m, 2H), 3.22-3.45 (m, 4H (under H₂O signal)), 3.43 (s, 2H), 3.78 (m, 2H), 3.97 (m, 5H), 4.28 (m, 2H), 6.83 (d, 1H), 7.05 (d, 2H), 7.10 (m, 1H), 7.21 (m, 1H), 7.33 (m, 1H), 7.41 (d, 2H), 7.47 (s, 1H), 7.75 (m, 1H), 8.12 (s, 1H), 8.81 (s, 1H).

### Example 11

### Preparation of Compound 301 in Table 3

This compound was prepared using the following scheme: 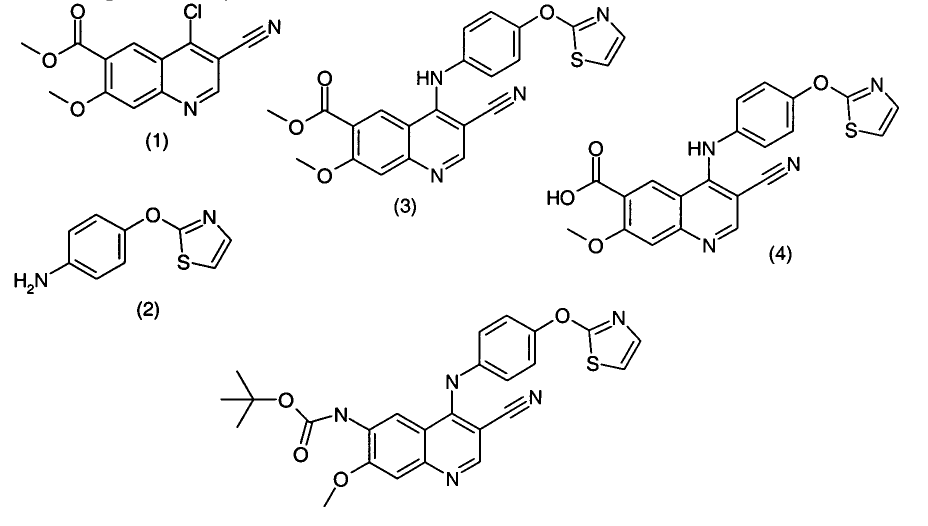 Reaction conditions: 100°C/4hrs/NEt₃/Diphenylphosphorylazide/t-BuOH
Chromatography: yes
Mass Spectrum m/e 490 (M+H)⁺.
NMR Spectrum (d-6-DMSO, d values) 1.48 (s, 9H), 4.01 (s, 3H), 7.26 (d, 1H), 7.33 (d, 1H), 7.45 (m, 1H), 7.49 (m, 2H), 7.53 (d, 2H), 8.70 (s, 1H), 8.82 (s, 1H), 8.97 (s, 1H).
Intermediate (3)
Reaction conditions: 100°C/18hrs/n-PrOH
Mass Spectrum m/e 433 (M+H)⁺.
Intermediate (4)
Reaction conditions: RT/36hrs/LiOH/MeOH/water
Mass Spectrum m/e 418 (M+H)⁺.

### Example 12

### Preparation of Compound 183 in Table 1

Intermediate 17 in Table 1 was reacted with cyclopropylamine and N-methylmorpholine at room temperature for 48hours in the presence of DMAP, EDC and DCM to yield the desired product.
Mass Spectrum m/e 624.5 (M+H)⁺
NMR Spectrum (d-6-DMSO, d values) 0.42 (m, 2H), 0.61 (m, 2H), 2.30 (m, 2H), 2.63 (m, 1H), 3.11 (m, 2H), 3.35 (2H under H₂O peak), 3.49 (m, 2H), 3.79 (m, 2H), 3.97 (m, 5H), 4.30 (m, 2H), 7.08 (m, 7H), 7.40 (d, 2H), 7.45 (s, 1H), 7.78 (s, 1H), 8.08 (s, 1H), 8.84 (s, 1H).

### Example 13

### Preparation of Compound No 430 in Table 1

This compound was prepared using the following scheme: 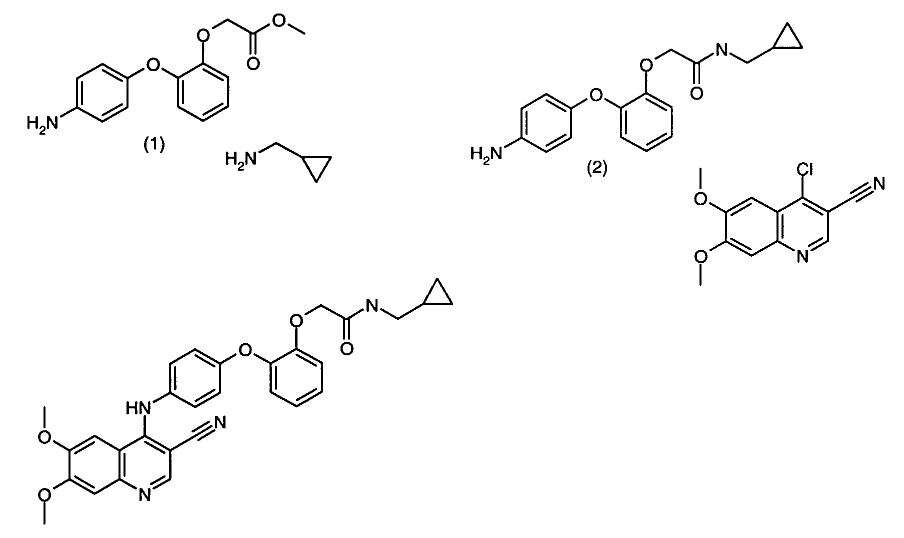 100°C/18hrs/n-PrOH
Chromatography: yes
Mass Spectrum m/e 525 (M+H)⁺
NMR Spectrum (d-6-DMSO, d values) 0.182 (m, 2H), 0.41 (m, 2H), 0.94 (m, 1H), 3.02 (t, 2H), 4.00 (m, 6H), 4.52 (s, 2H), 7.14 (m, 6H), 7.47 (m, 3H), 7.70 (t, 1H), 8.16 (s, 1H), 8.94 (s, 1H).

The aniline starting material (1) was prepared as described above in relation to Intermediate I5.

This was converted to Intermediate (2) above by reaction with cyclopropanemethylamine in methanol at room temperature for 18hrs.
Mass Spectrum m/e 313.5 (M+H)⁺

### Example 14

Using a method analogous to that of Example 13, the R^{7'} group was modified to form a different group R⁷ in the anilines used as starting materials in accordance with the following general scheme: 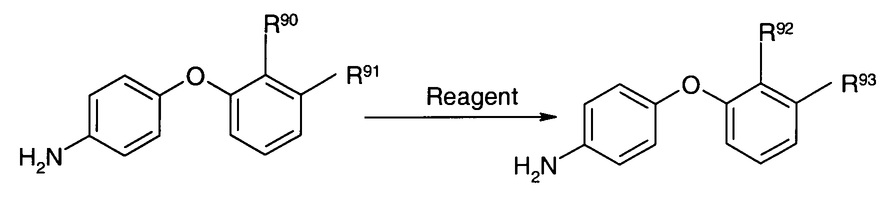 prior to conversion to the corresponding compound of formula (I) as summarised in the following Table 12.

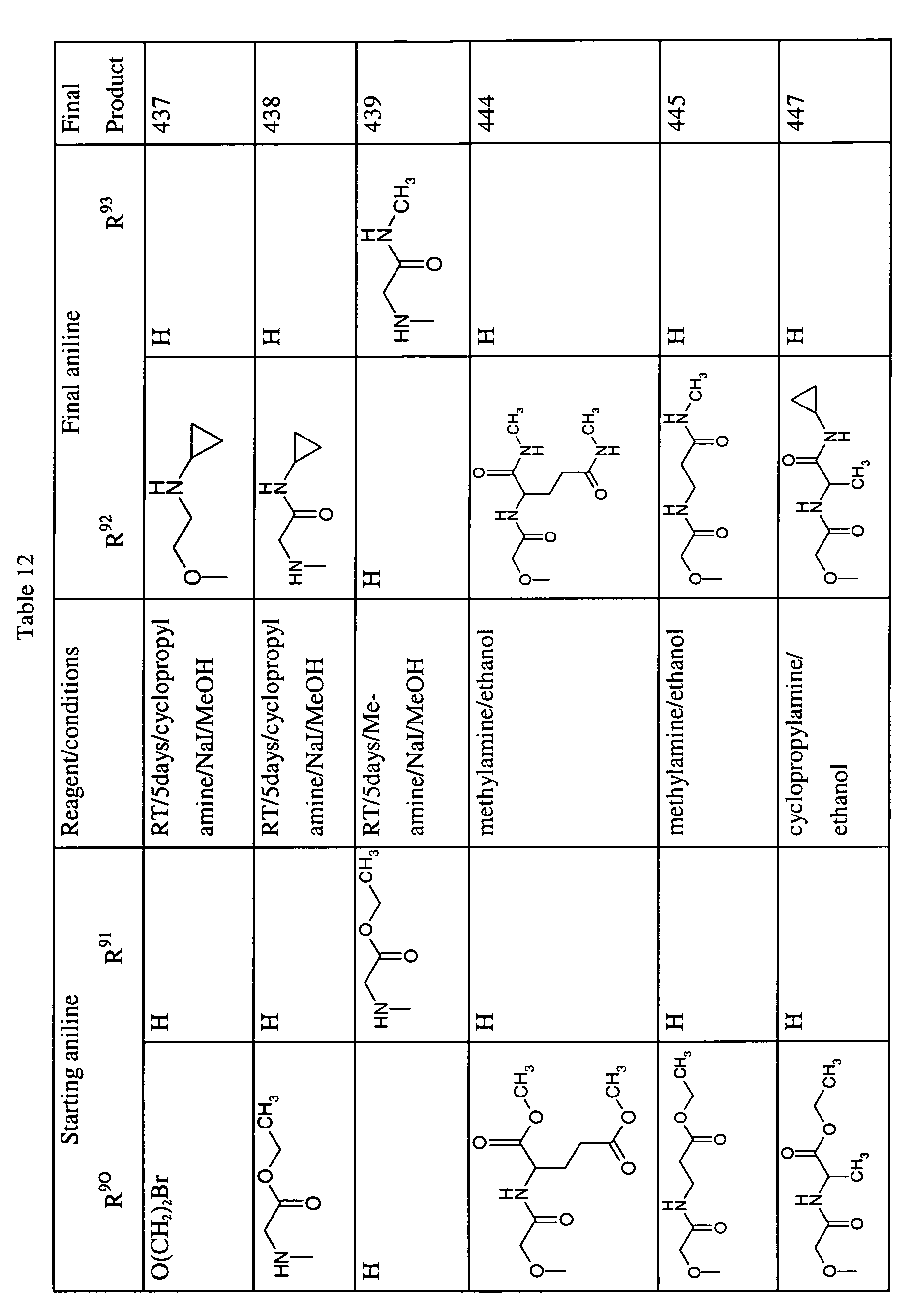

### Example 15

In the preparation of other compounds of formula (I) the R^{7'} group was modified to form a different group R⁷ in the nitrobenzyl compounds of formula (VII) used as starting materials in accordance with the following general scheme:

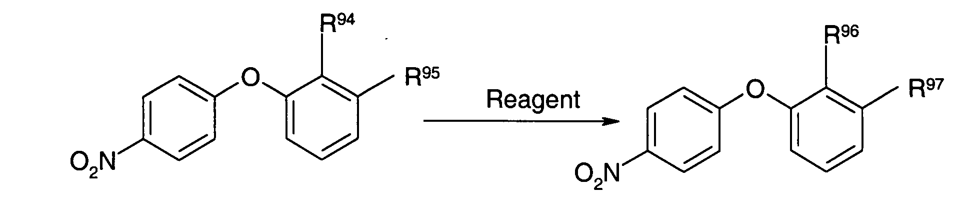

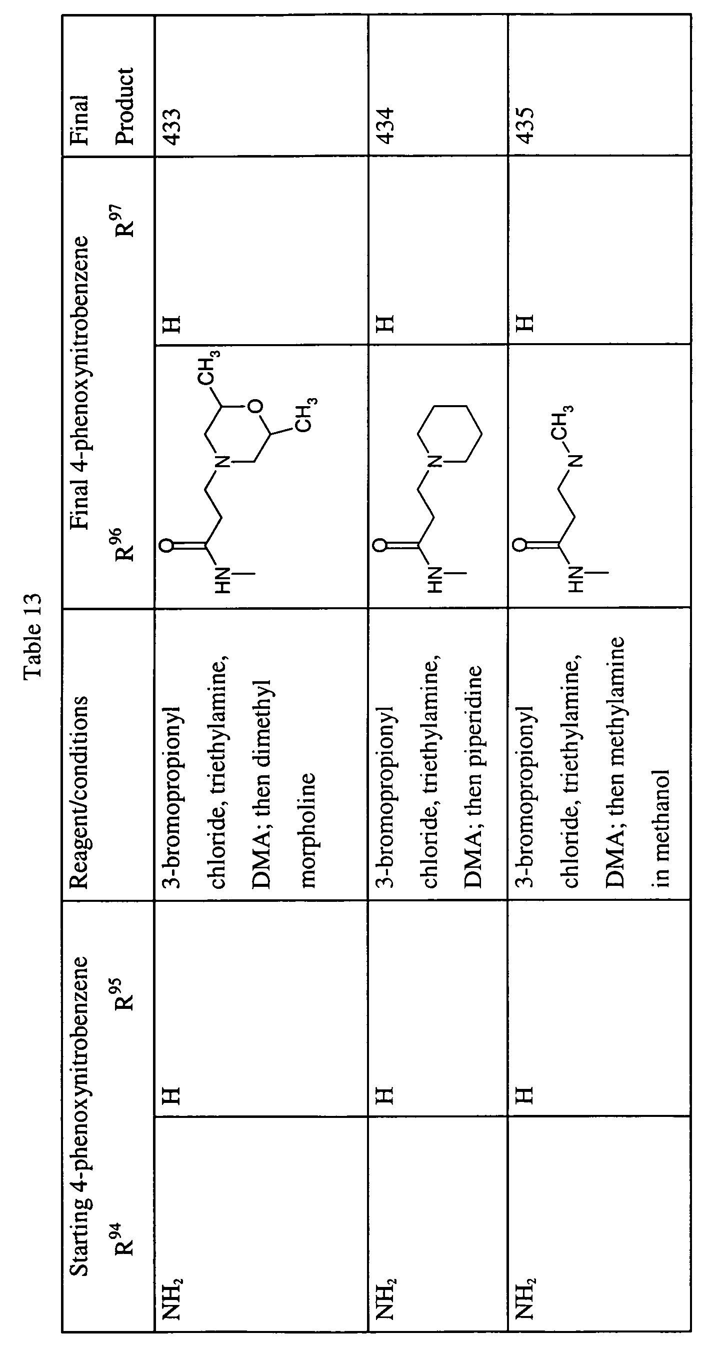

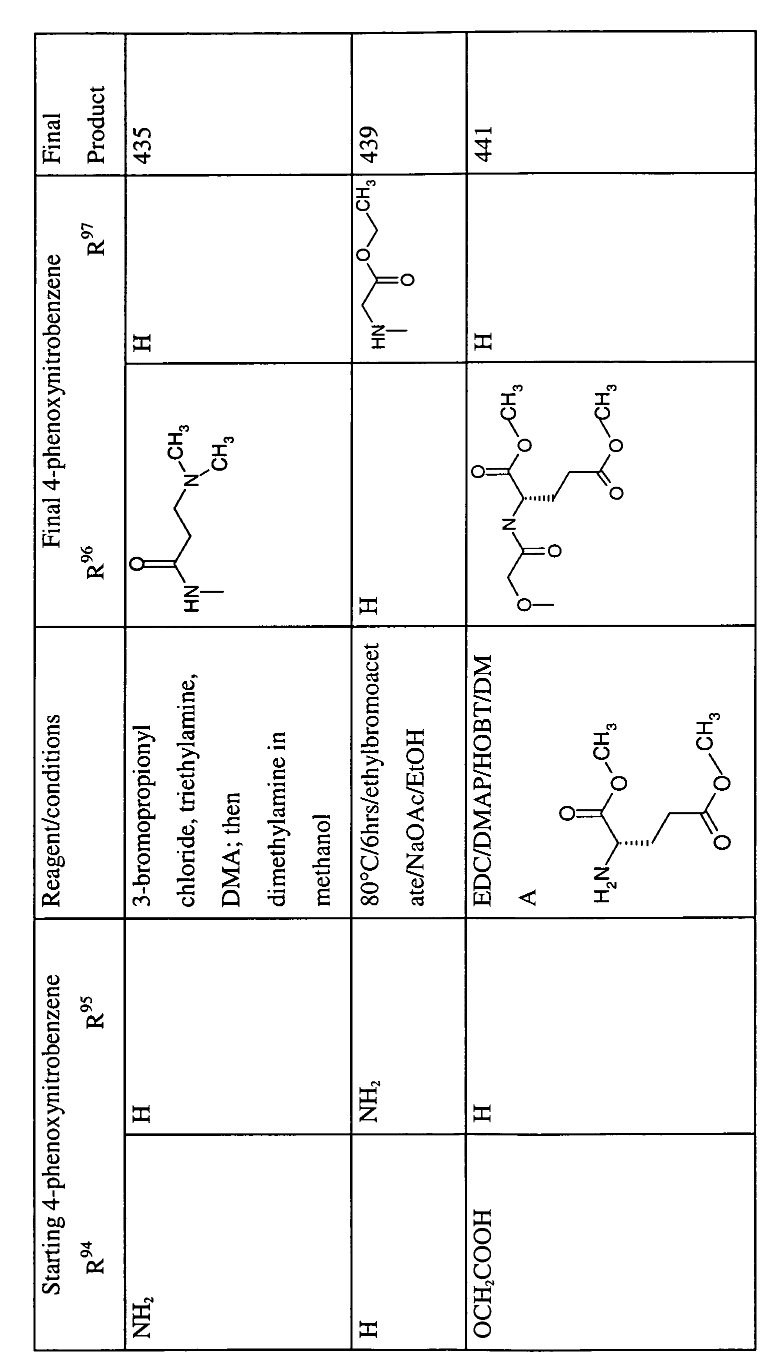

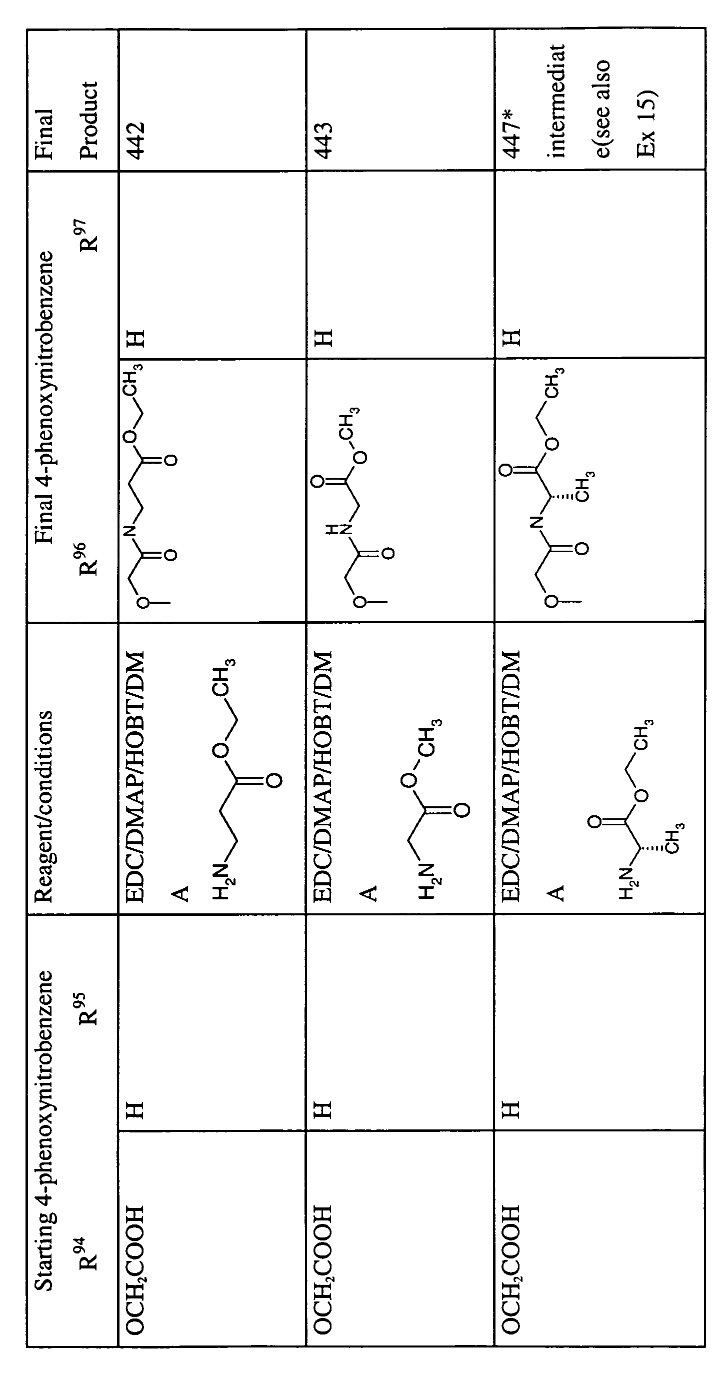

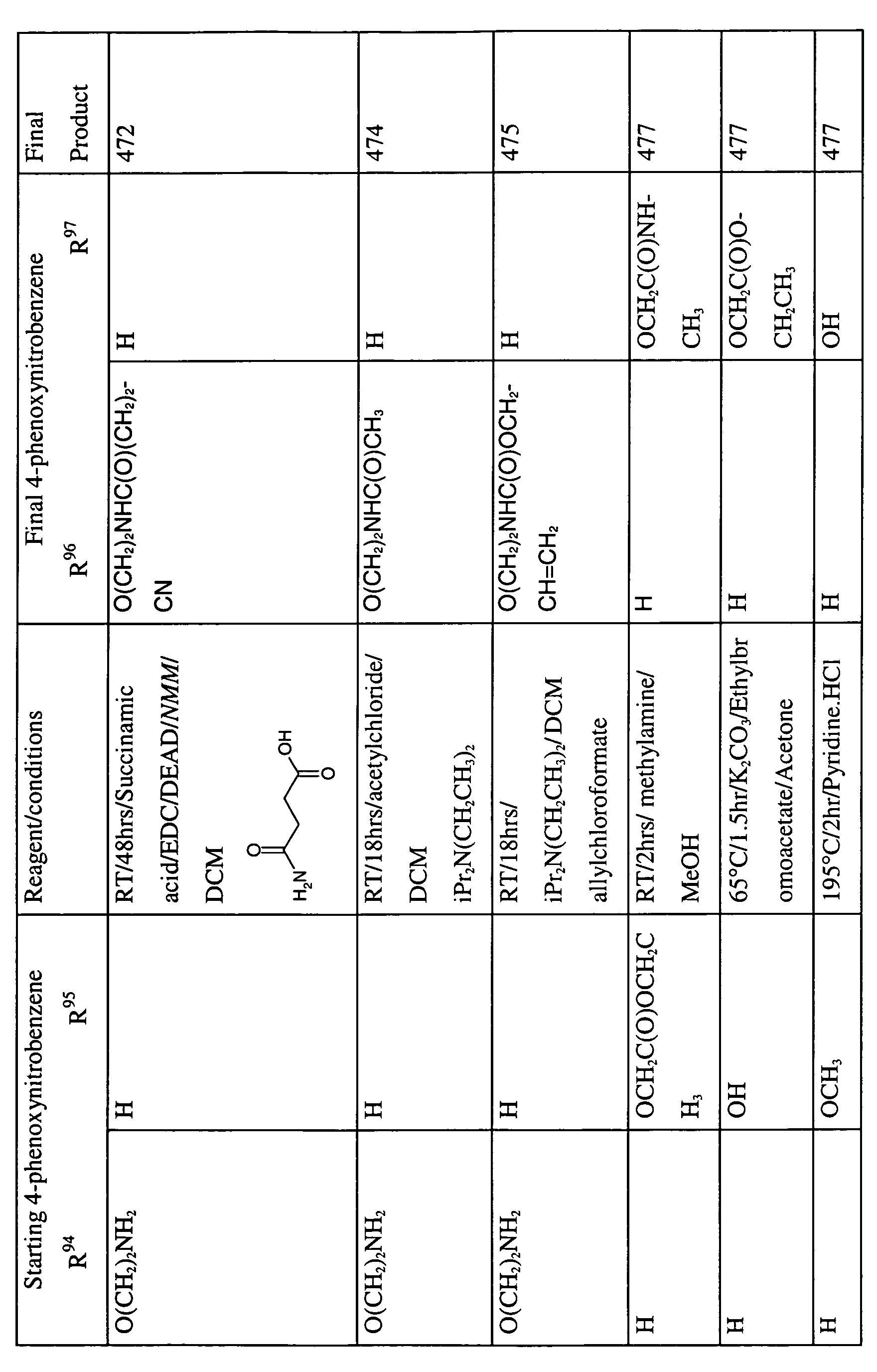

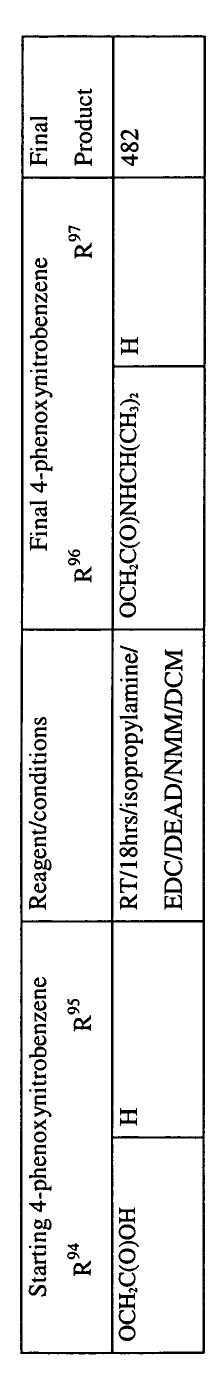

### Biological Data

### Assay for inhibitors of the MAP kinase pathway

To evaluate inhibitors of the MAPK pathway a coupled assay was carried out which measures phosphorylation of serine/threonine residues present in the substrate in the presence or absence of inhibitor. Recombinant glutathione S-transferase fusion protein containing human p45MEK1 (GST-MEK) was activated by c-raf (Sf9 insect cell lysate from triple baculoviral infection with c-raf/ras/lck) and used for the assay. Active GST-MEK was first used to activate a recombinant glutathione S-transferase fusion protein containing p44MAP kinase (GST-MAPK) in the presence of ATP and Mg²⁺ for 60min at room temperature in the presence or absence of potential inhibitors. The activated GST-MAPK was then incubated with myelin basic protein (MBP) as substrate for 10min at room temperature in the presence of ATP, Mg²⁺ and ³³P-ATP. The reaction was stopped by addition of 20% v/v phosphoric acid. Incorporation of ³³P into the myelin basic protein was determined by capture of the substrate on a filter mat, washing and counting using scintillation methods. The extent of inhibition was determined by comparison with untreated controls.

The final assay solution contained 10mM Tris, pH 7.5, 0.05mM EGTA, 8.33µM [γ³³P]ATP, 8.33mM Mg(OAc)₂, 0.5mM sodium orthovanadate, 0.05%w/v BSA, 6.5ng GST-MEK, 1µg GST-MAPK and 16.5µg MBP in a reaction volume of 60µl.

Compounds tested of the present invention had IC₅₀ results typically less than 0.5µM. For example, Compound No 252 gave an IC₅₀ of 0.15µM.

### In vitro MAP kinase assay

To determine whether compounds were inhibiting GST-MEK or GST-MAPK, a direct assay of MAPK activity was employed. GST-MAPK was activated by a constitutively active GST-MEK fusion protein containing two point mutations (S217E, S221E) and used for the assay in the presence and absence of potential inhibitors. The activated GST-MAPK was incubated with substrate (MBP) for 60min at room temperature in the presence of ATP, Mg²⁺ and ³³P-ATP. The reaction was stopped by addition of 20% v/v phosphoric acid. Incorporation of ³³P into the myelin basic protein was determined by capture of the substrate on a filter mat, washing and counting using scintillation methods.

The final assay solution contained 12mM Tris, pH 7.5, 0.06mM EGTA, 30µM [γ³³P]ATP, 10mM Mg(OAc)₂, 0.6mM sodium orthovanadate, 0.06%w/v BSA, 28ng GST-MAPK and 16.5µg MBP in a reaction volume of 60µl.

Compounds of the invention showed activity in this screen.

### Cell proliferation assays

Cells were seeded into multi-well plates at 20 000 - 40 000 cells/ml in growth medium containing 5% FCS and incubated overnight at 37°C. The compounds were prepared in fresh medium at an appropriate concentration and added to the wells containing the cells. These were then incubated for a further 72 hours. Cells were then either removed from the wells by incubating with trypsin/EDTA and counted using a Coulter counter, or treated with XTT/PMS in PBSA and optical densities read at 450nm. Compounds tested of the present invention had IC₅₀ results typically less than 30µM. For example, Compound No 250 gave an IC50 of 7.76 mM in HT29 human colon tumour cells; Compound No 32 gave an IC50 of 1.5µM in HT29 cells and an IC50 of 0.6µM in MC26 mouse colon tumour cells.

## Claims

1. A compound of formula (I) 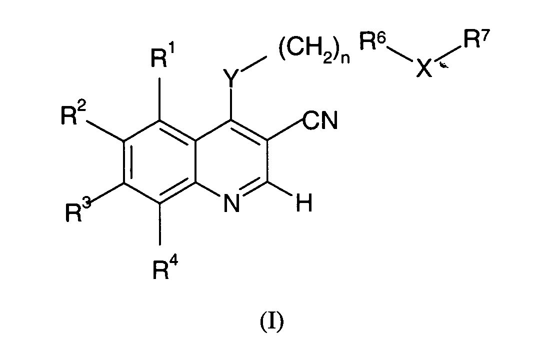 or a pharmaceutically acceptable salt thereof.
wherein:
n is 0-1;
X and Y are independently selected from -NH-, -O-, -S-, or -NR⁸- where R⁸ is alkyl of 1-6 carbon atoms and X may additionally comprise a CH₂ group;
R⁷ is a group (CH₂)ₘR⁹ where m is 0,or an integer of from 1-3 and R⁹ is a substituted phenyl ring or substituted cycloalkyl ring from 3 to 8 carbon atoms, wherein the substituents comprise at least one alkoxy group of 1-6 carbon atoms at the alpha position and optionally one or more further substitutents;
R⁶ is a divalent cycloalkyl of 3 to 7 carbon atoms, which may be optionally further substituted with one or more alkyl of 1 to 6 carbon atom groups; or is a divalent pyridinyl, pyimidinyl, or phenyl ring; wherein the pyridinyl, pyrimidinyl, or phenyl ring may be optionally further substituted with one or more groups selected from halogen, alkyl of 1-6 carbon atoms, alkenyl of 2-6 carbon atoms, alkynyl of 2-6 carbon atoms, azido, hydroxyalkyl of 1-6 carbon atoms, halomethyl, alkoxymethyl of 2-7 carbon atoms, alkanoyloxymethyl of 2-7 carbon atoms, alkoxy of 1-6 carbon atoms, alkylthio of 1-6 carbon atoms, hydroxy, trifluoromethyl, cyano, nitro, carboxy, carboalkoxy of 2-7 carbon atoms, carboalkyl of 2-7 carbon atoms, phenoxy, phenyl, thiophenoxy, benzoyl, benzyl, amino, alkylamino of 1-6 carbon atoms, dialkylamino of 2 to 12 carbon atoms, phenylamino, benzylamino, alkanoylamino of 1-6 carbon atoms, alkenoylamino of 3-8 carbon atoms, alkynoylamino of 3-8 carbon atoms, and benzoylamino;
R₁, R₂, R₃ and R₄ are each independently selected from hydrogen, hydroxy, halogeno, cyano, nitro, trifluoromethyl, C₁₋₃alkyl, -NR¹¹R¹² (wherein R¹¹ and R¹², which may be the same or different, each represents hydrogen or C₁₋₃alkyl), or a group R¹³-X¹-(CH₂)ₓ wherein x is 0 to 3, X¹ represents -O-, -CH₂-, -OCO-, carbonyl, -S-, -SO-, -SO₂-, -NR¹⁴CO-, -SO₂NR¹⁶-, -NR¹⁷SO₂- or -NR¹⁸- (wherein R¹⁴, R¹⁶, R¹⁷ and R¹⁸ each independently represents hydrogen, C₁₋₃alkyl or C₁₋₃alkoxyC₂₋₃alkyl) and R¹³ is selected from one of the following sixteen groups:
1) C₁₋₅alkyl which may be unsubstituted or which may be substituted with one or more groups selected from hydroxy, fluoro and amino;
2) C₁₋₅alkylX²COR¹⁹ (wherein X² represents -O- or -NR²⁰- (wherein R²⁰ represents hydrogen, C₁₋₃alkyl or C₁₋₃alkoxyC₂₋₃alkyl) and R¹⁹ represents -NR²¹R²²- or -OR²³- (wherein R²¹, R²² and R²³ which may be the same or different each represents hydrogen, C₁₋₃alkyl or C₁₋₃alkoxyC₂₋₃alkyl));
3) C₁₋₅alkylX³R²⁴ (wherein X³ represents -O-, -S-, -SO-, -SO₂-, -OCO-, -NR²⁵CO-, -CONR²⁶-, -SO₂NR²⁷-, -NR²⁸SO₂- or -NR²⁹- (wherein R²⁵, R²⁶, R²⁷, R²⁸ and R²⁹ each independently represents hydrogen, C₁₋₃alkyl or C₁₋₃alkoxyC₂₋₃alkyl) and R²⁴ represents hydrogen, C₁₋₃alkyl, cyclopentyl, cyclohexyl or a 5 or 6 membered saturated heterocyclic group with one or two heteroatoms, selected independently from O, S and N, which C₁₋₃alkyl group may bear one or two substituents selected from oxo, hydroxy, halogeno and C₁₋₄alkoxy and which cyclic group may bear one or two substituents selected from oxo, hydroxy, halogeno, C₁₋₄alkyl, C₁₋₄hydroxyalkyl and C₁₋₄alkoxy);
4) C₁₋₅alkylX⁴C₁₋₅alkylX⁵R³⁰ (wherein X⁴ and X⁵ which may be the same or different are each -O-, -S-, -SO-, -SO₂-, -NR³¹CO-, -CONR³²-, -SO₂NR³³-, -NR³⁴SO₂- or -NR³⁵- (wherein R³¹, R³², R³³, R³⁴ and R³⁵ each independently represents hydrogen, C₁₋₃alkyl or C₁₋₃alkoxyC₂₋₃alkyl) and R³⁰ represents hydrogen or C₁₋₃alkyl);
5) C₁₋₅alkylR³⁶ (wherein R³⁶ is a 5 or 6 membered saturated heterocyclic group with one or two heteroatoms, selected independently from O, S and N, which heterocyclic group may bear one or two substituents selected from oxo, hydroxy, halogeno, C₁₋₄alkyl, C₁₋₄hydroxyalkyl and C₁₋₄alkoxy);
6) (CH₂)_{q}X⁶R³⁷ (wherein q is an integer from 0 to 5, X⁶ represents a direct bond, -O-, -S-, -SO-, -SO₂-, -NR³⁸CO-, -CONR³⁹-, -SO₂NR⁴⁰-, -NR⁴¹SO₂- or -NR⁴²- (wherein R³⁸, R³⁹, R⁴⁰, R⁴¹ and R⁴² each independently represents hydrogen, C₁₋₃alkyl or C₁₋₃alkoxyC₂₋₃alkyl) and R³⁷ is a phenyl group, a pyridone group or a 5 or 6 membered aromatic heterocyclic group with 1 to 3 heteroatoms selected from O, N and S, which phenyl, pyridone or aromatic heterocyclic group may carry up to 5 substituents selected from hydroxy, halogeno, amino, C₁₋₄alkyl, C₁₋₄alkoxy, C₁₋₄hydroxyalkyl, C₁₋₄hydroxyalkoxy, C₁₋₄aminoalkyl, C₁₋₄alkylamino, carboxy, cyano, -CONR⁴³R⁴⁴ and -NR⁴⁵COR⁴⁶ (wherein R⁴³, R⁴⁴, R⁴⁵ and R⁴⁶, which may be the same or different, each represents hydrogen, C₁₋₄alkyl or C₁₋₃alkoxyC₂₋₃alkyl));
7) C₂₋₆alkenylR³⁶ (wherein R³⁶ is as defined hereinbefore);
8) C₂₋₆alkynylR³⁶ (wherein R³⁶ is as defined hereinbefore);
9) X⁷R⁴⁷ (wherein X⁷ is -SO₂-, -O- or -CONR⁴⁸R⁴⁹- (wherein R⁴⁸ and R⁴⁹, which may be the same or different, each represents hydrogen, C₁₋₃alkyl or C₁₋₃alkoxyC₂₋₃alkyl) and R⁴⁷ represents C₁₋₅alkyl which may be unsubstituted or which may be substituted with one or more groups selected from hydroxy, fluoro and amino) with the provisos that when X⁷ is -SO₂-, X¹ is -O-, when X⁷ is -O-, X¹ is carbonyl, when X⁷ is -CONR⁴⁸R⁴⁹-, X¹ is -O- or NR¹⁸ (wherein R⁴⁸, R⁴⁹ and R¹⁸ are as defined hereinbefore);
10) C₂₋₆alkenylR³⁷ (wherein R³⁷ is as defined hereinbefore);
11) C₂₋₆alkynylR³⁷ (wherein R³⁷ is as defined hereinbefore);
12) C₂₋₆alkenylX⁸R³⁷ (wherein X⁸ represents -O-, -S-, -SO-, -SO₂-, -NR⁵⁰CO-, -CONR⁵¹-, -SO₂NR⁵²-, -NR⁵³SO₂- or -NR⁵⁴- (wherein R⁵⁰, R⁵¹, R⁵², R⁵³ and R⁵⁴ each independently represents hydrogen, C₁₋₃alkyl or C₁₋₃alkoxyC₂₋₃alkyl) and R³⁷ is as defined hereinbefore);
13) C₂₋₆alkynylX⁹R³⁷ (wherein X⁹ represents -O-, -S-, -SO-, -SO₂-, -NR⁵⁵CO-, -CONR⁵⁶-, -SO₂NR⁵⁷-, -NR⁵⁸SO₂- or -NR⁵⁹- (wherein R⁵⁵, R⁵⁶, R⁵⁷, R⁵⁸ and R⁵⁹ each independently represents hydrogen, C₁₋₃alkyl or C₁₋₃alkoxyC₂₋₃alkyl) and R³⁷ is as defined hereinbefore);
14) C₁₋₃alkylX¹⁰C₁₋₃alkylR³⁷ (wherein X¹⁰ represents -O-, -S-, -SO-, -SO₂-, -NR⁶⁰CO-, -CONR⁶¹-, -SO₂NR⁶²-, -NR⁶³SO₂- or -NR⁶⁴- (wherein R⁶⁰, R⁶¹, R⁶², R⁶³ and R⁶⁴ each independently represents hydrogen, C₁₋₃alkyl or C₁₋₃alkoxyC₂₋₃alkyl) and R³⁷ is as defined hereinbefore);
15) R³⁶ (wherein R³⁶ is as defined hereinbefore); and
16) C₁₋₃alkylX¹⁰C₁₋₃alkylR³⁶ (wherein X¹⁰ and R³⁶ are as defined hereinbefore).

2. A compound according to claim 1 of formula (II) 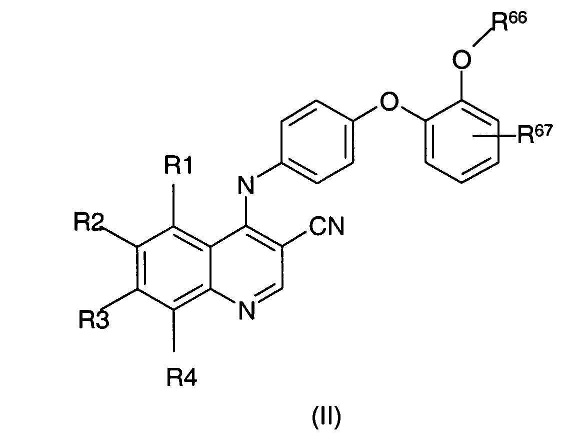 or a pharmaceutically acceptable salt thereof.
where R¹, R², R³ and R⁴ are as defined in claim 1, R⁶⁶ is an optionally substituted C₁₋₆ alkyl and R⁶⁷ is selected from hydrogen, halogen, alkyl of 1-6 carbon atoms, alkenyl of 2-6 carbon atoms, alkynyl of 2-6 carbon atoms, azido, hydroxyalkyl of 1-6 carbon atoms, halomethyl, alkoxymethyl of 2-7 carbon atoms, alkanoyloxymethyl of 2-7 carbon atoms, alkoxy of 1-6 carbon atoms, alkylthio of 1-6 carbon atoms, hydroxy, trifluoromethyl, cyano, nitro, carboxy, carboalkoxy of 2-7 carbon atoms, carboalkyl of 2-7 carbon atoms, phenoxy, phenyl, thiophenoxy, benzoyl, benzyl, amino, alkylamino of 1-6 carbon atoms, dialkylamino of 2 to 12 carbon atoms, phenylamino, benzylamino, alkanoylamino of 1-6 carbon atoms, alkenoylamino of 3-8 carbon atoms, alkynoylamino of 3-8 carbon atoms, and benzoylamino.

3. A compound according to claim 1 or a pharmaceutically acceptable salt thereof, wherein R⁹ is a phenyl ring or a cycloalkyl ring which is substituted at the alpha position with a methoxy group.

4. A compound according to claim 1 or a pharmaceutically acceptable salt thereof, of the formula:

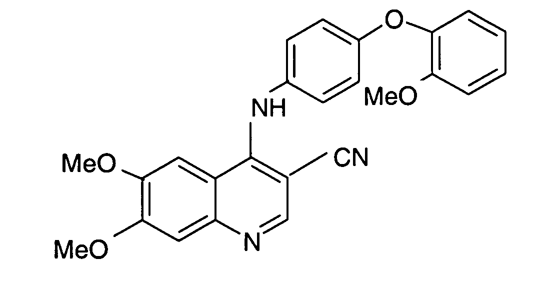

5. A pharmaceutical composition comprising a compound of formula (I) as defined in claim 1 or a pharmaceutically acceptable salt thereof in combination with a pharmaceutically acceptable carrier or excipient.

6. A method of preparing a compound of formula (I) as defined in claim 1 which method comprises either
(a) reacting a compound of formula (III) 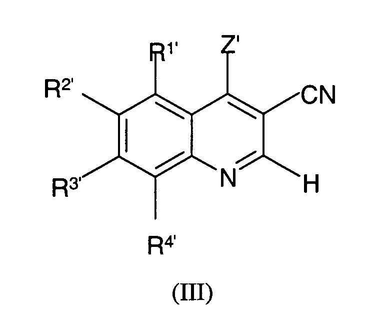 where R^{1'}, R^{2'}, R^{3'}, R^{4'} represent R¹, R², R³ and R⁴ respectively as defined in relation to formula (I) or a precursor thereof, and Z' is a leaving group, with a compound of formula (IV)
H- Y(CH₂)ₙR⁶XR^{7'} (IV)
where R⁶, Y, X, and n are as defined in relation to formula (I), and R^{7'} is a group R⁷ or a precursor thereof; or
(b) reacting a compound of formula (V) 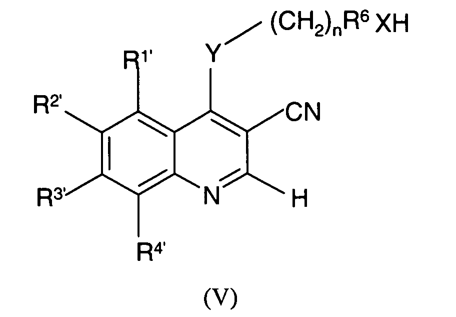 where R^{1'}, R^{2'}, R^{3'}, R^{4'} are as defined in relation to fomula (III) R⁶, X, Y and n are as defined in relation to formula (I), with a compound of formula (VI)
R^{7'}-Z" (VI)
where R^{7'} is as defined in relation to formula (IV) and Z" is a leaving group; and thereafter if necessary or desired converting precursor groups R^{1'}, R^{2'}, R^{3'}, R^{4'} and R^{7'} to groups of formula R¹, R², R³, R⁴ and R⁷ respectively, or converting a group R¹, R², R³, R⁴ and R⁷ to a different such group.

7. A compound for use in therapy comprising a compound of formula (I) as defined in claim 1, or a pharmaceutically acceptable salt thereof.

8. The use of a compound of formula (I) as defined in claim 1 or a pharmaceutical acceptable salt thereof in the preparation of a medicament for use in the inhibition of MEK enzymes.
